# EUROPEAN PATENT SPECIFICATION

(11) **EP 1 697 304 B1**
(45) Date of publication and mention of the grant of the patent: **20.02.2008**
(21) Application number: 04820002.6
(22) Date of filing: 16.11.2004
(51) Int. Cl.: C07C 233/73, C07C 271/16, C07C 311/04, C07C 317/44, C07C 323/62, C07D 209/42, C07D 209/46, C07D 213/81, C07D 217/06, C07D 333/70, A61K 31/166, A61K 31/265, A61K 31/18, A61K 31/381, A61K 31/4035

(54) **PEROXISOME PROLIFERATOR ACTIVATED RECEPTOR MODULATORS**
MODULATOREN DES PEROXISOMPROLIFERATORAKTIVIERTEN REZEPTORS
MODULATEURS DES RECEPTEURS ACTIVES PAR LES PROLIFERATEURS DE PEROXYSOMES

(30) Priority: 25.11.2003 US 525020 P
(43) Date of publication of application: 06.09.2006
(73) Proprietor: ELI LILLY AND COMPANY, Indianapolis, Indiana 46285 (US)
(72) Inventor: CANADA, Emily, Jane, Indianapolis, IN 46226 (US); GOSSETT, Lynn, Stacy, Indianapolis, IN 46217 (US); MANTELO, Nathan, Bryan, Brownsburg, IN 46112 (US); SHI, Qing, Carmel, Indiana 46033 (US); WANG, Minmin, Fishers, IN 46038 (US); WARSHAWSKY, Alan, M., Carmel, IN 46032 (US); XU, Yanping, Indiana 46062 (US)
(74) Representative: Burnside, Ivan John
(86) International application number: PCT/US2004/035528
(87) International publication number: WO 2005/054176

(56) References cited:
- WO-A-01/34094
- US-A1- 2003 105 078

## Description

### FIELD OF THE INVENTION

The present invention relates to compounds of peroxisome proliferator activated receptor (PPAR) agonists, more specifically compounds of PPAR gamma-delta dual agonists, which are useful for the treatment and/or prevention of disorders modulated by a PPAR agonist.

### BACKGROUND OF THE INVENTION

The peroxisome proliferator activated receptors (PPARs) are members of the nuclear receptor gene family that are activated by fatty acids and fatty acid metabolites. The PPARs belong to the subset of nuclear receptors that function as heterodimers with the 9-*cis* retinoic acid receptor (RXR). Three subtypes, which are designated as PPARα, PPARγ and PPARδ are found in species ranging from *Xenopus* to humans.

PPARα is the main subtype in the liver and has facilitated analysis of the mechanism by which peroxisome proliferators exert their pleiotropic effects. PPARα is activated by a number of medium and long-chain fatty acids, and it is involved in stimulating β-oxidation of fatty acids. PPARα is also involved with the activity of fibrates and fatty acids in rodents and humans. Fibric acid derivatives such as clofibrate, fenofibrate, bezafibrate, ciprofibrate, beclofibrate and etofibrate, as well as gemfibrozil, produce a substantial reduction in plasma triglycerides along with moderate reduction in low-density lipoprotein (LDL) cholesterol, and they are used particularly for the treatment of hypertriglyceridemia.

PPARγ is the main subtype in adipose tissue and involved in activating the program of adipocyte differentiation. PPARγ is not involved in stimulating peroxisome proliferation in the liver. There are two isomers of PPARγ:PPARγ1 and PPARγ2, which differ only in that PPARγ2 contains an additional 28 amino acids present at the amino terminus. The DNA sequences for the PPARγ receptors are described in Elbrecht, et al., BBRC 224;431-437 (1996). Although peroxisome proliferators, including the fibrates and fatty acids, activate the transcriptional activity of PPAR's, only prostaglandin J₂ derivatives have been identified as natural ligands for PPARγ, which also binds the antidiabetic agents thiazolidinediones with high affinity. The physiological functions of PPARα and PPARγ in lipid and carbohydrate metabolism were uncovered once it was recognized that they were the receptors for the fibrate and glitazone drugs, respectively.

PPARα and PPARγ receptors have been implicated in diabetes mellitus, cardiovascular disease, obesity, and gastrointestinal disease, such as inflammatory bowel disease and other inflammation related illnesses. Such inflammation related illnesses include, but are not limited to Alzheimer's disease, Crohn's disease, rheumatoid arthritis, psoriasis, and ischemia reprofusion injury. By contrast, PPARδ (also referred to as PPARβ and NUC1) is not reported to be receptor for any known class of drug molecules, and its role in mammalian physiology has remained undefined. The human nuclear receptor gene PPARδ (hPPARδ) has been cloned from a human osteosarcoma cell cDNA library and is fully described in A. Schmidt et al., Molecular Endocrinology, 6:1634-1641 (1992).

Diabetes is a disease in which a mammal's ability to regulate glucose levels in the blood is impaired because the mammal has a reduced ability to convert glucose to glycogen for storage in muscle and liver cells. In Type I diabetes, this reduced ability to store glucose is caused by reduced insulin production. "Type II Diabetes" or "non-insulin dependent diabetes mellitus" (NIDDM) is the form of diabetes, which is due to a profound resistance to insulin stimulating or regulatory effect on glucose and lipid metabolism in the main insulin-sensitive tissues, muscle, liver and adipose tissue. This resistance to insulin responsiveness results in insufficient insulin activation of glucose uptake, oxidation and storage in muscle and inadequate insulin repression of lipolysis in adipose tissue and of glucose production and secretion in liver. When these cells become desensitized to insulin, the body tries to compensate by producing abnormally high levels of insulin and hyperinsulemia results. Hyperinsulemia is associated with hypertension and elevated body weight. Since insulin is involved in promoting the cellular uptake of glucose, amino acids and triglycerides from the blood by insulin sensitive cells, insulin insensitivity can result in elevated levels of triglycerides and LDL (known as the "bad" cholesterol) which are risk factors in cardiovascular diseases. The constellation of symptoms which includes hyperinsulemia combined with hypertension, elevated body weight, elevated triglycerides and elevated LDL is known as Syndrome X.

Hyperlipidemia is a condition which is characterized by an abnormal increase in serum lipids, such as cholesterol, triglycerides and phospholipids. These lipids do not circulate freely in solution in plasma, but are bound to proteins and transported as macromolecular complexes called lipoproteins. One form of hyperlipidemia is hypercholesterolemia, characterized by the existence of elevated LDL cholesterol levels. The initial treatment for hypercholesterolemia is often a diet low in fat and cholesterol coupled with appropriate physical exercise. Drug intervention is initiated if LDL-lowering goals are not met by diet and exercise alone. It is desirable to lower elevated levels of LDL cholesterol and increase levels of HDL cholesterol. Generally, it has been found that increased levels of HDL are associated with lower risk for coronary heart disease (CHD). See Gordon, et al., Am. J. Med., 62, 707-714 (1977); Stampfer, et al., N. England J. Med., 325, 373- 381 (1991); and Kannel, et al., Ann. Internal Med., 90, 85-91 (1979). An example of an HDL raising agent is nicotinic acid, but the quantities needed to achieve HDL elevation are associated with undesirable effects, such as flushing.

There are several treatments currently available for treating diabetes mellitus but these treatments still remain unsatisfactory and have limitations. While physical exercise and reduction in dietary intake of calories will improve the diabetic condition, compliance with this approach can be poor because of sedentary lifestyles and excess food consumption, in particular high fat-containing food. Therefore, treatment with hypoglycemics, such as sulfonylureas (e.g., chlorpropamide, tolbutamide, tolazamide and acetohexamide) and biguanides (e.g. phenformin and metformin) are often necessary as the disease progresses. Sulfonylureas stimulate the β cells of the pancreas to secrete more insulin as the disease progresses. However, the response of the β cells eventually fails and treatment with insulin injections is necessary. In addition, both sulfonylurea treatment and insulin injection have the life threatening side effect of hypoglycemic coma, and thus patients using these treatments must carefully control dosage.

It has been well established that improved glycemic control in patients with diabetes (Type I and Type II) is accompanied by decreased microvasclular complications (DCCT and UKPDS). Due to difficulty in maintaining adequate glycemic control over time in patients with Type II diabetes, the use of insulin sensitizers in the therapy of Type II diabetes is growing. There is also a growing body of evidence that PPARγ agonist, insulin sensitizer, may have benefits in the treatment of Type II diabetes beyond their effects in improving glycemic control.

In the last decade a class of compounds known as thiazolidinediones (TZD) (e.g. U.S. Pat. Nos. 5,089,514; 4,342,771; 4,367,234; 4,340,605; and 5,306,726) have emerged as effective antidiabetic agents that have been shown to increase the sensitivity of insulin sensitive tissues, such as skeletal muscle, liver and adipose, to insulin. Increasing insulin sensitivity rather than the amount of insulin in the blood reduces the likelihood of hypoglycemic coma. Although thiazolidinediones have been shown to increase insulin sensitivity by binding to PPARγ receptors, this treatment also produces unwanted side effects such as weight gain and edema and, for troglitazone, liver toxicity. Recently, the compounds that are not TZDs have also been reported as PPAR modulators.

Adams et al. (WO 97/28115, WO 97/28135 and US Patent No. 5,895,051) discloses acetylphenols, which are useful as antiobesity and antidiabetic compounds.

Leibowitz et al. (WO 97/28149) discloses compounds which are PPARδ agonists and useful for treating cardiovascular diseases and related conditions.

Brooks et al. (WO 02/100813) discloses compounds of PPAR modulators that are useful for treating type II diabetes and other PPAR-mediated diseases and conditions.

In view of the above, an objective of the present invention is to provide new pharmaceutical agents which modulate PPAR receptors to prevent, treat and/or alleviate these diseases or conditions while reducing and or eliminating one or more of the unwanted side effects associated with the current treatments.

### SUMMARY OF THE INVENTION

The present invention relates to a compound of novel peroxisome proliferator activated receptor (PPAR) agonist having a structural formula I, 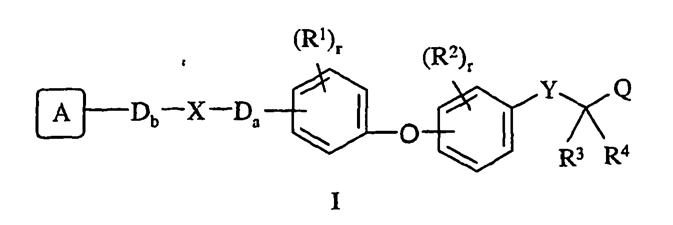 or a pharmaceutically acceptable salt or stereoisomer thereof, wherein:
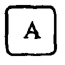 is: a) aryl,
   b) a 5- to 10-membered heteroaryl wherein the heteroaryl containing at least one heteroatom selected from N, O or S,
   c) C₃-C₈ cycloalkyl,
   d) aliphatic group, or
   e) heterocyclyl,
   wherein aryl, heteroaryl, cycloalkyl, heterocyclyl and aliphatic group being optionally substituted with one or more groups independently selected from R⁸;
Dₐ and D_{b} are each independently:
   a bond or
   -[C(R^{c})(R^{d})]ₙ, wherein R^{c} and R^{d} are each independently hydrogen, C₁-C₆ alkyl or aryl;
Q is: -C(O)OR⁵ or R^{5A};
X is: NR⁶C[O]ₚ,
   NR⁶S(O)₂,
   C[O]ₚ, NR⁶,
   S(O)₂NR⁶ or
   NR⁷;
Y is: a bond, CH₂, S or O; 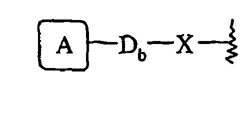 is: 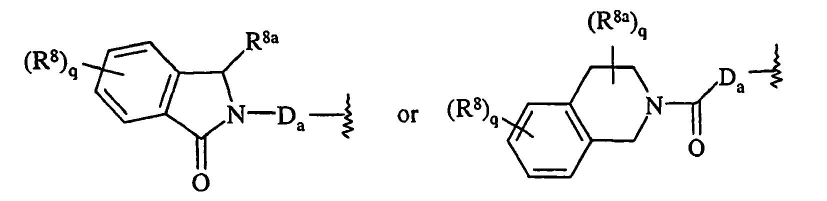
n and r are each independently: 1, 2, 3 or 4;
q is: 1, 2, 3, 4 or 5;
p is: 1 or 2;
R¹ and R² are each independently: hydrogen, C₁-C₆ alkyl, halo or haloalkyl;
R³ and R⁴ are each independently:
   hydrogen,
   halo,
   C₁-C₆ alkyl,
   C₁-C₆ alkoxy or
   aryloxy;
R³ and R⁴ are together a 3- to 6- membered carbocyclyl or heterocyclyl;
R⁵ is: hydrogen, C₁-C₆ alkyl or aminoalkyl;
R^{5A} is: carboxamide, sulfonamide, acylsulfonamide, tetrazole, 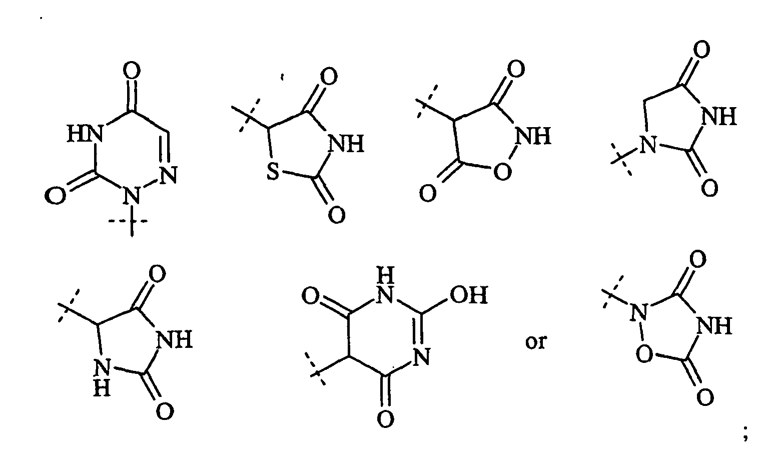
R⁶ is each independently:
   hydrogen,
   C₁-C₁₂alkyl,
   arylalkyl,
   C₃-C₈ cycloalkyl, or
   (CH₂)ₙC(O)aryl,
   wherein alkyl, arylalkyl and cycloalkyl group being optionally substituted with one or more groups independently selected from R⁸;
R⁷ is: hydrogen,
   acyl, or
   sulfonyl;
R⁸ and R^{8a} are each independently:
   hydrogen,
   C₁-C₆ alkyl,
   C₁-C₆ alkoxy,
   nitro,
   cyano,
   halo,
   haloalkyl,
   haloalkyloxy,
   aryl,
   heteroaryl,
   benzyl,
   aryloxy,
   SR⁹,
   S[O]ₚR⁹ or
   C[O]ₚR⁹; and
R⁹ is: hydrogen, C₁-C₆ alkyl, or C₃-C₈ cycloalkyl.

The compounds of the present invention are useful in the treatment and/or prevention of diseases or condition relates to hyperglycemia, dyslipidemia, Type II diabetes, Type I diabetes, hypertriglyceridemia, syndrome X, insulin resistance, heart failure, diabetic dyslipidemia, hyperlipidemia, hypercholesteremia, hypertension, obesity, anorexia bulimia, anorexia nervosa, cardiovascular disease and other diseases where insulin resistance is a component.

In one embodiment, the present invention also relates to a pharmaceutical composition comprising a compound of the present invention, or a pharmaceutically acceptable salt, solvate or hydrate thereof and a pharmaceutically acceptable carrier.

### DETAILED DESCRIPTION OF THE INVENTION

The compounds of the present invention are directed to peroxisome proliferator activated receptor (PPAR) agonists, more specifically compounds of PPARγ/δ dual agonists, which are useful for the treatment and/or prevention of disorders modulated by a PPAR, such as Type II diabetes, hyperglycemia, dyslipidemia, Type I diabetes, hypertriglyceridemia, syndrome X, insulin resistance, heart failure, diabetic dyslipidemia, hyperlipidemia, hypercholesteremia, hypertension, obesity, anorexia bulimia, anorexia nervosa, cardiovascular disease and other related diseases.

An embodiment of the present invention is a compound of novel peroxisome proliferator activated receptor (PPAR) agonists having a structural formula I, 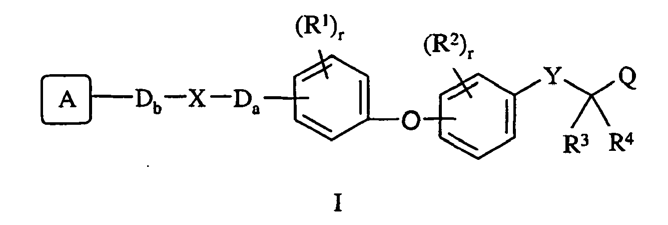 or a pharmaceutically acceptable salt or stereoisomer thereof, wherein:
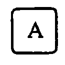 is: a) aryl,
   b) a 5- to 10-membered heteroaryl wherein the heteroaryl containing at least one heteroatom selected from N, O or S,
   c) C₃-C₈ cycloalkyl,
   d) aliphatic group, or
   e) heterocyclyl,
   wherein aryl, heteroaryl, cycloalkyl, heterocyclyl and aliphatic group being optionally substituted with one or more groups independently selected from R⁸;
Dₐ and D_{b} are each independently:
   a bond or
   -[C(R^{c})(R^{d})]ₙ, wherein R^{c} and R^{d} are each independently hydrogen, C₁-C₆ alkyl or aryl;
Q is: -C(O)OR⁵ or R^{5A} ;
X is: NR⁶C[O]ₚ,
   NR⁶S(O)₂,
   C[O]_{p,}NR⁶,
   S(O)₂NR⁶ or
   NR⁷;
Y is: a bond, CH₂, S or O; 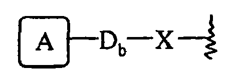
is: 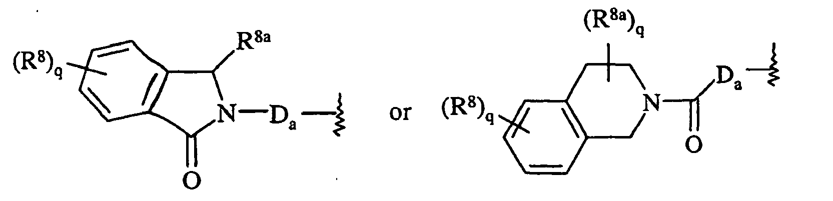
n and r are each independently: 1, 2, 3 or 4;
qis: 1,2,3,4 or 5;
p is: 1 or 2;
R¹ and R² are each independently: hydrogen, C₁-C₆ alkyl, halo or haloalkyl;
R³ and R⁴ are each independently:
   hydrogen,
   halo,
   C₁-C₆ alkyl,
   C₁-C₆ alkoxy or
   aryloxy;
R³ and R⁴ are together a 3- to 6- membered carbocyclyl or heterocyclyl;
R⁵ is: hydrogen, C₁-C₆ alkyl or aminoalkyl;
R^{5A} is: carboxamide, sulfonamide, acylsulfonamide, tetrazole, 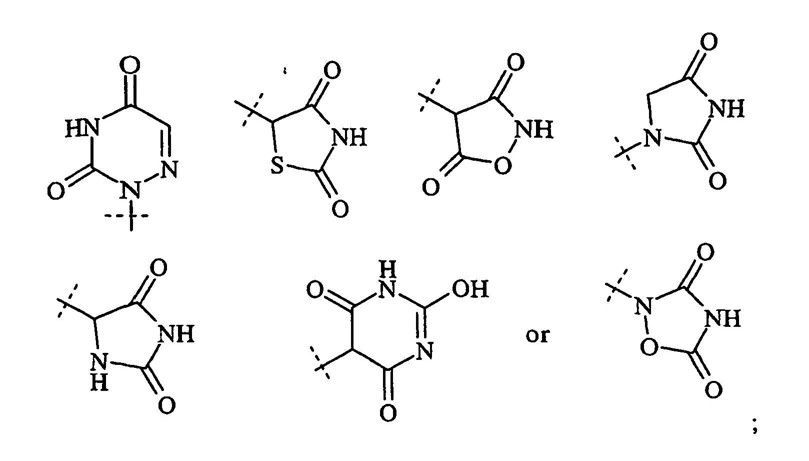
R⁶ is each independently:
   hydrogen,
   C₁-C₁₂ alkyl,
   arylalkyl,
   C₃-C₈ cycloalkyl, or
   (CH₂)ₙC(O)aryl,
   wherein alkyl, arylalkyl and cycloalkyl group being optionally substituted with one or more groups independently selected from R⁸;
R⁷ is: hydrogen,
   acyl, or
   sulfonyl;
R⁸ and R^{8a} are each independently:
   hydrogen,
   C₁-C₆ alkyl,
   C₁-C₆alkoxy,
   nitro,
   cyano,
   halo,
   haloalkyl,
   haloalkyloxy,
   aryl,
   heteroaryl,
   benzyl,
   aryloxy,
   SR⁹,
   S[O]pR⁹ or
   C[O]pR⁹; and
R⁹ is: hydrogen, C₁-C₆ alkyl, or C₃-C₈ cycloalkyl.

The compound as recited above, wherein aryl or heteroaryl are selected from the group consisting of phenyl, naphthyl, indolyl, isoindolyl, benzoimidazolyl, quinolinyl, isoquinolinyl, pyridyl, benzothiophenyl and benzofuranyl.

A preferred embodiment of the present invention is a compound having a structural formula II, 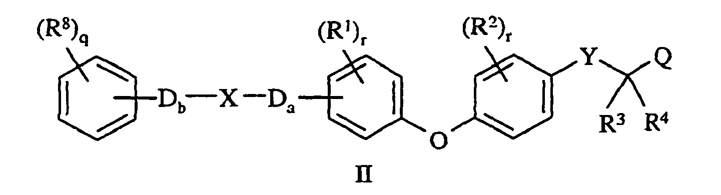 or a pharmaceutically acceptable salt or stereoisomer thereof, wherein:
q is 1,2,3,4, or 5.

The compound as recited above in formula II, wherein R⁸ is disubtituted in 2 and 4 positions, or trisubstituted in 2, 4, and 6 positions of phenyl ring relative to -D_{b}-.

Another preferred embodiment of the present invention is a compound having a structural formula III, 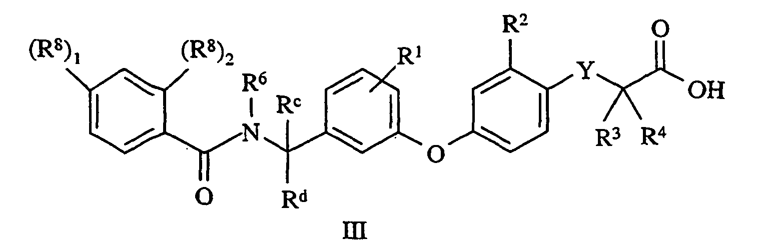 or a pharmaceutically acceptable salt or stereoisomer thereof, wherein:
Y is: O or CH₂;
R¹ is: hydrogen, halo or C₁-C₄ alkyl;
R², R³ and R⁴, R⁶, R^{c} and R^{d} are each independently: hydrogen or C₁-C₄ alkyl;
(R⁸)₁ and (R⁸)₂ are each independently:
hydrogen, halo, haloalkyl or haloalkyloxy, cyano, nitro, C₁-C₆ alkyl, C₁-C₆ alkoxy or SR⁹; R⁶ is: hydrogen or C₁-C₄ alkyl; and
R⁹ is: hydrogen or C₁-C₄ alkyl or C₃-C₆ cycloalkyl.

Yet another preferred embodiment of the present invention is the compound having a structural formula IV, 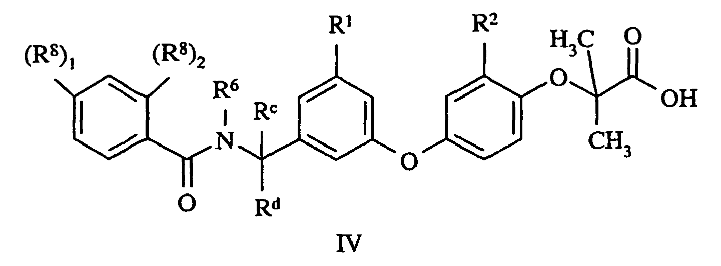 or a pharmaceutically acceptable salt or stereoisomer thereof, wherein:
R¹ and R² are each independently: hydrogen, halo or C₁-C₄ alkyl;
R^{c}, R^{d} and R⁶ are each independently: hydrogen or methyl; and
(R⁸)₁ and (R⁸)₂ are each independently:
   hydrogen, F, Cl, Br, OMe, CF₃, OCF₃, SCH₃, NO₂, cyano, methyl, ethyl, isobutyl, isopropyl or *tert*-butyl.

Yet another preferred embodiment of the present invention is the compound having a structural formula V, 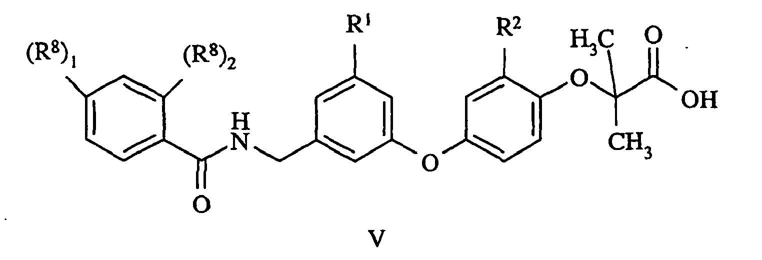 or a pharmaceutically acceptable salt or stereoisomer thereof, wherein:
R¹ and R² are each independently: hydrogen, methyl, ethyl or fluoro; and
(R⁸)₁ and (R⁸)₂ are each independently:
   hydrogen, F, Cl, Br, OMe, CF₃, OCF₃, SCH₃, NO₂, cyano, methyl, ethyl, isobutyl, isopropyl or *tert*-butyl.

Yet more preferred embodiment of the present invention is the compound having a structural formula VI, 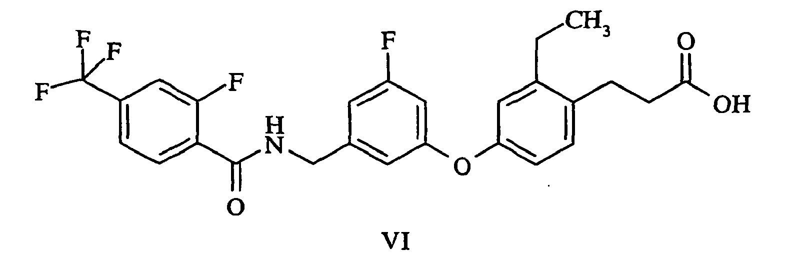 or a pharmaceutically acceptable salt or stereoisomer thereof.

Yet another preferred embodiment of the present invention is the compound having a structural formula VII, 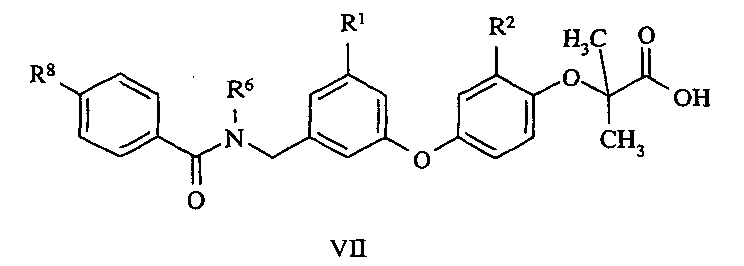 or a pharmaceutically acceptable salt or stereoisomer thereof, wherein:
R¹ and R² are each independently: hydrogen, halo or C₁-C₄ alkyl;
R⁶ is: hydrogen or C₁-C₄ alkyl;
R⁸ is: hydrogen, halo, haloalkyl or haloalkyloxy, cyano, nitro, C₁-C₆ alkyl, C₁-C₆ alkoxy or SR⁹; and
R⁹ is: hydrogen or C₁-C₄ alkyl or C₃-C₆ cycloalkyl.

The compound as recited above in formula VII, wherein R¹, R² and R⁶ are each independently hydrogen or methyl; and R⁸ is hydrogen, F, Cl, Br, OMe, CF₃, OCF₃, SCH₃, NO₂, methyl, ethyl, isobutyl, isopropyl or *tert*-butyl.

Yet another preferred embodiment of the present invention is the compound having a structural formula VIII, 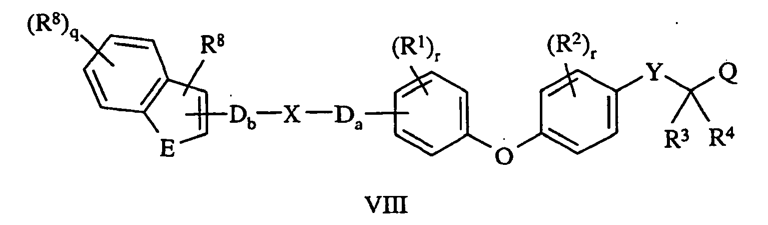 or a pharmaceutically acceptable salt or stereoisomer thereof, wherein:
q is 1,2,3 or 4; and
E is S, O or NR¹⁰ wherein R¹⁰ is hydrogen or C₁-C₄ alkyl.

Yet another preferred embodiment of the present invention is the compound having a structural formula IX, 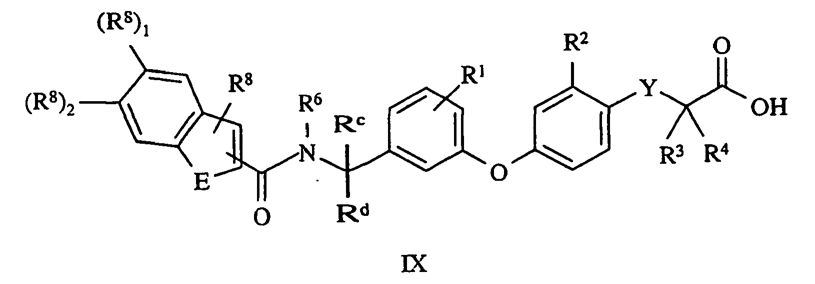 or a pharmaceutically acceptable salt or stereoisomer thereof, wherein:
Y is: O or CH₂;
E is: S, O, NH or NCH₃, NCH₂CH₃;
R¹ is: hydrogen, C₁-C₄ alkyl, halo or haloalkyl;
R², R³ and R⁴, R⁶, R^{c} and R^{d} are each independently: hydrogen or C₁-C₄ alkyl;
(R⁸)₁ and (R⁸)₂ are each independently: hydrogen, halo, haloalkyl, haloalkyloxy, cyano, nitro, C₁-C₆ alkyl or C₁-C₆ alkoxy; and
R⁸ is: hydrogen or C₁-C₄ alkyl.

Yet another preferred embodiment of the present invention is the compound having a structural formula X, 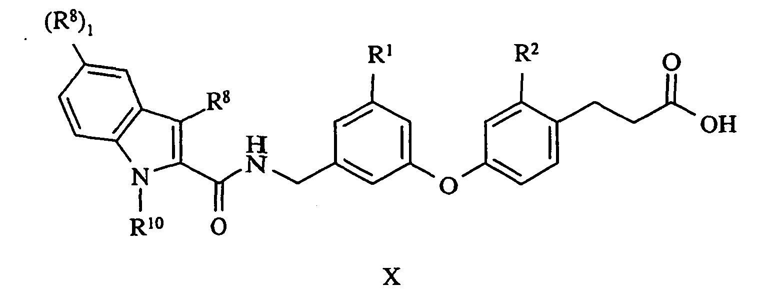 or a pharmaceutically acceptable salt or stereoisomer thereof, wherein:
R¹ and R² are each independently: hydrogen, halo or C₁-C₄ alkyl;
(R⁸)₁ is: hydrogen, F, Cl, Br, OMe, CF₃, OCF₃, SCH₃, NO₂, cyano, nitro, methyl, ethyl, isobutyl, isopropyl or *tert-*butyl;
R⁸ is: hydrogen, methyl, ethyl or propyl; and
R¹⁰ is: hydrogen, methyl or ethyl.

Yet another preferred embodiment of the present invention is the compound having a structural formula XI, 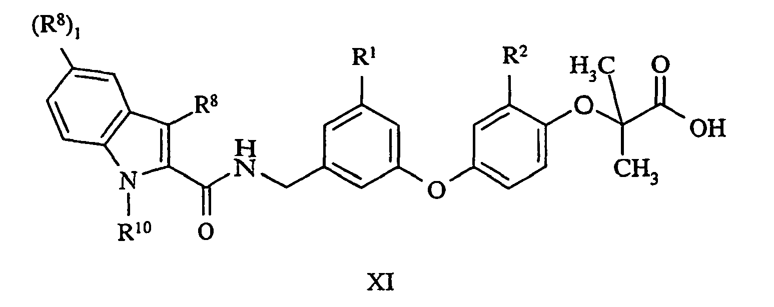 or a pharmaceutically acceptable salt or stereoisomer thereof, wherein:
R¹ and R² are each independently: hydrogen, halo or C₁-C₄ alkyl;
(R⁸)₁ is: hydrogen, F, Cl, Br, OMe, CF₃, OCF₃, SCH₃, NO₂, cyano, nitro, methyl, ethyl, isobutyl, isopropyl or *tert*-butyl;
R⁸ is: hydrogen, methyl, ethyl or propyl; and
R¹⁰ is: hydrogen, methyl or ethyl.

Yet more preferred embodiment of the present invention is the compound having a structural formula XII, 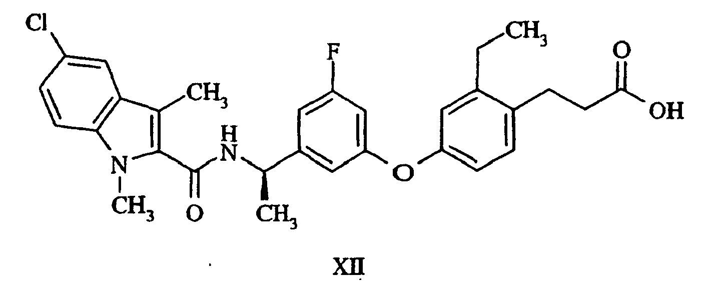 or a pharmaceutically acceptable salt.

Yet another preferred embodiment of the present invention is the compound having a structural formula XIII, 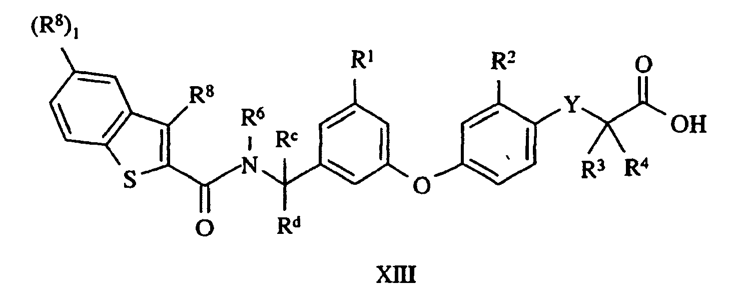 or a pharmaceutically acceptable salt or stereoisomer thereof, wherein:
Y is: O or CH₂;
R¹ is: hydrogen, C₁-C₄alkyl, halo or haloalkyl;
R², R³, R⁴, R⁶, R^{c} and R^{d} are each independently: hydrogen or C₁-C₄ alkyl;
R⁸ are each independently: hydrogen or C₁-C₄ alkyl; and
(R⁸)₁ is: hydrogen, halo, haloalkyl or haloalkyloxy, cyano, nitro, C₁-C₆alkyl or C₁-C₆ alkoxy.

The compound as recited above in formula XIII, wherein Y is O or CH₂; R¹ is hydrogen, methyl, F, Br or Cl; R² is hydrogen, methyl or ethyl; R³, R⁴, R⁶, R⁸, R^{c} and R^{d} are each independently hydrogen or methyl; and (R⁸)₁ is hydrogen, F, Cl, Br, OMe, CF₃, OCF₃, SCH₃, NO₂, cyano, nitro, methyl, ethyl, isobutyl, isopropyl or *tert-*butyl.

Yet another preferred embodiment of the present invention is the compound having a structural formula XIV, 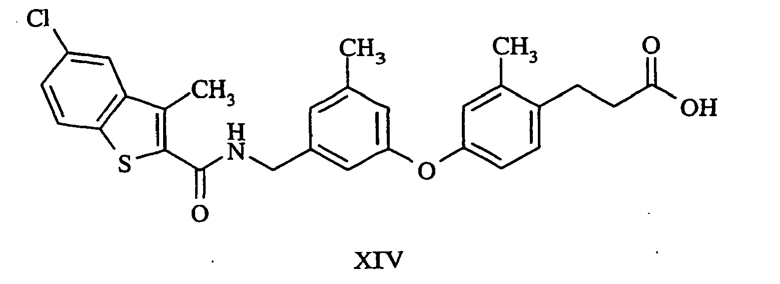 or a pharmaceutically acceptable salt.

Yet more preferred embodiment of the present invention is the compound having a structural formula XV, 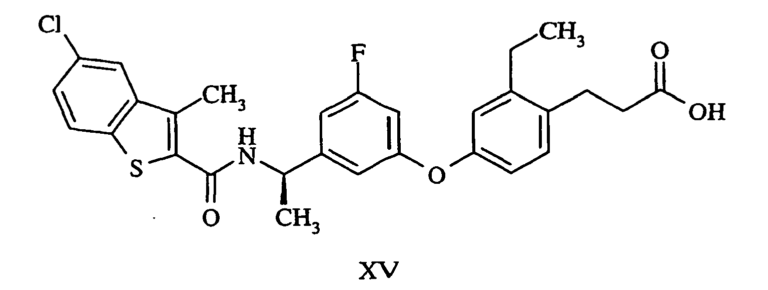 or a pharmaceutically acceptable salt.

Yet another preferred embodiment of the present invention is the compound having a structural formula XVI, 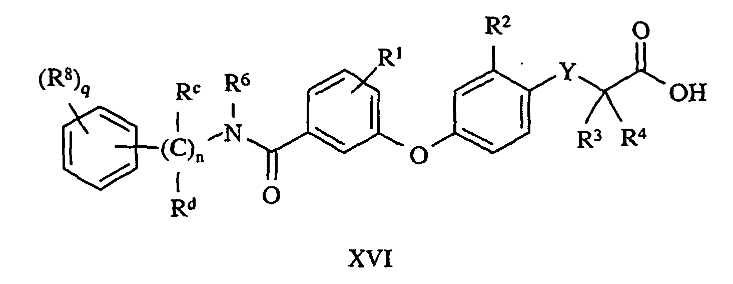 or a pharmaceutically acceptable salt or stereoisomer thereof, wherein
n is 1, 2, 3, or 4.

The compound as recited above in formula XVI, wherein Y is O or CH₂; R¹, R², R³, R⁴ R^{c} and R^{d} are each independently hydrogen or C₁-C₄ alkyl; n is 1 or 2; R⁶ is hydrogen, C₁-C₆ alkyl or arylalkyl; and R⁸ is hydrogen, C₁-C₆ alkoxy, halo or haloalkyl.

Yet another preferred embodiment of the present invention is the compound having a structural formula XVII, 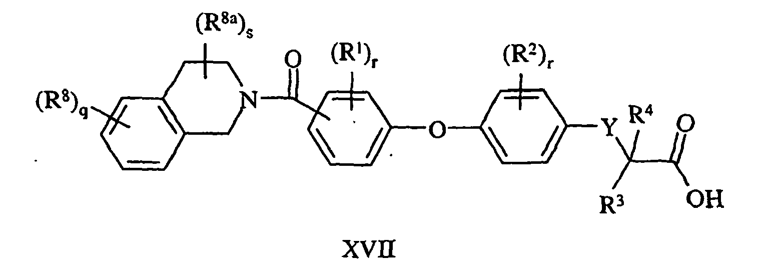 or a pharmaceutically acceptable salt or stereoisomer thereof, wherein:
R^{8a} is hydrogen, C₁-C₄ alkyl or aryl; and s is 1, 2, 3,4, 5 or 6.

Yet another preferred embodiment of the present invention is the compound having a structural formula XVIII, 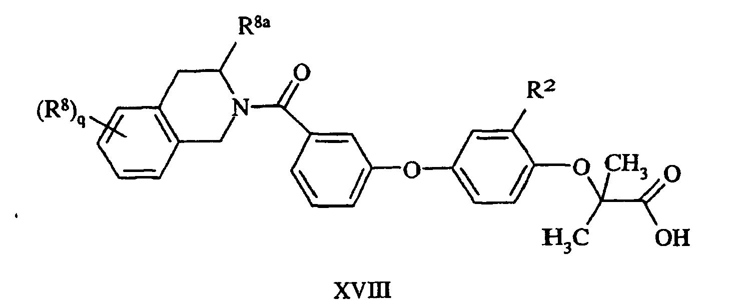 or a pharmaceutically acceptable salt or stereoisomer thereof, wherein:
R² is: hydrogen or C₁-C₄ alkyl,
R⁸ is: hydrogen, C₁-C₆alkyl, C₁₋₆alkoxy, halo, haloalkyl or haloalkyloxy;
R^{8a} is: hydrogen, methyl, or phenyl; and
q is: 1 or 2.

Yet another preferred embodiment of the present invention is the compound having a structural formula XIX, 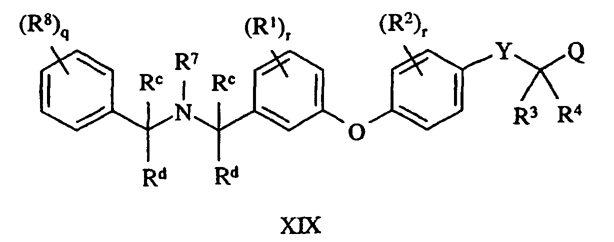 or a pharmaceutically acceptable salt or stereoisomer thereof, wherein:

The compound as recited above in formula XIX, wherein Q is COOH; R⁷ is hydrogen, methanesulfonyl or acetyl; and R^{c} and R^{d} are each hydrogen.

Yet more preferred embodiment of the present invention is the compound selected from the group consisting of:

| No | Structure | Name |
|---|---|---|
| 1 | 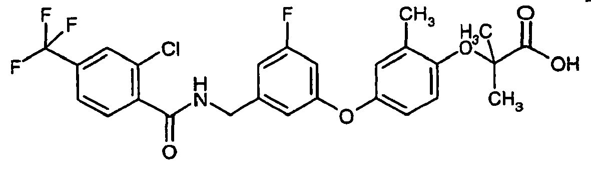 | 2-(4-{3-[(2-Chloro-4-trifluoromethyl-benzoylamino)-methyl]-5-fluoro-phenoxy}-2-methyl-phenoxy)-2-methyl-propionic acid |
| 2 | 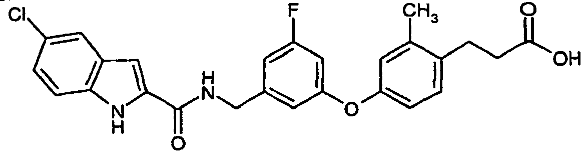 | 3-[4-(3-{[(5-Chloro-1H-indole-2-carbonyl)-amino]-methyl)-5-fluoro-phenoxy)-2-methyl-phenyl]-propionic acid |
| 3 | 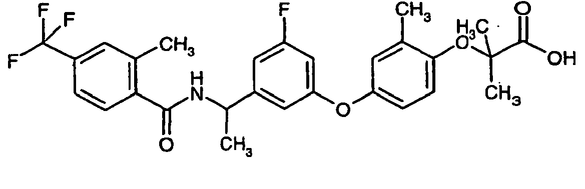 | 2-(4-{3-Fluoro-5-[1-(2-methyl-4-trifluoromethyl-benzoylamino)-ethyl]-phenoxy}-2-methyl-phenoxy)-2-methyl-propionic acid (isomer 1) |
| 4 | 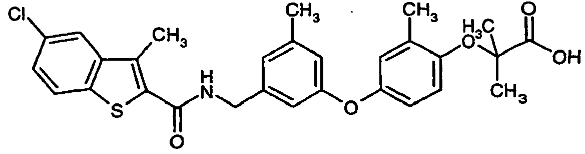 | 2-[4-(3-{[(5-Chloro-3-methyl-benzo[b]thiophene-2-carbonyl)-amino]-methyl}-5-methyl-phenoxy)-2-methyl-phenoxy]-2-methyl-propionic acid |
| 5 | 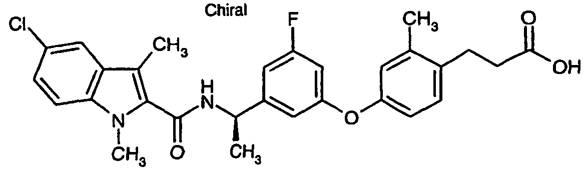 | (R)-3-[4-(3-{1-[(5-Chloro-1,3-dimethyl-1H-indole-2-carbonyl)-amino]-ethyl}-5-fluoro-phenoxy)-2-methyl-phenyl]-propionic acid |
| 6 | 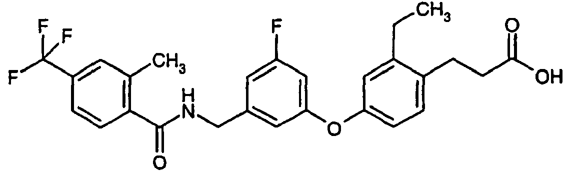 | 3-(2-Ethyl-4-{3-fluoro-5-[(2-methyl-4-trifluoromethyl-benzoylamino)-methyl]-phenoxy}-phenyl)-propionic acid |
| 7 | 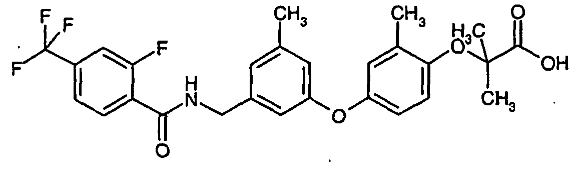 | 2-(4- {3-[(2-Fluoro-4-trifluoromethyl-benzoylamino)-methyl]-5-methyl-phenoxy}-2-methyl-phenoxy)-2-methyl-propionic acid |
| 8 | 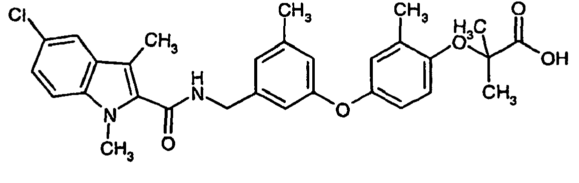 | (R)-2-[4-(3-{[(5-Chloro-1,3-dimethyl-1H-indole-2-carbonyl)-amino]-methyl)-5-methyl-phenoxy)-2-methyl-phenoxy]-2-methyl-propionic acid |
| 9 | 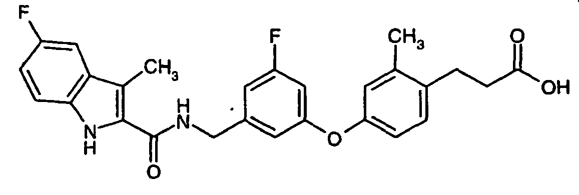 | 3-[4-(3-Fluoro-5-{[(5-fluoro-3-methyl-1H-indole-2-carbonyl)-amino]-methyl} -phenoxy)-2-methyl-phenyl]-propionic acid |
| 10 | 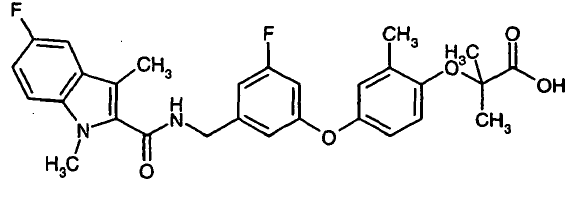 | 2-[4-(3-Flu oro-5-{[(5-fluoro-1,3-dimethyl-1H-indole-2-carbonyl)-amino]-methyl}-phenoxy)-2-methyl-phenoxy]-2-methyl-propionic acid |
| 11 | 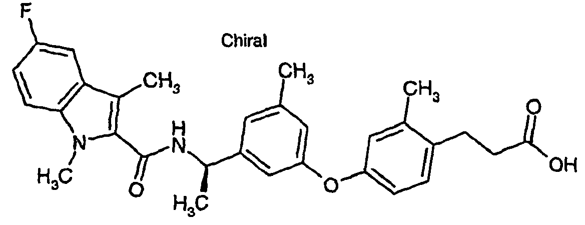 | (R) -3-[4-(3-{1-[(5-Fluoro-1,3-dimethyl-1H-indole-2-carbonyl)-amino]-ethyl} -5-methyl-phenoxy)-2-methyl-pbenyl]-propionic acid |
| 12 | 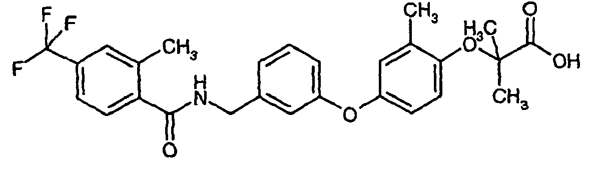 | 2-Methyl-2-(2-methyl-4-{3-[(2-methyl-4-trifluoromethyl-benzoylamino)-methyl]-phenoxy}-phenoxy)-propionic acid |
| 13 | 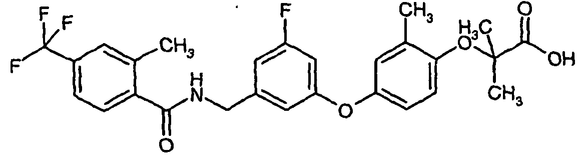 | 2-(4-{(3-Fluoro-5-[(2-methyl-4-trifluoromethyl-benzoylamino)-methyl]-phenoxy}-2-methyl-phenoxy)-2-methyl-propionic acid |
| 14 | 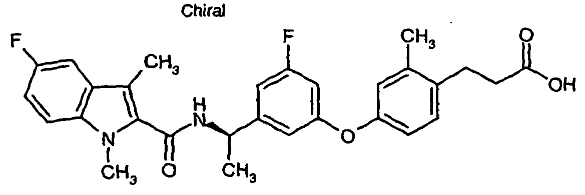 | (R) -3-[4-(3-Fluoro-5-{1-[(5-fluoro-1,3-dimethyl-1H-indole-2-carbonyl)-amino]-ethyl}-phenoxy)-2-methyl-phenyl]-propionic acid |
| 15 | 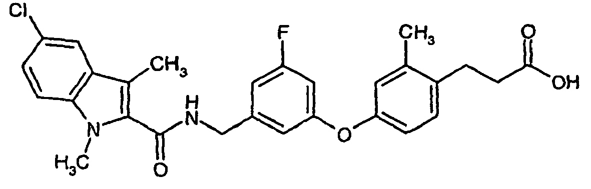 | 3-[4-(3-{[(5-Chloro-1,3-dimethyl-1H-i nd ol e-2-carbonyl)-amino]-methyl}-5-fluoro-phenoxy)-2-methyl-phenyl]-propionic acid |
| 16 | 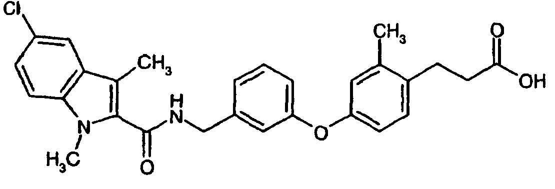 | 3-[4-(3-{[(5-Chloro-1,3-dimethyl-1H-indole-2-carbonyl)-amino]-methyl}-phenoxy)-2-methyl-phenyl]-propionic acid |
| 17 | 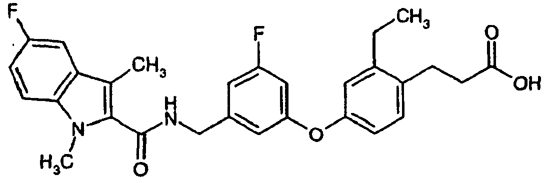 | 3-[2-Ethyl-4-(3-fluoro-5-{[(5-fluoro-1,3-dimethyl-1H-indole-2-carbonyl)-amino]-methyl}-phenoxy)-phenyl]-propionic acid |
| 18 | 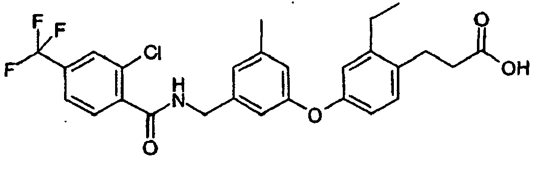 | 3-(4-{3-[(2-Chloro-4-trifluoromethyl-benzoylamino)-methyl]-5-methyl-phenoxy}-2-ethyl-phenyl)-propionic acid |

Also encompassed by the present invention is a pharmaceutical composition comprising a pharmaceutically acceptable carrier and a compound of the present invention or a pharmaceutically acceptable salt, solvate or hydrate thereof.

Also encompassed by the present invention is use of a compound of the present invention or a pharmaceutically acceptable salt thereof for the manufacture of a medicament for lowering blood-glucose in a mammal.

Also encompassed by the present invention is use of a compound of the present invention or a pharmaceutically acceptable salt thereof for the manufacture of a medicament for treating and/or preventing disease or condition in a mammal selected from the group consisting of hyperglycemia, dyslipidemia, Type II diabetes, Type I diabetes, hypertriglyceridemia, syndrome X, insulin resistance, heart failure, diabetic dyslipidemia, hyperlipidemia, hypercholesteremia, hypertension, obesity, anorexia bulimia, anorexia nervosa, cardiovascular disease and other diseases where insulin resistance is a component,

Also encompassed by the present invention is use of a compound of the present invention or a pharmaceutically acceptable salt thereof for the manufacture of a medicament for treating and/or preventing diabetes mellitus in a mammal.

Also encompassed by the present invention is use of a compound of the present invention or a pharmaceutically acceptable salt thereof for the manufacture of a medicament for treating and/or preventing cardiovascular disease in a mammal.

Also encompassed by the present invention is treating and/or preventing syndrome X in a mammal.

Also encompassed by the present invention is use of a compound of the present invention and a pharmaceutically acceptable salt, solvate, hydrate or stereoisomer thereof, for the manufacture of a medicament for the treatment of a condition modulated by a PPAR.

The terms used to describe the present invention have the following meanings unless otherwise indicated.

An "aliphatic group" is non-aromatic consisting solely of carbon and hydrogen and may optionally contain one or more units of unsaturation, e.g., double-bonds ("alkenyl") and/or triple-bonds ("alkynyl"). An aliphatic group may be straight chained, branched or cyclic. When straight chained or branched, an aliphatic group typically contains between about 1 and about 12 carbon atoms, more typically between about 1 and about 6 carbon atoms. When cyclic, an aliphatic group typically contains between about 3 and about 10 carbon atoms, more typically between about 3 and about 7 carbon atoms. Aliphatic groups are preferably C₁-C₁₂ straight chained and/or branched alkyl groups (i.e., completely saturated aliphatic groups), more preferably C₁-C₆straight chained and/or branched alkyl groups. Examples include, but are not limited to, methyl, ethyl, *n*-propyl, *iso*-propyl, *n*-butyl, *sec*-butyl, *tert*-butyl and the like.

The term "alkyl," unless otherwise indicated, refers to those alkyl groups of a designated number of carbon atoms of either a straight or branched saturated configuration, including substituted alkyl. The term "alkyl" used herein also includes "alkylene group" of either straight or branched saturated configuration, including substituted alkylene. Examples of "alkyl" include, but are not limited to: methyl, ethyl, n-propyl, isopropyl, n-butyl, isobutyl, sec-butyl and tert-butyl, pentyl, hexyl, isopentyl and the like. Examples of "alkylene group" is -[C(R^{c})(R^{d})]ₙ- where n is a positive integer, and R^{c} and R^{d} are independently hydrogen or C₁-C₆ alkyl. Preferably, n is an integer from about 1 to about 6, and more preferably from about 1 to about 4. A "branched (or substituted) alkylene group" is an alkylene group in which one or more methylene hydrogen atoms are replaced with a substituent, such as methyl, ethyl or the like. Alkyl as defined above may be optionally substituted with a designated number of substituents as set forth in the embodiment recited above.

The term "alkenyl" means carbon chains which contain at least one carbon-carbon double bond, and which may be linear or branched or combinations thereof. Examples of alkenyl include vinyl, allyl, isopropenyl, pentenyl, hexenyl, heptenyl, 1-propenyl, 2-butenyl, 2-methyl-2-butenyl, and the like.

The term "alkynyl" means carbon chains which contain at least one carbon-carbon triple bond, and which may be linear or branched or combinations thereof. Examples of alkynyl include ethynyl propargyl, 3-methyl-1-pentynyl, 2- heptynyl and the like.

The term "alkoxy" represents an alkyl group of indicated number of carbon atoms attached through an oxygen bridge, such as methoxy, ethoxy, propoxy, isopropoxy, butoxy, tert-butoxy, pentoxy, and the like. Alkoxy as defined above may be optionally substituted with a designated number of substituents as set forth in the embodiment recited above.

The term "cycloalkyl" refers to a saturated or partially saturated carbocycle containing one or more rings of from 3 to 12 carbon atoms, more typically 3 to 6 carbon atoms. Examples of cycloalkyl includes, but are not limited to cyclopropyl, cyclobutyl, cyclopentyl, cyclohexyl and cycloheptyl, and the like. Cycloalkyl as defined above may also includes a tricycle, such as adamantyl. Cycloalkyl as defined above may be optionally substituted with a designated number of substituents as set forth in the embodiment recited above.

The term "halo" refers to fluoro, chloro, bromo and iodo.

The term "haloalkyl" is a C₁-C₆ alkyl group, which is substituted with one or more halo atoms selected from F, Br, Cl and I. Examples of haloalkyl group are trifluoromethyl, CH₂CF₃ and the like.

The term "haloalkyloxy" represents a C₁-C₆ haloalkyl group attached through an oxygen bridge, such as OCF₃. The "haloalkyloxy" as defined above may be optionally substituted with a designated number of substituents as set forth in the embodiment recited above.

The term "aryl" includes carbocyclic aromatic ring systems (e.g. phenyl), fused polycyclic aromatic ring systems (e.g. naphthyl and anthracenyl) and aromatic ring systems fused to carbocyclic non-aromatic ring systems (e.g., 1,2,3,4-tetrahydronaphthyl). The "aryl" as defined above may be optionally substituted with a designated number of substituents as set forth in the embodiment recited above.

The term "aryloxy" represents an aryl group attached through an oxygen bridge, such as phenoxy (-O-phenyl). The "aryloxy" as defined above may be optionally substituted with a designated number of substituents as set forth in the embodiment recited above.

The term "heteroaryl" group, as used herein, is an aromatic ring system having at least one heteroatom such as nitrogen, sulfur or oxygen and includes monocyclic, bicyclic or tricyclic aromatic ring of 5- to 14-carbon atoms containing one or more heteroatoms selected from O, N, or S. The heteroaryl as defined above also includes heteroaryl fused with another heteroaryl, aryl fused with heteroaryl or aryl fused with heterocyclyl as defined herein. The "heteroaryl" may also be optionally substituted with a designated number of substituents as set forth in the embodiment recited above. Examples of heteroaryl are, but are not limited to: furanyl, thienyl (also referred to as "thiophenyl"), thiazolyl, imidazolyl, indolyl, isoindolyl, isooxazolyl, oxazoyl, pyrazolyl, pyrrolyl, pyrazinyl, pyridyl, pyrimidyl, pyrimidinyl and purinyl, cinnolinyl, benzofuranyl, benzoimidazolyl, benzothienyl (or benzothiophenyl), benzotriazolyl, benzoxazolyl, quinolinyl, isoxazolyl, isoquinolinyl 1,4 benzodioxan, or 2,3-dihydrobenzofuranyl and the like.

The term "heterocyclyl" refers to a non-aromatic ring which contains one or more heteroatoms selected from O, N or S, which includes a monocyclic, bicyclic or tricyclic ring of 5- to 14-carbon atoms containing one or more heteroatoms selected from O, N or S. The "heterocyclyl" as defined above may be optionally substituted with a designated number of substituents as set forth in the embodiment recited above. Examples of heterocyclyl include, but are not limited to, morpholine, piperidine, piperazine, pyrrolidine, and thiomorpholine.

The term "carbocyclyl" (also referred as "nonaromatic carbocyclic ring") refers to a saturated or partially saturated nonaromatic carbocyclic ring. Examples of carbocyclyl are, but are not limited to, cyclopentyl, cyclohexyl, cyclopentenyl, cyclohexenyl and the like.

An "arylalkyl" as used herein is an aryl substituent that is linked to a compound by an alkyl group having from one to six carbon atoms (e.g., C₁-C₆ alkyl-aryl). The "arylalkyl" as defined above may be optionally substituted with a designated number of substituents as set forth in the embodiment recited above.

The "acyl" represent an "alkyl-C(=O)- " group. Preferred acyl group are those in which the alkyl group is lower alkyl, such C₁-C₆ alkyl. Example of "acyl" is acetyl.

A "sulfonyl" is represent an "alkyl-S(O)₂-" group Preferred sulfonyl group are those in which the alkyl group is lower alkyl, such C₁-C₆ alkyl. Example of "sulfonyl" is mathanesulfonyl, ethansulfonyl and the like.

The "aminoalkyl" as used herein contains both a basic amino group (NH₂) and an alkyl group as defined above.

The term R^{5A} (or acid bioisosteres) as used herein includes, but are not limited to, carboxamide, sulfonamide, acylsulfonamide, tetrazole or the following moiety. 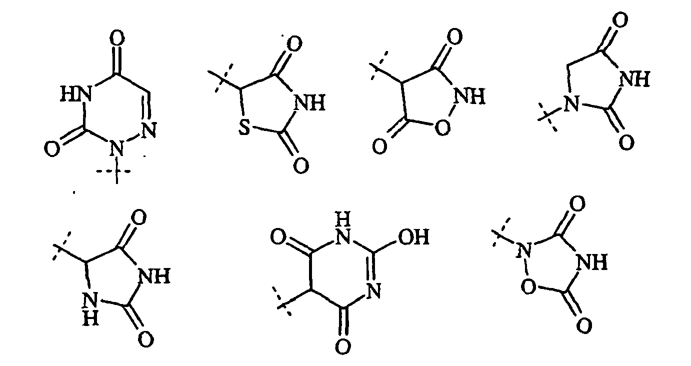 Carboxamide, sulfonamide, acylsulfonamide and tetrazole may be optionally substituted with one or more suitable substituents selected from haloalkyl, aryl, heteroaryl, and C₁-C₆ alkyl. The heteroalkyl, aryl, heteroaryl and alkyl may further optionally substituted with one or more substituents selected from the list provided for R⁸. The examples of R^{5A} are, but not limited to, hydroxamic acid, acyl cyanamide, tetrazoles, sulfinylazole, sulfonylazole, 3-hydroxyisoxazole, hydroxythiadiazole, sulphonate and acylsulfonamide.

The term "active ingredient" means the compounds generically described by Formula I as well as the salts, solvates and prodrugs of such compounds.

The term "pharmaceutically acceptable" means that the carrier, diluents, excipients and salt must be compatible with the other ingredients of the composition, and not deleterious to the recipient thereof. Pharmaceutical compositions of the present invention are prepared by procedures known in the art using well-known and readily available ingredients.

"Preventing" refers to reducing the likelihood that the recipient will incur or develop any of the pathological conditions described herein.

"Treating" refers to mediating a disease and/or condition, and preventing and/or mitigating its further progression or ameliorating the symptoms associated with the disease or condition.

"Pharmaceutically-effective amount" means that amount of a compound of the present invention, or of its salt, solvate, hydrate or prodrug thereof that will elicit the biological or medical response of a tissue, system or mammal. Such an amount can be administered prophylactically to a patient thought to be susceptible to development of a disease or condition. Such amount when administered prophylactically to a patient can also be effective to prevent or lessen the severity of the mediated condition. Such an amount is intended to include an amount, which is sufficient to modulate a PPAR receptor such as a PPARα, PPARγ, PPARδ or PPARγ/δ receptor to mediate a disease or condition. Conditions mediated by PPAR receptors include, for example, diabetes mellitus, cardiovascular disease, Syndrome X, obesity and gastrointestinal disease. Additional conditions associated with the modulation of a PPAR receptor include inflammation related conditions, which include, for example, IBD (inflammatory bowel disease), rheumatoid arthritis, psoriasis, AJzheimer's disease, Chrohn's disease and ischemia reprofusion injury (stroke and miocardial infarction).

A "mammal" is an individual animal that is a member of the taxonomic class Mammalia. The class Mammalia includes humans, monkeys, chimpanzees, gorillas, cattle, swine, horses, sheep, dogs, cats, mice, rats and the like.

Administration to a human is most preferred. A human to whom the compounds and compositions of the present invention are administered has a disease or condition in which control blood glucose levels are not adequately controlled without medical intervention, but wherein there is endogenous insulin present in the human's blood. Non-insulin dependent diabetes mellitus (NIDDM) is a chronic disease or condition characterized by the presence of insulin in the blood, even at levels above normal, but resistance or lack of sensitivity to insulin action at the tissues.

Those skilled in the art will recognize that sterocenters exist in compound of the present invention. Accordingly, the present invention includes all possible stereoisomers and geometric isomers of the presently claimed compounds including racemic compounds and the optically active isomers.

The compounds of the present invention contain one or more chiral centers and exist in different optically active forms. When compounds of the present invention contain one chiral center, the compounds exist in two enantiomeric forms and the present invention includes both enantiomers and mixtures of enantiomers, such as racemic mixtures. Resolution of the final product, an intermediate or a starting material may be effected by any suitable method known in the art, for example by formation of diastereoisomeric salts which may be separated by crystallization; formation of diastereoisomeric derivatives or complexes which may be separated by crystallization and gas-liquid or liquid chromatography; selective reaction of one enantiomer with an enantiomer-specific reagent such as enzymatic esterification; and gas-liquid or liquid chromatography in a chiral environment such as on a chiral support, for example silica with a bound chiral ligand or in the presence of a chiral solvent. See also Sterochemistry of Carbon Compounds by E.L. Eliel (Mcgraw Hill, 1962) and *Tables of Resolving Agents* by S. H. Wilen. It will be appreciated that where the desired enantiomer is converted into another chemical entity by one of the separation procedures described above, a further step is required to liberate the desired enantiomeric form. Alternatively, specific enantiomers may be synthesized by asymmetric synthesis using optically active reagents, substrates, catalysts or solvents, or by converting one enantiomer into the other by asymmetric transformation.

When a compound of the present invention has more than one chiral substituents, it may exist in diastereoisomeric forms. The diastereoisomeric pairs may be separated by methods known to those skilled in the art, for example chromatography or crystallization and the individual enantiomers within each pair may be separated as described above. The present invention includes each diastereoisomer of compounds of formula I and mixtures thereof.

Certain compounds of the present invention may exist in different stable conformational forms, which may be separable. Torsional asymmetry due to restricted rotation about an asymmetric single bond, for example because of steric hindrance or ring strain, may permit separation of different conformers. The present invention includes each conformational isomer of compounds of formula I and mixtures thereof.

Certain compound of the present invention may exist in zwitterionic form, and the present invention includes each zwitterionic form of compounds of formula I and mixtures thereof.

Certain compounds of the present invention and their salts may exist in more than one crystal form. Polymorphs of compounds of formula I form part of the present invention and may be prepared by crystallization of a compound of formula I under different conditions, such as using different solvents or different solvent mixtures for recrystallization; crystallization at different temperatures; and various modes of cooling ranging from very fast to very slow cooling during crystallization. Polymorphs may also be obtained by heating or melting a compound of formula I followed by gradual or fast cooling. The presence of polymorphs may be determined by solid probe NMR spectroscopy, IR spectroscopy, differential scanning calorimetry, powder X-ray diffraction or other available techniques.

Certain compounds of the present invention and their salts may exist in more than one crystal form, which includes each crystal form and mixtures thereof.

Certain compounds of the present invention and their salts may also exist in the form of solvates, for example hydrates, and thus the present invention includes each solvate and mixtures thereof.

"Pharmaceutically-acceptable salt" refers to salts of the compounds of formula I, which are substantially non-toxic to mammals. Typical pharmaceutically acceptable salts include those salts prepared by reaction of the compounds of the present invention with a mineral, organic acid: an organic base or inorganic base. Such salts are known as base addition salts, respectively. It should be recognized that the particular counterion forming a part of any salt of the present invention is not of a critical nature so long as the salt as a whole is pharmaceutically acceptable and the counterion does not contribute undesired qualities to the salt as a whole.

By virtue of its acidic moiety, a compound of the present invention forms salts with pharmaceutically acceptable bases. Some examples of base addition salts include metal salts such as aluminum; alkali metal salts such as lithium, sodium or potassium; and alkaline earth metal salts such as calcium, magnesium, ammonium, or substituted ammonium salts. Examples of substituted ammonium salts include, for instance, those with lower alkylamines such as trimethylamine and triethylamine; hydroxyalkylamines such as 2-hydroxyethylamine, bis-(2-hydroxyethyl)-amine or tri-(2-hydroxyethyl)-amine; cycloalkylamines such as bicyclohexylamine or dibenzylpiperidine, N-benzyl-β-phenethylamine, dehydroabietylamine, N,N'-bisdehydro-abietylamine, glucamine, N-piperazine methylglucamine; bases of the pyridine type such as pyridine, collidine, quinine or quinoline; and salts of basic amino acids such as lysine and arginine.

Examples of inorganic bases include, without limitation, sodium hydroxide, potassium hydroxide, potassium carbonate, sodium carbonate, sodium bicarbonate, potassium bicarbonate, calcium hydroxide, calcium carbonate, and the like.

Compounds of the present invention, which are substituted with a basic group, may exist as salts with pharmaceutically acceptable acids. The present invention includes such salts. Examples of such salts include hydrochlorides, hydrobromides, sulfates, methanesulfonates, nitrates, maleates, acetates, citrates, fumarates, tartrates [e.g. (+)-tartrates, (-)-tartrates or mixtures thereof including racemic mixtures], succinates, benzoates and salts with amino acids such as glutamic acid. These salts may be prepared by methods known to those skilled in the art.

The compounds of the present invention (or salt or prodrug, etc.) may also form a solvate with water (e.g., hydrate) or an organic solvent, and the present invention encompasses any solvate, hydrate or any mixtures thereof.

The compounds of present invention, which bind to and activate the PPARs, lower one or more of glucose, insulin, triglycerides, fatty acids and/or cholesterol, and are therefore useful for the treatment and/or prevention of hyperglycemia, dyslipidemia and in particular Type II diabetes as well as other diseases including syndrome X, Type I diabetes, hypertriglyceridemia, insulin resistance, diabetic dyslipidemia, hyperlipidemia, hypercholesteremia, heart failure, coagaulopathy, hypertension, and cardiovascular diseases, especially arteriosclerosis. In addition, these compounds are indicated to be useful for the regulation of appetite and food intake in subjects suffering from disorders such as obesity, anorexia bulimia and anorexia nervosa.

The compounds and compositions of the present invention are also useful to treat acute or transient disorders in insulin sensitivity, which sometimes occurs following a surgery, trauma, myocardial infarction and the like. The compounds and compositions of the present invention are also useful for lowering serum triglyceride levels. Elevated triglyceride level, whether caused by genetic predisposition or by a high fat diet, is a risk factor for the development of heart disease, stroke, and circulatory system disorders and diseases. The physician of ordinary skill will know how to identify humans who can benefit from administration of the compounds and compositions of the present invention.

The present invention further provides a compound of formula I, or a tautomeric form thereof and/or a pharmaceutically acceptable salt thereof and/or a pharmaceutically acceptable solvate thereof for use in the treatment and/or prophylaxis of hyperglycemia in a human or non-human mammal.

The compounds of the present invention are useful as therapeutic substances in preventing or treating Syndrome X, diabetes mellitus and related endocrine and cardiovascular disorders and diseases in human or non-human animals.

The present invention also relates to the use of a compound of formula I as described above for the manufacture of a medicament for treating a PPARγ or PPARδ mediated condition, separately or in combination.

A therapeutically effective amount of a compound of the present invention can be used for the preparation of a medicament useful for treating Syndrome X, diabetes, treating obesity, lowering tryglyceride levels, raising the plasma level of high density lipoprotein, and for treating, preventing or reducing the risk of developing arteriosclerosis, and for preventing or reducing the risk of having a first or subsequent atherosclerotic disease event in mammals, particularly in humans. In general, a therapeutically effective amount of a compound of formula I of the present invention typically reduces serum glucose levels, more specifically HbA1c, of a patient by about 0.7% or more; typically reduces serum triglyceride levels of a patient by about 20% or more: and increases serum HDL levels in a patient. Preferably, HDL levels can be increased by about 30% or more.

Additionally, an effective amount of a compound of the present invention and a therapeutically effective amount of one or more active agents selected from antihyperlipidemic agent, plasma HDL-raising agents, antihypercholesterolemic agents, fibrates, vitamins, aspirin, insulin secretogogues, insulin and the like can be used together for the preparation of a medicament useful for the above described treatments.

Advantageously, compositions containing the compound of the present invention or their salts may be provided in dosage unit form, preferably each dosage unit containing from about 1 to about 500 mg. It is understood that the amount of the compounds or compounds of the present invention that will be administered is determined by a physician considering of all the relevant circumstances.

Syndrome X includes pre-diabetic insulin resistance syndrome and the resulting complications thereof, insulin resistance, non-insulin dependent diabetes, dyslipidemia, hyperglycemia obesity, coagulopathy, hypertension and other complications associated with diabetes. The treatments mentioned herein include the above and encompass the treatment and/or prophylaxis of any one of or any combination of the following: pre-diabetic insulin resistance syndrome, the resulting complications thereof, insulin resistance, Type II or non-insulin dependent diabetes, dyslipidemia, hyperglycemia, obesity and the complications associated with diabetes including cardiovascular disease, especially arteriosclerosis.

The compositions are formulated and administered in the same general manner as detailed herein.

The compounds of the present invention and their pharmaceutically acceptable salt, solvate or hydrate thereof have valuable pharmacological properties and can be used in pharmaceutical compositions containing a therapeutically effective amount of a compound of the present invention, or a pharmaceutically acceptable salt, ester or prodrug thereof, in combination with one or more pharmaceutically acceptable excipients. Excipients are inert substances such as, without limitation carriers, diluents, fillers, flavoring agents, sweeteners, lubricants, solubilizers, suspending agents, wetting agents, binders, disintegrating agents, encapsulating material and other conventional adjuvants. Proper excipient is dependent upon the route of administration chosen. Pharmaceutical compositions typically contain from about 1 to about 99 weight percent of the active ingredient, which is a compound of the present invention.

Preferably, the pharmaceutical formulation is in unit dosage form. A "unit dosage form" is a physically discrete unit containing a unit dose suitable for administration in human subjects or other mammals. For example, a unit dosage form can be a capsule or tablet, or a number of capsules or tablets. A "unit dose" is a predetermined quantity of the active compound of the present invention, calculated to produce the desired therapeutic effect, in association with one or more pharmaceutically acceptable excipients. The quantity of active ingredient in a unit dose may be varied or adjusted from about 0.1 to about 1000 milligrams or more according to the particular treatment involved.

The dosage regimen utilizing the compounds of the present invention is selected by one of ordinary skill in the medical or veterinary arts considering various factors, such as without limitation, the species, age, weight, sex, medical condition of the recipient, the severity of the condition to be treated, the route of administration, the level of metabolic and excretory function of the recipient, the dosage form employed, the particular compound and salt thereof employed, and the like.

Preferably, the compounds of the present invention are administered in a single daily dose, or the total daily dose may be administered in divided doses of two, three or more times per day. Where delivery is via transdermal forms, administration is continuous.

Suitable routes of administration of pharmaceutical compositions of the present invention include, for example, oral, eye drop, rectal, transmucosal, topical or intestinal administration; parenteral delivery (bolus or infusion), including intramuscular, subcutaneous, intramedullary injections, as well as intrathecal, direct intraven-tricular, intravenous, intraperitoneal, intranasal, or intraocular injections. The compounds of the present invention can also be administered in a targeted drug delivery system, such as in a liposome coated with endothelial cell-specific antibody.

For oral administration, the compounds of the present invention can be formulated readily by combining the active compounds with pharmaceutically acceptable carriers well known in the art. Such carriers enable the compounds of the present invention to be Formulated as tablets, pills, powders, sachets, granules, dragees, capsules, liquids, elixirs, tinctures, gels, emulsions, syrups, slurries, suspensions and the like, for oral ingestion by a patient to be treated. Pharmaceutical preparations for oral use can be obtained by combining the active compound with a solid excipient, optionally grinding a resulting mixture, and processing the mixture of granules, after adding suitable auxiliaries, if desired, to obtain tablets or dragee cores.

For oral administration in the form of a tablet or capsule, the active ingredient may be combined with an oral, non-toxic, pharmaceutically-acceptable carrier, such as, without limitation, lactose, starch, sucrose, glucose, methyl cellulose, calcium carbonate, calcium phosphate, calcium sulfate, sodium carbonate, mannitol, sorbitol, and the like; together with, optionally, disintegrating agents, such as, without limitation, cross-linked polyvinyl pyrrolidone, maize, starch, methyl cellulose, agar, bentonite, xanthan gum, alginic acid: or a salt thereof such as sodium alginate, and the like; and, optionally, binding agents, for example, without limitation, gelatin, acacia, natural sugars, beta-lactose, corn sweeteners, natural and synthetic gums, acacia, tragacanth, sodium alginate, carboxymethyl-cellulose, polyethylene glycol, waxes, and the like; and, optionally, lubricating agents, for example, without limitation, magnesium stearate, sodium stearate, stearic acid: sodium oleate, sodium benzoate, sodium acetate, sodium chloride, talc, and the like. When a dosage unit form is a capsule, it may contain, in addition to materials of the above type, a liquid carrier such as a fatty oil.

Solid forms include powders, tablets and capsules. A solid carrier can be one or more substances, which may also act as flavoring agents, lubricants, solubilisers, suspending agents, binders, tablet disintegrating agents and encapsulating material.

In powders, the carrier is a finely divided solid, which is in admixture with the finely divided active ingredient. In tablets, the active ingredient is mixed with a carrier having the necessary binding properties in suitable proportions and compacted in the shape and size desired.

Various other materials may be present as coatings or to modify the physical form of the dosage unit. For instance, tablets may be coated with shellac, sugar or both. A syrup or elixir may contain, in addition to the active ingredient, sucrose as a sweetening agent, methyl and propylparabens as preservatives, a dye and a flavoring such as cherry or orange flavor.

Sterile liquids include suspensions, emulsions, syrups, and elixirs. The active ingredient can be dissolved or suspended in a pharmaceutically acceptable carrier, such as sterile water, sterile organic solvent, or a mixture of both sterile water and sterile organic solvent.

The active ingredient can also be dissolved in a suitable organic solvent, for example, aqueous propylene glycol. Other compositions can be made by dispersing the finely divided active ingredient in aqueous starch or sodium carboxymethyl cellulose solution or in a suitable oil.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations, which can be used orally, include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added.

All formulations for oral administration should be in dosages suitable for such administration. Particularly suitable compositions for oral administration are unit dosage forms such as tablets and capsules.

For parental administration, the compounds of the present invention or salts thereof can be combined with sterile aqueous or organic media to form injectable solutions or suspensions. Formulations for injection may be presented in unit dosage form, such as in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that each syringability exists. It must be stable under the conditions of manufacture and storage and must be preserved against any contamination. The carrier can be solvent or dispersion medium containing, for example, water, preferably in physiologically compatible buffers such as Hanks' solution, Ringer's solution, or physiological saline buffer, ethanol, polyol (e.g. glycerol, propylene glycol and liquid polyethylene glycol), propylene glycol and liquid polyethylene glycol), suitable mixtures thereof, and vegetable oils. Under ordinary conditions of storage and use, these preparations contain a preservative to prevent the growth of microorganisms.

The injectable solutions prepared in this manner can then be administered intravenously, intraperitoneally, subcutaneously, or intramuscularly, with intramuscular administration being preferred in humans.

For transmucosal administration, penetrants appropriate to the barrier to be permeated are used in the formulation. Such penetrants are generally known in the art. The active compounds can also be administered intranasally as, for example, liquid drops or spray.

For buccal administration, the compositions may take the form of tablets or lozenges Formulated in a conventional manner.

For administration by inhalation, the compounds for use according to the present invention are conveniently delivered in the form of a dry powder inhaler, or an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of pressurized aerosol the dosage unit may be determined by providing a valve to deliver a metered amount. Capsules and cartridges of gelatin for use in an inhaler or insufflator may be formulated containing a powder mix of the compound and a suitable powder base such as lactose or starch.

Pharmaceutical compositions of the present invention can be manufactured in a manner that is itself known, e.g., by means of conventional mixing, dissolving, granulating, dragee-making, levigating, emulsifying, encapsulating, entrapping or lyophilizing processes.

In making the compositions of the present invention, the active ingredient will usually be admixed with a carrier, or diluted by a carrier, or enclosed within a carrier, which may be in the form of a capsule, sachet, paper or other container. When the carrier serves as a diluent, it may be a solid, lyophilized solid or paste, semi-solid, or liquid material which acts as a vehicle, or can be in the form of tablets, pills, powders, lozenges, elixirs, suspensions, emulsions, solutions, syrups, aerosols (as a solid or in a liquid medium), or ointment, containing for example up to 10% by weight of the active compound. The compounds of the present invention are preferably formulated prior to administration.

### Binding and Cotransfection Studies

The in vitro potency of compounds in modulating PPARγ, PPARα and PPARδ receptors are determined by the procedures detailed below. DNA-dependent binding (ABCD binding) is carried out using Scintillation Proximity Assay (SPA) technology with PPAR receptors. Tritium-labeled PPARα and PPARγ agonists are used as radioligands for generating displacement curves and IC₅₀ values with compounds of the present invention. Cotransfection assays are carried out in CV-1 cells. The reporter plasmid contains an acylCoA oxidase (AOX) PPRE and TK promoter upstream of the luciferase reporter cDNA. Appropriate PPARs and RXRα are constitutively expressed using plasmids containing the CMV promoter. Since for PPARα and PPARβ, interference by endogenous PPARγ in CV-1 cells is an issue, in order to eliminate such interference, a GAL4 chimeric system is used in which the DNA binding domain of the transfected PPAR is replaced by that of GAL4, and the GAL4 response element is utilized in place of the AOX PPRE. Receptor activation by compounds of the present invention is determined relative to PPARα agonist and PPARγ agonist reference molecules to obtain percent efficacies. EC50 values are determined by computer fit to a concentration-response curve. A typical range for concentration determination is from 1nM to 10µM. For binding or cotransfection studies with receptors other than PPARs, similar assays are carried out using appropriate ligands, receptors, reporter constructs and etc. for that particular receptor. In some cases, a single high concentration of agonist (10 µM) was used.

These studies are carried out to evaluate the ability of compounds of the present invention to bind to and/or activate various nuclear transcription factors, particularly huPPARα ("hu" indicates "human"), huPPARγ and huPPARδ. These studies provide in-vitro data concerning efficacy and selectivity of compounds of the present invention. Furthermore, binding and cotransfection data for compounds of the present invention are compared with corresponding data for reference compounds that act on either huPPARα or huPPARγ. The typical range of concentration for binding is from 1nM to 10µM. The concentration of test compound required to effect 50% maximal activation of PPARα (IC₅₀α) and PPARγ (IC₅₀γ) is determined.

The compounds of the present invention, in general, have IC₅₀ or EC₅₀ in the range of about 1nM to about 1000 nM for either PPAR alpha, gamma or delta, preferably below about 50 nM for PPAR delta and below about 500 nM for PPAR gamma.

### Evaluation of Triglyceride and Cholesterol Level in HuapoAI Transgenic Mice

Five to six week old male mice, transgenic for human apoAI [C57B1/6-tgn(apoa1) 1rub, Jackson Laboratory, Bar Harbor, ME] are housed five per cage (10"x20"x8" with aspen chip bedding) with food (Purina 5001) and water available at all times. After an acclimation period of 2 weeks, animals are individually identified by ear notches, weighed and assigned to groups based on body weight. Beginning the following morning, mice are dosed daily by oral gavage for 7 days using a 20 gauges, 1½" curved disposable feeding needle. Treatments are test compounds (30 mg/kg), a positive control (fenofibrate, 100 mg/kg) or vehicle [1 % carboxymethylcellulose (w/v)/ 0.25% Tween80 (w/v); 0.2 ml/mouse]. Prior to termination on day 7, mice are weighed and dosed. Three hours after dosing, animals are anesthetized by inhalation of isoflurane (2-4%) and blood obtained via cardiac puncture (0.7-1.0 ml). Whole blood is transferred to serum separator tubes (Vacutainer SST), chilled on ice and permitted to clot. Serum is obtained after centrifugation at 4°C and frozen until analysis for triglycerides, total cholesterol, compound levels and serum lipoprotein profile by fast protein liquid chromatography (FPLC) coupled to an inline detection system. After sacrifice by cervical dislocation, the liver, heart and epididymal fat pads are excised and weighed.

The animals dosed with vehicle have average triglycerides values of about 60 to 80 mg/dl, which are reduced by the positive control fenofibrate (33-58 mg/dl with a mean reduction of 37%). The animals dosed with vehicle have average total serum cholesterol values of about 140 to 180 mg/dl, which are increased by fenofibrate (about 190 to 280 mg/dl with a mean elevation of 41%). When subject to FPLC analysis, pooled sera from vehicle-treated hu apoAI transgenic mice have a high-density lipoprotein cholesterol (HDLc) peak area, which ranges from 47v-sec to 62v-sec. Fenofibrate increases the amount of HDLc (68-96v-sec with a mean percent increase of 48%). Test compounds evaluated in terms of percent increase in the area under the curve. Representative compounds of the present invention are tested using the above methods or substantially similar methods.

### Evaluation of Glucose Levels in db/db Mice

Five week old male diabetic (db/db) mice [C57B1Ks/j-m +/+ Lepr(db), Jackson Laboratory, Bar Harbor, ME] or lean littermates (db+) are housed 6 per cage (10"x20"x8" with aspen chip bedding) with food (Purina 5015) and water available at all times. After an acclimation period of 2 weeks, animals are individually identified by ear notches, weighed and bled via the tail vein for determination of initial glucose levels. Blood is collected (100 µl) from unfasted animals by wrapping each mouse in a towel, cutting the tip of the tail with a scalpel, and milking blood from the tail into a heparinized capillary tube balanced on the edge of the bench. Sample is discharged into a heparinized microtainer with gel separator (VWR) and retained on ice. Plasma is obtained after centrifugation at 4°C and glucose is measured immediately. Remaining plasma is frozen until the completion of the experiment, and glucose and triglycerides are assayed in all samples. Animals are grouped based on initial glucose levels and body weights. Beginning the following morning, mice are dosed daily by oral gavage for 7 days using a 20 gauge, 1½" curved disposable feeding needle. Treatments are test compounds (30 mg/kg), a positive control agent (30 mg/kg) or vehicle [1% carboxymethylcellulose (w/v)/0.25% Tween80 (w/v); 0.3 ml/mouse]. On day 7, mice are weighed and bled (tail vein) for about 3 hours after dosing. Twenty-four hours after the 7^{th} dose (i.e., day 8), animals are bled again (tail vein). Samples obtained from conscious animals on days 0, 7 and 8 are assayed for glucose. After 24 hour bleed, animals are weighed and dosed for the final time. Three hours after dosing on day 8, animals are anesthetized by inhalation of isoflurane, and blood obtained is via cardiac puncture (0.5-0.7 ml). Whole blood is transferred to serum separator tubes, chilled on ice and permitted to clot. Serum is obtained after centrifugation at 4°C and frozen until analysis for compound levels. After sacrifice by cervical dislocation, the liver, heart and epididymal fat pads are excised and weighed.

The animals dosed with vehicle have average triglycerides values of about 170 to 230 mg/dl, which are reduced by the positive PPARγ control (about 70 to 120 mg/dl with a mean reduction of 50%). Male db/db mice are hyperglycemic (average glucose of about 680 to 730 mg/dl on the 7^{th} day of treatment), while lean animals have average glucose levels between about 190 and 230 mg/dl. Treatment with the positive control agent reduces glucose significantly (about 350 to 550 mg/dl with a mean decrease towards normalization of 56%).

Glucose is measured colorimetrically by using commercially purchased reagents (Sigma #315-500). According to the manufacturers, the procedures are modified from published work (McGowan et al. Clin Chem, 20:470-5 (1974) and Keston, A. Specific colorimetric enzymatic analytical reagents for glucose. Abstract of papers 129th Meeting ACS, 31C (1956).); and depend on the release of a mole of hydrogen peroxide for each mole of analyte coupled with a color reaction first described by Trinder (Trinder, P. Ann Clin Biochem, 6:24 (1969)). The absorbance of the dye produced is linearly related to the analyte in the sample. The assays are further modified for use in a 96 well format. Standards (Sigma #339-11, Sigma #16-11, and Sigma #CC0534 for glucose, triglycerides and total cholesterol, respectively), quality control plasma (Sigma # A2034), and samples (2 or 5 µl/well) are measured in duplicate using 200 µl of reagent. An additional aliquot of sample, pipetted to a third well and diluted in 200 µl water, provided a blank for each specimen. Plates are incubated at room temperature (18, 15, and 10 minutes for glucose, triglycerides and total cholesterol, respectively) on a plate shaker and absorbance read at 500 nm (glucose and total cholesterol) or 540 nm (triglycerides) on a plate reader. Sample absorbance is compared to a standard curve (100-800, 10-500, and 100-400 mg/dl for glucose, triglycerides and total cholesterol, respectively). Values for the quality control sample are consistently within the expected range and the coefficient of variation for samples is below 10%. All samples from an experiment are assayed at the same time to minimize inter-assay variability.

Serum lipoproteins are separated and cholesterol is quantitated with an inline detection system. Sample is applied to a Superose® 6 HR 10/30-size exclusion column (Amersham Pharmacia Biotech) and eluted with phosphate buffered saline-EDTA at 0.5 ml/min. Cholesterol reagent (Roche Diagnostics Chol/HP 704036) at 0.16 ml/min is mixed with the column effluent through a T-connection, and the mixture is passed through a 15 m x 0.5 mm id knitted tubing reactor immersed in a 37°C water bath. The colored product produced in the presence of cholesterol is monitored in the flow stream at 505 nm, and the analog voltage from the monitor is converted to a digital signal for collection and analysis. The change in voltage corresponding to change in cholesterol concentration is plotted against time, and the area under the curve corresponding to the elution of VLDL, LDL and HDL is calculated (Perkin Elmer Turbochrome software).

The compounds of the present invention can be prepared according to the procedures of the following schemes and examples, which may further illustrate details for the preparation of the compounds of the present invention. The compounds illustrated in the schemes and examples are, however, not to be construed as forming the only genus that is considered as the present invention.

### General Reaction Scheme

The compounds of the present invention, in general, may be prepared according to the Reaction Schemes shown below.

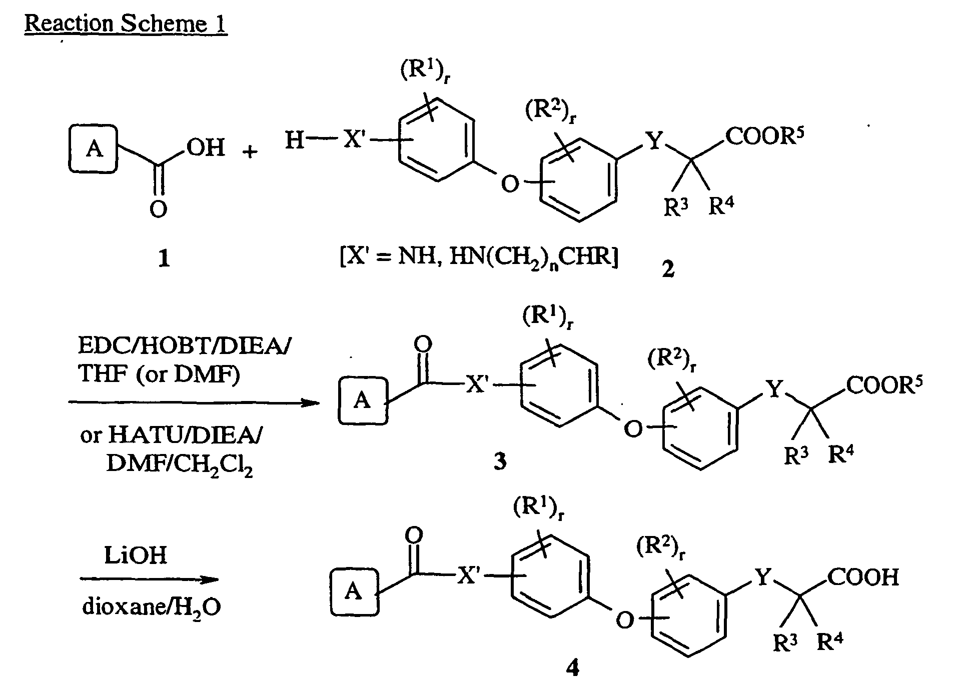

As shown in Reaction Scheme 1, treatment of carboxylic acid **1** and amine **2** with EDC and HOBT or HATU in the presence of DIEA provides ester **3.** Hydrolysis of ester under LiOH, dioxane and H₂O provides final acid **4.**

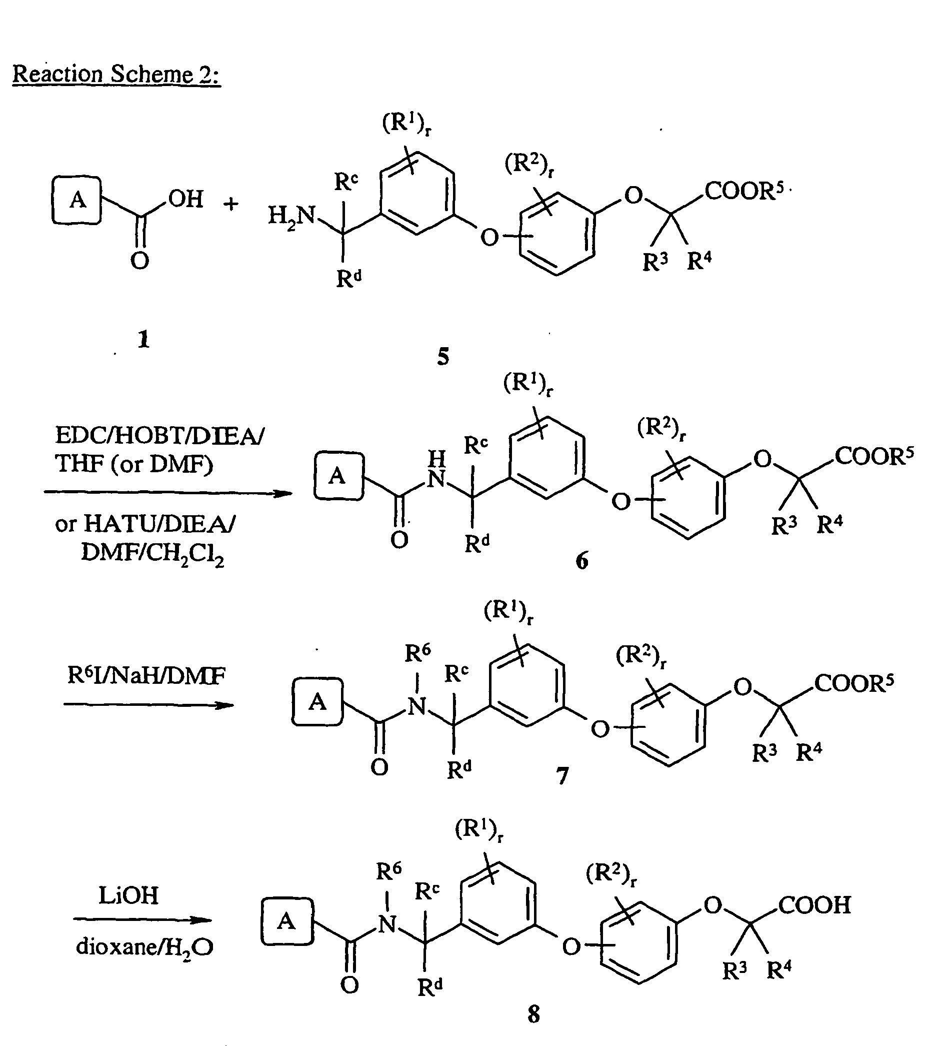

Similar to Reaction Scheme 1, treatment of carboxylic acid **1** and amine **5** with EDC and HOBT or HATU in the presence of DIEA provides ester **6** as shown in Reaction Scheme 2. Alkylation of **6** gives compound **7,** which then undergoes a hydrolysis to provide final acid **8.**

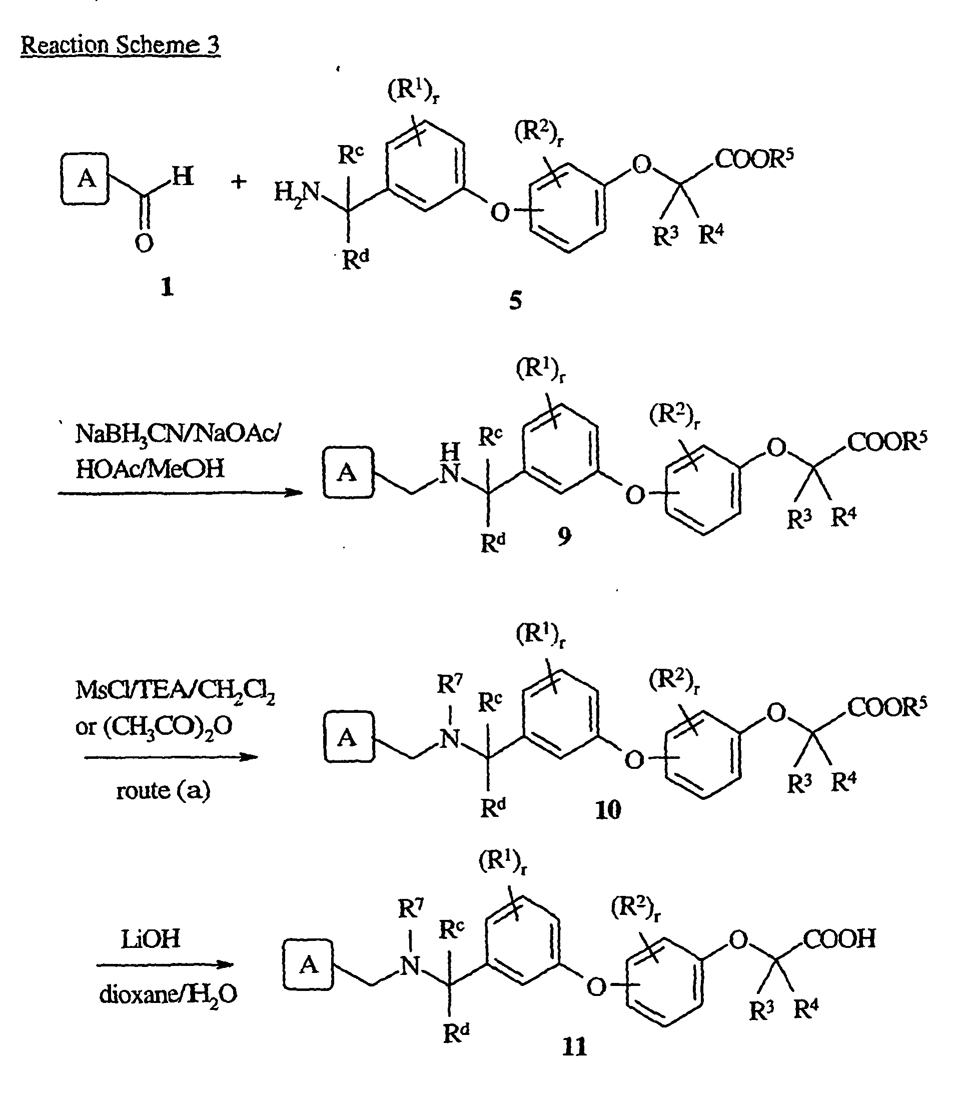

### Alternative route (b)

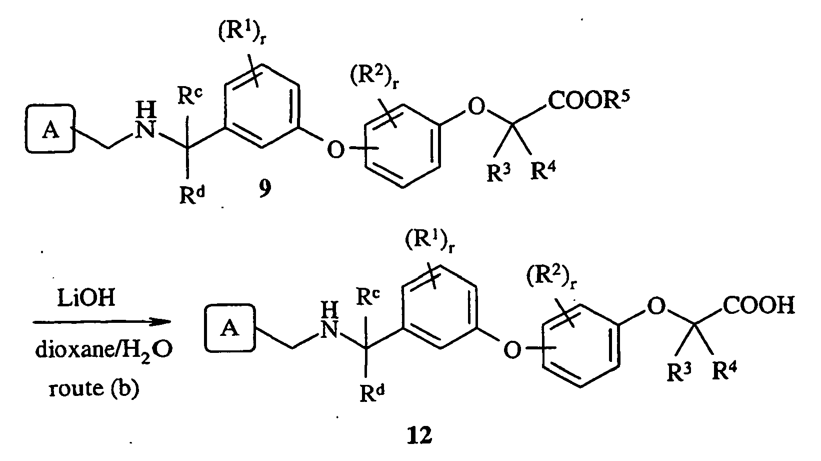

As shown in Reaction Scheme 3, treatment of aldehyde **1** and amine **5** with NaBH₃CN in the presence of NaOAc and HOAc provides amine **9.** Mesylation or acylation of amine **9** gives sulfonamide or amide **10.** Hydrolysis of ester under LiOH, dioxane and H₂O provides final acid **11.** Alternatively as shown in route (b), amine **9** undergoes a hydrolysis to provide final acid **12.**

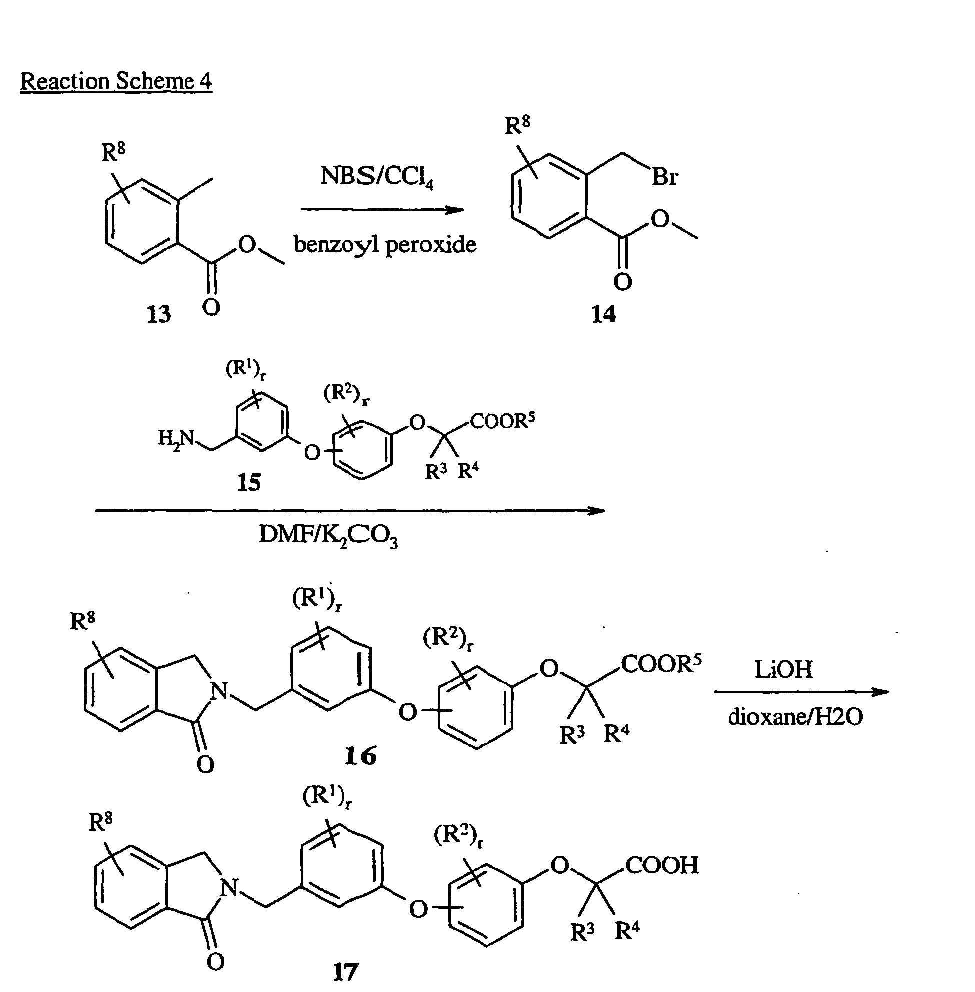

As shown in Reaction Scheme 4, bromination of **13** gives bromide **14.** Treatment of bromide **14** with the amine **15** under the basic condition provides lactam **16,** which then undergoes a hydrolysis to provide final acid **17.**

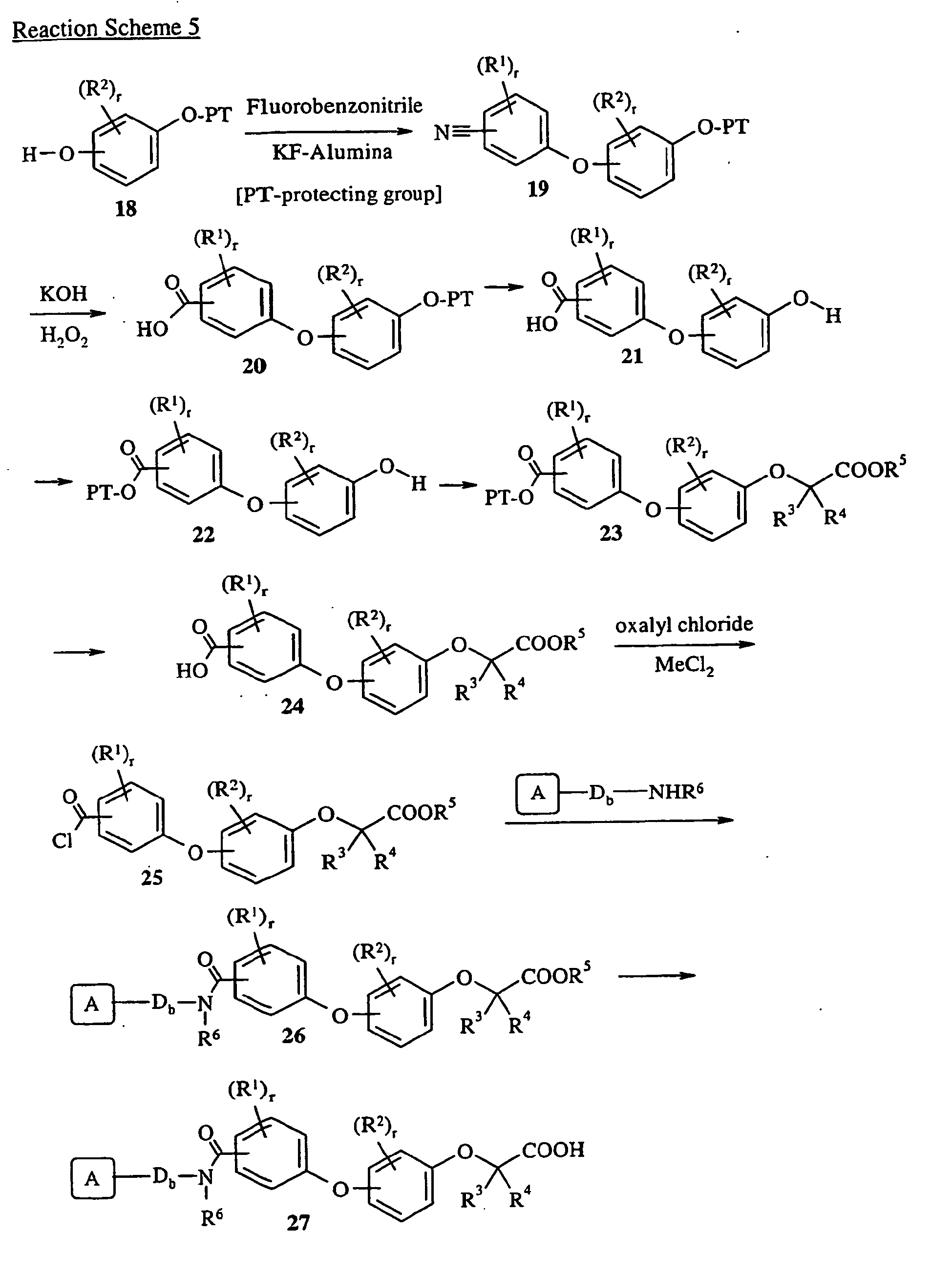

As shown in Reaction Scheme 5, a protected phenol **18** is reacted with fluorobenzonitrile in the presence of KF-alumina in an appropriate solvent to give the protected phenoxybenzonitrile **19.** The nitrile is hydrolyzed in a suitable condition, such as in the presence of aqueous base and hydrogen peroxide to produce the acid **20.** The protecting group (PT), such as benzyl group, is removed to give **21.** The acid is then protected with a suitable protecting group, such as benzyl group to give **22,** which is then reacted with a suitable ester-protected haloalkyl, such as ethylbromoacetate to give compound **23.** The protecting group is removed, and the acid **24** is reacted with a chlorinating agent, such as oxalyl chloride to produce compound **25.** The acid chloride compound is then reacted with an amine, such as benzylamine or cyclohexylamine in a suitable solvent, such as dichloromethane with a suitable base, such as triethylamine to give the amide ester compound **26.** The ester is cleaved using aqueous base to give the final acid compound **27.**

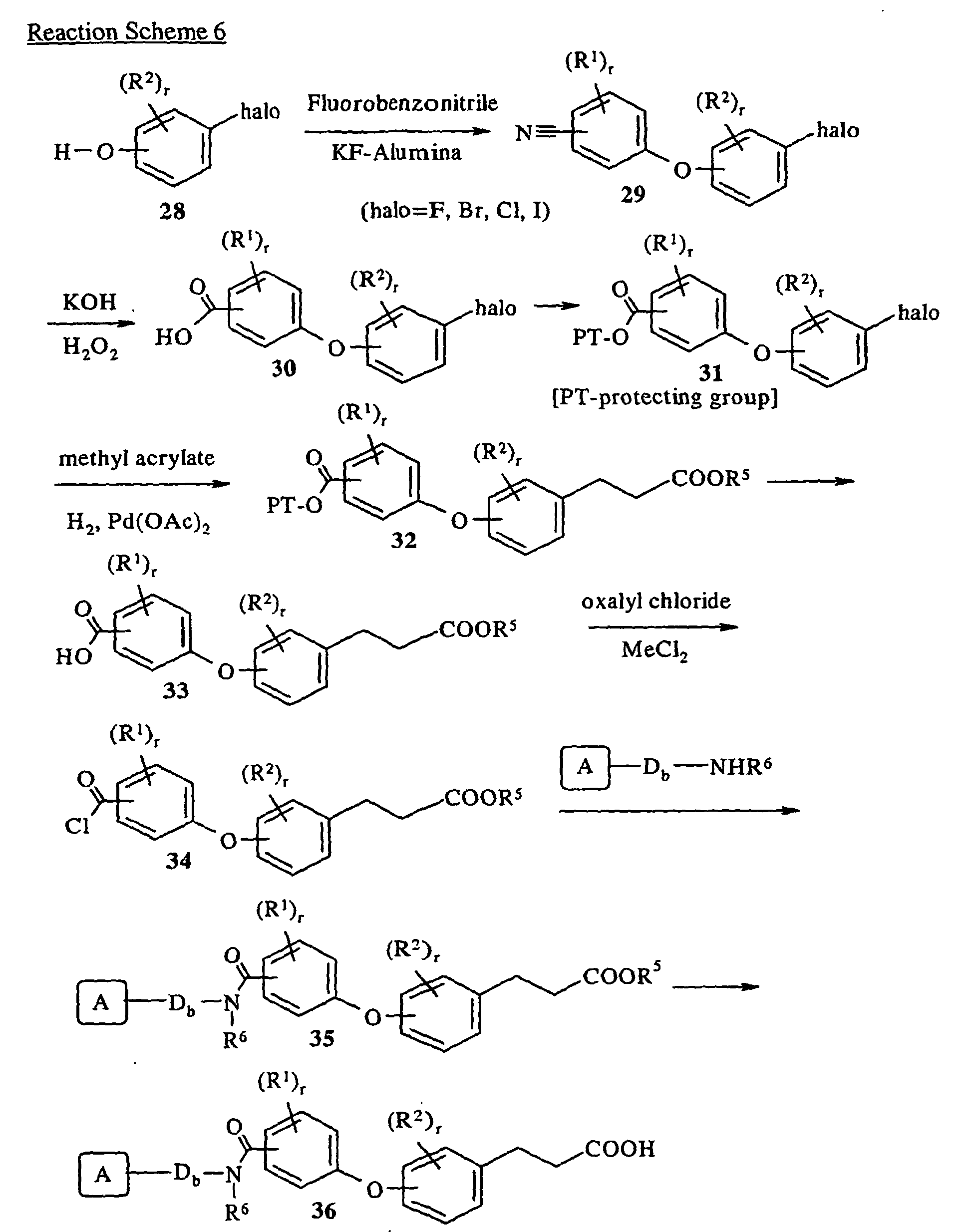

As shown in Reaction Scheme 6, a halophenol **28** is reacted with fluorobenzonitrile in the presence of KF-alumina in an appropriate solvent to give the halophenoxybenzonitrile **29.** The nitrile is hydrolyzed in a suitable condition, such as in aqueous base and hydrogen peroxide to produce the acid **30.** The acid is protected with a suitable protecting group, such as benzyl group to give **31,** which is then reacted in a palladium catalyzed Heck reaction with methyl acrylate to give the intermediate **32.** Compound **32** is reduced under standard hydrogenation condition to give the acid compound **33.** The acid is reacted with a chlorinating agent, such as oxalyl chloride to produce compound **34.** The acid chloride compound is reacted with an amine, such as benzylamine or cyclohexylamine in a suitable solvent, such as dichloromethane with a suitable base, such as triethylamine to give the amide ester **35.** The ester is cleaved using aqueous base to give the final acid product **36.**

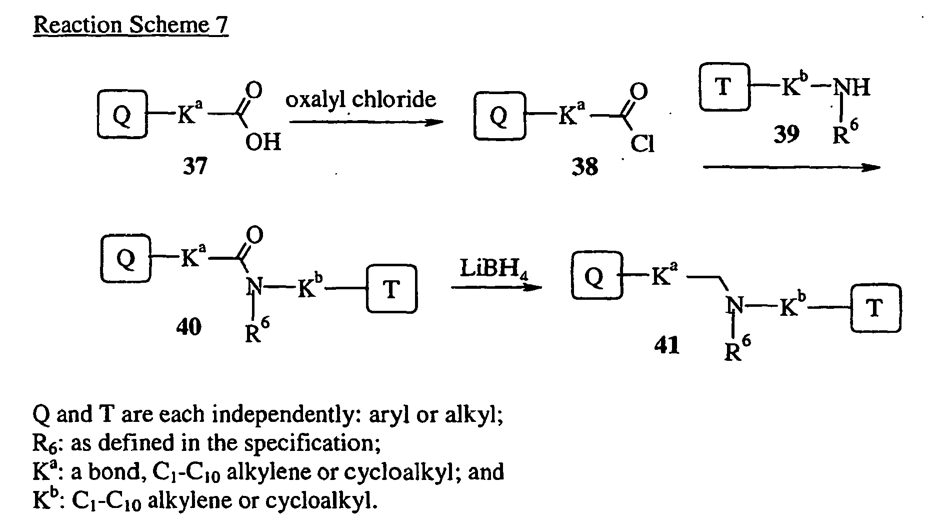

As shown in Reaction Scheme 7, the acid **37** is dissolved in a suitable solvent, such as dichloromethane, and treated with a chlorinating agent such as oxalyl chloride. The acid chloride **38** is then added to the amine **39** in a suitable solvent, such as dichloromethane, with a base such as triethylamine. The resulting amide **40** is then reduced using a reducing agent, such as lithium borohydride with trimethylsilyl chloride in a suitable solvent, such a tetrahydrofuran to give the final amine compound **41.**

In the Schemes, Procedures and Examples below, various reagent symbols and abbreviations have the following meanings.
- ACN: Acetonitrile
- BINAP: 2,2'-Bis(diphenylphosphino)-1,1'-binaphthyl
- Boc: t-butoxycarbonyl
- CBZ: benzyloxycarbonyl
- DCM: dichloromethane
- DEAD: diethyl azodicarboxylate
- DIAD: diisopropyl azodicarboxylate
- DIPEA: diisopropylethylamine
- DMAP: 4-dimethylamino pyridine
- DMF: N,N-dimethylformamide
- DMSO: dimethylsulfoxide
- eq (equiv): equivalent(s)
- ESI-MS: electron spray ion-mass spectroscopy
- Et: ethyl
- EtOAc: ethyl acetate
- h: hours
- HOAc: acetic acid
- HPLC: high performance liquid chromatography
- HRMS: high resolution mass
- LRMS: low resolution mass
- LAH: lithium aluminum hydride
- Me: methyl
- Ms: methanesulfonyl
- NBS: N-bromosuccinimide
- Pd₂(dba)₃: tris(dibenzylideneacetone) dipalladium(0)
- Ph: phenyl
- Pr: propyl
- rt (r.t.): room temperature
- TBAI: tetrabutylammonium iodide
- TBS: tertbutyldimethylsilyl
- TFA: trifluoroacetic acid
- TEA: triethylamine
- THF: tetrahydrofuran
- TLC: thin-layer chromatography

### Preparation of Intermediates

### Intermediate 1

### 5-Fluoro-3-methyl-1H-indole-2-carboxylic acid

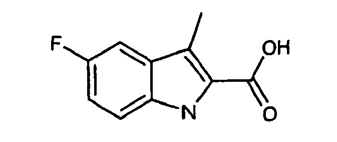

### Step A

### 5-Fluoro-3-methyl-1H-indole-2-carboxylic acid ethyl ester

4-Fluoroaniline (2.5 g, 22.5 mmol) is added to a solution of hydrochloric acid (5.5 mL in 9.6 mL water) and cooled to -5 °C. A solution of sodium nitrite is added dropwise (1.7 g, 24.7 mmol in 2 mL water). After the addition, reaction is stirred for 15 minutes at 0 °C, then pH is adjusted to 3-4 with sodium acetate (2.0 g, 24.3 mmol). In a separate flask, a solution of potassium hydroxide (1.4 g, 24.7 mmol in 2 mL water) is added to a solution of ethyl-2-ethylacetoacetate (3.9 g, 24.7 mmol) in ethanol (20 mL) at 0 °C followed by 28 g of ice. The diazonium solution is added immediately to the alkaline solution. The pH is adjusted to 5-6 with sodium acetate and stirred for 3 hours at 0 °C. Additional water is added and extracted 3 times with ethyl acetate, washed with brine, dried over sodium sulfate and concentrated under reduced pressure giving an oily residue. Oily residue is dissolved in a few mL ethanol and added dropwise at 78 °C to a solution of HCl in dioxane (50 mL, 4N) and HCl in ethanol (30 mL, 1N). After the addition, reaction is heated at 78 °C for approximately 2-3 hours. The reaction is cooled, then solution is concentrated under reduced pressure, water added, and extracted 3 times with dichloromethane, washed with brine, dried over sodium sulfate, and concentrated under reduced pressure. The resulting slurry is triturated with a minimum of dichloromethane and filtered providing pure title compound (1.38 g). Recrystallization of the mother liquor material (hexane) provided additional pure product (0.68 g, 41% overall). GC/MS: M·⁺ 221; ¹HNMR (400 MHz, CDCl₃).

### Step B

### 5-Fluoro-3-methyl-1H-indole-2-carboxylic acid

Compound of Step A (1.38 g, 6.24 mmol) is dissolved in dioxane (130 mL) and lithium hydroxide hydrate (3.9 g, 93.5 mmol), dissolved in water (65 mL), is added. Mixture is stirred at rt overnight under nitrogen, then acidified with 5 N HCl. Water is added and extracted with EtOAc, washed with brine, dried over sodium sulfate and concentrated under reduced pressure to give the title compound (0.76 g, 63%). Mass (ES): 192 (M-H⁺); ¹H NMR (400 MHz, CDCl₃).

### Intermediate 2

### 5-Fluoro-1,3-dimethyl-1H-indole-2-carboxylic acid

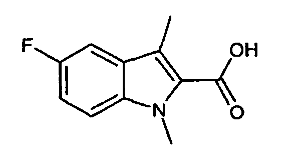

### Step A

### 5-Fluoro-1,3-dimethyl-1H-indole-2-carboxylic acid ethyl ester

To a suspension of 60% sodium hydride (162 mg, 4.1 mmol) in dry DMF (10 mL), cooled to 0 °C under nitrogen, is added dropwise the compound from Step A of Example 1 (600 mg, 2.7 mmol), dissolved in DMF (2 mL). The mixture is stirred at 0 °C for 45 minutes, then iodomethane (0.26 mL, 4.1 mmol) is added dropwise. Bath is removed after 1 hour and stirring is continued overnight. Reaction is quenched with ice water and a precipitate formed, which is filtered and dried providing title compound (600. mg, 94%) that is utilized without further purification. GC/MS: M⁺ 235; ¹HNMR (400 MHz, CDCl₃).

### Step B

### 5-Fluoro-1,3-dimethyl-1H-indole-2-carboxylic acid

The title compound is prepared according to Step B of Intermediate 1, utilizing 5-fluoro-1,3-dimethyl-1H-indole-2-carboxylic acid ethyl ester. Mass (ES⁻): 206 (M-H⁺); ¹H NMR (400 MHz, CDCl₃).

### Intermediate 3

### 5-Chloro-3-methyl-1H-indole-2-carboxylic acid

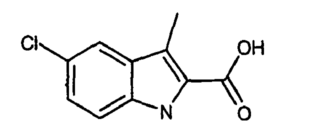

The title compound is prepared according to Intermediate 1 utilizing 4-chloroaniline and ethyl-2-ethylacetoacetate. Mass (ES⁻): 208 (M-H⁺); ¹H NMR (400 MHz, CDCl₃).

### Intermediate 4

### 5-Chloro-1,3-dimethyl-1H-indole-2-carboxylic acid

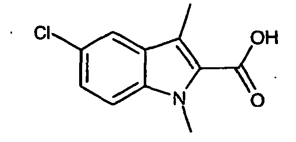

The title compound is prepared according to Intermediate 2 utilizing 5-chloro-3-methyl-1H-indole-2-carboxylic acid ethyl ester (Intermediate 3, Step A) and iodomethane. Mass (ES⁻): 222 (M-H⁺); ¹H NMR (400 MHz, CDCl₃).

### Intermediate 5

### 3-Methyl-5-trifluoromethyl-1H-indole-2-carboxylic acid

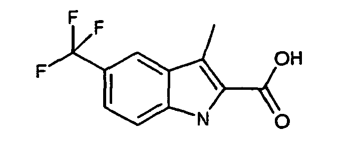

The title compound is prepared according to Intermediate 1 utilizing 4-trifluormethylaniline and ethyl-2-ethylacetoacetate. Mass (ES⁻): 242 (M-H⁺); ¹H NMR (400 MHz, CDCl₃).

### Intermediate 6

### 1,3-Dimethyl-5-trifluoromethyl-1H-indole-2-carboxylic acid

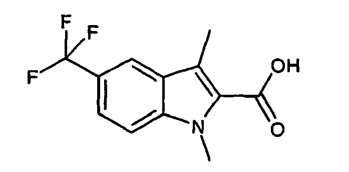

The title compound is prepared according to Intermediate 2 utilizing 1,3-dimethyl-5-trifluoromethyl-1H-indole-2-carboxylic acid ethyl ester (Intermediate 5, Step A) and iodomethane. Mass (ES⁻): 256 (M-H⁺); ¹H NMR (400 MHz, CDCl₃).

### Intermediate 7

### 5-Chloro-3-propyl-1H-indole-2-carboxylic acid

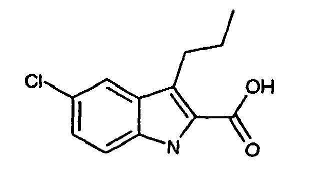

The title compound is prepared according to Intermediate 1 utilizing 4-chloroaniline and ethyl-2-n-butylacetoacetate. Mass (ES⁻): 236 (M-H⁺); ¹H NMR (400 MHz, CDCl₃).

### Intermediate 8

### 5-Chloro-1-methyl-3-propyl-1H-indole-2-carboxylic acid

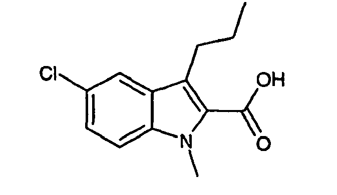

The title compound is prepared according to Intermediate 2 utilizing 5-chloro-3-propyl-1H-indole-2-carboxylic acid ethyl ester (Intermediate 7, Step A) and iodomethane. Mass (ES⁻): 250 (M-H⁺); ¹H NMR (400 MHz, CDCl₃).

### Intermediate 9

### 1-Ethyl-5-fluoro-3-methyl-1H-indole-2-carboxylic acid

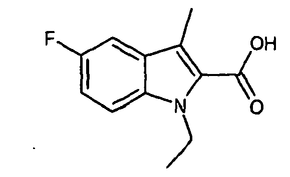

The title compound is prepared according to Intermediate 2 utilizing 5-fluoro-3-methyl-1H-indole-2-carboxylic acid ethyl ester (Intermediate 1, Step A) and iodoethane. Mass (ES⁻): 250 (M-H⁺); ¹H NMR (400 MHz, CDCl₃).

### Intermediate 10

### 2-Methyl-4-trifluoromethyl-benzoic acid

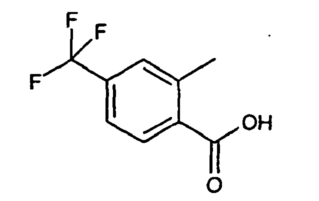

The compound of *N,N,N,N*,-tetramethylethylenediamine (19.8 mL, 131.9 mmol) is added to dry THF (120 mL) under nitrogen and cooled to -78 °C with a dry ice acetone bath. Sec-butyl lithium (94 mL, 131.9 mmol, 1.4 M in cyclohexane) is added dropwise keeping the temperature between -78 °C to -70 °C with overhead stirring. Suspension is stirred 15 minutes, then 4-trifluoromethyl-benzoic acid (11.4 g, 59.8 mmol) dissolved in 70 mL THF is added dropwise within the above temperature range and allowed to warm to -50 °C to -40 °C. Stirring is continued for 2 hours within that temperature range. Mixture is cooled to -78 °C and iodomethane (14.9 mL, 239.2 mmol) is added in 2-3 minutes. Bath is removed after 10 minutes and the reaction is stirred overnight. Reaction is quenched with water, extracted with ether, and the aqueous layer acidified with 5N HCl. Acidified solution is extracted with ether, washed with brine, dried over sodium sulfate and concentrated under reduced pressure. Purification by flash chromatography, eluting with 10 % EtOAc in hexane then 25 % EtOAc in hexane provides the title compound (2.4 g, 20 %). Mass (ES⁻): 203 ¹HNMR (400 MHz, CDCl₃).

### Intermediate 11

### 2-Bromo-4-trifluoromethyl-benzoic acid

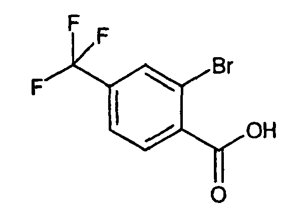

Anhydrous copper (II) bromide (1.34 g, 6.0 mmol) and *t*-butyl nitrite (90%) (0.86 g, 7.5 mmol) are added to dry acetonitrile (20 mL) under nitrogen and the reaction is heated to 65 °C. 2-Amino-4-trifluoromethyl-benzoic acid (1.03 g, 5.0 mmol) is added in 3 portions and stirred for 15 minutes. Reaction is cooled and 5 N HCl is added, extracted with ether, washed with brine, dried over sodium sulfate and concentrated under reduced pressure. Purification by flash chromatography, eluting with 15 % EtOAc in hexane then 25 % EtOAc in hexane provides the title compound (0.80 g, 60 %). Mass (ES⁻): 267; ¹HNMR (400 MHz, CDCl₃).

### Intermediate 12

### 2-Chloro-4-trifluoromethyl-benzoic acid

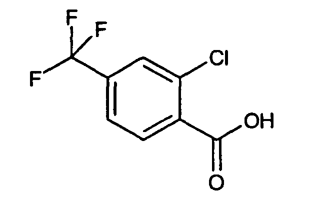

The title compound is prepared according to Intermediate 11 utilizing 2-amino-4-trifluoromethyl-benzoic acid and copper (II) chloride. Mass (ES⁻): 223 (M-H⁺); ¹H NMR (400 MHz, CDCl₃).

### Intermediate 13

### 2-Methyl-4-trifluoromethoxybenzoic acid

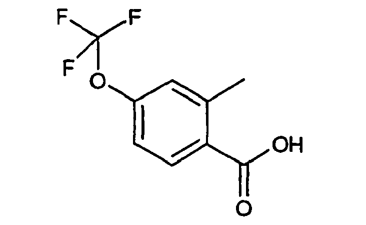

The title compound is prepared according to Intermediate 10 utilizing 4-trifluoromethoxy-benzoic acid. Mass (ES⁻): 219 (M-H⁺); ¹H NMR (400 MHz, CDCl₃).

### Intermediate 14

### 5-Chloro-benzo[b]thiophene-2-carboxylic acid

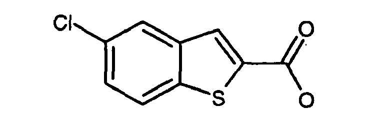

The title compound is prepared according to Intermediate 1, Step B, utilizing 5-chloro-benzo[b]thiophene-2-carboxylic acid methyl ester. Mass (ES⁻): 211 (M-H⁺); ¹H NMR (400 MHz, CDCl₃).

### Intermediate 15

### 7-Bromo-1H-indole-2-carboxylic acid

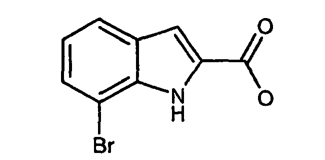

The title compound is prepared according to Intermediate 1, Step B, utilizing 7-bromo-1H-indole-2-carboxylic acid ethyl ester. Mass (ES⁻): 238 (M-H⁺); ¹H NMR (400 MHz, CDCl₃).

### Intermediate 16

### 3-{4-[3-(1-Amino-ethyl)-5-methyl-phenoxy]-2-ethyl-phenyl}-propionic acid ethyl ester

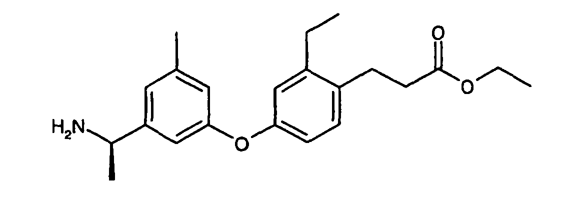

### Step A

### 3-[4-(3-Bromo-5-methyl-phenoxy)-2-ethyl-phenyl]-propionic acid ethyl ester

The compounds of 3,5-dibromotoluene (13.5 g, 54.0 mmol), 3-(2-ethyl-4-hydroxyphenyl)-propionic acid ethyl ester (3.0 g, 13.5 mmol) (Intermediate 59), cesium carbonate (5.27 g, 16.2 mmol), copper (I) chloride (0.67 g, 6.7 mmol), and 2,2,6,6-tetramethyl-3,5-heptanedione (0.62 g, 3.4 mmol) are added to 1-methyl-2-pyrrolidinone (35 ml) and heated overnight at 120 °C under nitrogen. After cooling, HCl acid (50 mL, 1N) is added to quench the reaction. Additional water is added and extracted 3 times with ethyl ether, washed with brine, dried over sodium sulfate and concentrated under reduced pressure. Purification by flash chromatography, eluting with 5 % EtOAc in hexane then 10 % EtOAc in hexane provides the title compound (2.7 g, 51 %). MS(ES⁺): 410 (M+NH₄⁺); ¹HNMR (400 MHz, CDCl₃).

### Step B

### 3-[4-(3-Acetyl-5-methyl-phenoxy)-2-ethyl-phenyl]-propionic acid ethyl ester

The compound from Step A (2.7 g, 6.9 mmol), is dissolved in toluene (85 mL), and tributyl(1-ethoxyvinyl)tin (2.56 mL, 7.6 mmol) and dichlorobis(triphenylphosphine)palladium(II) (0.48 g, 0.69 mmol) are added. The solution is degassed and heated at 100°C under nitrogen for 5 hours. After the reaction is cool, HCl acid (40 mL, 1N) is added and stirred for 1 hour. Reaction mixture is filtered through Celite and washed with EtOAc. Organic layer is separated, washed with brine, dried over sodium sulfate and concentrated under reduced pressure. Oil is dissolved in ethyl ether (300 mL) and an aqueous solution of KF (200 mL, 0.1N) is added. Mixture is stirred overnight. Precipitate is removed by filtration, washed with ethyl ether, and the organic layer removed. The water layer is extracted twice with ethyl ether, then the ethers layers are combined, washed with brine, dried over sodium sulfate and concentrated under reduced pressure. Purification by flash chromatography, eluting with 5 % EtOAc in hexane then 10 % EtOAc in hexane provides the title compound (1.3 g, 52 %). MS(ES⁺): 372 (M+NH₄⁺); ¹HNMR (400MHz, CDCl₃).

### Step C

### 3-{2-Ethyl-4-[3-(1-hydroxy-ethyl)-5-methyl-phenoxy]-phenyl}-propionic acid ethyl ester

(R)-2-methyl-CBS-oxazaborolidine (0.34 mL, 0.36 mmol, 1N in toluene) is dissolved in dry toluene (3.5 mL) and borane-*N,N*-diethylaniline complex (0.64 mL, 3.6 mmol) is added. Flask is immersed in a water bath and the compound from Step B (1.3 g, 3.6 mmol) is dissolved in toluene (9.0 mL) and is added dropwise to the solution keeping the temperature between 19 °C and 23 °C. Reaction is stirred 45 minutes under nitrogen. Reaction is quenched with methanol (1.2 mL), followed by 1N HCl (2.2 mL) and stirred for 15 minutes. Aqueous layer is separated, extracted twice with ethyl acetate. Organic layers are combined, washed with brine, dried over sodium sulfate and concentrated under reduced pressure. Purification by flash chromatography, eluting with 10 % EtOAc in hexane then 20 % EtOAc in hexane provides the title compound (1.1 g, 85 %). MS(ES⁺): 374 (M+NH₄⁺); ¹HNMR (400 MHz, CDCl₃).

### Step D

### 3-{4-[3-(1-Azido-ethyl)-5-methyl-phenoxy]-2-ethyl-phenyl)-propionic acid ethyl ester

Compound from Step C (1.1 g, 3.1 mmol) and diphenylphosphoryl azide (0.81 mL, 3.8 mmol) are dissolved in toluene (10.0 mL), cooled to 0°C, and DBU is added dropwise. Reaction is stirred 2 hours under nitrogen at 0°C, then at rt overnight. Azidotrimethylsilane (0.41 mL, 3.1 mmol) and tetrabutylammonium fluoride (1M in THF, 3.1 mL, 3.1 mmol) are added to the solution and heated at 40 °C for 8 hours. Solvent is removed under reduced pressure. Purification by flash chromatography, eluting with 10 % EtOAc in hexane then 15 % EtOAc in hexane provides the title compound (0.93 g, 78 %). MS(ES⁺): 399 (M+NH₄⁺); ¹HNMR (400 MHz, CDCl₃).

### Step E

### 3-{4-[3-(1-Amino-ethyl)-5-methyl-phenoxy]-2-ethyl-phenyl}-propionic acid ethyl ester

Compound from Step D (0.93 g, 2.3 mmol) and triphenylphosphine (0.96 g, 3.8 mmol) are stirred overnight in THF (20 mL) and water (2 mL). Solvent is removed under reduced pressure with bath at rt, extracted into ethyl acetate, washed with brine, dried over sodium sulfate and concentrated under reduced pressure. Purification by SCX column, eluting with 10 % ammonia (2.0 M in methanol) in dichloromethane provides the title compound (0.93 g, 78 %). MS(ES⁺): 399 (M+NH₄⁺); ¹HNMR (400 MHz, CDCl₃).

### Intermediate 17

### 2-[4-(3-Aminomethyl-phenoxy)-2-methyl-phenoxy]-2-methyl-propionic acid ethyl ester

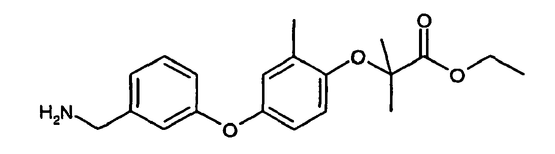

### Step A

### 2-[4-(3-Cyano-phenoxy)-2-methyl-phenoxy]-2-methyl-propionic acid ethyl ester

The compounds of 2-(4-hydroxy-2-methyl-phenoxy)-2-methyl-propionic acid ethyl ester (4.5 g, 18.9 mmol) (WO 20030721) 3-cyanophenylboronic acid (5.5 g, 37.8 mmol), dried copper (II) acetate (6.8 g, 37.8 mmol), and dried molecular sieve (11.3 g) are added to dichloromethane (225 ml). Pyridine (15.3 mL, 189.0 mmol) is added dropwise at rt. Reaction is stirred overnight with a drying tube in place. Reaction mixture is filtered through Celite and washed with dichloromethane. Filtrate is concentrated under reduced pressure. Purification by flash chromatography, eluting with 10 % EtOAc in hexane then 15 % EtOAc in hexane provides the title compound (3.0 g, 47 %). MS(ES⁺): 340 (M+H⁺); ¹HNMR (400 MHz, CDCl₃).

### Step B

### 2-[4-(3-Aminomethyl-phenoxy)-2-methyl-phenoxy]-2-methyl-propionic acid ethyl ester

Compound from Step A (3.0 g, 8.9 mmol) is reacted overnight in a Parr shaker with 5 % Pd/C (0.3 g) in glacial acetic acid (422 mL) at rt with 40 psi hydrogen. Reaction mixture is filtered, most of acetic acid removed under reduced pressure with rt bath. Ethyl acetate is added to oily residue. Solution is washed with diluted sodium bicarbonate, washed with brine, dried over sodium sulfate and concentrated under reduced pressure providing the title compound (2.8 g, 92 %) that is utilized without purification. MS(ES⁺): 343 (M+H⁺); ¹HNMR (400 MHz, CDCl₃).

### Intermediate 18

### 2-[4-(4-Aminomethyl-phenoxy)-2-methyl-phenoxy]-2-methyl-propionic acid ethyl ester

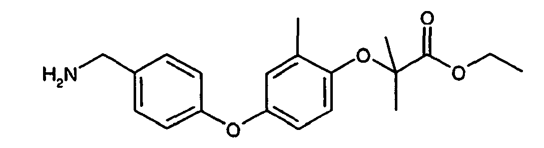

The title compound is prepared according to Intermediate 17 utilizing 4-cyanophenylboronic acid and 2-(4-hydroxy-2-methyl-phenoxy)-2-methyl-propionic acid ethyl ester (WO 2003072102). Mass (ES⁺): 366 (M+Na⁺); ¹H NMR (400 MHz, CDCl₃).

### Intermediate 19

### 2-[4-(3-Aminomethyl-4-methyl-phenoxy)-2-methyl-phenoxy]-2-methyl-propionic acid ethyl ester

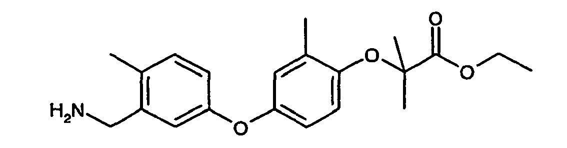

2-[4-(3-Cyano-4-methyl-phenoxy)-2-methyl-phenoxy]-2-methyl-propionic acid ethyl ester (240. mg, 0.68 mmol) (Intermediate 51) is reacted overnight in a Parr shaker with Raney nickel (0.2 g) in ammonia (2N solution in methyl alcohol, 50 mL) at 40°C with 60 psi hydrogen. Reaction mixture is filtered and concentrated under reduced pressure with rt bath providing the title compound (245 g, quantitative) that is utilized without purification. MS(ES⁺): 358 (M+H⁺); ¹HNMR (400 MHz, CDCl₃).

### Intermediate 20

### 2-[4-(3-Aminomethyl-5-methyl-phenoxy)-2-methyl-phenoxy]-2-methyl-propionic acid ethyl ester

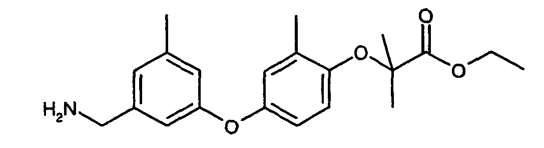

The title compound is prepared according to Intermediate 19 utilizing 2-[4-(3-cyano-5-methyl-phenoxy)-2-methyl-phenoxy]-2-methyl-propionic acid ethyl ester (Intermediate 55). Mass (ES⁺): 358 (M+H⁺); ¹H NMR (400 MHz, CDCl₃).

### Intermediate 21

### 2-[4-(5-Aminomethyl-2-methyl-phenoxy)-2-methyl-phenoxy]-2-methyl-propionic acid ethyl ester

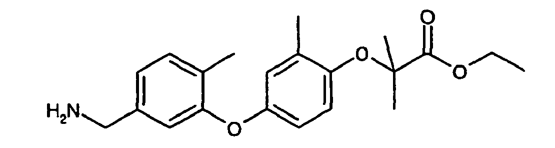

The title compound is prepared according to Intermediate 19 utilizing 2-[4-(5-cyano-2-methyl-phenoxy)-2-methyl-phenoxy]-2-methyl-propionic acid ethyl ester (Intermediate 54). Mass (ES⁺): 358 (M+H⁺); ¹H NMR (400 MHz, CDCl₃).

### Intermediate 22

### 2-[4-(3-Aminomethyl-4-fluoro-phenoxy)-2-methyl-phenoxy]-2-methyl-propionic acid ethyl ester

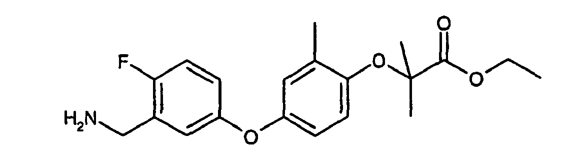

The title compound is prepared according to Intermediate 19 utilizing 2-[4-(3-cyano-4-fluoro-phenoxy)-2-methyl-phenoxy]-2-methyl-propionic acid ethyl ester (Intermediate 53). Mass (ES⁺): 362 (M+H⁺); ¹H NMR (400 MHz, CDCl₃).

### Intermediate 23

### 3-{4-[3-(1-Amino-ethyl)-5-fluoro-phenoxy]-2-ethyl-phenyl}-propionic acid ethyl ester

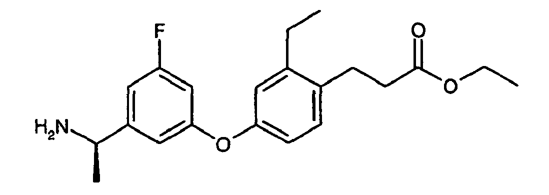

The title compound is prepared according to Intermediate 16 utilizing 1,3-dibromo-5-fluoro-benzene and 3-(2-ethyl-4-hydroxy-phenyl)-propionic acid ethyl ester (Intermediate 59). Mass (ES⁺): 360 (M+H⁺); ¹HNMR (400 MHz, CDCl₃).

### Intermediate 24

### 3-{4-[3-(1-Amino-ethyl)-phenoxy]-2-methyl-phenyl}-propionic acid methyl ester

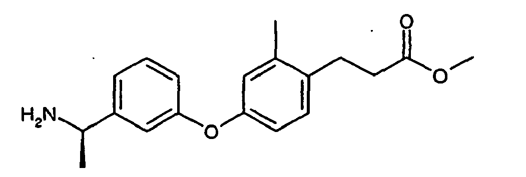

The title compound is prepared according to Intermediate 16, Steps A, C, D, and E utilizing 3'-bromoacetophenone and 3-(4-hydroxy-2-methyl-phenyl)-propionic acid methyl ester (J. Chem. Soc. Perkin Trans. 1; 4; 1990; 1041-1045). Mass (ES⁺): 314 (M+H⁺); ¹H NMR (400 MHz, CDCl₃).

### Intermediate 25

### 3-{4-[3-(1-Amino-ethyl)-5-fluoro-phenoxy]-2-methyl-phenyl}-propionic acid methyl ester

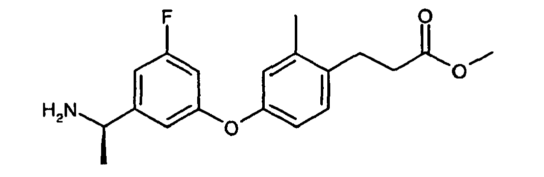

The title compound is prepared according to Intermediate 16 utilizing 1,3-dibromo-5-fluoro-benzene and 3-(4-hydroxy-2-methyl-phenyl)-propionic acid methyl ester (J.Chem.Soc.Perkin Trans. 1; 4; 1990; 1041-1045). Mass (ES⁺): 332 (M+H⁺); ¹H NMR (400 MHz, CDCl₃).

### Intermediate 26

### 3-{4-[3-(1-Amino-ethyl)-5-methyl-phenoxy]-2-methyl-phenyl}-propionic acid methyl ester

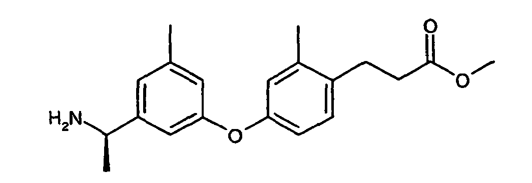

The title compound is prepared according to Intermediate 16 utilizing 3,5-dibromotoluene and 3-(4-hydroxy-2-methyl-phenyl)-propionic acid methyl ester *(*J.Chem.Soc.Perkin Trans. 1; 4; 1990; 1041-1045). Mass (ES⁺): 328 (M+H⁺); ¹H NMR (400 MHz, CDCl₃).

### Intermediate 27

### 3-[4-(3-Aminomethyl-5-fluoro-phenoxy)-2-ethyl-phenyl]-propionic acid ethyl ester

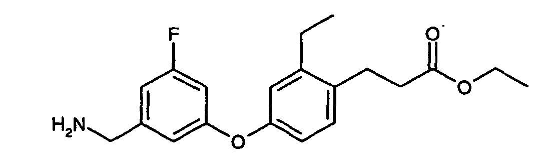

The title compound is prepared according to Intermediate 33 utilizing 3,5-difluorobenzonitrile and 3-(2-ethyl-4-hydroxy-phenyl)-propionic acid ethyl ester (Intermediate 59). Mass (ES⁺): 360 (M+H⁺); ¹H NMR (400 MHz, CDCl₃).

### Intermediate 28

### 3-{4-[2-(2-Amino-ethyl)-phenoxy]-2-methyl-phenyl}-propionic acid ethyl ester

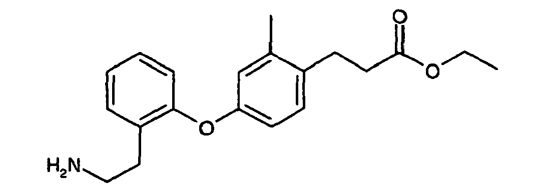

3-{4-[2-(2-tert-Butoxycarbonylamino-ethyl)-phenoxy]-2-methyl-phenyl}-propionic acid ethyl ester (600 mg, 1.4 mmol) (Intermediate 56) is dissolved in dioxane (5 mL) and cooled with an ice bath. Cooled HCl in dioxane (30 mL, 4N) is added and reaction is stirred for 1.5 hours, and then concentrated under reduced pressure. Oil is purified through an SCX column eluting with 10 % ammonia (2N in methanol) in dichloromethane providing the title compound (390 mg, 85 %). Mass (ES⁺): 328 (M+H⁺); ¹H NMR (400 MHz, CDCl₃).

### Intermediate 29

### 2-{4-[3-(2-Amino-ethyl)-phenoxy]-2-methyl-phenoxy}-2-methyl-propionic acid ethyl ester

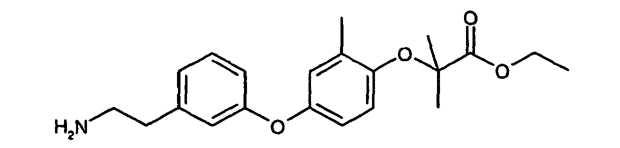

The title compound is prepared according to Intermediate 28 utilizing 2-{4-[3-(2-tert-butoxycarbonylamino-ethyl)-phenoxy]-2-methyl-phenoxy}-2-methyl-propionic acid ethyl ester (Intermediate 58). Mass (ES⁺): 358 (M+H⁺); ¹H NMR (400 MHz, CDCl₃).

### Intermediate 30

### 2-Ethyl-4-trifluoromethyl-benzoic acid

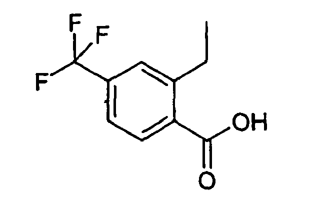

2-Methyl-4-trifluoromethyl-benzoic acid (0.10 g, 0.49 mmol) is dissolved in THF (8 ml), and the solution is cooled to -78 °C. LDA (2.0 M in heptane/THF/ethylbenzene, 0.55 mL, 1.1 mmol) is added. The mixture is warmed to -35 °C. After the dark purple color disappeared, the mixture is cooled back to -78 °C and LDA (2.0 M in heptane/THF/ethylbenzene, 0.55 mL, 1.1 mmol) is added, stirred at -35 °C for 1h. The reaction mixture is cooled back to -78 °C and MeI (0.15 ml, 2.4 mmol) is added, warmed to RT and stirred overnight. The mixture is concentrated, acidified with 5M HCl, extracted with EtOAc, washed with brine, dried over Na₂SO₄. Removal of solvents give a residue (0.20 g) as a mixture of 56 % the title compound and 44 % of the starting material. This mixture is used without isolation and the purification is done after peptide coupling. Mass (ES⁻): 217 (M-H⁺); ¹HNMR (400 MHz, CDCl₃).

### Intermediate 31

### 2-Phenoxy-4-trifluoromethyl-benzoic acid

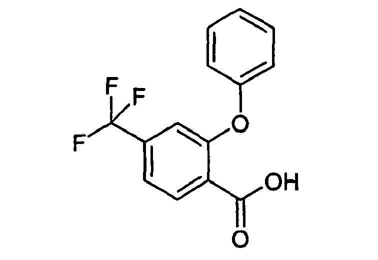

A mixture of 2-fluoro-4-(trifluoromethyl) benzoic acid (2.50 g, 12 mmol), phenol (2.26 g, 24 mmol) and Cs₂CO₃ (11.73g, 36 mmol) in DMF (20 ml) is heated at 100°C under N₂ overnight. The mixture is cooled to RT, quenched with H₂O, acidified with 5 M HCl, extracted with EtOAc, washed with brine, dried over Na₂SO₄, filtered and concentrated. Purification by reversed phase HPLC provides the title compound. Mass (ES⁻): 281.3 (M-H⁺); ¹HNMR (400 MHz, CDCl₃).

### Intermediate 32

### 2-Phenoxy-4-trifluoromethyl-benzaldehyde

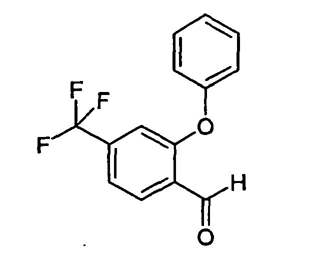

A mixture of 2-fluoro-4-(trifluoromethyl)benzaldehyde (4.97g, 26 mmol), phenol (2.64g, 28 mmol) and Cs₂CO₃ (16.94g, 26 mmol) in DMF (20 ml) is heated at 85 °C under N₂ overnight. The reaction mixture is cooled to RT. Water is added, extracted with EtOAc, washed with brine, dried over Na₂SO₄, filtered and concentrated. Purification by chromatography, eluting with 5% EtOAc in hexane then 15% EtOAc in hexane provides the title compound (5.30g). Mass (ES⁻): 265.3 (M-H⁺); ¹HNMR (400 MHz, CDCl₃).

### Intermediate 33

### 3-[4-(3-Aminomethyl-phenoxy)-2-methyl-phenyl]-propionic acid methyl ester

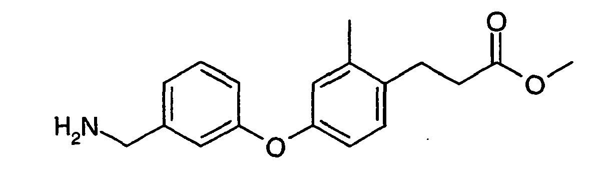

### Step A

### 3-[4-(3-Cyano-phenoxy)-2-methyl-phenyl]-propionic acid methyl ester

A mixture of 3-fluorobenzonitrile (4.82 ml, 45 mmol), 3-(4-Hydroxy-2-methyl-phenyl)-propionic acid methyl ester (J. Chem.Soc.Perkin Trans. 1; 4; 1990; 1041-1045) (8.74g, 45 mmol) and Cs₂CO₃ (29.32g, 90 mmol) in DMF (60 ml) is heated at 100 °C under N₂ overnight. The reaction mixture is cooled to RT, quenched with ice-H₂O, extracted with EtOAc, washed with brine, dried over Na₂SO₄, filtered and concentrated. Purification by chromatography, eluting with 10% EtOAc in hexane then 15% EtOAc in hexane provides the title compound (8.56g). MS: (ES⁺) 296.1 (M+H⁺); ¹H NMR (400 MHz, CDCl₃).

### Step B

### 3-[4-(3-Aminomethyl-phenoxy)-2-methyl-phenyl]-propionic acid methyl ester

The compound from Step A (6.16g, 21mmol) is dissolved in HOAc (75 ml). 5% Pd/C (0.62g) is added. The mixture is hydrogenated at RT under 40 - 60 Psi overnight. The reaction mixture is filtered, concentrated, dissolved in EtOAc, washed with dilute NaHCO₃ and brine, dried over Na₂SO₄, filtered and concentrated again to provide the title compound (5.97g). MS: (ES⁺) 300.2 (M+H¹); ¹H NMR (400 MHz, CDCl₃).

### Intermediate 34

### 3-{4-[3-(1-Amino-ethyl)-5-fluoro-phenoxy]-2-methyl-phenyl}-propionic acid methyl ester

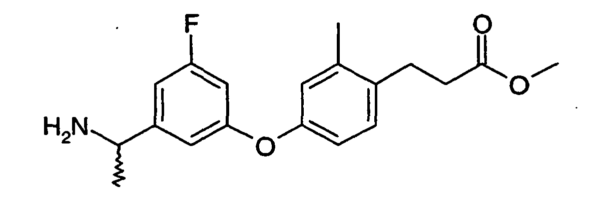

### Step A

### 3-[4-(3-Bromo-5-fluoro-phenoxy)-2-methyl-phenyl]-propionic acid methyl ester

The title compound is prepared as described in Intermediate 16, Step A utilizing 1,3-Dibromo-5-fluoro-benzene and 3-(4-hydroxy-2-methyl-phenyl)-propionic acid methyl ester (J.Chem.Soc.Perkin Trans. 1; 4; 1990; 1041-1045). MS: (ES⁺) 385 (M+NH₄⁺);¹H NMR (400 MHz, CDCl₃).

### Step B

### 3-[4-(3-Acetyl-5-fluoro-phenoxy)-2-methyl-phenyl]-propionic acid methyl ester

The title compound is prepared as described in Intermediate 16, Step B utilizing the compound Step A above. MS:(ES⁺) 331(M+H⁺) ; ¹H NMR (400 MHz, CDCl₃).

### Step C

### 3-{4-[3-Fluoro-5-(1-hydroxyamino-ethyl)-phenoxy]-2-methyl-phenyl}-propionic acid methyl ester

The compound from Step B (0.54 g, 1.64 mmol) is dissolved in EtOH (8 ml). NH₂OH·HCl (0.11g, 1.64 mmol), NaOAc (0.27g, 3.28 mmol) and H₂O are added, heated under reflux for 1h. The reaction mixture is concentrated. Water is added, extracted with EtOAc, washed with brine, dried over Na₂SO₄, filtered and concentrated again to provide the title compound (0.56g). MS: (ES⁺) 346.2 (M+H⁺); ¹H NMR (400 MHz, CDCl₃).

### Step D

### 3-{4-[3-(1-Amino-ethyl)-5-fluoro-phenoxy]-2-methyl-phenyl}-propionic acid methyl ester

The compound from Step C (0.56g, 1.62 mmol) is dissolved in a mixture of MeOH (9.0 ml), AcOH (9.0 ml) and H₂O (4.4 ml). Zn dust (0.42g, 6.49 mmol) is added, stirred at RT for 6 h. The reaction mixture is filtered, concentrated. The residue is dissolved in EtOAc, washed with dilute NaHCO₃, brine, dried over Na₂SO₄, filtered and concentrated again to provide the title compound (0.53g). MS: (ES⁺) 332.1 (M+H⁺); ¹H NMR (400 MHz, CDCl₃).

### Intermediate 35

### [4-(3-Aminomethyl-phenoxy)-2-methyl-phenoxy]-acetic acid methyl ester

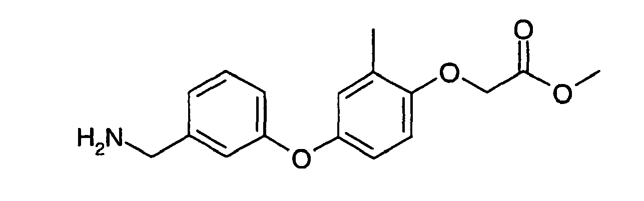

The title compound is prepared according to the procedure described in Intermediate 33 utilizing 3-fluoro-benzonitrile and (4-hydroxy-2-methyl-phenoxy)-acetic acid methyl ester. MS:(ES⁺) 302.1 (M+H⁺); ¹H NMR (400 MHz, CDCl₃).

### Intermediate 36

### 3-[4-(3-Aminomethyl-5-fluoro-phenoxy)-2-methyl-phenyl]-propionic acid methyl ester

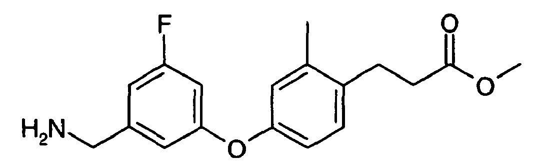

The title compound is prepared according to the procedure described in Intermediate 33 utilizing 3,5-difluoro-benzonitrile and 3-(4-hydroxy-2-methyl-phenyl)-propionic acid methyl ester (J.Chem.Soc.Perkin Trans.1; 4; 1990; 1041-1045). MS: (ES⁺) 318.2 (M+H⁺); ¹H NMR (400 MHz, CDCl₃).

### Intermediate 37

### 3-[4-(2-Aminomethyl-phenoxy)-2-methyl-phenyl]-propionic acid methyl ester

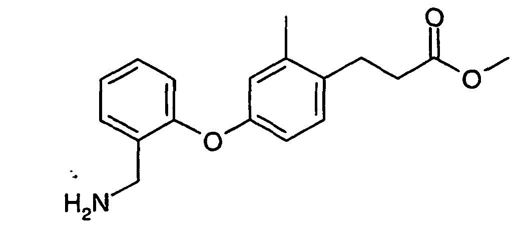

The title compound is prepared according to the procedure described in Intermediate 33 utilizing 2-fluoro-benzonitrile and 3-(4-hydroxy-2-methyl-phenyl)-propionic acid methyl ester (J.Chem.Soc.Perkin Trans. 1; 4; 1990; 1041-1045). MS: (ES⁺) 300.12 (M+H⁺); ¹H NMR (400 MHz, CDCl₃).

### Intermediate 38

### 3-[4-(3-Aminomethyl-5-trifluoromethyl-phenoxy)-2-methyl-phenyl]-propionic acid methyl ester

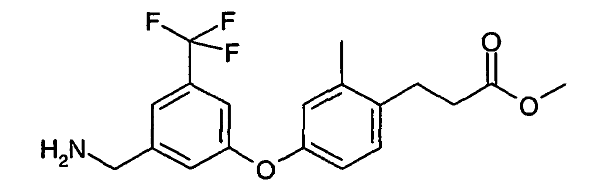

The title compound is prepared according to the procedure described in Intermediate 33 utilizing 3-fluoro-5-trifluoromethyl-benzonitrile and 3-(4-hydroxy-2-methyl-phenyl)-propionic acid methyl ester (J. Chem. Soc. Perkin Trans. 1 ; 4; 1990; 1041-1045). MS: (ES⁺) 368.1 (M+H⁺); ¹H NMR (400 MHz, CDCl₃).

### Intermediate 39

### 3-[4-(5-Aminomethyl-2-trifluoromethyl-phenoxy)-2-methyl-phenyl]-propionic acid methyl ester

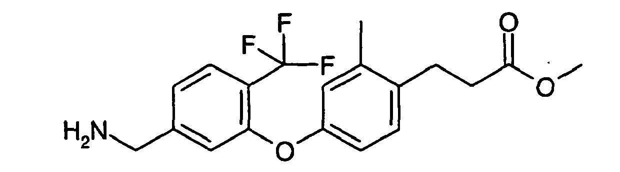

The title compound is prepared according to the procedure described in Intermediate 33 utilizing 3-fluoro-4-trifluoromethyl-benzonitrile and 3-(4-hydroxy-2-methyl-phenyl)-propionic acid methyl ester (J. Chem.Soc.Perkin Trans. 1; 4; 1990; 1041-1045). MS: (ES⁺) 368.1 (M+H⁺); ¹H NMR (400 MHz, CDCl₃).

### Intermediate 40

### 3-[4-(3-Aminomethyl-4-trifluoromethyl-phenoxy)-2-methyl-phenyl]-propionic acid methyl ester

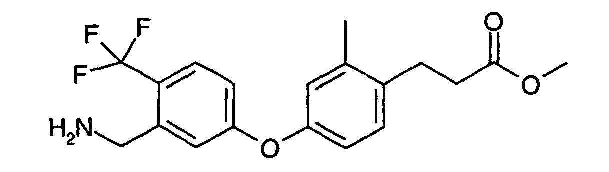

### Step A

### 3-[4-(3-Cyano-4-trifluoromethyl-phenoxy)-2-methyl-phenyl]-propionic acid methyl ester

The title compound is prepared according to the procedure described in Intermediate 33, Step A, utilizing 5-fluoro-2-trifluoromethyl-benzonitrile and 3-(4-hydroxy-2-methyl-phenyl)-propionic acid methyl ester *(*J.Chem.Soc.Perkin Trans. 1 ; 4; 1990; 1041-1045). MS: (ES⁺) 363.9.1 (M+H⁺); ¹H NMR (400 MHz, CDCl₃).

### Step B

### 3-[4-(3-Aminomethyl-4-trifluoromethyl-phenoxy)-2-methyl-phenyl]-propionic acid methyl ester

The compound from Step A (0.35 g, 0.96 mmol) is dissolved in 2 M NH₃ in MeOH (50 ml). Raney Nickel (0.2 g) is added. The mixture is hydrogenated at 40 °C under 60 Psi overnight. The reaction mixture is filtered and concentrated to provide the crude title compound (0.44g). MS: (ES⁺) 368.06 (M+H⁺).

### Intermediate 41

### 2-{4-[3-(1-Amino-ethyl)-5-fluoro-phenoxy]-2-methyl-phenoxy}-2-methyl-propionic acid ethyl ester

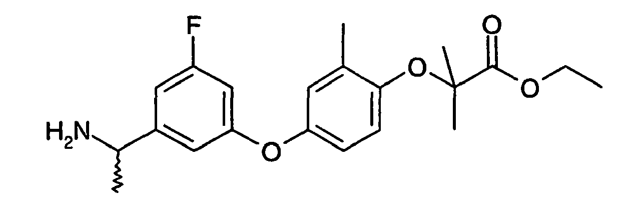

The title compound is prepared according to the procedure described in Intermediate 34 utilizing 1,3-dibromo-5-fluoro-benzene and 2-(4-hydroxy-2-methyl-phenoxy)-2-methyl-propionic acid ethyl ester (WO 2003072102). MS: (ES⁺) 376.2 (M+H⁺); ¹H NMR (400 MHz, CDCl₃).

### Intermediate 42

### 3-[4-(3-Aminomethyl-5-fluoro-phenoxy)-2-methyl-phenyl]-2,2-dimethyl-propionic acid methyl ester

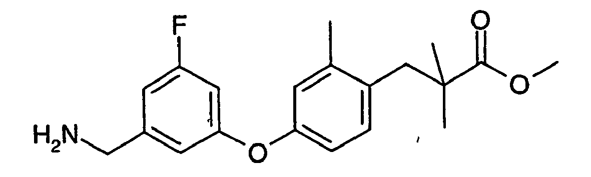

The title compound is prepared according to the procedure described in Intermediate 33 utilizing 3,5-difluoro-benzonitrile and 3-(4-hydroxy-2-methyl-phenyl)-2,2-dimethyl-propionic acid methyl ester (Intermediate 60). MS: (ES⁺) 346.2 (M+H⁺); ¹H NMR (400 MHz, CDCl₃).

### Intermediate 43

### 3-{4-[3-(1-Amino-propyl)-5-fluoro-phenoxy]-2-methyl-phenyl}-propionic acid methyl ester

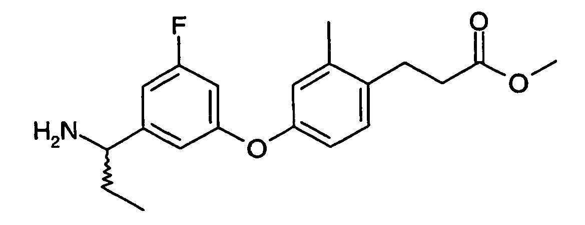

### Step A

### 3-[4-(3-Fluoro-5-propionyl-phenoxy)-2-methyl-phenyl]-propionic acid methyl ester

The title compound is prepared according to the procedure described in Intermediate 33, Step A utilizing 3,5-difluoropropiophenone and 3-(4-hydroxy-2-methylphenyl)-propionic acid methyl ester *(*J.Chem.Soc.Perkin Trans.1; 4; 1990; 1041-1045). MS: (ES⁺) 345.2 (M+H⁺); ¹H NMR (400 MHz, CDCl₃).

### Step B

### 3-{4-[3-Fluoro-5-(1-hydroxyamino-propyl)-phenoxy]-2-methyl-phenyl}-propionic acid methyl ester

The title compound is prepared according to the procedure described in Intermediate 34, Step C utilizing the compound from Step A. MS: (ES⁺) 360.2 (M+H⁺); ¹H NMR (400 MHz, CDCl₃).

### Step C

### 3-{4-[3-(1-Amino-propyl)-5-fluoro-phenoxy]-2-methyl-phenyl}-propionic acid methyl ester

The title compound is prepared according to the procedure described in Intermediate 34, Step D utilizing the compound from Step B. MS: (ES⁺) 346.3 (M+H⁺); ¹H NMR (400 MHz, CDCl₃).

### Intermediate 44

### 3-{4-[3-(1-Amino-propyl)-5-fluoro-phenoxy]-2-methyl-phenyl}-propionic acid methyl ester

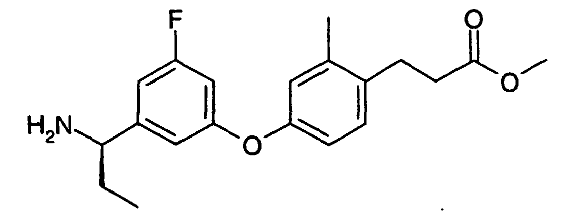

The title compound is prepared according to the procedure described in Intermediate 16 utilizing 3-[4-(3-fluoro-5-propionyl-phenoxy)-2-methyl-phenyl]-propionic acid methyl ester (Intermediate 43, Step A). Chiral HPLC t_{R} = 6.532 min (LC column: CHIRALPAK AD; 4.6 x 250 mm; 100% MeOH with 0.2 % DMEA; flow rate: 1.0 ml/min); ¹H NMR (400 MHz, CDCl₃).

### Intermediate 45

### 3-[4-(3-Aminomethyl-5-methyl-phenoxy)-2-methyl-phenyl]-propionic acid methyl ester

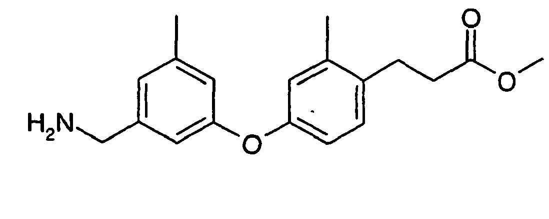

### Step A

### 3-[4-(3-Bromo-5-cyano-phenoxy)-2-methyl-phenyl]-propionic acid methyl ester

The title compound is prepared according to the procedure described in Intermediate 16, Step A utilizing 3,5-dibromo-benzonitrile and 3-(4-hydroxy-2-methylphenyl)-propionic acid methyl ester *(*J. Chem. Soc. Perkin Trans. 1, 4, 1990, 1041-1045). MS: (ES⁺) 393.2 (M+NH₄⁺); ¹H NMR (400 MHz, CDCl₃).

### Step B

### 3-[4-(3-Cyano-5-methyl-phenoxy)-2-methyl-phenyl]-propionic acid methyl ester

The compound from Step A above (0.60 g, 1.6 mmol) is dissolved in DMF (10 ml). Trimethylboroxine (0.22 ml, 1.6 mmol), (Ph₃P)₄Pd (0.18 g, 0.16 mmol) and K₂CO₃ (0.66 g) are added. The reaction mixture is heated at 115 °C under N₂ overnight. The reaction mixture is filtered through Celite, washed with EtOAc. The filtrate is concentrated. The residue is purified by chromatography, eluting with 10% EtOAc in hexane then 15% EtOAc in hexane providing the title compound (0.40 g). MS: (ES^{327.5} (M+NH₄⁺); ¹H NMR (400 MHz, CDCl₃).

### Step C

### 3-[4-(3-Aminomethyl-5-methyl-phenoxy)-2-methyl-phenyl]-propionic acid methyl ester

The title compound is prepared according to the procedure described in Intermediate 33, Step B utilizing the compound from Step B above. The crude product is purified by SCX column eluting with 10 % 2M NH₃ in MeOH /CH₂Cl₂. MS: (ES⁺) 314.5 (M+H⁺); ¹H NMR (400 MHz, CDCl₃).

### Intermediate 46

### 3-[4-(3-Aminomethyl-5-methyl-phenoxy)-2-ethyl-phenyl]-propionic acid ethyl ester

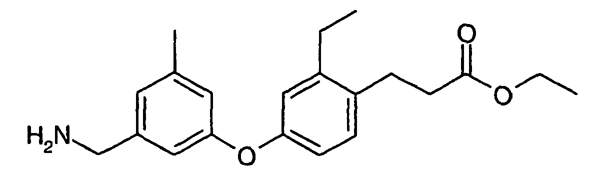

The title compound is prepared according to the procedure described in Intermediate 45 utilizing 3,5-dibromo-benzonitrile and 3-(2-ethyl-4-hydroxy-phenyl)-propionic acid ethyl ester (Intermediate 59). MS: (ES⁺) 342.5 (M+H⁺); ¹H NMR (400 MHz, CDCl₃).

### Intermediate 47

### 2-[4-(3-aminomethyl-5-fluoro-phenoxy)-2-methyl-phenoxy]-2-methyl-propionic acid ethyl ester

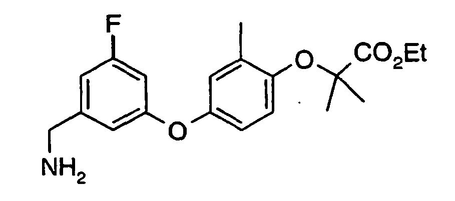

### Step A

### 2-[4-(3-cyano-5-flurorphenoxy)-2-methylphenoxy]-2-methylpropionic acid ethyl ester

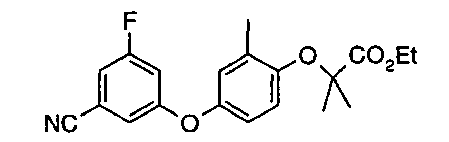

3,5-difluorobenzonitrile (1.286 g, 9.24 mmol) is added to a suspension of KF/Al₂O₃ (5.0 g, 40% KF), 18-crown-6 (220 mg, 0.84 mmol) and compound (a) from Intermediate 55, Step A (2.0 g, 8.4 mmol) in CH₃CN (150 mL, HPLC grade). The mixture is warmed to reflux. After 2 h, the reaction is allowed to reach r.t., filtered trough Celite and washed with EtOAc/brine. The organic layer is dried, filtered and concentrated, and the crude residue is flash chromatographed on SiO₂ (5-10% EtOAc/hexanes), affording 1.95 g of the title compound (65%, white solid).

### Step B

### 2-[4-(3-methylamino-5-flurorphenoxy)-2-methylphenoxy]-2-methylpropionic acid ethyl ester

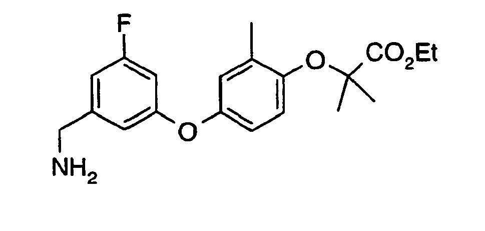

Pd/C (140 mg, 10% Pd on activated C, 0.027 mmol) is added to a solution of compound obtained from Step A (1.70 g, 4.927 mmol) in glacial AcOH (200 mL). The mixture is stirred at r.t. under H₂ atmosphere (40 psi) overnight (c.a. 14 h) and filtered trough Celite (CH₂Cl₂ washings). The AcOH is neutralized with NaHCO₃ and the mixture partitioned between CH₂Cl₂ and H₂O. The organic layer is dried, filtered and concentrated. The crude residue is flash chromatographed on SiO₂ (0.2% ethyldimethylamine, 30% iPrOH, 70% hexane) to afford about 1.35 g of the title compound (79%, colorless oil).

### Intermediate 48

### [3-(3-Aminomethyl-5-fluoro-phenoxy)-phenyl]-acetic acid methyl ester

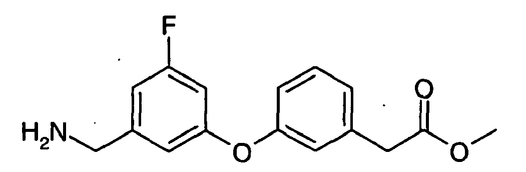

The title compound is prepared according to the procedure described in Intermediate 33 utilizing 3,5- difluoro-benzonitrile and (3-hydroxy-phenyl)-acetic acid methyl ester (WO 2003072102). MS: (ES⁺) 290.3 (M+H⁺); ¹H NMR (400 MHz, CDCl₃).

### Intermediate 49

### 2-[4-(3-Aminomethyl-phenoxy)-phenoxy]-2-methyl-propionic acid ethyl ester

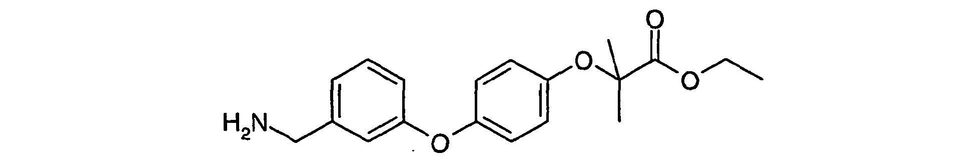

The title compound is prepared according to the procedure described in Intermediate 40 utilizing 3-fluorobenzonitrile and 2-(4-hydroxy-phenoxy)-2-methyl-propionic acid ethyl ester (WO 2002081454) except that the reaction is carried out at rt and 500 Psi for Step B. MS: (ES⁺) 330.3 (M+H⁺); ¹H NMR (400 MHz, CDCl₃).

### Intermediate 50

### 3-Methyl-5-trifluoromethyl-benzo[b]thiophene-2-carboxylic acid

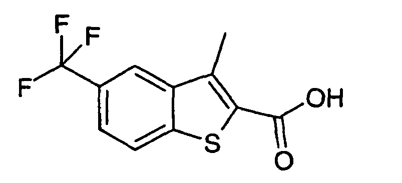

### Step A

### 1-(2-Methylsulfanyl-5-trifluoromethyl-phenyl)-ethanone

A solution of 2-fluoro-5-trifluoromethyl acetophenone (3.0 g, 14.5 mmol) in dry DMF (20 mL) was treated with sodium thiomethoxide (1.22 g, 17.4 mmol) and the reaction was stirred for 2 hours at rt under N₂. The reaction was quenched with 1 N HCl (10 mL), diluted with Et₂O and then extracted twice with water. The organic layer was dried (Na₂SO₄) and the solvent removed *in vacuo* to afford crude product that was absorbed on silica gel and then column purified using 5/1 hexanes/acetone to afford 2.88 g (85%) of the product. R_{f} = 0.57 (1/1 hexanes/acetone). ¹H NMR (400 MHz, CDCl₃) δ 8.05 (s, 1H), 7.69 (d, 1H, *J* = 8.31 Hz), 7.42 (d, 1H, *J* = 8.80 Hz), 2.68 (s, 3H), 2.48 (s, 3H).

### Step B

### 3-Methyl-5-trifluoromethyl-benzo[b]thiophene-2-carboxylic acid

A mixture of 1-(2-methylsulfanyl-5-trifluoromethyl-phenyl)-ethanone (made above) (2.06 g, 8.79 mmol) and bromoacetic acid (7.33 g, 52.8 mmol) in acetic acid (20 mL) was heated to reflux and stirred for 20 hours under N₂. The reaction was cooled and water was added to form a slurry. The slurry was filtered and the solids rinsed with water to afford 1.59 g (69%) of the product after drying in a vacuum oven at 45 °C. R_{f} = 0.18 (1/1 hexanes/acetone). ¹H NMR (400 MHz, CDCl₃). MS (ES⁻) *m*/*z* mass calcd for C₁₁H₇O₂SF₃ 260, found 259 (M - 1, 100%).

### Intermediate 51

### 2-[4-(3-cyano-4-methylphenoxy)-2-methylphenoxy]-2-methylpropionic acid ethyl ester

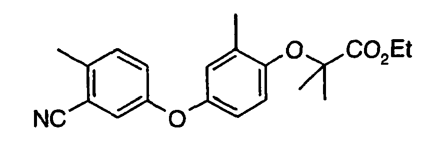

### Step A

### 2-[4-(3-cyano-4-nitrophenoxy)-2-methylphenoxy]-2-methylpropionic acid ethyl ester

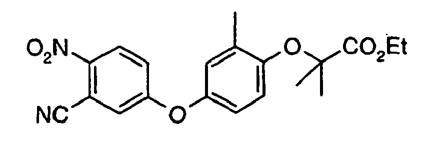

A mixture of 9.6 g (58 mmol) of 2,5-difluorobenzonitrile, 13.8 g (58 mmol) of 2-(4-hydroxy-2-methylphenoxy)-2-methylpropionic acid ethyl ester (WO 2003072102), 34.5 g of 40% w/w potassium fluoride-alumina, 1.56 g (5.8 mmol) of 18-crown-6 in CH₃CN is refluxed under argon atmosphere. After 1 hour, the reaction mixture is cooled to rt, partitioned between equal parts of ether and water, and shaked vigorously. The aqueous layer and alumina sediments are separated and the resulting organic phase is washed once with a saturated potassium chloride solution. The organic phase is dried over MgSO₄, and concentrated in vacuo. The crude is used in the next step without further purification. About 19.6 g of 2-[4-(3-cyano-4-nitrophenoxy)-2-methylphenoxy]-2-methylpropionic acid ethyl ester are obtained.

### Step B

### 2-[4-(4-amino-3-cyanophenoxy)-2-methylphenoxy]-2-methylpropionic acid ethyl ester

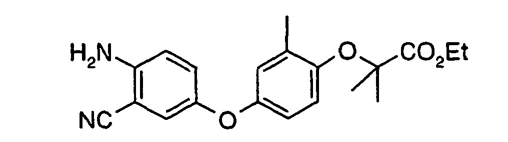

A mixture of 7 g (18.2 mmol) of 2-[4-(3-cyano-4-nitrophenoxy)-2-methylphenoxy]-2-methylpropionic acid ethyl ester, 700 mg of Pd(C) 10% in 50 ml of EtOH is hydrogenated for two hours at rt. The mixture is filtered over celite and washed with EtOH. The solvent is evaporated to give 6.4 g of the title compound, which is used for the next step without further purification.

### Step C

### 2-[4-(4-bromo-3-cyanophenoxy)-2-methylphenoxy]-2-methylpropionic acid ethyl ester

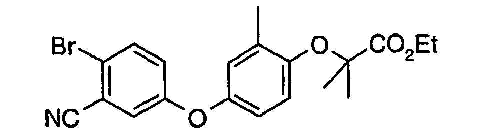

To a mixture of 2.5 ml (21.2 mmol) of ^{t}BuONO and 3.79 g (17 mmol) of CuBr₂in acetonitrile at 65°C under argon atmosphere is added 5 g (14.1 mmol) of 2-[4-(4-amino-3-cyanophenoxy)-2-methylphenoxy]-2-methylpropionic acid ethyl ester in 10 ml of acetonitrile. After the addition is washed, the reaction is stirred at the same temperature for 15 min. The reaction is allowed to reach rt, and HCl (2N) is added. The mixture is extracted with ether (3x20 ml). The organic phase is washed with HCl (2N), dried over MgSO₄ and concentrated to give 5.8 g of the title compound, which is used for the next step without further purification.

### Step D

### 2-[4-(3-cyano-4-methylphenoxy)-2-methylphenoxy]-2-methylpropionic acid ethyl ester

A sealed tube is charged with 3 g (7.2 mmol) of 2-[4-(4-bromo-3-cyanophenoxy)-2-methylphenoxy]-2-methylpropionic acid ethyl ester, 80 mg (0.35 mmol) of Pd(OAc)₂, 192 mg (0.7 mmol) of P(o-tolyl)₃, 1.1 ml (7.9 mmol) Sn(CH₃)₄ and 3 ml of Et₃N in 5 ml of DMF and is heated at 115°C under argon atmosphere overnight. To the mixture, HCl (2N) is added (10ml), and the mixture is extracted with ether (3x50 ml). The organic phase is dried over MgSO₄ and concentrated. The desired product is purificated by silica gel chromatogaphy using hexane/ethyl acetate 15:1 as eluent to obtain 680 mg (27%) of the title compound. MS Data (Ion Trap): m/z 353.8 [M+H] as base peak, m/z 376.1 [M+Na] and m/z 511.7.

### Intermediate 52

### 2-{4-[3-(3-amino-propyl)-phenoxy]-2-methyl-phenoxy}-methyl-propionic acid ethyl ester

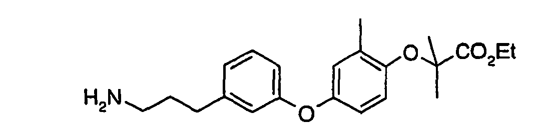

### Step A

### 2-[4-(3-formilphenoxy)-2-methylphenoxy]-2-methypropionic acid ethyl ester

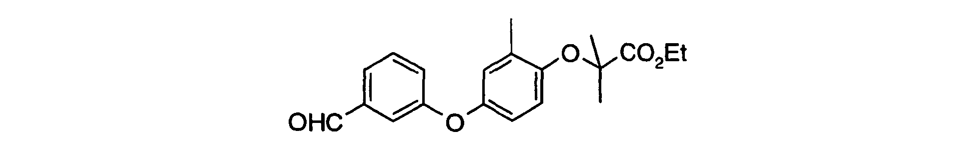

A sealed tube is charged with Pd(OAc)₂ (36 mg, 0.14 mmol), 2-(ditertbutylphosphino)biphenyl (76 mg, 0.4 mmol) and K₃PO₄ (3.56 g, 14 mmol). The tube is evacuated and back-filled with argon and fitted with a rubber septum. Toluene (10 ml) followed by 3-bromo benzaldehyde (0.81 ml, 7 mmol) and 2-(4-hydroxy-2-methylphenoxy)-2-methylpropionic acid ethyl ester (WO 2003072102) (2 g, 8.4 mmol) in toluene (10 ml) are added via syringe. The tube is sealed under argon and stirred at 110 °C for two days. The mixture is diluted with 30 ml of diethyl ether, filtered through a pad of celite and concentrated to leave a crude oil. The resulting oil is purified by flash chromatogaphy on silica gel using 10:1 hexanes/ethyl acetate as eluent to obtain 900 mg of the title compound.

### Step B

### 2-{4-[3-(2-cyanovinyl)-phenoxy]-2-methylphenoxy}-2-methypropionic acid ethyl ester

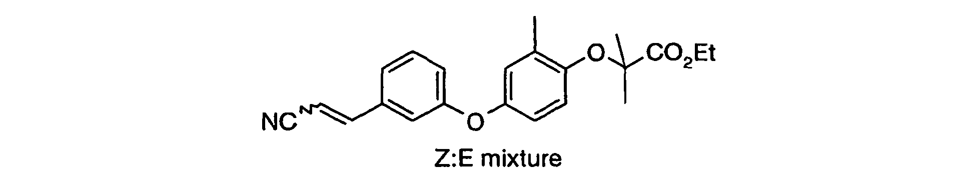

To a solution of diethyl-(cyanomethyl)-phosphonate (0.466 ml, 2.63mmol) in THF (5 ml) at -78°C is added dropwise ^{t}BuOK (2.63 ml, 2.63 mmol) under a nitrogen blanket. The solution is stirred for 30 min., and 2-[4-(3-formilphenoxy)-2-methylphenoxy]-2-methypropionic acid ethyl ester (900 mg, 2.63 mmol) in THF (10 ml) is added slowly at the same temperature. The mixture is stirred at -78°C for another 30 min. and then warmed to 0°C, quenched with 25% NH₄Cl (aq), extracted with EtOAc, washed with brine, dried over MgSO₄, filtered and concentrated. Purification: silica gel chromatogaphy. Eluent 12:1 hexanes/ethyl acetate affords 825 mg of a mixture 70:30 of E/Z isomers of the title compound.

### Step C

### 2-{4-[3-(3-amino-propyl)-phenoxy]-2-methyl-phenoxy}-2-methyl-propionic acid ethyl ester

A mixture of 2-{4-[3-(2-cyanovinyl)-phenoxy]-2-methylphenoxy}-2-methypropionic acid ethyl ester (700 mg, 1.9 mmol) and 70 mg of Pd(C) 10% in 10ml of EtOH is hydrogenated one hour at rt. Then, it is filtered over celite and washed with EtOH. The solvent is evaporated, and the crude is purified using silica gel chromatogaphy, 25:1 hexane/ethyl acetate as eluent to give 460 mg of the title compound. MS Data (Ion Trap): m/z 390 [M+Na] as base peak, m/z 368 [M+H], m/z 294 and 254 (in positive mode).

### Intermediate 53

### 2-[4-(3-cyano-4-fluorophenoxy)-2-methylphenoxy]-2-methylpropionic acid ethyl ester

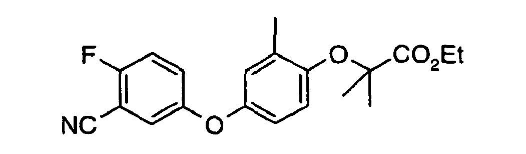

### Step A

### 1-bromo-2,5-difluoro-4-nitrobenzene

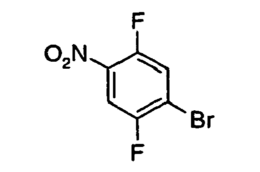

To a solution of 2-bromo-1,4-difluorobenzene (10 g, 51.8 mmol) in 40 ml of sulfuric acid at 0°C is added dropwise 35 ml of nitric acid while maintaining an internal temperature below 20°C. The mixture is then poured into ice and extracted with ether (3x100 ml). The organic phase is washed with NaHCO₃ (3 times), dried over MgSO₄ and evaporated. The crude is purificated by silica gel chromatogaphy (hexane/acetone 10:1) to afford 11.25 g of the title compound.

### Step B

### 2,5-difluoro-4-nitrobenzonitrile

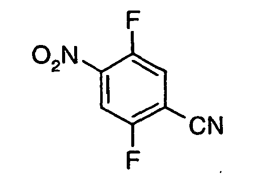

A mixture of 1-bromo-2,5-difluoro-4-nitrobenzene (5g, 21 mmol) and 2.44 g (27 mmol) of CuCN in 20 ml of DMF is heated at 160°C in a sealed tube, under argon atmosphere overnight. The mixture is washed with 50 ml of water and NH₄OH (3x20 ml) and then extracted with CH₂Cl₂ (3 x 100ml). The organic phase is dried over MgSO₄ and evaporated to give 3.8 g of the title compound, which is used in next step without further purification.

### Step C

### 2-[4-(5-cyano-4-fluoro-2-nitrophenoxy)-2-methylphenoxy]-2-methylpropionic acid ethyl ester

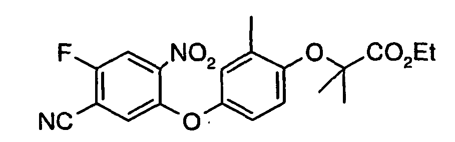

A mixture of 2,5-difluoro-4-nitrobenzonitrile (1.6 g, 8.7 mmol), 2.1 g (8.7 mmol) of 2-(4-hydroxy-2-methylphenoxy)-2-methylpropionic acid ethyl ester (WO 2003072102), 5.25 g of 40% w/w potassium fluoride-alumina, 230 mg (0.87 mmol) of 18-crown-6 in CH₃CN is refluxed under argon atmosphere. After 1 hour, the mixture is cooled to rt, partitioned between equal parts of ether and water, and shaked vigorously. The aqueous layer and alumina sediments are separated, and the resulting organic phase is washed once with a saturated potassium chloride solution. The organic phase is dried over MgSO₄, and concentrated in vacuo. The crude is purificated by silica gel chromatogaphy (hexane/ethyl acetate 15: 1) to yield 2,4 g of the title compound.

### Step D

### 2-[4-2-amino-5-cyano-4-fluorophenoxy)-2-methylphenoxy]-2-methylpropionic acid ethyl ester

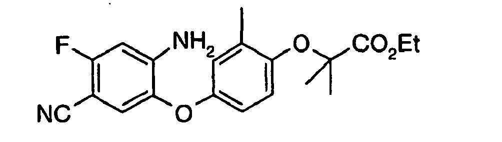

A mixture of 2-[4-(5-cyano-4-fluoro-2-nitrophenoxy)-2-methylphenoxy]-2-methylpropionic acid ethyl ester (1.5 g, 3.7 mmol), 150 mg of Pd(c) 10% in 30 ml of EtOH is hydrogenated overnight at rt. Then, it is filtered over celite and washed with EtOH. The solvet is evaporated to give 1.4 g of the title compound, which is used for the next step without further purification.

### Step E

### 2-[4-(3-cyano-4-fluorophenoxy)-2-methylphenoxy]-2-methylpropionic acid ethyl ester

To a solution of 2-[4-(2-amino-5-cyano-4-fluorophenoxy)-2-methylphenoxy]-2-methylpropionic acid ethyl ester (1.4 g, 3.7 mmol) in a mixture of 80ml THF/ 8 ml water is added 2.91 ml (52.6 mmol) of H₃PO₂, catalytic amount of Cu₂O and a solution of 312 mg (4.5 mmol) of NaNO₂ in 3 ml of water. After stirring at rt overnight , the mixture is diluted with an aqueous NaHCO₃ solution and extracted with ethyl acetate (3x100 ml). he combined organic extracts are washed with aqueous NH₄Cl, dried over MgSO₄ and evaporated to dryness. Purification by silica gel chromatogaphy (hexane/ethyl acetate 10: 1) provides about 479 mg of the title compound. MS Data: (ion trap/ESI +): m/z 380.1 [M+Na] as base peak and m/z 358.1 [M+H].

### Intermediate 54

### 2-[4-(5-cyano-2-methylphenoxy)-2-methylphenoxy]-2-methylpropionic acid ethyl ester

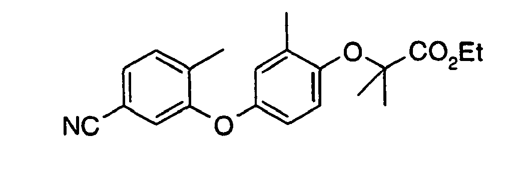

### Step A

### 3-fluoro-4-methyl-5-nitrobenzonitrile

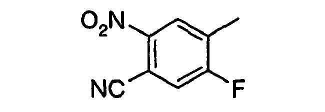

Add potassium nitrate (4.51 g, 44.54 mmol) in three or four portions to 3-fluoro-4-methylbenzonitrile (6.02 g, 44.54 mmol) in concentrated H₂SO₄ (50 ml) at 0°C and stir. Stir the deep orange solution for 2 hours at 0°C and then for 1 hour at rt. Add the mixture over 800 ml of ice and extract with 150 ml of AcOEt. Allow the mixture to warm at rt and separate the organic layer. Extract again the aqueous layer with 100 ml of AcOEt. Combine the organic extracts and washed with water (50 ml) and brine (50 ml). Dry the organic layer over magnesium sulfate, filter, and concentrate under reduced pressure to give the crude product (7.64 g, 93% yield). Although the product can be used without further purification, flash chromatogapy can be performed in silica using hexane:ethyl acetate (10:1) as eluent.

### Step B

### 2-[4-(5-cyano-4-nitro-2-methylphenoxy)-2-methylphenoxy]-2-methylpropionic acid ethyl ester

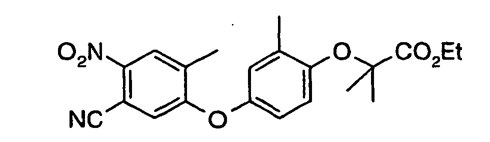

Prepare a solution of 3-fluoro-4-methyl-5-nitrobenzonitrile (2.85 g, 15.49 mmol) and ethyl 2,2,-dimethyl-2-(3'-methyl-4'-hydroxy)phenyloxyacetate (WO 2003072102) (3.69 g, 15.49 mmol) in 150 ml of acetonitrile, and add 7.12 g of potassium fluoride (40% in alumina) and 18-crown-6 ether (409 mg, 1.55 mmol) and heat to reflux for 2 hours. Cool the mixture at rt, and add equal parts of water and diethyl ether (80 ml + 80 ml). Separate the aqueous layer and the alumina sediments and wash the organic layer with water (20 ml) and brine (20 ml). Dry the organic layer with sodium sulfate, filter and concentrate under reduced pressure to give the crude product (5.77 g, 94 %), which can be used without further purification.

### Step C

### 2-[4-(5-cyano-4-amino-2-methylphenoxy)-2-methylphenoxy]-2-methylpropionic acid ethyl ester

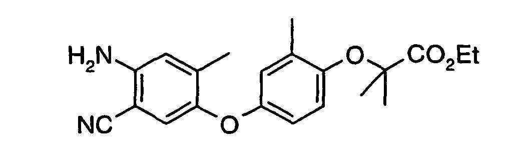

Prepare a solution of compound obtained from Step B (5.08 g, 12.76 mmol) in 150 ml of ethanol and add a slurry of 508 mg of Pd/C (10%) in 20 ml of ethanol. Close the flask with a septum, purge it several times with H₂ and stir it overnight under positive pressure of H₂. Afterward, filter the slurry through a short pad of celite and evaporate the solution under reduced pressure to give the crude product (4.60 g, 98%), which can be used without further purification.

### Step D

### 2-[4-(5-cyano-2-methylphenoxy)-2-methylphenoxy]-2-methylpropionic acid ethyl ester

Add 30 ml of HCl (2N) to a solution of Step C (2.07 g, 5.63 mmol) in 100 ml of THF-AcOH (9:1) and stir for 5 min. Add 1.7 ml of H₂O₂ (3%) and then add a solution of sodium nitrite (388 mg, 5.63 mmol) in water (2ml). Stir the mixture at 0°C for 30 min and then at rt for 2 hours. Wash the mixture with saturated solution of sodium bicarbonate for several times until the aqueous layer reaches pH 9. Separate the organic layer and wash it with water and then brine. Dry the organic layer with magnesium sulfate, filter and evaporate the solvent under reduce pressure to give the crude product (1.69 g, 85%). Purify the product by flash chromatogaphy in silica using hexane-ethyl acetate (6:1) as solvent. Mass spectrum (m/e): 354 (M+1)

### Intermediate 55

### 2-[4-(3-cyano-5-methylphenoxy)-2-methylphenoxy]-2-methylpropionic acid ethyl ester

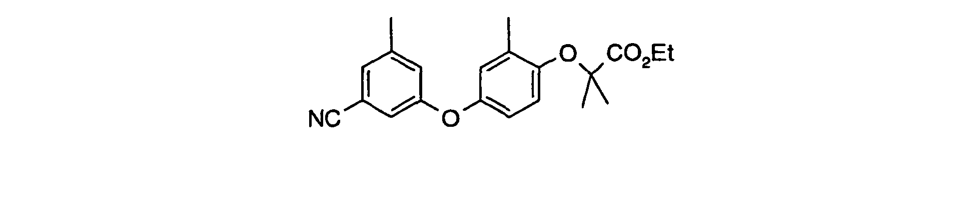

### Step A

### 2-[4-(3-cyano-5-nitrophenoxy)-2-methylphenoxy]-2-methylpropionic acid ethyl ester

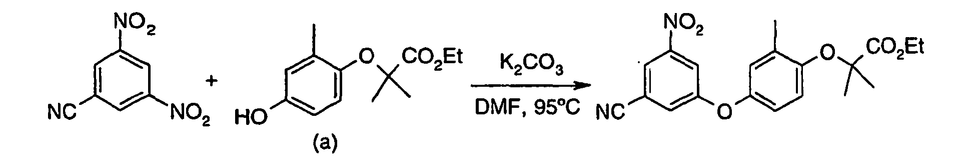

A mixture of 3,5-dinitrobenzonitrile (3.65 g, 18.90 mmol), compound (a) (WO 2003072102) (3 g, 12.60 mmol) and K₂CO₃ (2.1 g, 15.194 mmol) in DMF (40 mL, HPLC grade) is stirred at 95 °C. After 12 h, the mixture is poured into brine, and extracted with EtOAc. The organic layer is dried, filtered and concentrated, and the crude obtained is flash chromatographed on SiO₂ (10 % EtOAc/hexanes) affording 5.54 g of the arylether (94%, white solid).

### Step B

### 2-[4-(3-cyano-5-aminophenoxy)-2-methylphenoxy]-2-methylpropionic acid ethyl ester

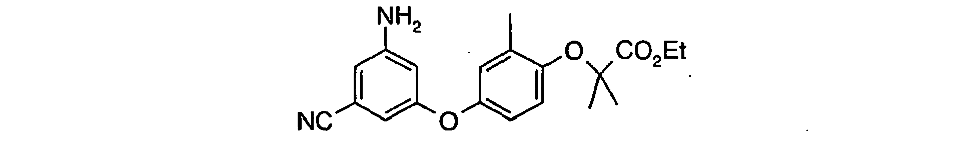

Pd/C (600 mg, 10% Pd on activated C, 0.564 mmol) is added to a solution of the nitro compound from Step B (4.3 g, 11.20 mmol) in EtOH (50 mL, HPLC grade). The reaction mixture is stirred at r.t. for 2 h under H₂ atmosphere (1 atm). The mixture is filtered trough Celite (EtOAc washings), and the solvent is removed in rotatory evaporator. The product purified by flash chromatography on SiO₂ (10-30% EtOAc/hexanes) to afford 3.94 g of the amino compound (99%, colorless oil).

### Step C

### 2-[4-(3-cyano-5-iodophenoxy)-2-methylphenoxy]-2-methylpropionic acid ethyl ester

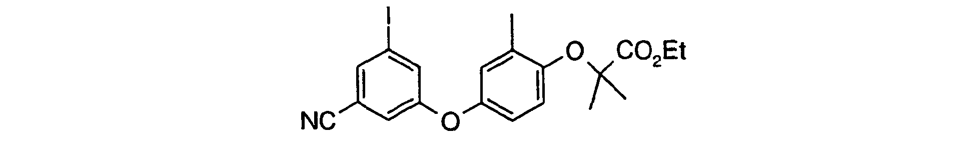

Ter-BuONO (1.5 mL, 12.61 mmol) is added to a 0°C cooled solution of the arylamine from Step B (400 mg, 1.13 mmol) and I₂ (1.72 g, 6.77 mmol) in CH₃CN (18 mL, HPLC grade). The mixture is allowed to reach r.t., poured into brine, and extracted with TBME. The organic layer is dried, filtered and concentrated. The resulting crude is flash chromatographed on SiO₂ (3% EtOAc/hexanes) to afford 416 mg of the title compound (79%, white solid).

### Step D

### 2-[4-(3-cyano-5-methylphenoxy)-2-methylphenoxy]-2-methylpropionic acid ethyl ester

Me₄Sn (292 mg, 1.631 mmol) is added to a solution of the iodide from Step C (380 mg, 0.8172 mmol) and Pd(PPh₃)₄ (95 mg, 0.08172 mmol) in DMF (12 mL, anhydrous). The mixture is warmed to 120 °C and stirred at that temperature for 90 min. It is allowed to reach r.t., filtered trough Celite and washed with EtOAc/brine. The organic layer is dried, filtered and concentrated. The crude residue is flash chromatographed on SiO₂ (3-5% EtOAc/hexanes) to afford 270 mg of the title compound (94%, colorless oil).

### Intermediate 56

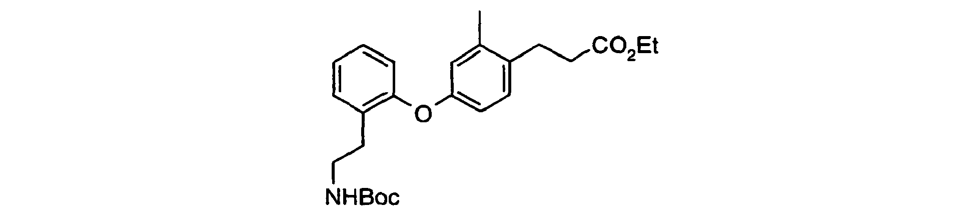

### Step A

### 3-methyl-4-bromobenzyloxyphenol

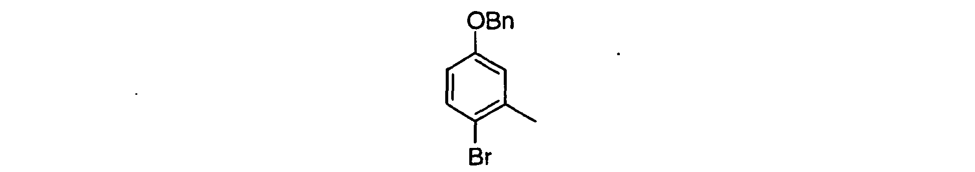

Benzyl bromide (3 mL, 25.2 mmol) is added to a suspension of 3-methyl-4-bromophenol (4.7 g, 25.13 mmol) and K₂CO₃ (3.5 g, 25.32 mmol) in CH₃CN (40 mL, HPLC grade), and the mixture is stirred at r.t. for 16 h. It is acidified with diluted HCl (1M) and partitioned between EtOAc and H₂O. The organic layer is dried, filtered and concentrated. The product is purified by flash chromatography on SiO₂ (1-2% EtOAc/hexanes) to afford about 6.5 g of the benzylated phenol (93%, white solid).

### Step B

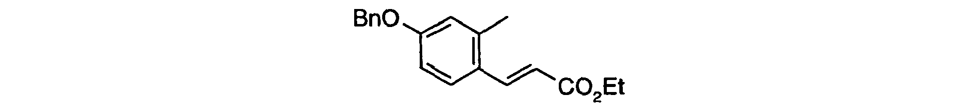

Ethyl acrylate (12 mL, 114.7 mmol) is added to a solution of 3-methyl-4-bromobenzyloxyphenol (6.5 g, 23.465 mmol), palladium acetate (560 mg, 2.494 mmol), P(o-tol)₃ (1.5 g, 4.928 mmol) and DIPEA (12 mL, 68.89 mmol) in EtCN (120 mL, HPLC grade). The mixture is warmed to 95°C and stirred at that temperature for 60 h. It is allowed to reach r.t., filtered trough Celite and partitioned between EtOAc and H₂O. The organic layer is dried, filtered and concentrated, and the resulting crude is flash chromatographed on SiO₂ (2-3% EtOAc/hexanes) to afford 6.55 g of the Heck product (94%, white solid).

### Step C

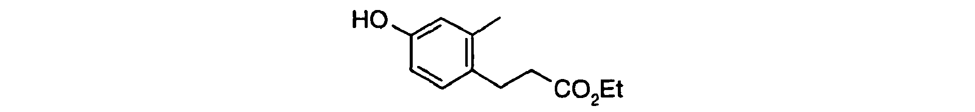

Palladium (1.2 g, 10% on activated carbon, 1.127 mmol) is added to a solution of the benzyloxyphenol (6.5 g, 21.96 mmol) in EtOH (120 mL), and the reaction mixture is stirred under H₂ atmosphere (H₂ balloon) overnight (c.a. 14 h). The reaction mixture is filtered trough Celite, and the solvent is removed in a rotatory evaporator. The crude residue is flash chromatographed on SiO₂ (5-10-15% EtOAc/hexanes) to afford 4.20 g of the title compound (92%, white solid).

### Step D

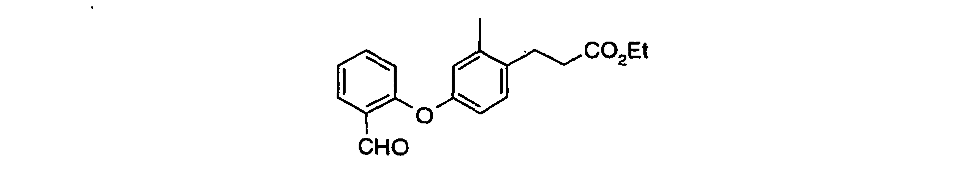

A mixture of 2-fluorobenzaldehyde (2.35 g, 18.934 mmol), the compound obtained from Step C (3.5 g, 16.827 mmol) and K₂CO₃ (1.35 g, 9.76 mmol) in anhydrous DMF (30 mL) is warmed to 140°C, and the mixture is stirred at that temperature for 2 h. It is allowed to reach r.t. and poured into brine. The organic layer is diluted with EtOAc, washed with brine and water, and then dried, filtered and concentrated. The resulting crude residue is flash chromatographed on SiO₂ (3-5% EtOAc/hexanes) to afford 2.38 g of the substitution product (45%, colorless oil).

### Step E

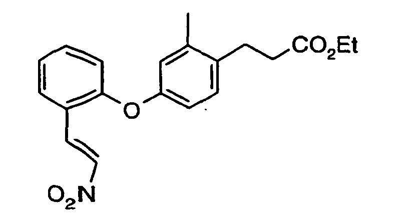

Nitromethane (1 mL, 18.46 mmol) is added to a suspension of aldehyde compound obtained from Step D (560 mg, 1.795 mmol) and ammonium acetate (500 mg, 6.486 mmol) in AcOH (glacial, 12 mL), and the mixture is warmed to 115°C. After 5 h, it is allowed to reach r.t., and then neutralized with NaHCO₃ (s) and partitioned between EtOAc and H₂O. The organic layer is dried, filtered and concentrated to give a crude residue that is flash chromatographed on SiO₂ (3% EtOAc/hexanes) affording 395g of the condensation product (62%, colorless oil).

### Step F

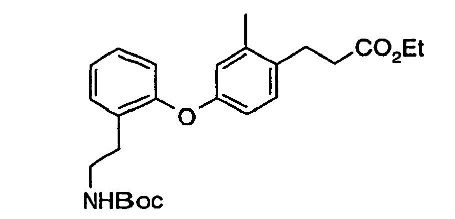

Palladium (100 mg, 10% on activated carbon, 0.094 mmol) is added to a solution of the nitroalkene obtained from Step E (400 mg, 1.1267 mmol) in EtOH (15 mL) and concentrated HCl (1 mL). The mixture is stirred under H₂ atmosphere (H₂ balloon) overnight (c.a. 14 h) and filtered trough Celite. The solvent is removed in a rotatory evaporator. The crude residue is dissolved in CH₂Cl₂ (20 mL) and Boc₂O (1 g), and Et₃N (1.5 mL) are added. The mixture is stirred at r.t. for 2 h, diluted with CH₂Cl₂ and washed with HCl (3% aqueous solution). The organic layer is dried, filtered and concentrated to give a crude that is flash chromatographed on SiO₂ (5% EtOAc/hexanes) to afford 302 mg of the title compound (63%, colorless oil).

### Intermediate 57

### 2-[4-(3-amino-5-flurorphenoxy)-2-methylphenoxy]-2-methylpropionic acid ethyl ester

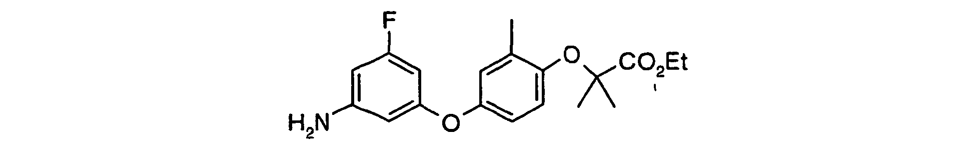

### Step A

### 2-[4-(3-nitro-5-flurorphenoxy)-2-methylphenoxy]-2-methylpropionic acid ethyl ester

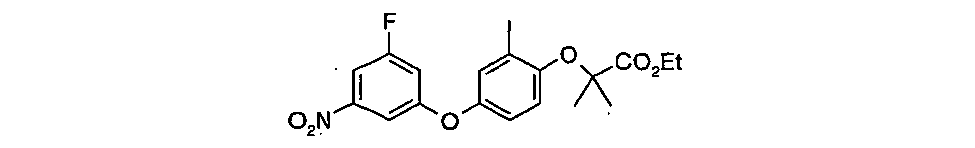

The compound of 3,5-difluoronitrobenzene (1.477 g, 9.28 mmol) is added to a suspension of compound (a) from Intermediate 55, Step A (2 g, 8.4 mmol), KF (4.5 g, 40% in Al₂O₃) and 18-crown-6 (210 mg, 0.8 mmol) in CH₃CN (40 mL). The mixture is warmed to reflux and stirred for 4 h. It is allowed to reach r.t,. and partitioned between EtOAc and brine. The organic layer is dried, filtered and concentrated to give a crude residue that is flash chromatographed on SiO₂ (3-5% EtOAc/hexanes) to afford 2.4 g of the coupling product (76%, colorless oil).

### Step B

### 2-[4-(3-amino-5-flurorphenoxy)-2-methylphenoxy]-2-methylpropionic acid ethyl ester

Palladium on carbon (300 mg, 10% Pd on activated C, 0.282 mmol) is added to a solution of nitro compound obtained from Step A (2.4 g, 6.366 mmol) in MeOH (80 mL). The mixture is stirred at r.t. for 2 h under H₂ atmosphere (balloon), and filtered trough Celite (EtOAc washings). The solvent is removed in rotary evaporator, and the product is purified by flash chromatography on SiO₂ (10% EtOAc/hexanes) to afford 2.0 g of title compound (90%, colorless oil).

### Intermediate 58

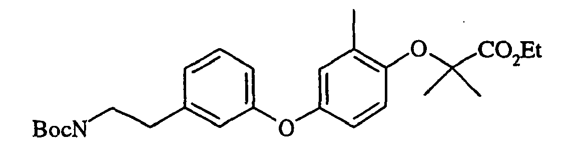

### Step A

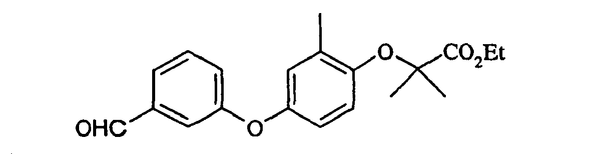

The compound (a) from Intermediate 55, Step A (1.6 g, 6.722 mmol) and boronic aldehyde (2 g, 13.35 mmol) are added to a suspension of Cu(OAc)₂ (2.45 g, 13.488 mmol) in a mixture of pyridine (5.4 mL) and CH₂Cl₂ (40 mL). The mixture is stirred at r.t. under air atmosphere in the presence of sieves for 36 h. It is filtered through Celite (CH₂Cl₂ washings) and partitioned between CH₂Cl₂ and diluted HCl (3% aqueous solution). The organic layer is dried, filtered and concentrated to give a crude residue that is flash chromatographed on SiO₂ (5-10% EtOAc/hexanes) affording 0.525 mg of unreacted starting compound (a) and 700 mg of the coupling product (30%, colorless oil).

### Step B

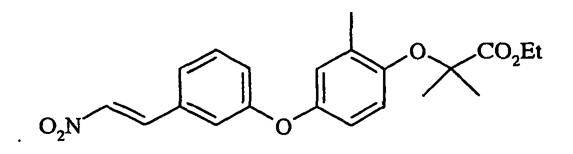

Nitromethane (4 mL, 73.84 mmol) is added to a suspension of aldehyde obtained from Step A (1.3 g, 3.8 mmol) and ammonium acetate (1.2 g, 15.566 mmol) in AcOH (glacial, 15 mL), and the mixture is warmed to 110°C. After 4 h 30 min., it is allowed to reach r.t., neutralized with NaHCO₃ (s) and partitioned between EtOAc and H₂O. The organic layer is dried, filtered and concentrated to give a crude residue that is flash chromatographed on SiO₂ (10% EtOAc/hexanes) affording 980 mg of the title compound (67%, colorless oil).

### Step C

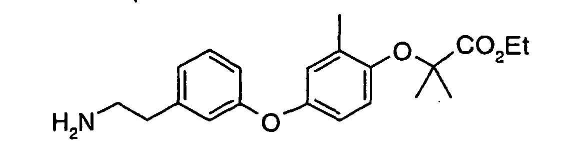

Palladium (100 mg, 10% on activated carbon, 0.094 mmol) is added to a solution of the nitroalkene obtained from Step B (1.0 g, 2.597 mmol) in a mixture of MeOH (25 mL) and concentrated HCl (1 mL). The mixture is stirred under H₂ atmosphere (H₂ balloon) overnight (c.a. 14 h) and filtered trough Celite. The solvent is removed in a rotatory evaporator. The crude residue is flash chromatographed on SiO₂ (10-20-30% iPrOH, 0.2% DIPEA/hexanes) to afford 200 mg of the title compound (22%, colorless oil).

### Step D

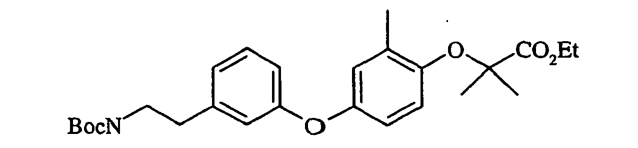

Boc₂O (165 mg, 0.7575 mmol) and Et₃N (0.25 mL, 1.82 mmol) are added to a solution of the amine obtained from Step C (180 mg, 0.606 mmol) in CH₂Cl₂ (12 mL). The mixture is stirred at r.t. for 15 min., diluted with CH₂Cl₂ and extracted with HCl (3% aqueous solution). The organic layer is dried, filtered and concentrated to give a crude residue that is flash chromatographed on SiO₂ (10-20% EtOAc/hexanes) affording 140 mg of the title compound (58%, colorless oil).

### Intermediate 59

### 3-(2-ethyl-4-hydroxy-phenyl)-propionic acid ethyl ester

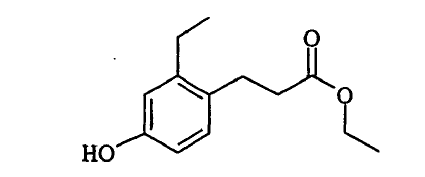

### Step A: Benzyl protection of 3-ethyl phenol

The 3-ethyl phenol (244g, 2.0 mol, 1.0 eq) and benzyl bromide (350.6 g, 2.05 mol, 1.025 eq.) are stirred in a 5-liter flask with 1.0 Liter DMF. A 15°C bath is applied to keep the reaction temperature at 20-25°C. The potassium carbonate (359.6g, 2.6 mol, 1.3 eq) is added with 350 ml DMF, and the mixture is stirred over night (14 hours) at 20-25°C. Tert-butyl methyl ether (MTBE, 650 ml) is added to the mixture. The solids in the mixture are filtered over a Hyflo pad, and are washed with 650 ml MTBE. The filtrate is washed with a solution 1150 ml 0.35 N HCl. The aqueous layer is back extracted with 500 ml MTBE, and the combined organic layers are washed with 500 ml water and then with 500 ml saturated NaCl solution. The organic layer is dried over magnesium sulfate, and then filtered and evaporated at 40°C on a rotary evaporator to afford about 424 g light yellow fluid oil.

### Step B: N-Bromination

The starting material (420g, 1.98 mol uncorr., 1 eq) and 1800 ml of acetonitrile are added to a 5L flask and stirred under nitrogen atmosphere. The N-bromosuccinimide (341.8 g, 1.92 mol, 0.97 eq) is added in four to five portions over 15-20 minutes. A 15°C water bath is applied to the flask to keep the temperature at less than 40°C. The NBS is rinsed into flask with 200 ml ACN. The reaction is stirred for 2 h, over which time the temperature decreased from 37°C to 20°C. The mixture is transferred to a rotary evaporator and evaporated to a mushy oil (app. 900 g). To this oil is added 2000 ml of MTBE, and the mixture is transferred to a separatory funnel. This is washed once with 750 ml of 1N HCl, once with 750ml of 0.3 N HCl, and once with 500 ml of saturated NaCl solution. The organic layer is dried over MgS04, and then evaporated on a 40°C bath *in vacuo* to afford about 567.8 g of light orange oil.

### Step C: Formylation

The starting material oil (509.2g, 1.75 mol, 1 eq) is dissolved in 1.0 L of THF and dried over night over 4 Å molecular sieves. The solution is filtered into a 12-L 4 neck flask, and rinsed in with 3.0 L THF. Under nitrogen atmosphere, the mixture is cooled to -70 to -72°C. n-Butyl Lithium, 2.5M in hexane (800ml, 2.0 mol, 1.15 eq) are added slowly over 1 hour 40 minutes while keeping the temperature below -68°C. The reaction is then stirred for 5 minutes, and then dry DMF (1L, 12.9 mol, 7.37 eq) is added as quickly as possible, while keeping the temperature below -48°C. The cooling is removed, and the reaction is warmed to -14°C. A solution of 1 N HCl (3.0 L) is added over 3 minutes. The mixture is transferred to a separatory flask and then extracted with 4L diethyl ether, and then with 3.5L diethyl ether. The combined ether layers are washed with a solution of 1L 1N HCl, 1L saturated NaCl solution and 2 L saturated NaCl solution. The ether solution is dried over 500 g magnesium sulfate and then filtered, washed with ether, and evaporated on 40°C bath to a constant weight to afford about 417.4 g brown oil (99.4% weight yield).

### Step D: Horner-Emmons

To a 12 flask fitted with a heating mantle, nitrogen atmosphere, and a stirrer is added 4 L ethanol (2B-3). The potassium carbonate (679.1 g, 4.01 mol, 3.15 equivalents), the aldehyde (375 g, 1.56 mol, 1 equiv.), and triethylphosphonoacetate (454.7 g, 2.03 mol, 1.3 eq) are added to the 12L flask, and then rinsed in with a total of 900 ml ethanol. The reaction is heated to reflux over 30 min., and then refluxed for 2h and 15 min., and then allowed to cool to rt. The mixture is filtered to remove the potassium salts, and the filtrate is evaporated to 1270 g to remove most of the ethanol. The oil is transferred to a separatory flask and added 4L ethyl acetate and washed with 5L 0.6 N HCl. The aqueous layer is back extracted with 4L 0.1N HCl, and then with 2L saturated NaCl solution. The organic layer is dried over magnesium sulfate, filtered, and then evaporated to a dark brown oil on a 40 bath to afford about 565 g.

### Step E: Hydrogenation

The starting material oil, 1000g (Theo. 2.62 mol from HEW), is dissolved in 4L ethyl acetate and added to a 3-gallon autoclave. About 250 g (42% water wet) of 10% palladium on carbon is added. The mixture is hydrogenated at 50 psi hydrogen pressure at 50°C. After the reaction is completed, the mixture is cooled to 20-25°C and held under 50 psi hydrogen pressure over night. The catalyst is filtered and washed with 1.5 L ethyl acetate. The solution is evaporated on 40°C bath to yield about 732.2 g of the crude final compound.

The final compound is purified as described below. A 5 kg Biotage Flash Chromatography column is pre-conditioned with 10L of 95:5 heptane:ethyl acetate. About 250 g of crude final product (120 wt% yield from Step 4 SM, GC 70%) is loaded on the column with 250 ml 10:1 1 heptane:ethyl acetate. The column is eluted with 20L of 95:5 heptane:ethyl acetate (4 L fractions) prior to impurities being eluted; then with 20 L of 95:5 heptane:ethyl acetate (2 L fractions). Fractions are analyzed by TLC (silica; 4:1 heptane:ethyl acetate) and/or by GC. At this point, the fractions contained <95% pure product. The elution is continued with 40 L of 80:20 heptane:ethyl acetate, with 4 L fractions being taken. The fractions containing the final product of <95% purity (by GC) are combined and evaporated to a clear oil (colorless to light yellow). (131.4g, 52.5% weight recovery, 63.1% yield from Step D, starting material of aldehyde)

### Intermediate 60

### 3-(4-Hydroxy-phenyl)-2,2-dimethyl-propionic acid methyl ester

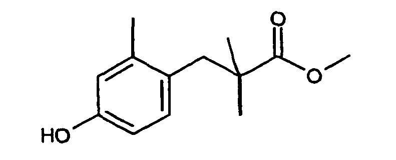

### Step A

### 2-Methyl-4-anisaldehyde

A mixture of 2,3-dimethylanisole (50g, 0.37 mol), Cu2+ sulfate pentahydrate (90 g, 0.36 mol), and potassium peroxydisulfate (301 g, 1.11 mol) in acetonitrile/water (1:1, 2.6 L) is stirred vigorously and heated to reflux for 30 minutes. The reaction is cooled to rt and extracted with CH₂Cl₂ (4L) and washed with water (2L). The layers are separated, and the aqueous layer is again extracted with CH₂Cl₂. The organic layers are combined and concentrated to afford about 55 g (~100%) product, which is taken on as is. 1H-NMR (DMSO-d6): 10.05 (s, 1H), 7.78 (m, 1H), 6.95 (m, 1H), 6.88 (s, 1H), 3.84 (s, 3H), 2.6 (s, 3H).

### Step B

### 4-Methoxy-2-methylbenzyl alcohol

NaBH₄ (14.82 g, 0.39 mol) is added to a solution of the compound from Step A (55 g, 0.37 mol) in EtOH (800 mL). The reaction is quenched with water (3L), acidified with 5N HCl, and extracted with Et₂O. The organics are separated and concentrated. The crude product is purified by Biotage 75L (Hexane:EtOAc, 9:1) to afford about 17.35 g (30%). 1H-NMR (CDCl₃): 7.22 (m, 1H), 6.7 (m, 2H), 4.64 (s, 2H), 3.8 (s, 3H), 2.4 (s, 3H).

### Step C

### Acetic acid 4-methoxy-2-methyl-benzyl ester

A solution of compound from Step B (17.35 g, 0.114 mol) in CH₂Cl₂ (900 mL) is cooled 0°C, and TEA (23.3 mL, 0.167 mol) and acetyl chloride (9.3 mL, 0.131 mol) are added. The reaction is stirred for 1 h and then quenched with 1N HCl, washed with aq. NaHCO₃, brine, dried (Na₂SO₄), and concentrated to afford an oil (22.14 g, ~100%). 1H-NMR (CDCl₃): 7.24 (m, 1H), 6.73 (m, 2H), 5.08 (s, 2H), 3.8 (s, 3H), 2.33 (s, 3H), 2.08 (s, 3H).

### Step D

### 3-(4-Methoxy-2-methyl-phenyl)-2,2-dimethyl-propionic acid methyl ester

The compound from Step C (22.14 g, 0.114 mol) is dissolved in CH₂Cl₂ and treated with 1-methoxy-1-trimethylsiloxy-2-methyl-1-propene (53.3 g, 0.306 mol) and Mg(ClO₄)₂ (2.58 g, 0.012 mol). The reaction is stirred overnight at rt. Upon completion, the reaction is washed with water, brine, and dried with Na₂SO₄. The crude product is purified (Biotage 75M (Hexane:EtOAc, 9:1® 8:2)) to obtain about 18.7 g (70%). 1H-NMR (CDCl₃): 6.97 (d, 1H), 6.7 (m, 2H), 3.8 (s, 3H), 3.64 (s, 3H), 2.85 (s, 2H), 2.3 (s, 3H), 1.2 (s, 6H).

### Step E

### 3-(4-Hydroxy-phenyl)-2,2-dimethyl-propionic acid methyl ester

BBr₃ (1M in CH₂Cl₂, 79 ml) is cooled to 0°C, and the compound from Step D (9.35 g, 0.0395 mol) is added dropwise over 10 minutes. After stirring for 1 h at 0°C, the reaction is quenched with 1:1 MeOH: CH₂Cl₂. The organics are concentrated, and the resulting oil is run through a plug of silica gel with Hexane:EtOAc (8:2). Fractions 1,2 are concentrated and about 7.5 g (85%) of the desired compound are isolated. 1H-NMR (CDCl₃): 6.87 (d, 1H), 6.6 (m, 2H), 4.9 (bs, 1H), 3.64 (s, 3H), 2.82 (s, 2H), 2.22 (s, 3H), 1.2 (s, 6H).

### Intermediate 61

### 3-[4-(3-Aminomethyl-5-chloro-phenoxy)-2-methyl-phenyl]-propionic acid methyl ester

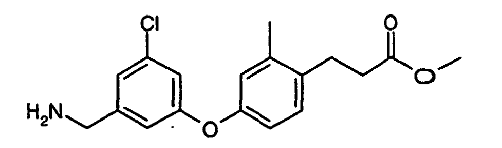

The title compound is prepared according to the procedure described in Intermediate 33 utilizing 3-chloro-5-fluoro-benzonitrile and 3-(4-hydroxy-2-methylphenyl)-propionic acid methyl ester (J. Chem.Soc.Perkin Trans- 1; 4; 1990; 1041-1045). MS: (ES+) 334 (M+H⁺); 1H NMR (400 MHz, CDCl₃).

### Example 1

### General Procedures for Coupling and Hydrolysis

### Step A-Coupling Step

Acid (1.0 eq), amine (1.0-1.5 eq), 1-(3-dimethylaminopropyl)-3-ethylcarbodiimide hydochloride (1.2-1.5 eq), 1-hydroxybenzotriazole hydrate 0.2-1.5 eq), and *N,N*-diisopropylethylamine (1.0 eq) are stirred overnight under nitrogen at rt in dry THF or dry DMF. Water is added, extracted with ethyl acetate, washed with 1 N HCl, then saturated sodium bicarbonate, followed by brine, dried over sodium sulfate and concentrated under reduced pressure. Purification by flash chromatography, eluting with 10-15 % EtOAc in hexane then 25 % EtOAc in hexane provides the intermediate ester shown in the above general procedure.

### Step B-Hydrolysis Step

Compound from Step A (1 eq) is hydrolyzed in dioxane/water (2:1 v/v, ~0.02M) with lithium hydroxide hydrate (7-13 eq). Reaction is stirred at rt overnight under nitrogen, and then acidified with 5 N HCl. Water is added, extracted with EtOAc, washed with brine, dried over sodium sulfate and concentrated under reduced pressure to give the final acid.

### Example 2

### 3-(2-Ethyl-4-{3-fluoro-5-[1-(2-methyl-4-trifluoromethyl-benzoylamino)-ethyl]-phenoxy}-phenyl)-propionic acid

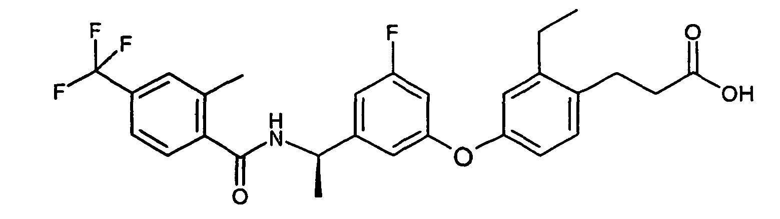

### Step A

### 3-(2-Ethyl-4-{3-fluoro-5-[1-(2-methyl-4-trifluoromethyl-benzoylamino)-ethyl]-phenoxy}-phenyl)-propionic acid ethyl ester

2-Methyl-4-trifluoromethyl-benzoic acid (41.3 mg, 0.20 mmol), 3-{4-[3-(1-amino-ethyl)-5-fluoro-phenoxy]-2-ethyl-phenyl}-propionic acid ethyl ester (Intermediate 23) (80.0 mg, 0.23 mmol), 1-(3-dimethylaminopropyl)-3-ethylcarbodiimide hydochloride (46.5 mg, 0.24mmol), 1-hydroxybenzotriazole hydrate (32.8 mg, 0.24 mmol), and *N,N* diisopropylethylamine (0.035 mL, 0.20 mmol) are stirred overnight under nitrogen at rt in dry THF (8 mL). Water is added, extracted with ethyl acetate, washed with 1 N HCl, then saturated sodium bicarbonate, followed by brine, dried over sodium sulfate and concentrated under reduced pressure. Purification by flash chromatography, eluting with 15 % EtOAc in hexane then 25 % EtOAc in hexane provides the title compound (84.1 mg, 76 %). MS(ES⁺): 546 (M+H⁺); ¹HNMR (400 MHz, CDCl₃)

### Step B

### 3-(2-Ethyl-4-{3-fluoro-5-[1-(2-methyl-4-trifluoromethyl-benzoylamino)-ethyl]-phenoxy}-phenyl)-propionic acid

Compound of Step A (84.1 mg, 0.15 mmol) is dissolved in dioxane (6 mL) and lithium hydroxide hydrate (85 mg, 2.02 mmol), dissolved in water(3 mL), is added. Reaction is stirred at rt overnight under nitrogen, then acidified with 5 N HCl. Water is added, extracted with EtOAc, washed with brine, dried over sodium sulfate and concentrated under reduced pressure to give the title compound (66.4 mg, 84 %). Exact mass calculated for C₂₈H₂₈F₄NO₄ (M+H+) 518.1954, found 518.1956; ¹NMR (400 MHz, CDCl3).

### Example 3

### 2-(4-{3-[(2,4-Bis-trifluoromethyl-benzoylamino)-methyl]-phenoxy} -2-methyl-phenoxy)-2-methyl-propionic acid

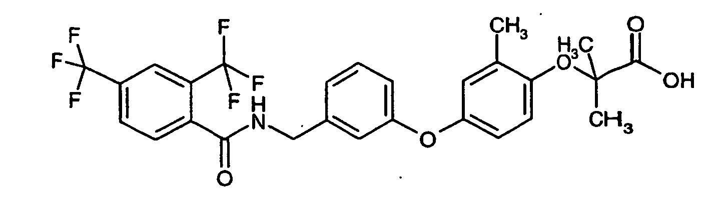

The title compound is prepared according to Example 1 utilizing 2,4-bis-trifluoromethyl-benzoic acid and 2-[4-(3-aminomethyl-phenoxy)-2-methyl-phenoxy]-2-methyl-propionic acid ethyl ester (Intermediate 17). Exact mass calcd for C₂₇H₂₄F₆NO₅ (M+H⁺): 556.1559, found 556.1561; ¹HNMR (400 MHz, CDCl₃).

### Example 4

### 2-(4-{3-[(2,4-Dimethyl-benzoylamino)-methyl]-phenoxy}-2-methyl-phenoxy)-2-methyl-propionic acid

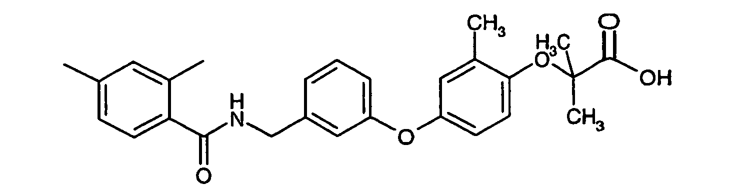

The title compound is prepared according to Example 1 utilizing 2,4-dimethyl-benzoic acid and 2-[4-(3-aminomethyl-phenoxy)-2-methyl-phenoxy]-2-methyl-propionic acid ethyl ester (Intermediate 17). Exact mass calcd for C₂₇H₃₀NO₅ (M+H⁺): 448.2240, found 448.2118; ¹H NMR (400 MHz, CDCl₃).

### Example 5

### 2-Methyl-2-(4-{3-[(2-methyl-4-trifluoromethyl-benzoylamino)-methyl]-phenoxy}-phenoxy)-propionic acid

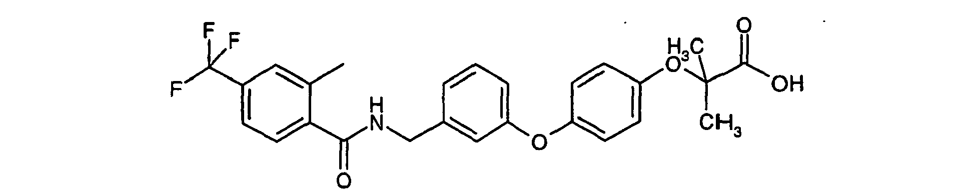

The title compound is prepared according to Example 1 utilizing 2-methyl-4-trifluoromethyl-benzoic acid (Intermediate 10) and 2-[4-(3-aminomethyl-phenoxy)-phenoxy]-2-methyl-propionic acid (Intermediate 49). Exact mass calcd for C₂₆H₂₅F₃NO₅ (M+H⁺): 488.1685, found 488.1674; ¹H NMR (400 MHz, CDCl₃).

### Example 6

### 2-Methyl-2-(4-{3-[(4-trifluoromethyl-benzoylamino)-methyl]-phenoxy}-phenoxy)-propionic acid

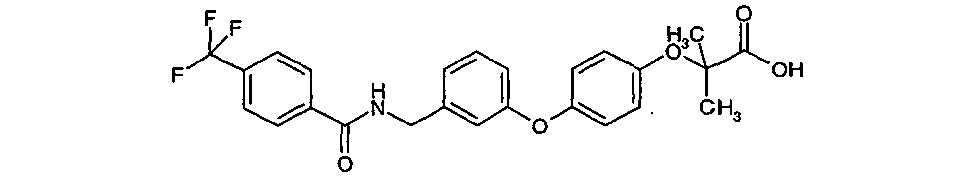

The title compound is prepared according to Example 1 utilizing 4-trifluoromethyl-benzoic acid and 2-[4-(3-aminomethyl-phenoxy)-phenoxy]-2-methyl-propionic acid (Intermediate 49). Exact mass calcd for C₂₅H₂₃F₃NO₅ (M+H⁺): 474.1588, found 474.1505; ¹H NMR (400 MHz, CDCl₃).

### Example 7

### 2-Methyl-2-(2-methyl-4- {3-[(2-methyl-4-trifluoromethyl-benzoylamino)-methyl]-phenoxy}-phenoxy)-propionic acid

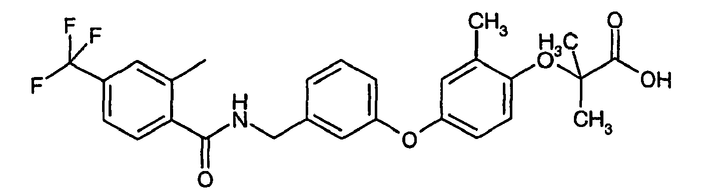

The title compound is prepared according to Example 1 utilizing 2-methyl-4-trifluoromethyl-benzoic acid (Intermediate 10) and 2-[4-(3-aminomethyl-phenoxy)-2-methyl-phenoxy]-2-methyl-propionic acid ethyl ester (Intermediate 17). Exact mass calcd for C₂₇H₂₇F₃NO₅ (M+H⁺): 502.1841, found 502.1844; ¹H NMR (400 MHz, CDCl₃).

### Example 8

### 2-{4-[3-(Isopropoxycarbonylamino-methyl)-phenoxy]-2-methyl-phenoxy}-2-methyl-propionic acid

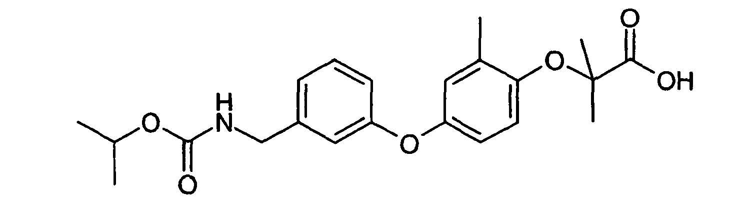

The title compound is prepared according to the general procedures described in Example 140 utilizing isopropyl chloroformate and 2-[4-(3-aminomethylphenoxy)-2-methylphenoxy]-2-methylpropionic acid ethyl ester (Intermediate 17). Mass (ES⁺): 402.3 (M+H⁺).

### Example 9

### 2-Methyl-2-(2-methyl-4-{3-[(2-methyl-4-trifluoromethoxy-benzoylamino)-methyl]-phenoxy}-phenoxy)-propionic acid

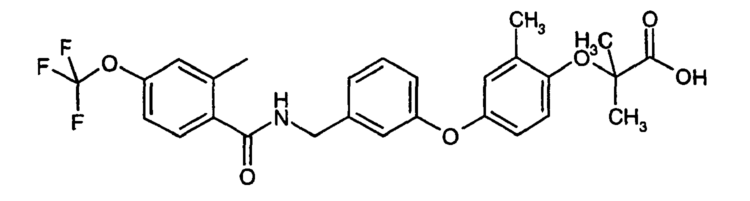

### Step A

### 2-Methyl-2-(2-methyl-4-{3-[(2-methyl-4-trifluoromethoxy-benzoylamino)-methyl]-phenoxy}-phenoxy)-propionic acid ethyl ester

The compound of 2-methyl-4-trifluoromethoxy-benzoic acid (30.0 mg, 0.14 mmol) (Intermediate13),2-[4-(3-aminomethyl-phenoxy)-2-methyl-phenoxy]-2-methyl-propionic acid ethyl ester (Intermediate 17) (47.0.mg, 0.14 mmol), *o*-(7-azobenzotriazol-1-yl)-*N,N,N',N'*-tetramethyluronium hexafluorophosphate (62.0 mg, 0.16 mmol), and *N,N-*diisopropylethylamine (0.023 mL, 0.14 mmol) are stirred overnight under nitrogen at rt in DCM (3 mL) and dry DMF (0.5 mL). Water is added, and the mixture is extracted with ethyl acetate. The mixture is washed with 1 N HCl and saturated sodium bicarbonate and brine, and then dried over sodium sulfate and concentrated under reduced pressure. Purification by flash chromatography, eluting with 15 % EtOAc in hexane and then 25 % EtOAc in hexane provides the title compound (41 mg, 55 %). MS(ES⁺): 546 (M+H⁺); ¹HNMR (400 MHz, CDCl₃).

### Step B

### 2-Methyl-2-(2-methyl-4-{3-[(2-methyl-4-trifluoromethoxy-benzoylamino)-methyl]-phenoxy}-phenoxy)-propionic acid

Compound of Step A (41.0 mg, 0.075 mmol) is dissolved in dioxane (4 mL) and lithium hydroxide hydrate (62.0 mg, 1.5 mmol), dissolved in water (2 mL), is added. Reaction is stirred at rt overnight under nitrogen, and then acidified with 5 N HC1. Water is added and the mixture is extracted with EtOAc, which is washed with brine, dried over sodium sulfate and concentrated under reduced pressure to give the title compound (35.0 mg, 90 %). Exact mass calcd for C₂₇H₂₇F₃NO₆ (M+H⁺): 518-1790, found 518.1797; ¹H NMR (400 MHz, CDCl₃).

### Example 10

### 2-(4-{3-[(4-Methoxy-2-methyl-benzoylamino)-methyl]-phenoxy}-2-methyl-phenoxy)-2-methyl-propionic acid

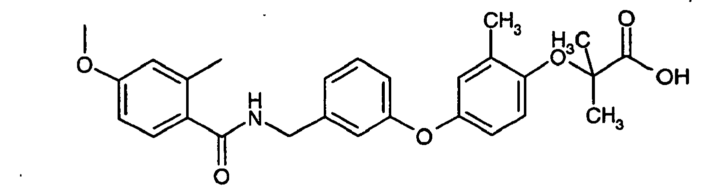

The title compound is prepared according to Example 1 utilizing 2-methyl-4-methoxy-benzoic acid and 2-[4-(3-aminomethyl-phenoxy)-2-methyl-phenoxy]-2-methyl-propionic acid ethyl ester (Intermediate 17). Exact mass calcd for C₂₇H₃₀NO₆ (M+H⁺): 464.2073, found 464.2082; ¹H NMR (400 MHz, CDCl₃).

### Example 11

### 2-(4- {3-[(3,4-Dimethyl-benzoylamino)-methyl]-phenoxy} -2-methyl-phenoxy)-2-methyl-propionic acid

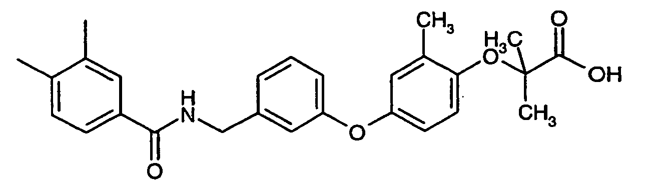

The title compound is prepared according to Example 1 utilizing 3,4-dimethyl-benzoic acid and 2-[4-(3-aminomethyl-phenoxy)-2-methyl-phenoxy]-2-methyl-propionic acid ethyl ester (Intermediate 17). Exact mass calcd for C₂₇H₃₀NO₅ (M+H⁺): 448.2124, found 448.2126; ¹H NMR (400 MHz, CDCl₃).

### Example 12

### 2-(4-{3-[(2,4-Dichloro-benzoylamino)-methyl]-phenoxy}-2-methyl-phenoxy)-2-methyl-propionic acid

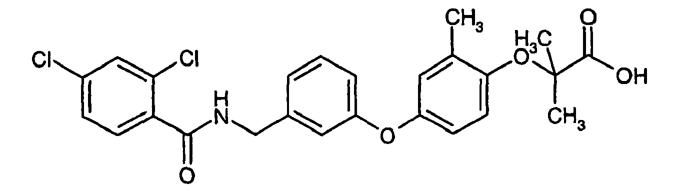

The title compound is prepared according to Example 1 utilizing 2,4-dichloro-benzoic acid and 2-[4-(3-aminomethyl-phenoxy)-2-methyl-phenoxy]-2-methyl-propionic acid ethyl ester (Intermediate 17). Exact mass calcd for C₂₅H₂₄Cl₂NO₅ (M+H⁺): 488.1032, found 488.1041; ¹H NMR (400 MHz, CDCl₃).

### Example 13

### 2-(4-{3-[(4-Ethyl-benzoylamino)-methyl]-phenoxy}-2-methyl-phenoxy)-2-methyl-propionic acid

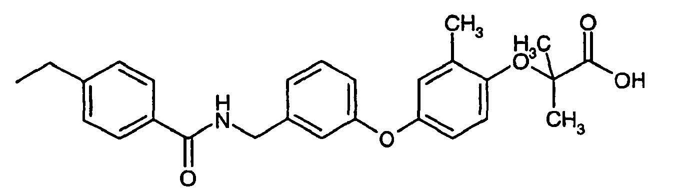

The title compound is prepared according to Example 1 utilizing 4-ethyl-benzoic acid and 2-[4-(3-aminomethyl-phenoxy)-2-methyl-phenoxy]-2-methyl-propionic acid ethyl ester (Intermediate 17). Exact mass calcd for C₂₇H₃₀NO₅ (M+H⁺): 448.2124, found 448.2099; ¹H NMR (400 MHz, CDCl₃).

### Example 14

### 2-(4-{3-[(2-Fluoro-4-trifluoromethyl-benzoylamino)-methyl]-phenoxy}-2-methyl-phenoxy)-2-methyl-propionic acid

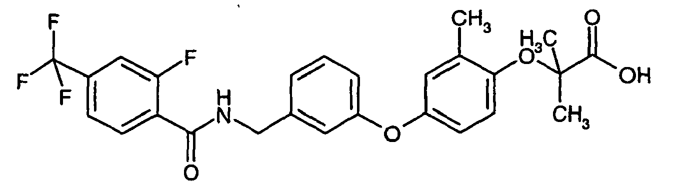

The title compound is prepared according to Example 1 utilizing 2-fluoro-4-trifluoromethyl-benzoic acid and 2-[4-(3-aminomethyl-phenoxy)-2-methyl-phenoxy]-2-methyl-propionic acid ethyl ester (Intermediate 17). Exact mass calcd for C₂₆H₂₄F₄NO₅ (M+H⁺): 506.1591, found 506.1581; ¹H NMR (400 MHz, CDCl₃).

### Example 15

### 2-Methyl-2-(2-methyl-4-(3-[(2-methyl-benzoylamino)-methyl]-phenoxy}-phenoxy)-propionic acid

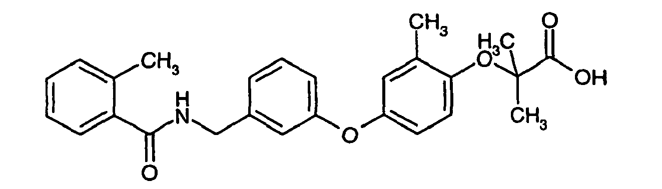

The title compound is prepared according to Example 1 utilizing 2-methylbenzoic acid and 2-[4-(3-aminomethyl-phenoxy)-2-methyl-phenoxy]-2-methyl-propionic acid ethyl ester (Intermediate 17). Exact mass calcd for C₂₆H₂₈NO₅ (M+H⁺): 434.1967, found 434.1954; ¹H NMR (400 MHz, CDCl₃).

### Example 16

### 2-Methyl-2-(2-methyl-4-{3-[(2-nitro-4-trifluoromethyl-benzoylamino)-methyl]-phenoxy}-phenoxy)-propionic acid

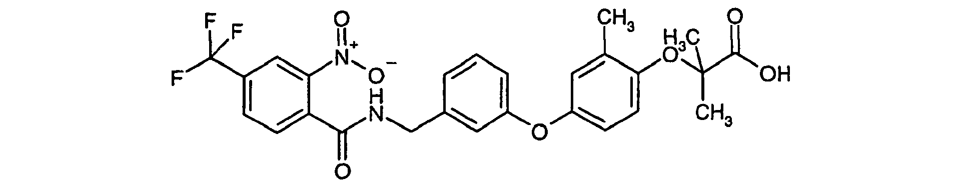

The title compound is prepared according to Example 1 utilizing 2-nitro-4-triflouromethyl-benzoic acid (US Pat. No. 4,868,833) and 2-[4-(3-aminomethyl-phenoxy)-2-methyl-phenoxy]-2-methyl-propionic acid ethyl ester (Intermediate 17). Exact mass calcd for C₂₆H₂₄F₃N₂O₇ (M+H⁺): 533.1536, found 533.1518; ¹H NMR (400 MHz, CDCl₃).

### Example 17

### 2-(4-{3-[(4-Acetyl-benzoylamino)-methyl]-phenoxy}-2-methyl-phenoxy)-2-methyl-propionic acid

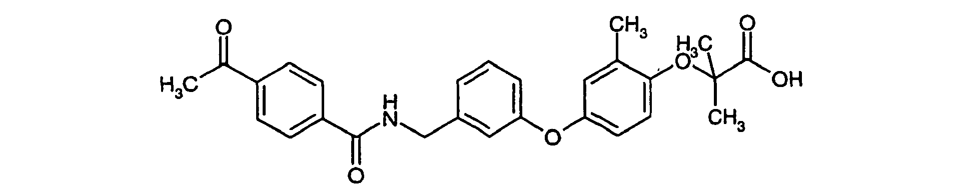

The title compound is prepared according to Example 1 utilizing 4-acetylbenzoic acid and 2-[4-(3-aminomethyl-phenoxy)-2-methyl-phenoxy]-2-methyl-propionic acid ethyl ester (Intermediate 17). Exact mass calcd for C₂₇H₂₈NO₆ (M+H⁺): 462.1897, found 462.1917; ¹H NMR (400 MHz, CDCl₃).

### Example 18

### 2-Methyl-2-[2-methyl-4-(3-{[2-(4-trifluoromethyl-phenyl)-acetylamino]-methyl}-phenoxy)-phenoxy]-propionic acid

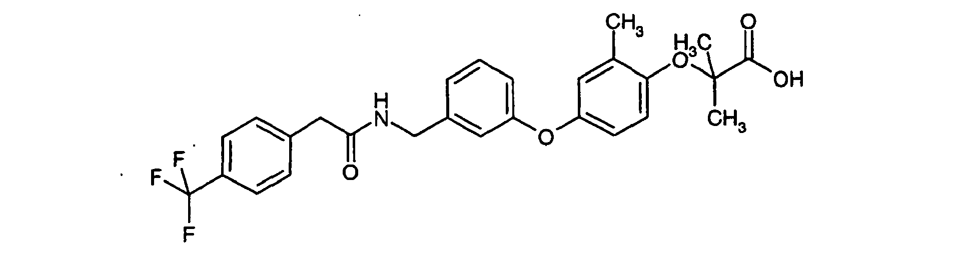

The title compound is prepared according to Example 1 utilizing (4-trifluoromethyl-phenyl)-acetic acid and 2-[4-(3-aminomethyl-phenoxy)-2-methyl-phenoxy]-2-methyl-propionic acid ethyl ester (Intermediate 17). Exact mass calcd for C₂₇H₂₇F₃NO₅ (M+H⁺): 502.1841, found 502.1832; ¹H NMR (400 MHz, CDCl₃).

### Example 19

### 2-Methyl-2-[2-methyl-4-(3-{[methyl-(2-methyl-4-trifluoromethoxy-benzoyl)-amino]-methyl }-phenoxy)-phenoxy]-propionic acid

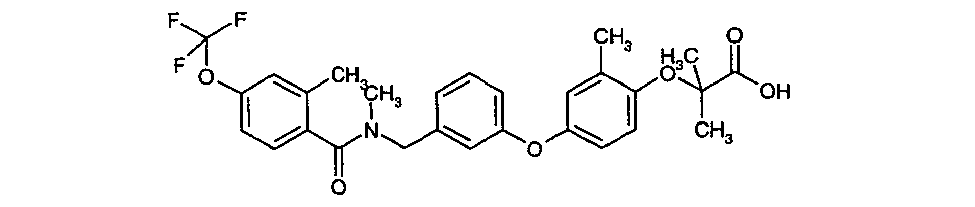

The ethyl ester of the title compound is prepared according to Intermediate 2, Step A by utilizing 2-ethyl-2-(2-methyl-4-{3-[(2-methyl-4-trifluoromethoxy-benzoylamino)-methyl]-phenoxy}-phenoxy)-propionic acid ethyl ester (Example 9, Step A), and is then hydrolyzed according to Example 9, Step B, providing the title compound. Exact mass calcd for C₂₈H₂₉F₃NO₆ (M+H⁺): 532.1947, found 532.1921; ¹H NMR (400 MHz, CDCl₃).

### Example 20

### 3-(2-Methyl-4-{3-[(2-methyl-4-trifluoromethyl-benzoylamino)-methyl]-phenoxy)-phenyl)-propionic acid

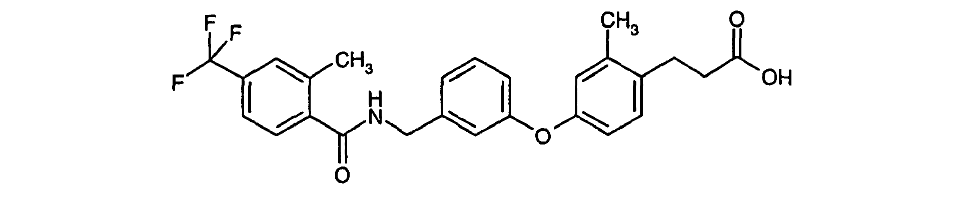

**T**he title compound is prepared according to Example 1 utilizing 2-methyl-4-trifluoromethyl-benzoic acid (Intermediate 10) and 3-[4-(3-aminomethyl-phenoxy)-2-methyl-phenyl]-propionic acid methyl ester (Intermediate 33). Exact mass calcd for C₂₆H₂₅F₃NO₄ (M+H⁺): 472.1736, found 472.1744; ¹H NMR (400 MHz, CDCl₃).

### Example 21

### 2-(4-{ 3-[(2-Methoxy-4-trifluoromethyl-benzoylamino)-methyl]-phenoxy}-2-methyl-phenoxy)-2-methyl-propionic acid

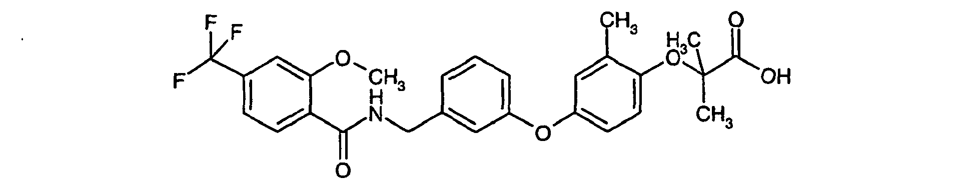

The title compound is prepared according to Example 1 utilizing 2-methoxy-4-trifluoromethyl-benzoic acid *(*J.Am.Chem.Soc.; 73; 1951; 2375) and 2-[4-(3-aminomethyl-phenoxy)-2-methyl-phenoxy]-2-methyl-propionic acid ethyl ester (Intermediate 17). Exact mass calcd for C₂₇H₂₇F₃NO₆ (M+H⁺): 518.1790, found 518.1788; ¹H NMR (400 MHz, CDCl₃).

### Example 22

### 2-(4-{3-[(2-Chloro-4-fluoro-benzoylamino)-methyl]-phenoxy}-2-methyl-phenoxy)-2-methyl-propionic acid

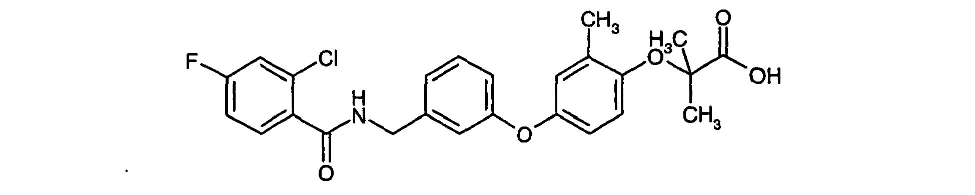

The title compound is prepared according to Example 1 utilizing 2-chloro-4-fluoro-benzoic acid and 2-[4-(3-aminomethyl-phenoxy)-2-methyl-phenoxy]-2-methyl-propionic acid ethyl ester (Intermediate 17). Exact mass calcd for C₂₅H₂₄ClFNO₅ (M+H⁺): 472.1327, found 472.1322; ¹H NMR (400 MHz, CDCl₃).

### Example 23

### 2-(4-{3-[(3-Fluoro-4-trifluoromethyl-benzoylamino)-methyl]-phenoxy}-2-methyl-phenoxy)-2-methyl-propionic acid

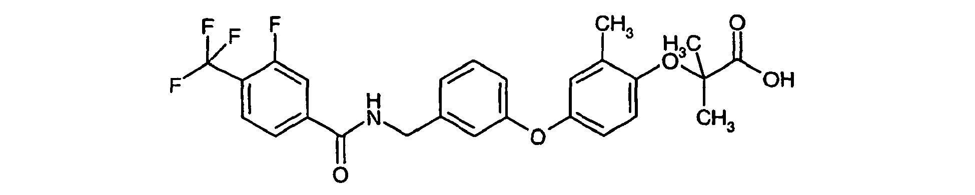

The title compound is prepared according to Example 1 utilizing 3-fluoro-4-trifluoromethyl-benzoic acid and 2-[4-(3-aminomethyl-phenoxy)-2-methyl-phenoxy]-2-methyl-propionic acid ethyl ester (Intermediate 17). Exact mass calcd for C₂₆H₂₄F₄NO₅ (M+H⁺): 506.1591, found 506.1593; ¹H NMR (400 MHz, CDCl₃).

### Example 24

### 2-(4-{3-[(2,3-Dimethyl-benzoylamino)-methyl]-phenoxy}-2-methyl-phenoxy)-2-methyl-propionic acid

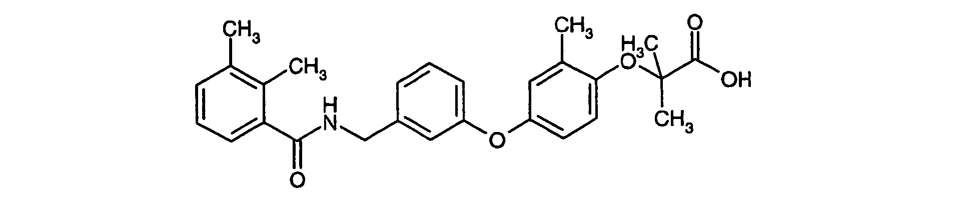

The title compound is prepared according to Example 1 utilizing 2,3-dimethyl-benzoic acid and 2-[4-(3-aminomethyl-phenoxy)-2-methyl-phenoxy]-2-methyl-propionic acid ethyl ester (Intermediate 17). Exact mass calcd for C₂₇H₃₀NO₅ (M+H⁺): 448.2124, found 448.2111; ¹H NMR (400 MHz, CDCl₃).

### Example 25

### 2-Methyl-2-(2-methyl-4- 3-[(2-methylsulfanyl-4-trifluoromethyl-benzoylamino)-methyl]-phenoxy}-phenoxy)-propionic acid

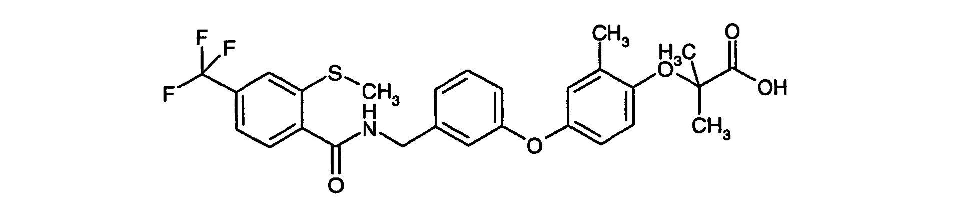

The title compound is prepared according to Example 1 utilizing 2-methylsulfanyl-4-trifluoromethyl-benzoic acid (WO 9902489) and 2-[4-(3-aminomethyl-phenoxy)-2-methyl-phenoxy]-2-methyl-propionic acid ethyl ester (Intermediate 17). Exact mass calcd for C₂₇H₂₇F₃NO₅S (M+H⁺): 534.1562, found 534.1542; ¹H NMR (400 MHz, CDCl₃).

### Example 26

### 2-(4-{3-[(2-Methanesulfinyl-4-trifluoromethyl-benzoylamino)-methyl]-phenoxy}-2-methyl-phenoxy)-2-methyl-propionic acid

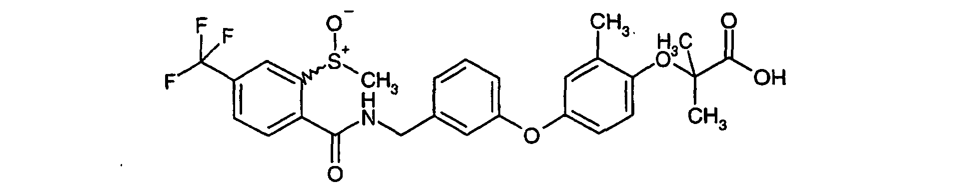

The compound of 2-methyl-2-(2-methyl-4-{3-[(2-methylsulfanyl-4-trifluoromethyl-benzoylamino)-methyl]-phenoxy}-phenoxy)-propionic acid (61.7 mg, 0.12mmol) (Example 25) is dissolved in chloroform (5 mL), cooled in an ice bath, and solid 3-chloroperoxybenzoic acid (77 %) (24.7 mg, 0.11mmol) is added. Solution is stirred for 15 minutes and quenched with water and then extracted with dichloromethane. The solution is washed with sodium bicarbonate, brine, and then dried with sodium sulfate and concentrated under reduced pressure providing the title compound (18.6 mg, 30 %) without further purification. Exact mass calcd for C₂₇H₂₇F₃NO₆S (M+H⁺): 550.1511, found 550.1490; ¹H NMR (400 MHz, CDCl₃).

### Example 27

### 2-(4-{3-[(4-Isobutyl-benzoylamino)-methyl]-phenoxy-2-methyl-phenoxy)-2-methyl-propionic acid

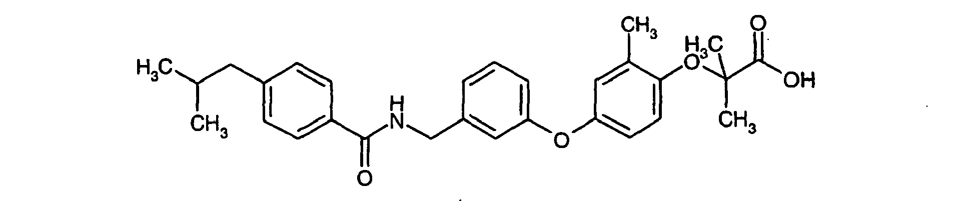

The title compound is prepared according to Example 1 utilizing 4-isobutyl-benzoic acid and 2-[4-(3-aminomethyl-phenoxy)-2-methyl-phenoxy]-2-methyl-propionic acid ethyl ester (Intermediate 17). Exact mass calcd for C₂₉H₃₄NO₅ (M+H⁺): 476.2437, found 476.2437; ¹H NMR (400 MHz, CDCl₃).

### Example 28

### 2-(4-{3-[(4-Isopropyl-benzoylamino)-methyl]-phenoxy}-2-methyl-phenoxy)-2-methyl-propionic acid

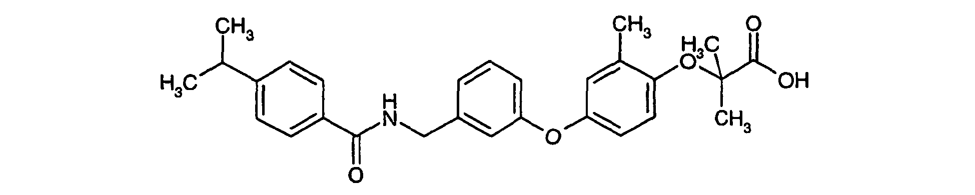

The title compound is prepared according to Example 1 utilizing 4-isopropyl-benzoic acid and 2-[4-(3-aminomethyl-phenoxy)-2-methyl-phenoxy]-2-methyl-propionic acid ethyl ester (Intermediate 17). Exact mass calcd for C₂₈H₃₂NO₅ (M+H⁺): 462.2299, found 462.2280; ¹H NMR (400 MHz, CDCl₃).

### Example 29

### 2-(4{3-[(2-Bromo-4-methyl-benzoylamino)-methyl]-phenoxy}-2-methyl-phenoxy)-2-methyl-propionic acid

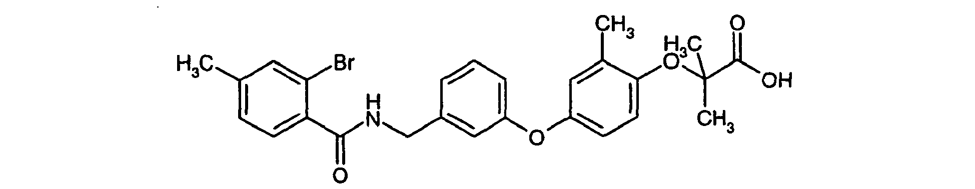

The title compound is prepared according to Example 1 utilizing 2-bromo-4-methyl-benzoic acid and 2-[4-(3-aminomethyl-phenoxy)-2-methyl-phenoxy]-2-methyl-propionic acid ethyl ester (Intermediate 17). Exact mass calcd for C₂₆H₂₇BrNO₅ (M+H⁺): 512.1073, found 512.1069; ¹H NMR (400 MHz, CDCl₃).

### Example 30

### 2-[4-(3-{[(5-Chloro-1H-indole-2-carbonyl)-amino]-methyl}-phenoxy)-2-methyl-phenoxy]-2-methyl-propionic acid

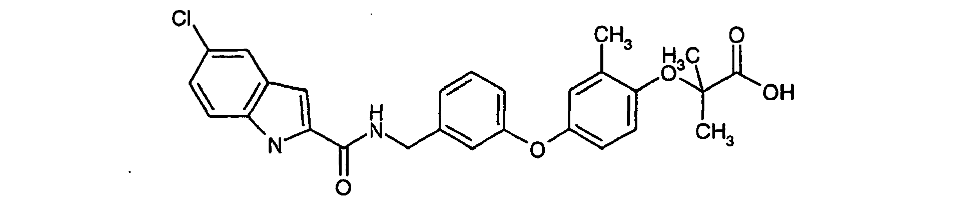

The title compound is prepared according to Example 1 utilizing 5-chloro-1H-indole-2-carboxylic acid and 2-[4-(3-aminomethyl-phenoxy)-2-methyl-phenoxy]-2-methyl-propionic acid ethyl ester (Intermediate 17). Exact mass calcd for C₂₇H₂₆ClN₂O₅ (M+H⁺): 493.1530, found 493.1547; ¹H NMR (400 MHz, CDCl₃).

### Example 31

### 3-(4- {3-[(2-Methoxy-4-trifluoromethyl-benzoylamino)-methyl]-phenoxy}-2-methylphenyl)-propionic acid

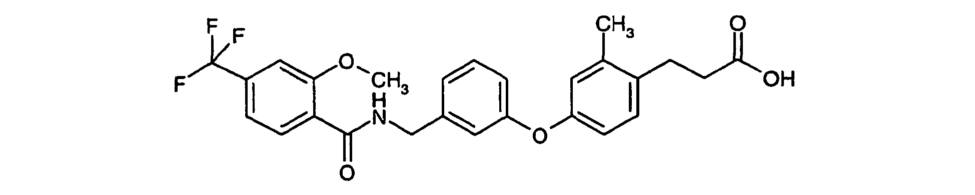

The title compound is prepared according to Example 1 utilizing 2-methoxy-4-trifluoromethyl-benzoic acid *(*J.Am.Chem.Soc.; 73; 1951; 2375) and 3-[4-(3-aminomethyl-phenoxy)-2-methyl-phenyl]-propionic acid methyl ester (Intermediate 33). Exact mass calcd for C₂₆H₂₅F₃NO₅ (M+H⁺): 488.1685, found 488.1680; ¹H NMR (400 MHz, CDCl₃).

### Example 32

### 3-(4-{3-[(2-Fluoro-4-trifluoromethyl-benzoylamino)-methyl]-phenoxy}-2-methylphenyl)-propionic acid

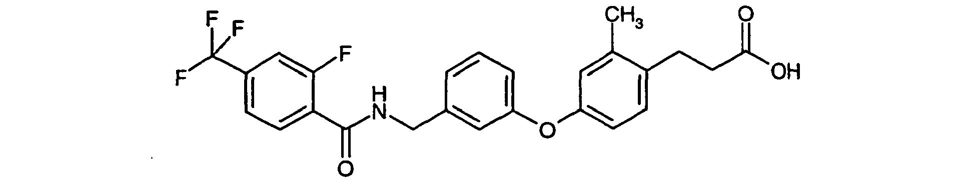

The title compound is prepared according to Example 1 utilizing 2-fluoro-4-trifluoromethyl-benzoic acid and 3-[4-(3-aminomethyl-phenoxy)-2-methyl-phenyl]-propionic acid methyl ester (Intermediate 33). Exact mass calcd for C₂₅H₂₂F₄NO₄ (M+H⁺): 476.1485, found 476.1503; ¹HNMR (400 MHz, CDCl₃).

### Example 33

### 2-Methyl-2-(2-methyl-4-{4-[(2-methyl-4-trifluoromethyl-benzoylamino)-methyl]-phenoxy}-phenoxy)-propionic acid

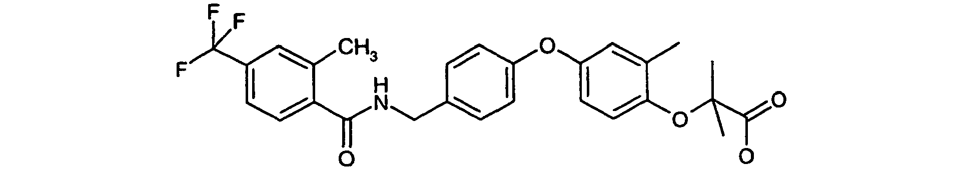

The title compound is prepared according to Example 1 utilizing 2-methyl-4-trifluoromethyl-benzoic acid (Intermediate 10) and 2-[4-(4-aminomethyl-phenoxy)-2-methyl-phenoxy]-2-methyl-propionic acid ethyl ester (Intermediate 18). Exact mass calcd for C₂₇H₂₇F₃NO₅ (M+H⁺): 502.1841, found 502.1832; ¹H NMR (400 MHz, CDCl₃).

### Example 34

### 2-[4-(3-1[(3-Chloro-5-trifluoromethyl-pyridine-2-carbonyl)-amino]-methyl}-phenoxy)-2-methyl-phenoxy]-2-methyl-propionic acid

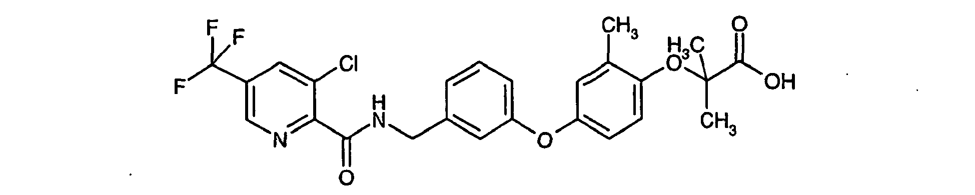

The title compound is prepared according to Example 1 utilizing 3-chloro-5-trifluoromethyl-pyridine-2-carboxylic acid and 2-[4-(3-aminomethyl-phenoxy)-2-methyl-phenoxy]-2-methyl-propionic acid ethyl ester (Intermediate 17). Exact mass calcd for C₂₅H₂₃ClF₃N₂O₅ (M+H⁺): 523.1248, found 523.1247; ¹H NMR (400 MHz, CDCl₃).

### Example 35

### 2-[4-(3-{[(1H-Indole-2-carbonyl)-amino]-methyl}-phenoxy)-2-methyl-phenoxy]-2-methyl-propionic acid

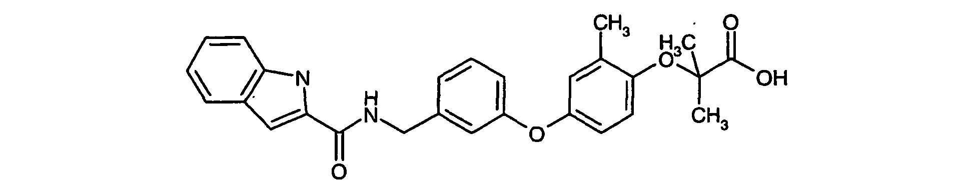

The title compound is prepared according to Example 1 utilizing 1H-indole-2-carboxylic acid and 2-[4-(3-aminomethyl-phenoxy)-2-methyl-phenoxy]-2-methyl-propionic acid ethyl ester (Intermediate 17). Exact mass calcd for C₂₇H₂₇N₂O₅ (M+H⁺): 459.1290, found 459.1918; ¹H NMR (400 MHz, CDCl₃).

### Example 36

### 3-[4-(3-{[(5-Chloro-benzo[b]thiophene-2-carbonyl)-amino]-methyl}-phenoxy)-2-methylphenyl]-propionic acid

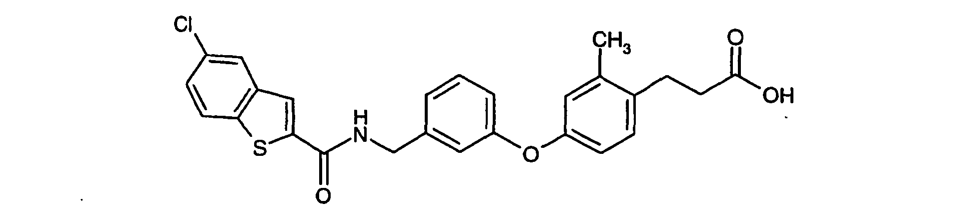

The title compound is prepared according to Example 1 utilizing 5-chloro-benzo[b]thiophene-2-carboxylic acid and 3-[4-(3-aminomethyl-phenoxy)-2-methylphenyl]-propionic acid methyl ester (Intermediate 33). Exact mass calcd for C₂₆H₂₃ClNO₄S (M+H⁺): 480.1036, found 480.1016; ¹H NMR (400 MHz, CDCl₃).

### Example 37

### 3-[4-(3-{[(5-Fluoro-1H-indole-2-carbonyl)-amino]-methyl}-phenoxy)-2-methyl-phenyl]-propionic acid

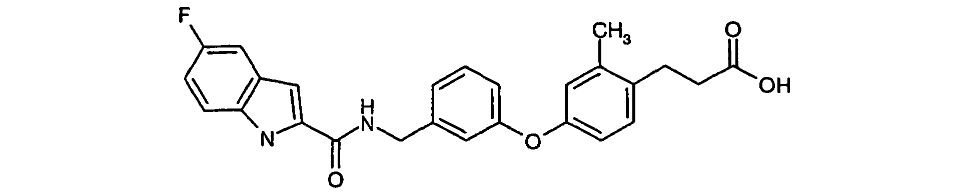

The title compound is prepared according to Example 1 utilizing 5-fluoro-1H-indole-2-carboxylic acid and 3-[4-(3-aminomethyl-phenoxy)-2-methyl-phenyl]-propionic acid methyl ester (Intermediate 33). Exact mass calcd for C₂₆H₂₄FN₂O₄ (M+H⁺): 447.1720, found 447.1720; ¹H NMR (400 MHz, CDCl₃).

### Example 38

### 3-[2-Methyl-4-(3-{[(5-trifluoromethoxy-1H-indole-2-carbonyl)-amino]-methyl}-phenoxy)-phenyl]-propionic acid

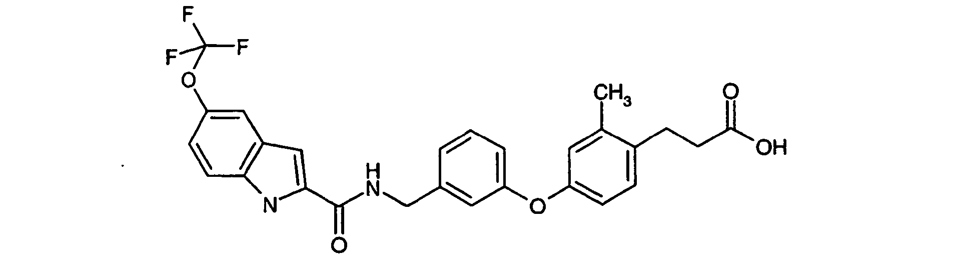

The title compound is prepared according to Example 1 utilizing 5-trifluoromethoxy-1H-indole-2-carboxylic acid and 3-[4-(3-aminomethyl-phenoxy)-2-methyl-phenyl]-propionic acid methyl ester (Intermediate 33). Exact mass calcd for C₂₇H₂₄F₃N₂O₅ (M+H⁺): 513.1637, found 513.1647; ¹H NMR (400 MHz, CDCl₃).

### Example 39

### 3-[2-Methyl-4-(3-{[(5-methyl-1H-indole-2-carbonyl)-amino]-methyl}-phenoxy)-phenyl]-propionic acid

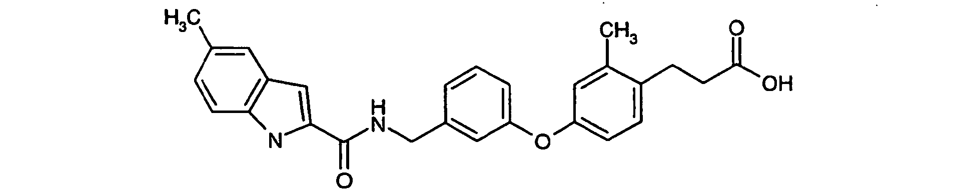

The title compound is prepared according to Example 1 utilizing 5-methyl-1H-indole-2-carboxylic acid and 3-[4-(3-aminomethyl-phenoxy)-2-methyl-phenyl]-propionic acid methyl ester (Intermediate 33). Exact mass calcd for C₂₇H₂₇N₂O₄ (M+H⁺): 443.1971, found 443.1966; ¹H NMR (400 MHz, CDCl₃).

### Example 40

### 3-[4-(3-{[(5-Chloro-3-methyl-benzo[b]thiophene-2-carbonyl)-amino]-methyl}-phenoxy)-2-methyl-phenyl]-propionic acid

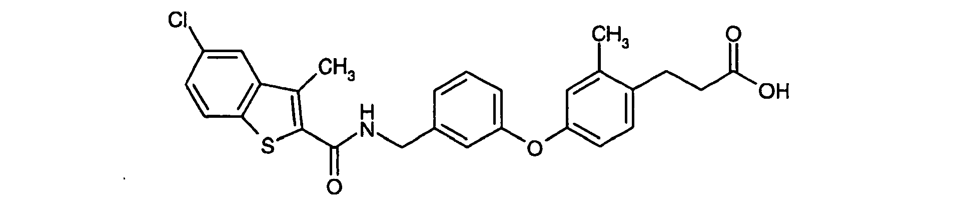

The title compound is prepared according to Example 1 utilizing 5-chloro-3-methyl-benzo[b]thiophene-2-carboxylic acid and 3-[4-(3-aminomethyl-phenoxy)-2-methyl-phenyl]-propionic acid methyl ester (Intermediate 33). Exact mass calcd for C₂₇H₂₅ClNO₄S (M+H⁺): 494.1193, found 494.1189; ¹H NMR (400 MHz, CDCl₃).

### Example 41

### 2-[4-(3-{[(5-Chloro-3-methyl-benzo[b]thiophene-2-carbonyl)-amino]-methyl}-phenoxy)-2-methyl-phenoxy]-2-methyl-propionic acid

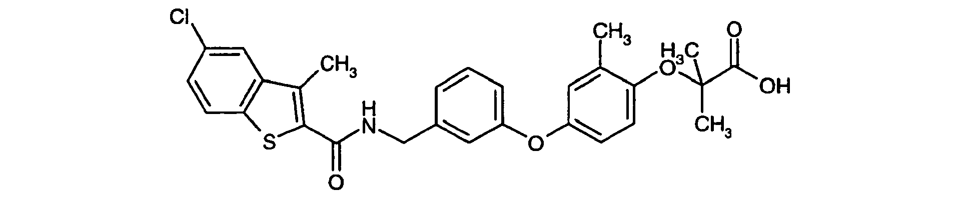

The title compound is prepared according to Example 9 utilizing 5-chloro-3-methyl-benzo[b]thiophene-2-carboxylic acid and 2-[4-(3-aminomethyl-phenoxy)-2-methyl-phenoxy]-2-methyl-propionic acid ethyl ester (Intermediate 17). Exact mass calcd for C₂₈H₂₇ClNO₅S (M+H⁺): 524.1298, found 524.1304; ¹H NMR (400 MHz, CDCl₃).

### Example 42

### 3 3-[2-Methyl-4-(3-{[(naphthalene-2-carbonyl)-amino]-methyl }-phenoxy)-phenyl]-propionic acid

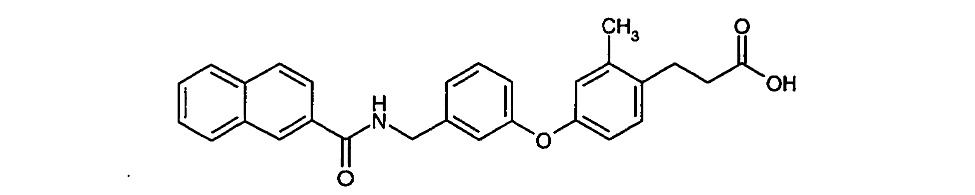

The title compound is prepared according to Example 1 utilizing naphthalene-2-carboxylic acid and 3-[4-(3-aminomethyl-phenoxy)-2-methyl-phenyl]-propionic acid methyl ester (Intermediate 33). Exact mass calcd for C₂₈H₂₆NO₄ (M+H⁺): 440.1862, found 440.1873; ¹H NMR (400 MHz, CDCl₃).

### Example 43

### 3-[4-(3-([(5-Cyano-3-methyl-1H-indole-2-carbonyl)-amino]-methyl}-phenoxy)-2-methyl-phenyl]-propionic acid

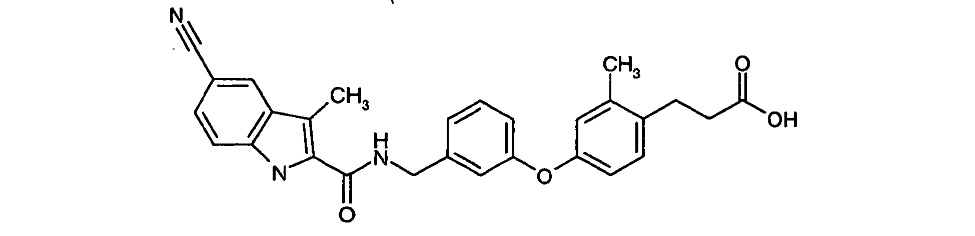

The title compound is prepared according to Example 1 utilizing 5-cyano-3-methyl-1H-indole-2-carboxylic acid (J. Med. Chem. 1997,40 (18), 2843-2857) and 3-[4-(3-aminomethyl-phenoxy)-2-methyl-phenyl]-propionic acid methyl ester (Intermediate 33). Exact mass calcd for C₂₈H₂₆N₃O₄ (M+H⁺): 468.1923, found 468.1903; ¹H NMR (400 MHz, CDCl₃).

### Example 44

### 3-[4-(3-{[(5-Cyano-1-methyl-1H-indole-2-carbonyl)-amino]-methyl}-phenoxy)-2-methyl-phenyl]-propionic acid

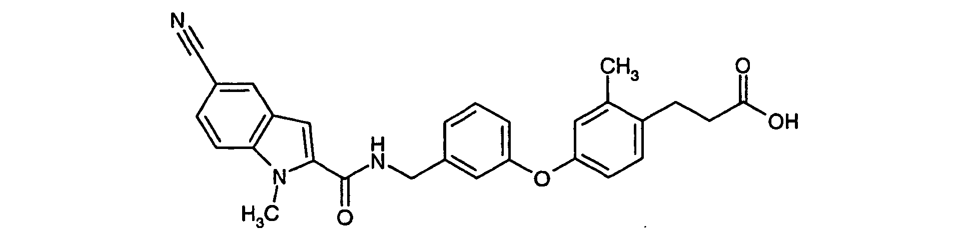

The title compound is prepared according to Example 1 utilizing 5-cyano-1-methyl-1H-indole-2-carboxylic acid (J. Med. Chem. 1997, 40 (18), 2843-2857) and 3-[4-(3-aminomethyl-phenoxy)-2-methyl-phenyl]-propionic acid methyl ester (Intermediate 33). Exact mass calcd for C₂₈H₂₆N₃O₄ (M+H⁺): 468.1923, found 468.1903; ¹H NMR (400 MHz, CDCl₃).

### Example 45

### 3-[4-(3-{[(4-Chloro-1H-indole-2-carbonyl)-amino]-methyl}-phenoxy)-2-methyl-phenyl]-propionic acid

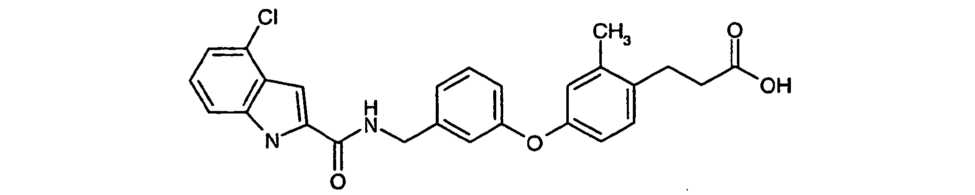

The title compound is prepared according to Example 1 utilizing 4-chloro-1H-indole-2-carboxylic acid and 3-[4-(3-aminomethyl-phenoxy)-2-methyl-phenyl]-propionic acid methyl ester (Intermediate 33). Exact mass calcd for C₂₆H₂₄ClN₂O₄ (M+H⁺): 463.1425, found 463.1399; ¹H NMR (400 MHz, CDCl₃).

### Example 46

### 3-[4-(3-{[(5-Chloro-3-phenyl-1H-indole-2-carbonyl)-amino]-methyl}-phenoxy)-2-methyl-phenyl]-propionic acid

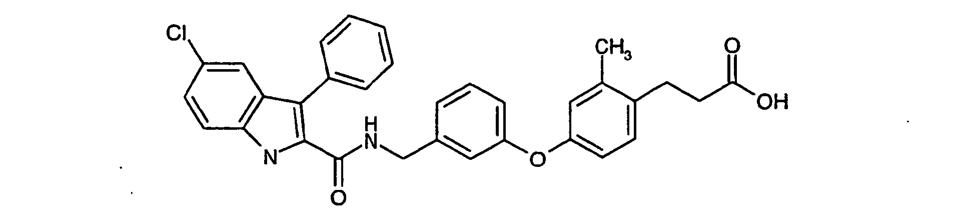

The title compound is prepared according to Example 1 utilizing 5-chloro-3-phenyl-1H-indole-2-carboxylic acid and 3-[4-(3-aminomethyl-phenoxy)-2-methylphenyl]-propionic acid methyl ester (Intermediate 33). Exact mass calcd for C₃₂H₂₈CIN₂O₄ (M+H⁺): 539.1738, found 539.1736; ¹H NMR (400 MHz, CDCl₃).

### Example 47

### 3-[4-(3-{[(5-Cyano-1H-indole-2-carbonyl)-amino]-methyl)-phenoxy)-2-methyl-phenyl]-propionic acid

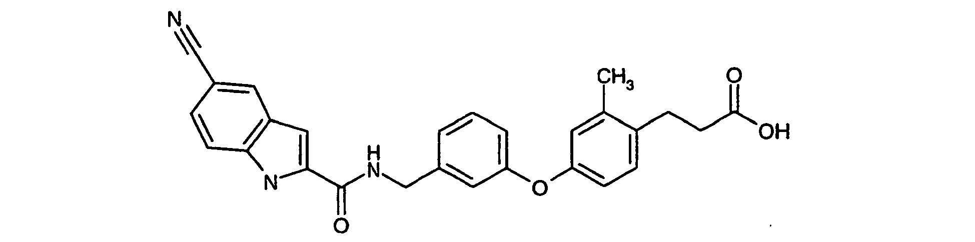

The title compound is prepared according to Example 9 utilizing 5-cyano-1H-indole-2-carboxylic acid (J. Med. Chem. 1997, 40 (18), 2843-2857) and 3-[4-(3-aminomethyl-phenoxy)-2-methyl-phenyl]-propionic acid methyl ester (Intermediate 33). Exact mass calcd for C₂₇H₂₄N₃O₄ (M+H⁺): 454.1767, found 454.1743; ¹H NMR (400 MHz, CDCl₃).

### Example 48

### 2-(4- {3-Fluoro-5-[(2-methyl-4-trifluoromethyl-benzoylamino)-methyl]-phenoxy}-2-methyl-phenoxy)-2-methyl-propionic acid

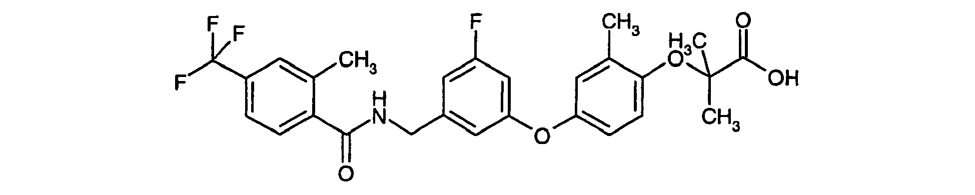

The title compound is prepared according to Example 1 utilizing 2-methyl-4-trifluoromethyl-benzoic acid (Intermediate 10) and 2-[4-(3-aminomethyl-5-fluoro-phenoxy)-2-methyl-phenoxy]-2-methyl-propionic acid ethyl ester (Intermediate 47). Exact mass calcd for C₂₇H₂₆F₄NO₅ (M+H⁺): 520.1747, found 520.1747; ¹H NMR (400 MHz, CDCl₃).

### Example 49

### 3-[4-(3-{([(6-Chloro-1H-indole-2-carbonyl)-amino]-methyl}-phenoxy)-2-methyl-phenyl]-propionic acid

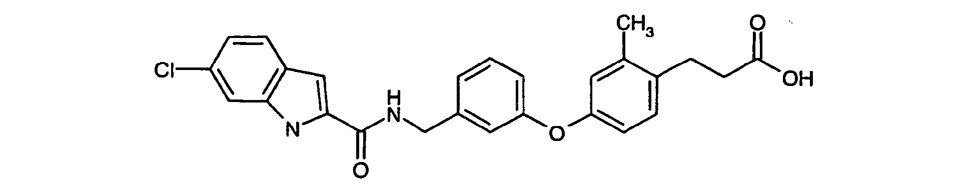

The title compound is prepared according to Example 1 utilizing 6-chloro-1H-indole-2-carboxylic acid and 3-[4-(3-aminomethyl-phenoxy)-2-methyl-phenyl]-propionic acid methyl ester (Intermediate 33). Exact mass calcd for C₂₆H₂₄ClN₂O₄ (M+H⁺): 463.1425, found 463.1427; ¹H NMR (400 MHz, CDCl₃).

### Example 50

### 3-[4-(3-{[(5-Chloro-3-methyl-1H-indole-2-carbonyl)-amino]-methyl}-phenoxy)-2-methyl-phenyl]-propionic acid

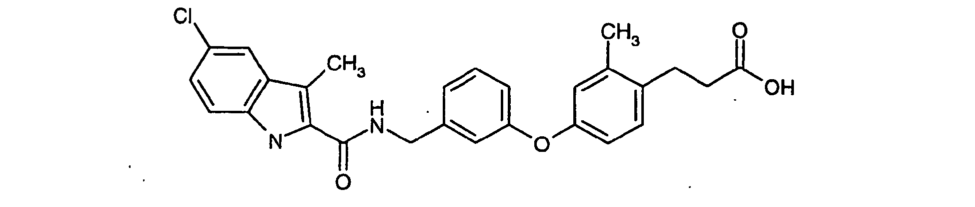

The title compound is prepared according to Example 1 utilizing 5-chloro-3-methyl- 1H-indole-2-carboxylic acid (Intermediate 3) and 3-[4-(3-aminomethyl-phenoxy)-2-methyl-phenyl]-propionic acid methyl ester (Intermediate 33). Exact mass calcd for C₂₇H₂₆ClN₂O₄ (M+H⁺): 477.1581, found 477.1584;¹H NMR (400 MHz, CDCl₃).

### Example 51

### 3-[2-Methyl-4-(3-{[(3-methyl-benzo[b]thiophene-2-carbonyl)-amino]-methyl}-phenoxy)-phenyl]-propionic acid

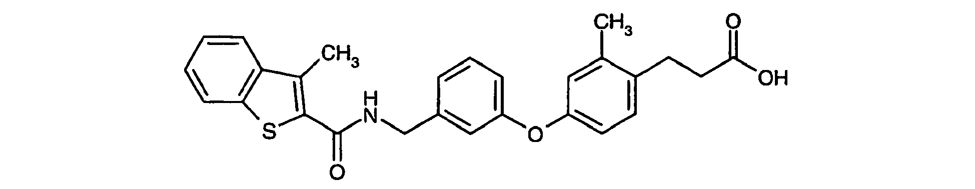

The title compound is prepared according to Example 1 utilizing 3-methyl-benzo[b]thiophene-2-carboxylic acid and 3-[4-(3-aminomethyl-phenoxy)-2-methylphenyl]-propionic acid methyl ester (Intermediate 33). Exact mass calcd for C₂₇H₂₆NO₄S (M+H⁺): 460.1582, found 460.1578; ¹H NMR (400 MHz, CDCl₃).

### Example 52

### 3-[4-(3-{[(Benzo[b]thiophene-2-carbonyl)-amino]-methyl)-phenoxy)-2-methyl-phenyl]-propionic acid

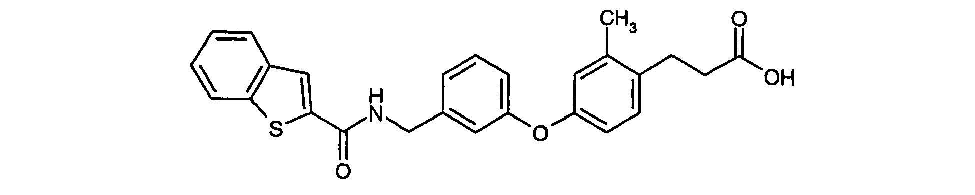

The title compound is prepared according to Example 1 utilizing benzo[b]thiophene-2-carboxylic acid and 3-[4-(3-aminomethyl-phenoxy)-2-methylphenyl]-propionic acid methyl ester (Intermediate 33). Exact mass calcd for C₂₆H₂₄NO₄S (M+H⁺): 446.1439, found 446.1439; ¹H NMR (400 MHz, CDCl₃).

### Example 53

### 3-(2-Methyl-4-{3-[(4,4,4-trifluoro-butyrylamino)-methyl]-phenoxy}-phenyl)-propionic acid

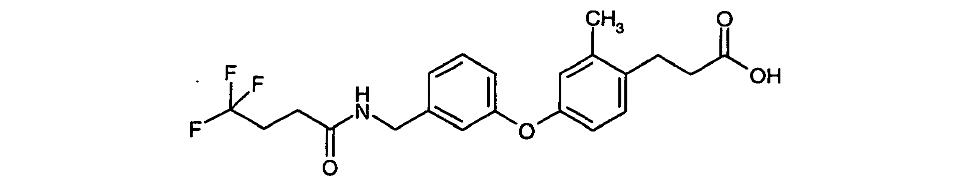

The title compound is prepared according to Example 1 utilizing 4,4,4-trifluoro-butyric acid and 3-[4-(3-aminomethyl-phenoxy)-2-methyl-phenyl]-propionic acid methyl ester (Intermediate 33). Exact mass calcd for C₂₁H₂₃F₃NO₄ (M+H⁺): 410.1579, found 410.1586; ¹H NMR (400 MHz, CDCl₃).

### Example 54

### 3-(4-{3-Fluoro-5-[(2-methyl-4-trifluoromethyl-benzoylamino)-methyl]-phenoxy}-2-methyl-phenyl)-propionic acid

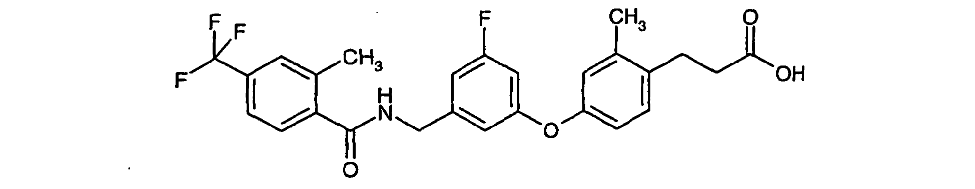

The title compound is prepared according to Example 1 utilizing 2-methyl-4-trifluoromethyl-benzoic acid (Intermediate 10) and 3-[4-(3-aminomethyl-5-fluoro-phenoxy)-2-methyl-phenyl]-propionic acid methyl ester (Intermediate 36). Exact mass calcd for C₂₆H₂₄F₄NO₄ (M+H⁺): 490.1641, found 490.1653; ¹H NMR (400 MHz, CDCl₃).

### Example 55

### 3-[4-(3-{[(5-Chloro-3-methyl-1H-indole-2-carbonyl)-amino]-methyl}-5-fluoro-phenoxy)-2-methyl-phenyl]-propionic acid

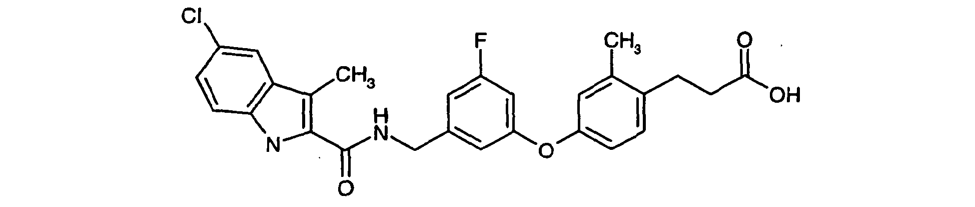

The title compound is prepared according to Example 1 utilizing 5-chloro-3-methyl-1H-indole-2-carboxylic acid (Intermediate 3) and 3-[4-(3-aminomethyl-5-fluoro-phenoxy)-2-methyl-phenyl]-propionic acid methyl ester (Intermediate 36). Exact mass calcd for C₂₇H₂₅ClFN₂O₄ (M+H⁺): 495.1487, found 495.1487; ¹H NMR (400 MHz, CDCl₃).

### Example 56

### 3-[4-(3-Fluoro-5-{ [(3-methyl-5-trifluoromethyl-1H-indole-2-carbonyl)-amino]-methyl}-phenoxy)-2-methyl-phenyl]-propionic acid

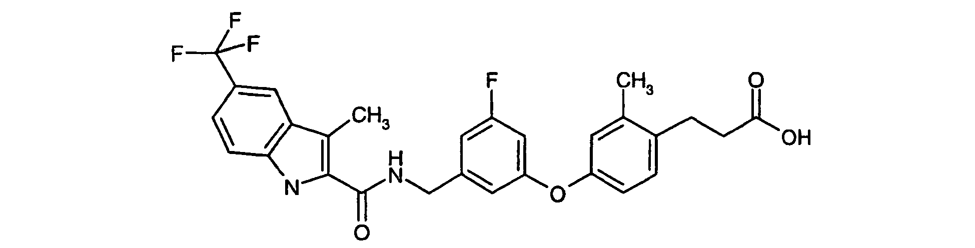

The title compound is prepared according to Example 1 utilizing 3-methyl-5-trifluoromethyl-1H-indole-2-carboxylic acid (Intermediate 5) and 3-[4-(3-aminomethyl-5-fluoro-phenoxy)-2-methyl-phenyl]-propionic acid methyl ester (Intermediate 36). Exact mass calcd for C₂₈H₂₅F₄N₂O₄ (M+H⁺): 529.1750, found 529.1757;¹H NMR (400 MHz, CDCl₃).

### Example 57

### 3-[2-Methyl-4-(3-{[(3-methyl-5-trifluoromethyl-1H-indole-2-carbonyl)-amino]-methyl)-phenoxy)-phenyl]-propionic acid

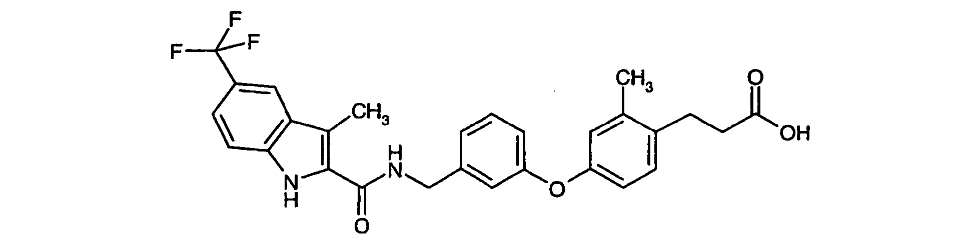

The title compound is prepared according to Example 1 utilizing 3-methyl-5-trifluoromethyl-1H-indole-2-carboxylic acid (Intermediate 5) and 3-[4-(3-aminomethyl-phenoxy)-2-methyl-phenyl]-propionic acid methyl ester (Intermediate 33). Exact mass calcd for C₂₈H₂₆F₃N₂O₄ (M+H⁺): 511.1844, found 511.1834; ¹H NMR (400 MHz, CDCl₃).

### Example 58

### 3-[4-(3-{[(5-Fluoro-3-methyl-1H-indole-2-carbonyl)-amino]-methyl}-phenoxy)-2-methyl-phenyl]-propionic acid

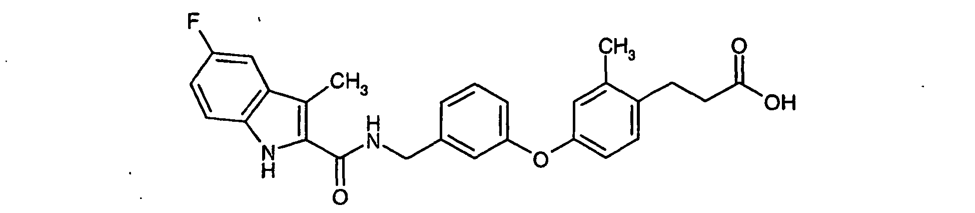

The title compound is prepared according to Example 1 utilizing 5-fluoro-3-methyl-1H-indole-2-carboxylic acid (Intermediate 1) and 3-[4-(3-aminomethyl-phenoxy)-2-methyl-phenyl]-propionic acid methyl ester (Intermediate 33). Exact mass calcd for C₂₇H₂₆F N₂O₄ (M+H⁺): 461.1877, found 461.1873; ¹H NMR (400 MHz, CDCl₃).

### Example 59

### 3-[4-(3-Fluoro-5-{[(5-fluoro-3-methyl-1H-indole-2-carbonyl)-amino]-methyl}-phenoxy)-2-methyl-phenyl]-propionic acid

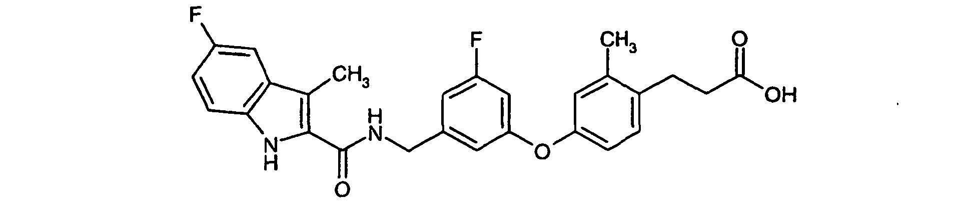

The title compound is prepared according to Example 1 utilizing 3-methyl-5-trifluoro-1H-indole-2-carboxylic acid (Intermediate 1) and 3-[4-(3-aminomethyl-5-fluoro-phenoxy)-2-methyl-phenyl]-propionic acid methyl ester (Intermediate 36). Exact mass calcd for C₂₇H₂₅F₂N₂O₄ (M+H⁺): 479.1782, found 479.1787; ¹H NMR (400 MHz, CDCl₃).

### Example 60

### 3-[4-(3-{[(5-Chloro-3-methyl-benzo[b]thiophene-2-carbonyl)-amino]-methyl}-5-fluoro-phenoxy)-2-methyl-phenyl]-propionic acid

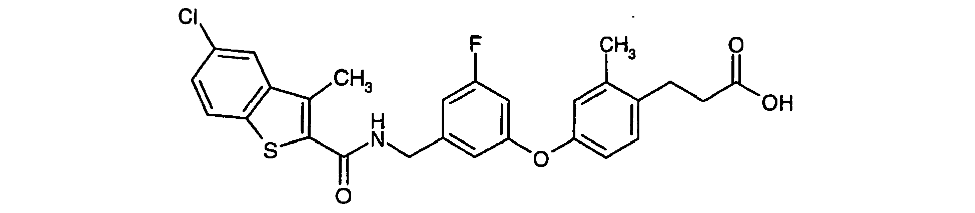

The title compound is prepared according to Example 1 utilizing 5-chloro-3-methyl-benzo[b]thiophene-2-carboxylic acid and 3-[4-(3-aminomethyl-5-fluoro-phenoxy)-2-methyl-phenyl]-propionic acid methyl ester (Intermediate 36). Exact mass calcd for C₂₇H₂₄ClFNO₄S (M+H⁺): 512.1099, found 512.1160; ¹H NMR (400 MHz, CDCl₃).

### Example 61

### 2-[4-(3-{[(5-Chloro-3-methyl-benzo[b]thiophene-2-carbonyl)-amino]-methyl}-5-fluoro-phenoxy)-2-methyl-phenoxy]-2-methyl-propionic acid

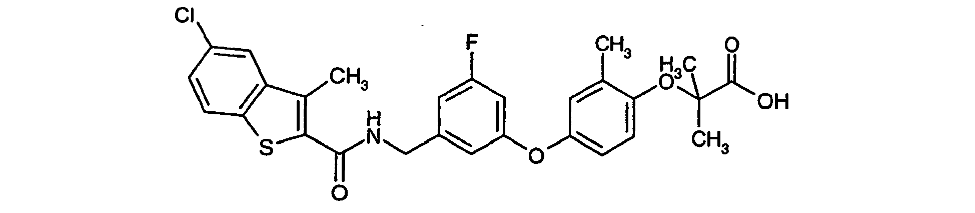

The title compound is prepared according to Example 1 utilizing 5-chloro-3-methyl-benzo[b]thiophene-2-carboxylic acid and 2-[4-(3-aminomethyl-5-fluoro-phenoxy)-2-methyl-phenoxy]-2-methyl-propionic acid ethyl ester (Intermediate 47). Exact mass calcd for C₂₈H₂₆ClFNO₅S (M+H⁺): 542.1204, found 542.1190; ¹H NMR (400 MHz, CDCl₃).

### Example 62

### 2-{4-[3-(5-Chloro-1-oxo-1,3-dihydro-isoindol-2-ylmethyl)-5-fluoro-phenoxy]-2-methyl-phenoxy}-2-methyl-propionic acid

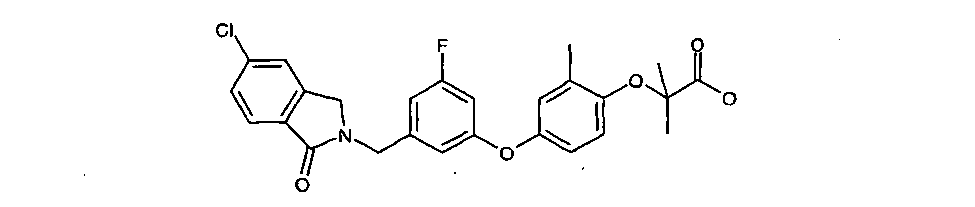

### Step A

### 4-chloro-2-methylbenzoic acid methyl ester

4-Chloro-2-methylbenzoic acid (3.4 g, 20.0 mmol) is dissolved in a solution of methanol (25 ml) and dichloromethane (200 ml) and tetramethylsilyl diazomethane (15 mL, 30.0 mmol, 2M in hexane) is added dropwise keeping the temperature near rt with some cooling. Reaction is stirred overnight at rt under nitrogen. Water is added, the organic layer is separated, washed with brine, dried over sodium sulfate and concentrated under reduced pressure providing the title compound with no purification (3.3 g, 90 %). GC/MS: M·⁺ 184; ¹HNMR (400 MHz, CDCl₃).

### Step B

### 2-Bromomethyl-4-chloro-benzoic acid methyl ester

The compound from Step A (1.0 g, 5.4 mmol), N-bromosuccinimide (1.1 g, 6.0 mmol), and benzoylperoxide (catalytic amount) are refluxed overnight in carbon tetrachloride (10 mL) under nitrogen. Upon cooling the solid is removed by filtration and the filtrate is concentrated under reduced pressure. Ether is added, and the mixture is washed with water and brine, and then dried over sodium sulfate, and concentrated under reduced pressure. The remaining solid is triturated with hexane and collected by filtration providing the title compound (0.65 g, 45 %). GC/MS: M.⁺ 263/264; ¹HNMR (400 MHz, CDCl₃).

### Step C

### 2-{4-[3-(5-Chloro-1-oxo-1,3-dihydro-isoindol-2-ylmethyl)-5-fluoro-phenoxy]-2-methyl-phenoxy}-2-methyl-propionic acid ethyl ester

Compound from Step B (76 mg, 0.29mmol), 2-[4-(3-aminomethyl-5-fluoro-phenoxy)-2-methyl-phenoxy]-2-methyl-propionic acid ethyl ester (Intermediate 47) (120 mg, 0.33 mmol), and potassium carbonate (92 mg, 0.66 mmol) are dissolved in dry DMF (10 mL) and stirred at rt overnight under nitrogen. Water is added to the reaction, and the mixture is extracted with ethyl acetate, washed with brine, dried over sodium sulfate and concentrated under reduced pressure. Purification by flash chromatography, eluting with 10 % EtOAc in hexane then 25 % EtOAc in hexane provides the title compound (38 g, 26 %). MS(ES⁺): 512 (M+H⁺); ¹HNMR (400 MHz, CDCl₃)

### Step D

### 2-{4-[3-(5-Chloro-1-oxo-1,3-dihydro-isoindol-2-ylmethyl)-5-fluoro-phenoxy]-2-methyl-phenoxy}-2-methyl-propionic acid

Compound of Step C (38 mg, 0.074 mmol) is dissolved in dioxane (4 mL) and lithium hydroxide hydrate (40 mg, 0.95 mmol), dissolved in water (2 mL), is added. Mixture is stirred at rt overnight under nitrogen, and then acidified with 5 N HCl. Water is added, and the mixture is extracted with EtOAc, washed with brine, dried over sodium sulfate and concentrated under reduced pressure to give the title compound (36.8 mg, quantitative). Exact mass calculated for C₂₆H₂₄ClFNO₅ (M+H⁺) 484.1327, found 484.1345; ¹H NMR (400 MHz, CDCl₃).

### Example 63

### 3-(4-{3-Fluoro-5-[(2-fluoro-4-trifluoromethyl-benzoylamino)-methyl]-phenoxy}-2-methyl-phenyl)-propionic acid

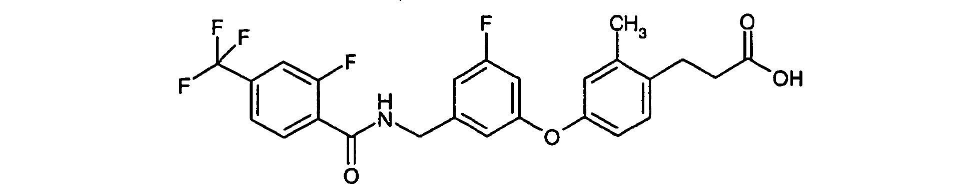

The title compound is prepared according to Example 1 utilizing 2-fluoro-4-trifluoromethyl-benzoic acid and 3-[4-(3-aminomethyl-5-fluoro-phenoxy)-2-methylphenyl]-propionic acid methyl ester (Intermediate 36). Exact mass calcd for C₂₅H₂₁F ₅NO₄ (M+H⁺): 494.1391, found 494.1379; ¹HNMR (400 MHz, CDCl₃).

### Example 64

### 3-(4-{3-Fluoro-5-[(2-methoxy-4-trifluoromethyl-benzoylamino)-methyl]-phenoxy}-2-methyl-phenyl)-propionic acid

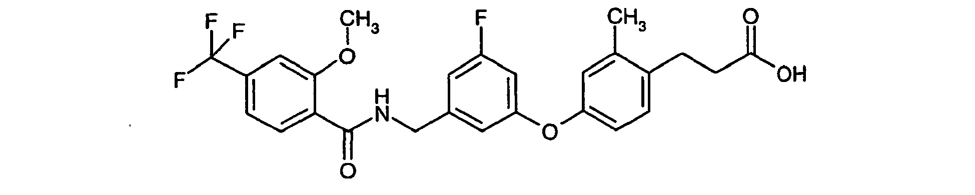

The title compound is prepared according to Example 1 utilizing 2-methoxy-4-trifluoromethyl-benzoic acid *(*J.Am.Chem.Soc.; 73; 1951; 2375) and 3-[4-(3-aminomethyl-5-fluoro-phenoxy)-2-methyl-phenyl]-propionic acid methyl ester (Intermediate 36). Exact mass calcd for C₂₆H₂₄F₄NO₅ (M+H⁺): 506.1591, found 506.1568; ¹H NMR (400 MHz, CDCl₃).

### Example 65

### 3-[4-(3-{[(5-Chloro-1,3-dimethyl-1H-indole-2-carbonyl)-amino]-methyl}-5-fluoro-phenoxy)-2-methyl-phenyl]-propionic acid

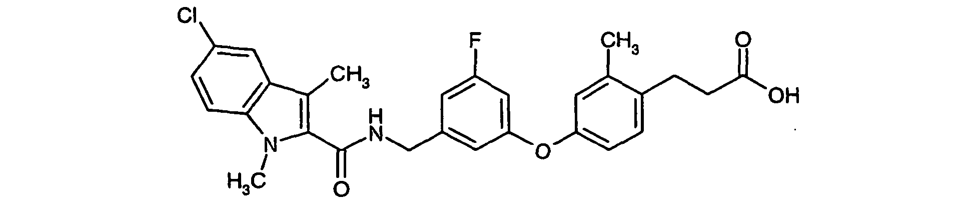

The title compound is prepared according to Example 1 utilizing 5-chloro-1,3-dimethyl-1H-indole-2-carboxylic acid (Intermediate 4) and 3-[4-(3-aminomethyl-5-fluoro-phenoxy)-2-methyl-phenyl]-propionic acid methyl ester (Intermediate 36). Exact mass calcd for C₂₈H₂₇ClFN₂O₄ (M+H⁺): 509.1643, found 509.1619; ¹H NMR (400 MHz, CDCl₃).

### Example 66

### 2-[4-(3-{[(5-Chloro-1,3-dimethyl-1H-indole-2-carbonyl)-amino]-methyl}-5-fluoro-phenoxy)-2-methyl-phenoxy]-2-methyl-propionic acid

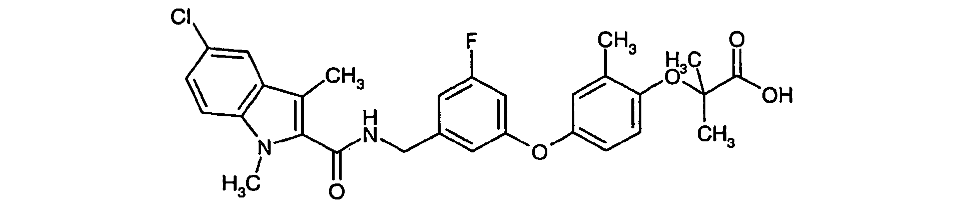

The title compound is prepared according to Example 1 utilizing 5-chloro-1,3-dimethyl-1H-indole-2-carboxylic acid (Intermediate 4) and 2-[4-(3-aminomethyl-5-fluoro-phenoxy)-2-methyl-phenoxy]-2-methyl-propionic acid ethyl ester (Intermediate 47). Exact mass calcd for C₂₉H₂₉ClFN₂O₅ (M+H⁺): 539.1749, found 539.1733; ¹H NMR (400 MHz, CDCl₃).

### Example 67

### 2-{4-[3-Fluoro-5-(1-oxo-1,3-dihydro-isoindol-2-ylmethyl)-phenoxy]-2-methyl-phenoxy}-2-methyl-propionic acid

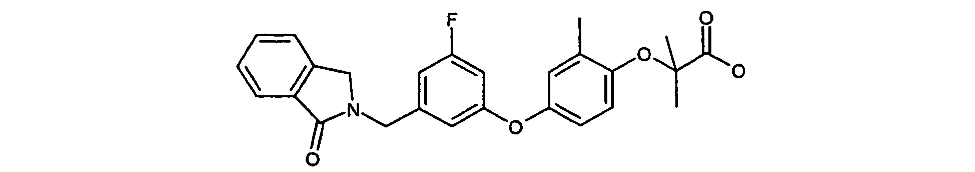

The title compound is prepared according to Example 62, Steps C and D, utilizing 2-bromomethyl-benzoic acid and 2-[4-(3-aminomethyl-5-fluoro-phenoxy)-2-methyl-phenoxy]-2-methyl-propionic acid ethyl ester (Intermediate 47). Exact mass calcd for C₂₆H₂₅FNO₅ (M+H⁺): 450.1717, found 450.1699; ¹H NMR (400 MHz, CDCl₃).

### Example 68

### 3-[4-(3-Fluoro-5-{([(5-fluoro-1,3-dimethyl-1H-indole-2-carbonyl)-amino]-methyl}-phenoxy)-2-methyl-phenyl]-propionic acid

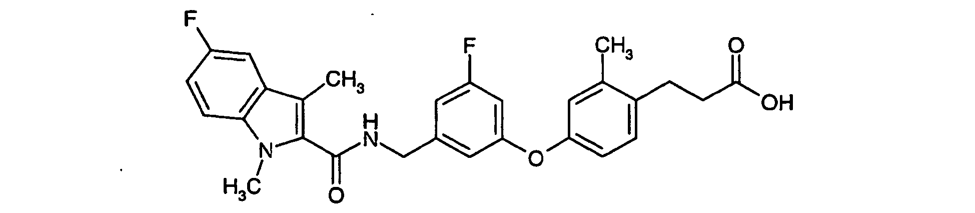

The title compound is prepared according to Example 1 utilizing 5-fluoro-1,3-dimethyl-1H-indole-2-carboxylic acid (Intermediate 2) and 3-[4-(3-aminomethyl-5-fluoro-phenoxy)-2-methyl-phenyl]-propionic acid methyl ester (Intermediate 36). Exact mass calcd for C₂₈H₂₇F₂N₂O₄ (M+H⁺): 493.1939, found 493.1932; ¹H NMR (400 MHz, CDCl₃).

### Example 69

### 2-[4-(3-Fluoro-5-{[(5-fluoro-1,3-dimethyl-1H-indole-2-carbonyl)-amino]-methyl}-phenoxy)-2-methyl-phenoxy]-2-methyl-propionic acid

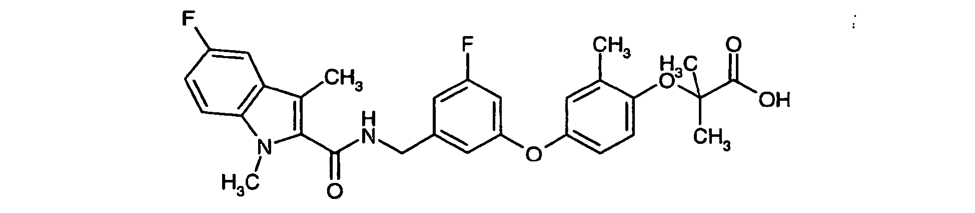

The title compound is prepared according to Example 1 utilizing 5-fluoro-1,3-dimethyl-1H-indole-2-carboxylic acid (Intermediate 2) and 2-[4-(3-aminomethyl-5-fluoro-phenoxy)-2-methyl-phenoxy]-2-methyl-propionic acid ethyl ester (Intermediate 47). Exact mass calcd for C₂₉H₂₉F₂N₂O₅ (M+H⁺): 523.2045, found 523.2038; ¹H NMR (400 MHz, CDCl₃).

### Example 70

### 3-[4-(3-{[(7-Bromo-1H-indole-2-carbonyl)-amino]-methyl}-phenoxy)-2-methyl-phenyl]-propionic acid

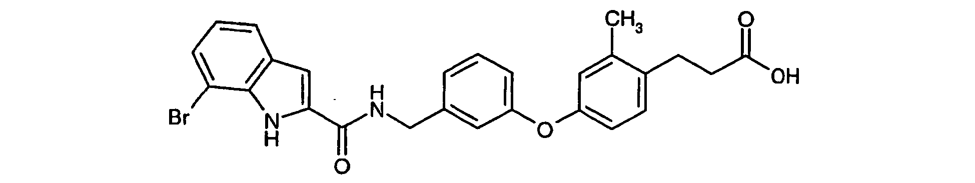

The title compound is prepared according to Example 1 utilizing 7-bromo-1H-indole-2-carboxylic acid and 3-[4-(3-aminomethyl-phenoxy)-2-methyl-phenyl]-propionic acid methyl ester (Intermediate 33). Exact mass calcd for C₂₆H₂₄BrN₂O₄ (M+H⁺): 507.0919, found 507.0929; ¹HNMR (400 MHz, CDCl₃).

### Example 71

### 2-(4-{3-[(2-Fluoro-4-trifluoromethyl-benzoylamino)-methyl]-4-methyl-phenoxy)-2-methyl-phenoxy)-2-methyl-propionic acid

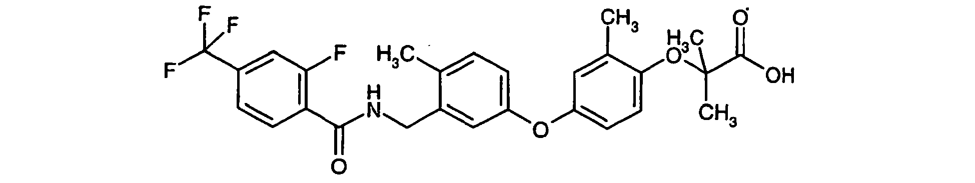

The title compound is prepared according to Example 1 utilizing 2-fluoro-4-trifluoromethyl-benzoic acid and 2-[4-(3-aminomethyl-4-methyl-phenoxy)-2-methyl-phenoxy]-2-methyl-propionic acid ethyl ester (Intermediate 19). Exact mass calcd for C₂₇H₂₆F₄NO₅ (M+H⁺): 520.1747, found 520.1730; ¹H NMR (400 MHz, CDCl₃).

### Example 72

### 3-[4-(3-{[(1,3-Dimethyl-5-trifluoromethyl-1H-indole-2-carbonyl)-amino]-methyl}-5-fluoro-phenoxy)-2-methyl-phenyl]-propionic acid

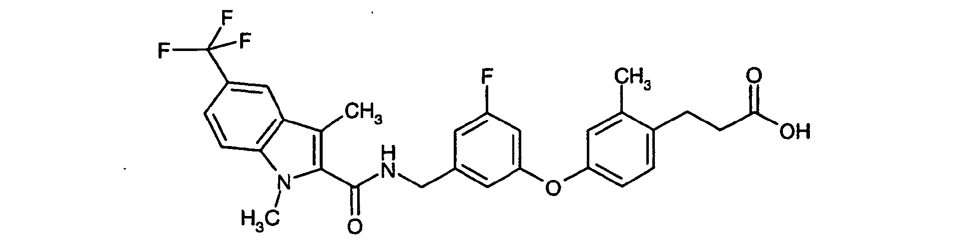

The title compound is prepared according to Example 1 utilizing 1,3-dimethyl-5-trifluoromethyl-1H-indole-2-carboxylic acid (Intermediate 6) and 3-[4-(3-aminomethyl-5-fluoro-phenoxy)-2-methyl-phenyl]-propionic acid methyl ester (Intermediate 36). Exact mass calcd for C₂₉H₂₇F₄N₂O₄ (M+H⁺): 543.1907, found 543.1883; ¹H NMR (400 MHz, CDCl₃).

### Example 73

### 2-[4-(3-{[(1,3-Dimethyl-5-trifluoromethyl-1H-indole-2-carbonyl)-amino]-methyl}-5-fluoro-phenoxy)-2-methyl-phenoxy]-2-methyl-propionic acid

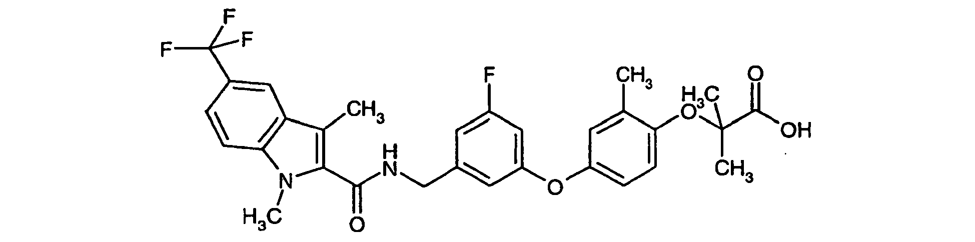

The title compound is prepared according to Example 1 utilizing 1,3-dimethyl-5-trifluoromethyl-1H-indole-2-carboxylic acid (Intermediate 6) and 2-[4-(3-aminomethyl-5-fluoro-phenoxy)-2-methyl-phenoxy]-2-methyl-propionic acid ethyl ester (Intermediate 47). Exact mass calcd for C₃₀H₂₉F₄N₂O₅ (M+H⁺): 573.2012, found 573.2009; ¹H NMR (400 MHz, CDCl₃).

### Example 74

### 2-[4-(3-{[(5-Chloro-1,3-dimethyl-1H-indole-2-carbonyl)-amino]-methyl}-phenoxy)-2-methyl-phenoxy]-2-methyl-propionic acid

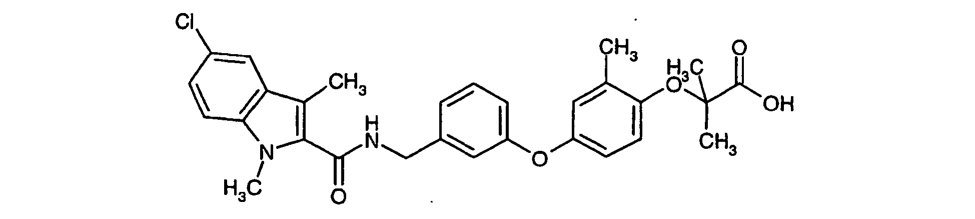

The title compound is prepared according to Example 1 utilizing 5-chloro-1,3-dimethyl-1H-indole-2-carboxylic acid (Intermediate 4) and 2-[4-(3-aminomethyl-phenoxy)-2-methyl-phenoxy]-2-methyl-propionic acid ethyl ester (Intermediate 17). Exact mass calcd for C₂₉H₃₀ClN₂O₅ (M+H⁺): 521.1854, found 521.1843; ¹H NMR (400 MHz, CDCl₃).

### Example 75

### 3-[4-(3-{ [(5-Chloro-1,3-dimethyl-1H-indole-2-carbonyl)-amino]-methyl}-phenoxy)-2-methyl-phenyl]-propionic acid

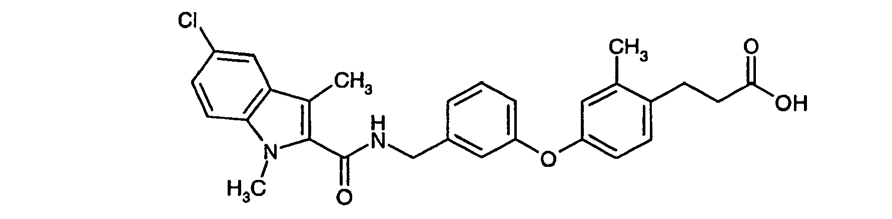

The title compound is prepared according to Example 1 utilizing 5-chloro-1,3-dimethyl-1H-indole-2-carboxylic acid (Intermediate 4) and 3-[4-(3-aminomethyl-phenoxy)-2-methyl-phenyl]-propionic acid methyl ester (Intermediate 33). Exact mass calcd for C₂₈H₂₈ClN₂O₄ (M+H⁺): 491.1737, found 491.1731; ¹H NMR (400 MHz, CDCl₃).

### Example 76

### 2-Methyl-2-(2-methyl-4-{3-methyl-5-[(2-methyl-4-trifluoromethyl-benzoylamino)-methyl]-phenoxy}-phenoxy)-propionic acid

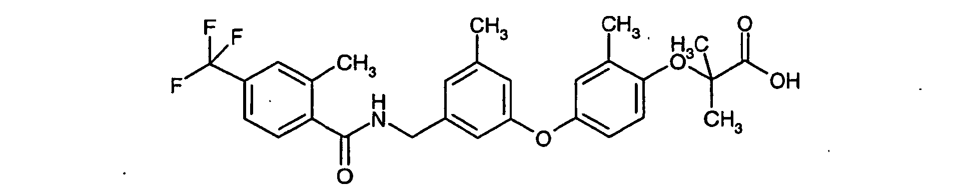

The title compound is prepared according to Example 1 utilizing 2-methyl-4-trifluoromethyl-benzoic acid (Intermediate 10) and 2-[4-(3-aminomethyl-5-methyl-phenoxy)-2-methyl-phenoxy]-2-methyl-propionic acid ethyl ester (Intermediate 20). Exact mass calcd for C₂₈H₂₉F₃NO₅ (M+H⁺): 516.1998, found 516.2005; ¹H NMR (400 MHz, CDCl₃).

### Example 77

### 2-(4-(3-[(2-Fluoro-4-trifluoromethyl-benzoylamino)-methyl]-5-methyl-phenoxyl-2-methyl-phenoxy)-2-methyl-propionic acid

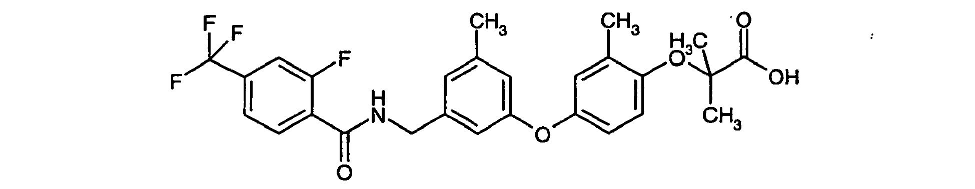

The title compound is prepared according to Example 1 utilizing 2-fluoro-4-trifluoromethyl-benzoic acid and 2-[4-(3-aminomethyl-5-methyl-phenoxy)-2-methyl-phenoxy]-2-methyl-propionic acid ethyl ester (Intermediate 20). Exact mass calcd for C₂₇H₂₆F₄NO₅ (M+H⁺): 520.1747, found 520.1774; ¹H NMR (400 MHz, CDCl₃).

### Example 78

### 3-[4-(3-{[(5-Fluoro-1,3-dimethyl-1H-indole-2-carbonyl)-amino]-methyl}-phenoxy)-2-methyl-phenyl]-propionic acid

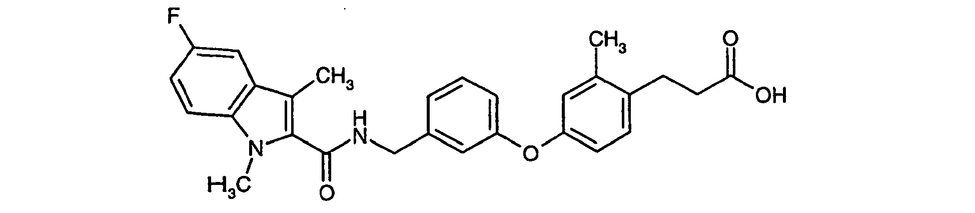

The title compound is prepared according to Example 1 utilizing 5-fluoro-1,3-dimethyl-1H-indole-2-carboxylic acid (Intermediate 2) and 3-[4-(3-aminomethyl-phenoxy)-2-methyl-phenyl]-propionic acid methyl ester (Intermediate 33). Exact mass calcd for C₂₈H₂₈FN₂O₄ (M+H⁺): 475.2033, found 475.2019; ¹H NMR (400 MHz, DMSO-D6).

### Example 79

### 2-[4-(3-{[(5-Fluoro-1,3-dimethyl-1H-indole-2-carbonyl)-amino]-methyl}-phenoxy)-2-methyl-phenoxy]-2-methyl-propionic acid

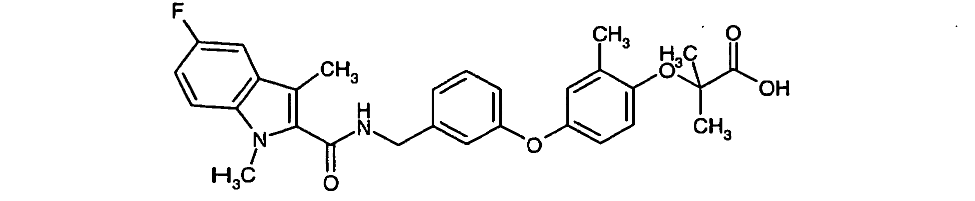

The title compound is prepared according to Example 1 utilizing 5-fluoro-1,3-dimethyl-1H-indole-2-carboxyl acid (Intermediate 2) and 2-[4-(3-aminomethyl-phenoxy)-2-methyl-phenoxy]-2-methyl-propionic acid ethyl ester (Intermediate 17). Exact mass calcd for C₂₉H₃₀FN₂O₅ (M+H⁺): 505.2139, found 505.2147; ¹H NMR (400 MHz, CDCl₃).

### Example 80

### 2-(4-{3-[3-(2-Fluoro-4-trifluoromethyl-benzoylamino)-propyl]-phenoxy}-2-methyl-phenoxy)-2-methyl-propionic acid

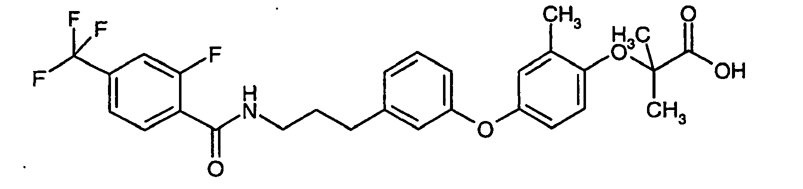

The title compound is prepared according to Example 1 utilizing 2-fluoro-4-trifluoromethyl-benzoic acid and 2-{4-[3-(3-amino-propyl)-phenoxy]-2-methyl-phenoxy}-2-methyl-propionic acid ethyl ester (Intermediate 52). Exact mass calcd for C₂₈H₂₈F₄NO₅ (M+H⁺): 534.1904, found 534.1890; ¹H NMR (400 MHz, CDCl₃).

### Example 81

### 2-[4-(3-{[(1,3-Dimethyl-5-trifluoromethyl-1H-indole-2-carbonyl)-amino]-methyl)-5-methyl-phenoxy)-2-methyl-phenoxy]-2-methyl-propionic acid

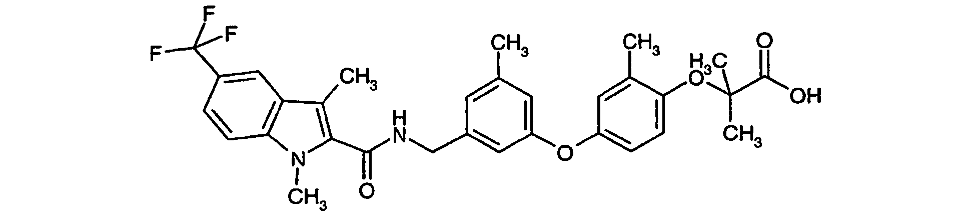

The title compound is prepared according to Example 1 utilizing 1,3-dimethyl-5-trifluoromethyl-1H-indole-2-carboxylic acid (Intermediate 6) and 2-[4-(3-aminomethyl-5-methyl-phenoxy)-2-methyl-phenoxy]-2-methyl-propionic acid ethyl ester (Intermediate 20). Exact mass calcd for C₃₁H₃₂F₃N₂O₅ (M+H⁺): 569.2263, found 569.2264; ¹H NMR (400 MHz, CDCl₃).

### Example 82

### 2-[4-(3-{ [(5-Chloro-1,3-dimethyl-1H-indole-2-carbonyl)-amino]-methyl -5-methyl-phenoxy)-2-methyl-phenoxy]-2-methyl-propionic acid

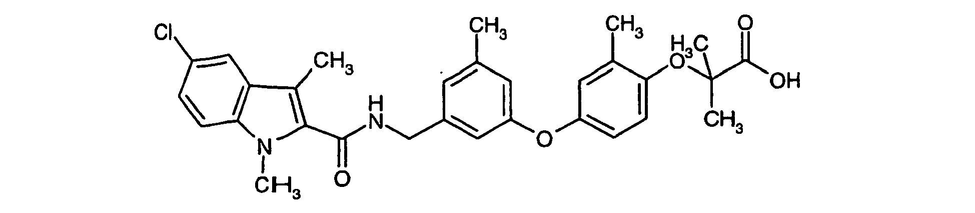

The title compound is prepared according to Example 1 utilizing 5-chloro-1,3-dimethyl-1H-indole-2-carboxylic acid (Intermediate 4) and 2-[4-(3-aminomethyl-5-methyl-phenoxy)-2-methyl-phenoxy]-2-methyl-propionic acid ethyl ester (Intermediate 20). Exact mass calcd for C₃₀H₃₂ClN₂O₅ (M+H⁺): 535.200, found 535.1976; ¹H NMR (400 MHz, CDCl₃).

### Example 83

### 2-[4-(3- {[(1,3-Dimethyl-5-trifluoromethyl-1H-indole-2-carbonyl)-amino]-methyl}-phenoxy)-2-methyl-phenoxy]-2-methyl-propionic acid

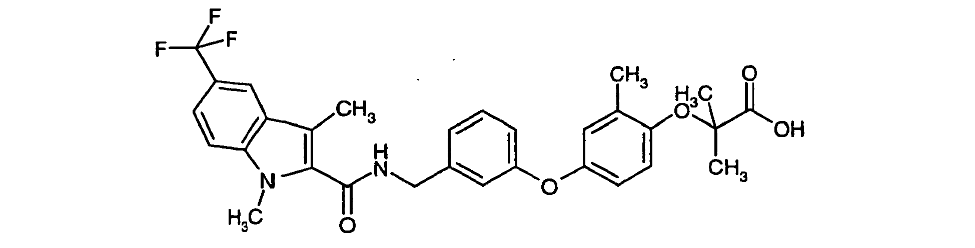

The title compound is prepared according to Example 1 utilizing 1,3-dimethyl-5-trifluoromethyl-1H-indole-2-carboxylic acid (Intermediate 6) and 2-[4-(3-aminomethyl-phenoxy)-2-methyl-phenoxy]-2-methyl-propionic acid ethyl ester (Intermediate 17). Exact mass calcd for C₃₀H₃₀CF₃N₂O₅ (M+H⁺): 555.2107, found 555.2088; ¹H NMR (400 MHz, CDCl₃).

### Example 84

### 3-[4-(3-{[(1,3-Dimethyl-5-trifluoromethyl-1H-indole-2-carbonyl)-amino]-methyl}-phenoxy)-2-methyl-phenyl]-propionic acid

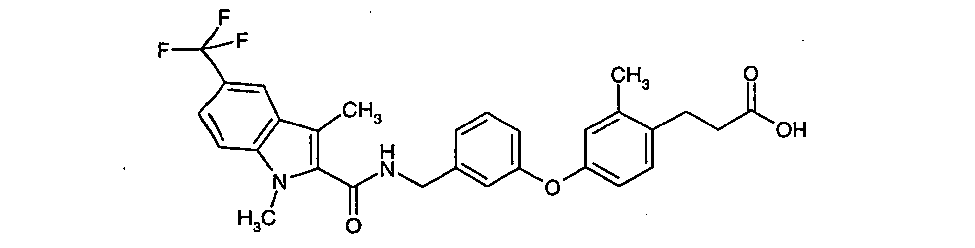

The title compound is prepared according to Example 1 utilizing 1,3-dimethyl-5-trifluoromethyl-1H-indole-2-carboxylic acid (Intermediate 6) and 3-[4-(3-aminomethyl-phenoxy)-2-methyl-phenyl]-propionic acid methyl ester (Intermediate 33). Exact mass calcd for C₂₉H₂₈F₃N₂O₄ (M+H⁺): 525.2001, found 525.1984; ¹H NMR (400 MHz, CDCl₃).

### Example 85

### 2-[4-(3-{[(5-Fluoro-3-methyl-1H-indole-2-carbonyl)-amino]-methyl}-phenoxy)-2-methyl-phenoxy]-2-methyl-propionic acid

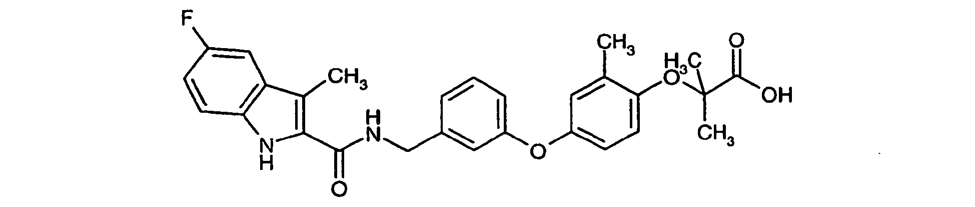

The title compound is prepared according to Example 1 utilizing 5-fluoro-3-methyl-1H-indole-2-carboxylic acid (Intermediate 1) and 2-[4-(3-aminomethyl-phenoxy)-2-methyl-phenoxy]-2-methyl-propionic acid ethyl ester (Intermediate 17). Exact mass calcd for C₂₈H₂₈FN₂O₅ (M+H⁺): 491.1982, found 491.1995; ¹H NMR (400 MHz, CDCl₃).

### Example 86

### 2-[4-(3-Fluoro-5-{[(5-fluoro-3-methyl-1H-indole-2-carbonyl)-amino]-methyl}-phenoxy)-2-methyl-phenoxy]-2-methyl-propionic acid

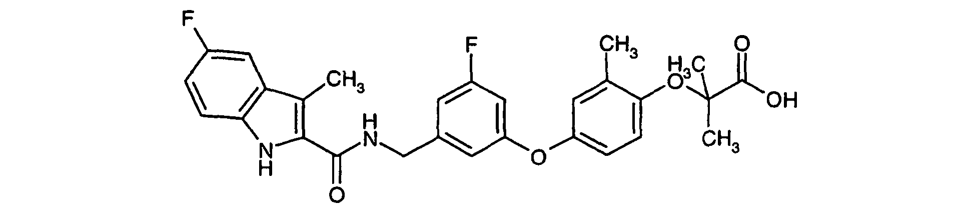

The title compound is prepared according to Example 1 utilizing 5-fluoro-3-methyl-1H-indole-2-carboxylic acid (Intermediate 1) and 2-[4-(3-aminomethyl-5-fluoro-phenoxy)-2-methyl-phenoxyl-2-rnethyl-propionic acid ethyl ester (Intermediate 47). Exact mass calcd for C₂₈H₂₇F₂N₂O₅ (M+H⁺): 509.1879, found 509.1888; ¹H NMR (400 MHz, CDCl₃).

### Example 87

### 2-(4-{5-[(2-Fluoro-4-trifluoromethyl-benzoylamino)-methyl]-2-methyl-phenoxy}-2-methyl-phenoxy)-2-methyl-propionic acid

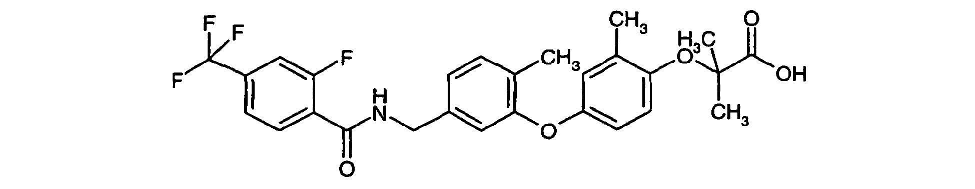

The title compound is prepared according to Example 1 utilizing 2-fluoro-4-trifluoro-benzoic acid and 2-[4-(5-aminomethyl-2-methyl-phenoxy)-2-methyl-phenoxy]-2-methyl-propionic acid ethyl ester (Intermediate 21). Exact mass calcd for C₂₇H₂₆F₄NO₅ (M+H⁺): 520.1747, found 520.1730; ¹H NMR (400 MHz, CDCl₃).

### Example 88

### 2-(4-{4-Fluoro-3-[(2-fluoro-4-trifluoromethyl-benzoylamino)-methyl]-phenoxy}-2-methyl-phenoxy)-2-methyl-propionic acid

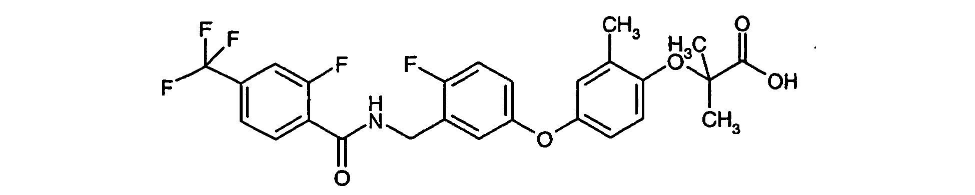

The title compound is prepared according to Example 1 utilizing 2-fluoro-4-trifluoro-benzoic acid and 2-[4-(3-aminomethyl-4-fluoro-phenoxy)-2-methyl-phenoxy]-2-methyl-propionic acid ethyl ester (Intermediate 22). Exact mass calcd for C₂₆H₂₃F₅NO₅ (M+H⁺): 524.1496, found 524.1494; ¹H NMR (400 MHz, CDCl₃).

### Example 89

### 3-[4-(3-{[(3-Chloro-1H-indole-6-carbonyl)-amino]-methyl}-phenoxy)-2-methyl-phenyl]-propionic acid

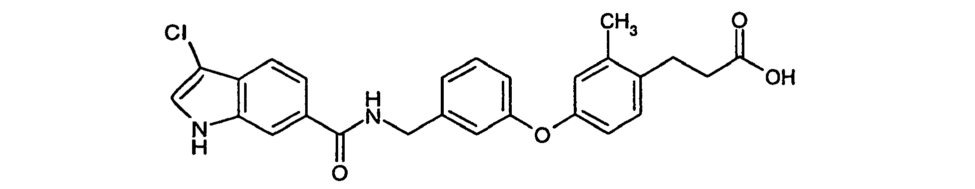

The title compound is prepared according to Example 1 utilizing 3-chloro-1H-indole-6-carboxylic acid (WO 9900128) and 3-[4-(3-aminomethyl-phenoxy)-2-methyl-phenyl]-propionic acid methyl ester (Intermediate 33). Exact mass calcd for C₂₆H₂₄ClN₂O₄ (M+H⁺): 463.1420, found 463.1425; ¹H NMR (400 MHz, CDCl₃).

### Example 90

### 3-[4-(3-Fluoro-5-{1-[(5-fluoro-1,3-dimethyl-1H-indole-2-carbonyl)-amino]-ethyl}-phenoxy)-2-methyl-phenyl]-propionic acid, isomer 1

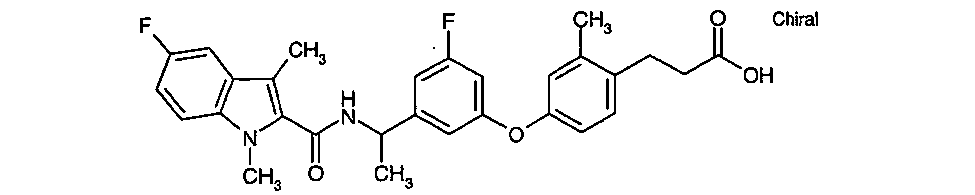

The methyl ester of the title compound is prepared according to Example 1, Step A utilizing 5-fluoro-1,3-dimethyl-1H-indole-2-carboxylic acid (Intermediate 2) and (R, S)-3-{4-[3-(1-amino-ethyl)-5-fluoro-phenoxy]-2-methyl-phenyl}-propionic acid methyl ester (Intermediate 34). Chiral chromatography is used to separate the ester enamtiomers. The title compound is then prepared according to Example 1, Step B utilizing the pure ester enamtiomer. Exact mass calcd for C₂₉H₂₉F₂N₂O₄ (M+H⁺): 507.2095, found 507.20.96; ¹H NMR (400 MHz, CDCl₃).

### Example 91

### 3-[4-(3-Fluoro-5-{1-[(5-fluoro-1,3-dimethyl-1H-indole-2-carbonyl)-amino]-ethyl}-phenoxy)-2-methyl-phenyl]-propionic acid, isomer 2

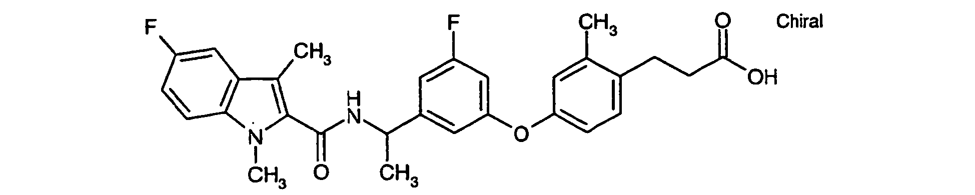

The methyl ester of the title compound is prepared according to Example 1, Step A utilizing 5-fluoro-1,3-dimethyl-1H-indole-2-carboxylic acid (Intermediate 2) and (R, S)-3-{4-[3-(1-amino-ethyl)-5-fluoro-phenoxy]-2-methyl-phenyl}-propionic acid methyl ester (Intermediate 34). Chiral chromatography is used to separate the ester enamtiomers. The title compound is then prepared according to Example 1, Step B utilizing the pure ester enamtiomer. Exact mass calcd for C₂₉H₂₉F₂N₂O₄ (M+H⁺): 507.2095, found 507.2096; ¹H NMR (400 MHz, CDCl₃).

### Example 92

### 3-[4-(3-{[(1,3-Dimethyl-5-trifluoromethyl-1H-indole-2-carbonyl)-amino]-methyl}-5-fluoro-phenoxy)-2-methyl-phenyl]-2,2-dimethyl-propionic acid

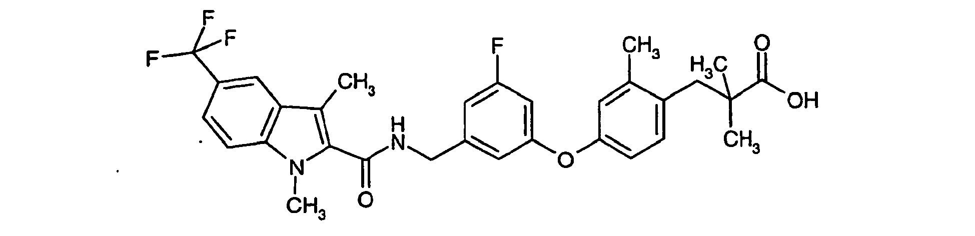

The title compound is prepared according to Example 1 utilizing 1,3-dimethyl-5-trifluoromethyl-1H-indole-2-carboxylic acid (Intermediate 6) and 3-[4-(3-aminomethyl-5-fluoro-phenoxy)-2-methyl-phenyl]-2,2-dimethyl-propionic acid methyl ester (Intermediate 42). Exact mass calcd for C₃₁H₃₁F₄N₂O₄ (M+H⁺): 571.2220, found 571.2208; ¹H NMR (400 MHz, CDCl₃).

### Example 93

### 2-(4-{3-Fluoro-5-[1-(2-methyl-4-trifluoromethyl-benzoylamino)-ethyl]-phenoxy}-2-methyl-phenoxy)-2-methyl-propionic acid, isomer 1

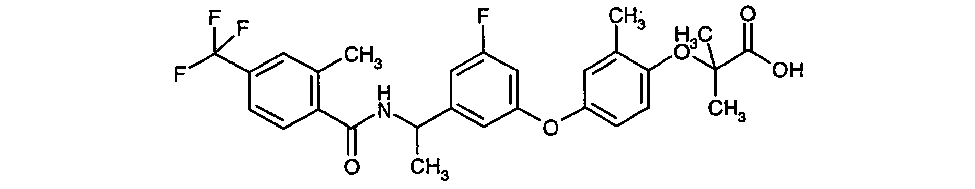

The methyl ester of the title compound is prepared according to Example 1, Step A utilizing 2-methyl-4-trifluoromethyl-benzoic acid (Intermediate 10) and (R, S)-2-{4-[3-(1-amino-ethyl)-5-fluoro-phenoxy]-2-methyl-phenoxy}-2-methyl-propionic acid methyl ester (Intermediate 41). Chiral chromatography is used to separate the ester enamtiomers. The title compound is then prepared according to Example 1, Step B utilizing the pure ester enamtiomer. Exact mass calcd for C₂₈H₂₈F₄NO₅ (M+H⁺): 534.1904, found 534.1887; ¹H NMR (400 MHz, CDCl₃).

### Example 94

### 2-(4-{3-Fluoro-5-[1-(2-methyl-4-trifluoromethyl-benzoylamino)-ethyl]-phenoxy}-2-methyl-phenoxy)-2-methyl-propionic acid, isomer 2

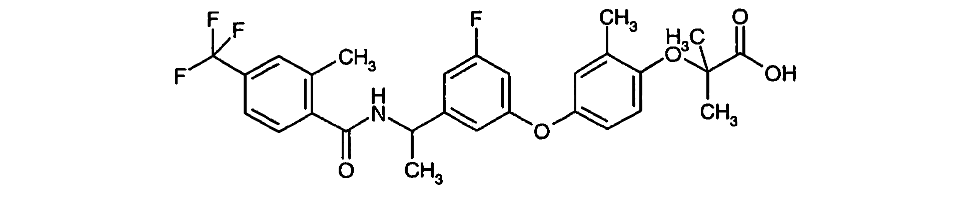

The methyl ester of the title compound is prepared according to Example 1, Step A utilizing 2-methyl-4-trifluoromethyl-benzoic acid (Intermediate 10) and (R, S)-2-{4-[3-(1-amino-ethyl)-5-fluoro-phenoxy]-2-methyl-phenoxy}-2-methyl-propionic acid methyl ester (Intermediate 41). Chiral chromatography is used to separate the ester enamtiomers. The title compound is then prepared according to Example 1, Step B utilizing the pure ester enamtiomer. Exact mass calcd for C₂₈H₂₈F₄NO₅ (M+H⁺): 534.1904, found 534.1884; ¹H NMR (400 MHz, CDCl₃).

### Example 95

### 2-Methyl-2-(2-methyl-4-{3-methyl-5-[(4-trifluoromethyl-benzoylamino)-methyl]-phenoxy}-phenoxy)-propionic acid

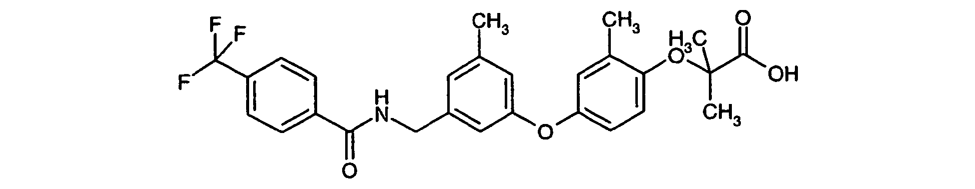

The title compound is prepared according to Example 1 utilizing 4-trifluoromethyl-benzoic acid and 2-[4-(3-aminomethyl-5-methyl-phenoxy)-2-methyl-phenoxy]-2-methyl-propionic acid ethyl ester (Intermediate 20). Exact mass calcd for C₂₇H₂₇F₃NO₅ (M+H⁺): 502.1841, found 502.1841; ¹HNMR (400 MHz, CDCl₃).

### Example 96

### 2-Methyl-2-[2-methyl-4-(3-methyl-5-{[methyl-(4-trifluoromethyl-benzoyl)-amino]-methyl }-phenoxy)-phenoxy]-propionic acid

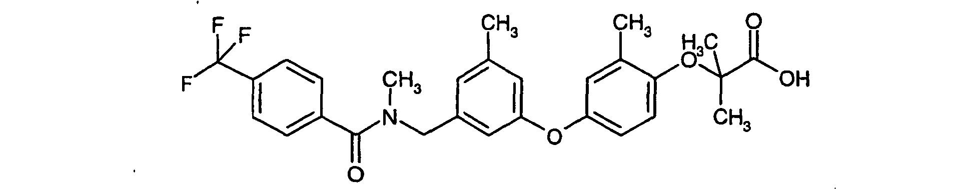

The title compound is prepared according to Intermediate 2, Steps A and B, utilizing 2-methyl- 2-(2-methyl-4- {3-methyl-5-[(4-trifluoromethyl-benzoylamino)-methyl]-phenoxy}-phenoxy)-propionic acid ethyl ester (Example 95, Step A). Exact mass calcd for C₂₈H₂₉F₃NO₅ (M+H⁺): 516.1998, found 516.1990; ¹H NMR (400 MHz, DMSO-D6).

### Example 97

### 3-(2-Ethyl-4-{3-fluoro-5-[(2-fluoro-4-trifluoromethyl-benzoylamino)-methyl]-phenoxy}-phenyl)-propionic acid

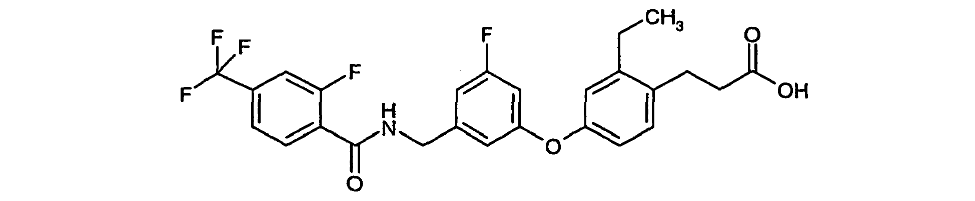

The title compound is prepared according to Example 1 utilizing 2-fluoro-5-trifluoromethyl-benzoic acid and 3-[4-(3-aminomethyl-5-fluoro-phenoxy)-2-ethylphenyl]-propionic acid ethyl ester (Intermediate 27). Exact mass calcd for C₂₆H₂₃F₅NO₄ (M+H⁺): 508.1547, found 508.1534; ¹H NMR (400 MHz, CDCl₃).

### Example 98

### 3-[4-(3-{[(5-Chloro-1,3-dimethyl-1H-indole-2-carbonyl)-amino]-methyl}-5-fluoro-phenoxy)-2-ethyl-phenyl]-propionic acid

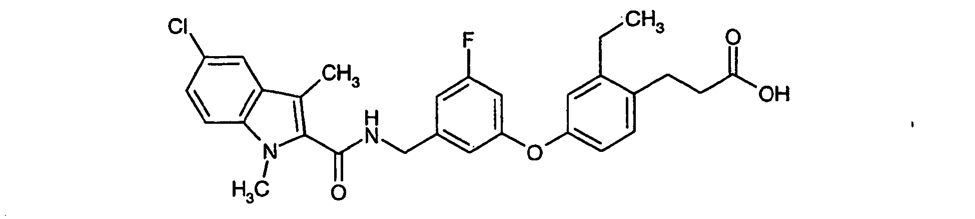

The title compound is prepared according to Example 1 utilizing 5-chloro-1,3-dimethyl-1H-indole-2-carboxylic acid (Intermediate 4) and 3-[4-(3-aminomethyl-5-fluoro-phenoxy)-2-ethyl-phenyl]-propionic acid ethyl ester (Intermediate 27). Exact mass calcd for C₂₉H₂₉ClFN₂O₄ (M+H⁺): 523.1800, found 523.1776; ¹H NMR (400 MHz, CDCl₃).

### Example 99

### 3-[2-Ethyl-4-(3-fluoro-5-{[(5-fluoro-3-methyl-1H-indole-2-carbonyl)-amino]-methyl}-phenoxy)-phenyl]-propionic acid

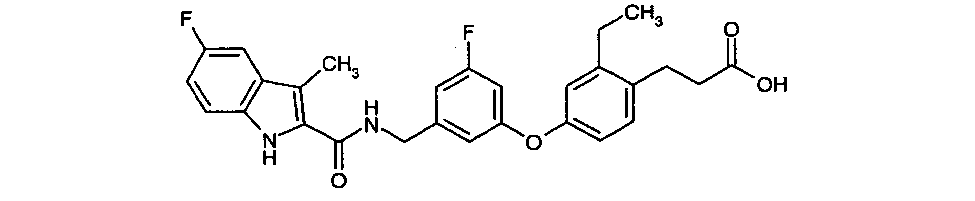

The title compound is prepared according to Example 1 utilizing 5-fluoro-3-methyl-1H-indole-2-carboxylic acid (Example 1) and 3-[4-(3-aminomethyl-5-fluoro-phenoxy)-2-ethyl-phenyl]-propionic acid ethyl ester (Intermediate 27). Exact mass calcd for C₂₈H₂₇F₂N₂O₄ (M+H⁺): 493.1939, found 493.1942; ¹H NMR (400 MHz, CDCl₃).

### Example 100

### (R)-3-[4-(3-{1-[(5-Chloro-1,3-dimethyl-1H-indole-2-carbonyl)-amino]-ethyl}-phenoxy)-2-methyl-phenyl]-propionic acid

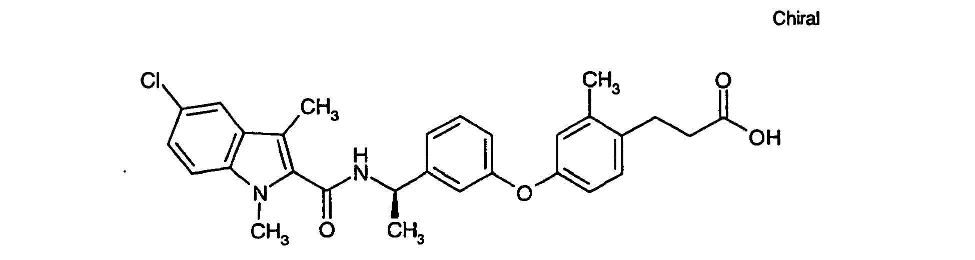

The title compound is prepared according to Example 1 utilizing 5-chloro-1,3-dimethyl-1H-indole-2-carboxylic acid (Intermediate 4) and (R)-3-{4-[3-(1-aminoethyl)-phenoxy]-2-methyl-phenyl}-propionic acid methyl ester (Intermediate 24). Exact mass calcd for C₂₉H₃₀ClN₂O₄ (M+H⁺): 505.1894, found 505.1909; ¹H NMR (400 MHz, CDCl₃).

### Example 101

### (R)-3-[4-(3-{1-[(5-Fluoro-1,3-dimethyl-1H-indole-2-carbonyl)-amino]-ethyl }-phenoxy)-2-methyl-phenyl]-propionic acid

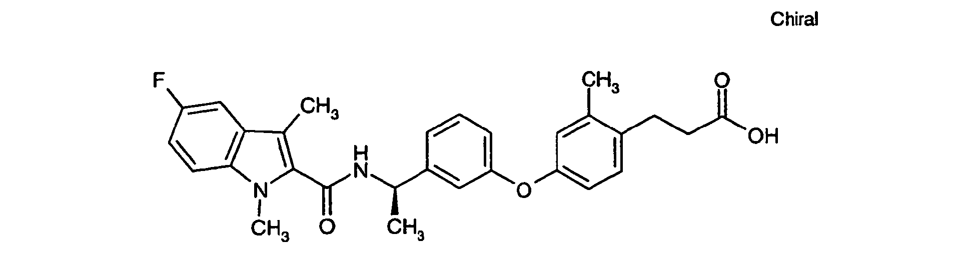

The title compound is prepared according to Example 1 utilizing 5-fluoro-1,3-dimethyl-1H-indole-2-carboxylic acid (Intermediate 2) and (R)-3-{4-[3-(1-aminoethyl)-phenoxy]-2-methyl-phenyl)-propionic acid methyl ester (Intermediate 24). Exact mass calcd for C₂₉H₃₀FN₂O₄ (M+H⁺): 489.2189, found 489.2190;¹H NMR (400 MHz, CDCl₃).

### Example 102

### (R)-3-[4-(3-(1-[(5-Fluoro-3-methyl-1H-indole-2-carbonyl)-aminol-ethyl)-phenoxy)-2-methyl-phenyl]-propionic acid

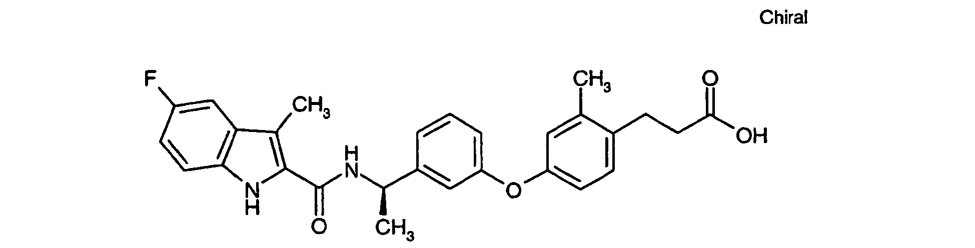

The title compound is prepared according to Example 1 utilizing 5-fluoro-3-methyl-1H-indole-2-carboxylic acid (Intermediate 1) and (R)-3-{4-[3-(1-amino-ethyl)-phenoxy]-2-methyl-phenyl}-propionic acid methyl ester (Intermediate 24). Exact mass calcd for C₂₈H₂₈FN₂O₄ (M+H⁺): 475.2021, found 475.2033; ¹H NMR (400 MHz, CDCl₃).

### Example 103

### 2-[4-(3-Fluoro-5-{1-[(3-methyl-benzo[b]thiophene-2-carbonyl)-amino]-ethyl}-phenoxy)-2-methyl-phenoxy]-2-methyl-propionic acid, isomer 1

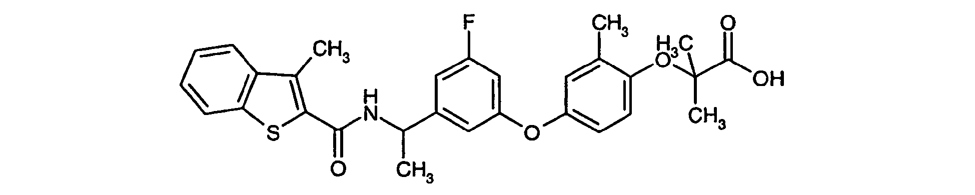

The ethyl ester of the title compound is prepared according to Example 1 Step A utilizing 3-methyl-benzo[b]thiophene-2-carboxylic acid and (R, S)-2-{4-[3-(1-amino-ethy)-5-fluoro-phenoxy]-2-methyl-phenoxy)-2-methyl-propionic acid ethyl ester (Intermediate 41). Chiral chromatography is used to separate the ester enamtiomers. The title compound is then prepared according to Example 1, Step B utilizing the pure ester enamtiomer. Exact mass calcd for C₂₉H₂₉FNO₅S (M+H⁺): 522.1750, found 522.1749; ¹H NMR (400 MHz, CDCl₃).

### Example 104

### 2-[4-(3-Fluoro-5-{1-[(3-methyl-benzo[b]thiophene-2-carbonyl)-amino]-ethyl}-phenoxy)-2-methyl-phenoxy]-2-methyl-propionic acid, isomer 2

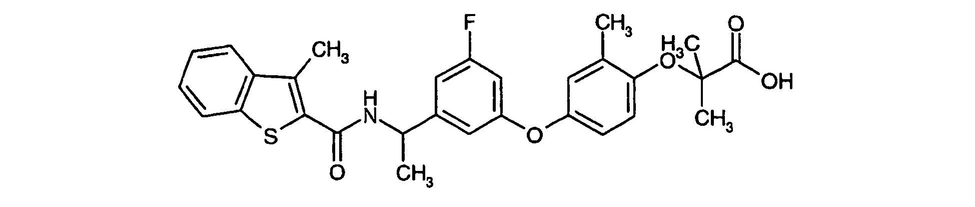

The ethyl ester of the title compound is prepared according to General Procedure, Step A as described in Example 1 by utilizing 3-methyl-benzo[b]thiopbene-2-carboxylic acid and (R, S)-2-{4-[3-(1-amino-ethyl)-5-fluoro-phenoxy]-2-methyl-phenoxy)-2-methyl-propionic acid ethyl ester (Intermediate 41). Chiral chromatography is used to separate the ester enamtiomers. The title compound is then prepared according to Example 1, Step B utilizing the pure ester enamtiomer. Exact mass calcd for C₂₉H₂₉FNO₅S (M+H⁺): 522.1750, found 522.1749; ¹H NMR (400 MHz, CDCl₃).

### Example 105

### 2-[4-(3-Fluoro-5-{1-[(5-fluoro-1,3-dimethyl-1H-indole-2-carbonyl)-amino]-ethyl}-phenoxy)-2-methyl-phenoxy]-2-methyl-propionic acid, isomer 1

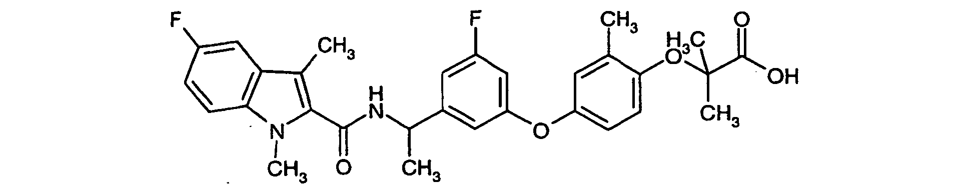

The ethyl ester of the title compound is prepared according to Example 1, Step A utilizing 5-fluoro-1,3-dimethyl-1H-indole-2-carboxylic acid (Intermediate 2) and (R, S)-2-{4-(3-(1-amino-ethyl)-5-fluoro-phenoxy]-2-methyl-phenoxy}-2-methyl-propionic acid ethyl ester (Intermediate 41). Chiral chromatography is used to separate the ester enamtiomers. The title compound is then prepared according to Example 1, Step B utilizing the pure ester enamtiomer. Exact mass calcd for C₃₀H₃₀F₂N₂O₅ (M+H⁺):, found; ¹H NMR (400 MHz, CDCl₃).

### Example 106

### 2-[4-(3-Fluoro-5-{1-[(5-fluoro-1,3-dimethyl-1H-indole-2-carbonyl)-amino]-ethyl}-phenoxy)-2-methyl-phenoxy]-2-methyl-propionic acid, isomer 2

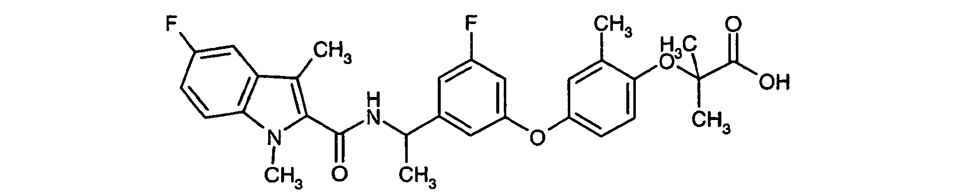

The ethyl ester of the title compound is prepared according to Example 1, Step A utilizing 5-fluoro-1,3-dimethyl-1H-indole-2-carboxylic acid (Intermediate 2) and (R, S)-2-{4-[3-(1-amino-ethyl)-5-fluoro-phenoxy]-2-methyl-phenoxy}-2-methyl-propionic acid ethyl ester (Intermediate 41). Chiral chromatography is used to separate the ester enamtiomers. The title compound is then prepared according to Example 1, Step B utilizing the pure ester enamtiomer. Exact mass calcd for C₃₀H₃₀F₂N₂O₅ (M+H⁺): found; ¹H NMR (400 MHz, CDCl₃).

### Example 107

### 3-(2-Methyl-4-{2-[2-(2-methyl-4-trifluoromethyl-benzoylamino)-ethyl]-phenoxy)-phenyl)-propionic acid

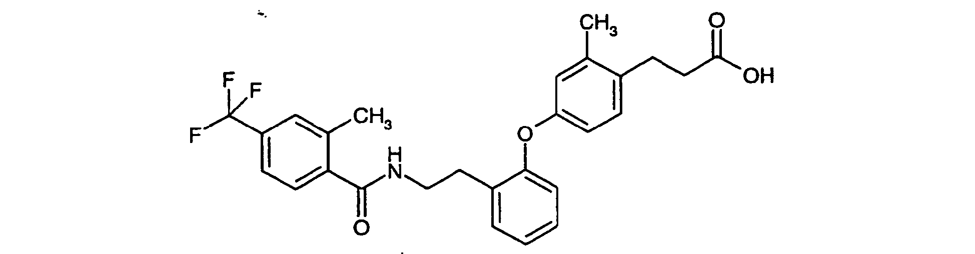

The title compound is prepared according to Example 1 utilizing 2-methyl-4-trifluoromethyl-benzoic acid (Intermediate 10) and 3-{4-[2-(2-amino-ethyl)-phenoxy]-2-methyl-phenyl}-propionic acid ethyl ester (Intermediate 28). Exact mass calcd for C₂₇H₂₇F₃NO₄ (M+H⁺): 486.1892, found 486.1893; ¹H NMR (400 MHz, CDCl₃).

### Example 108

### 3-[4-(2-{2-[(5-Fluoro-3-methyl-1H-indole-2-carbonyl)-amino]-ethyl)-phenoxy)-2-methyl-phenyl]-propionic acid

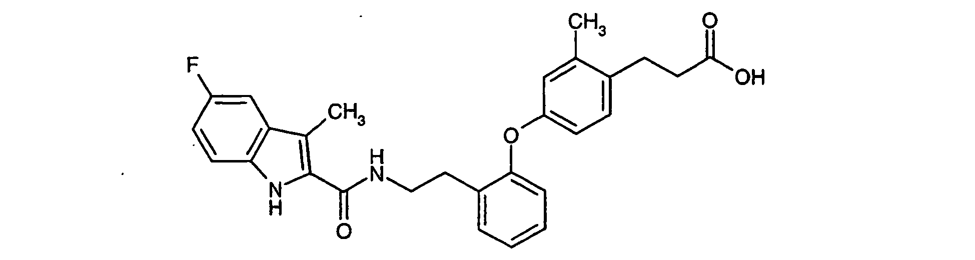

The title compound is prepared according to Example 1 utilizing 5-fluoro-3-methyl-1H-indole-2-carboxylic acid (Intermediate 1) and 3-{4-[2-(2-amino-ethyl)-phenoxy]-2-methyl-phenyl}-propionic acid ethyl ester (Intermediate 28). Exact mass calcd for C₂₈H₂₈FN₂O₄ (M+H⁺): 475.2033, found 475.2015; ¹H NMR (400 MHz, CDCl₃).

### Example 109

### 3-[4-(2-{2-[(5-Chloro-3-methyl-1H-indole-2-carbonyl)-amino]-ethyl }-phenoxy)-2-methyl-phenyl]-propionic acid

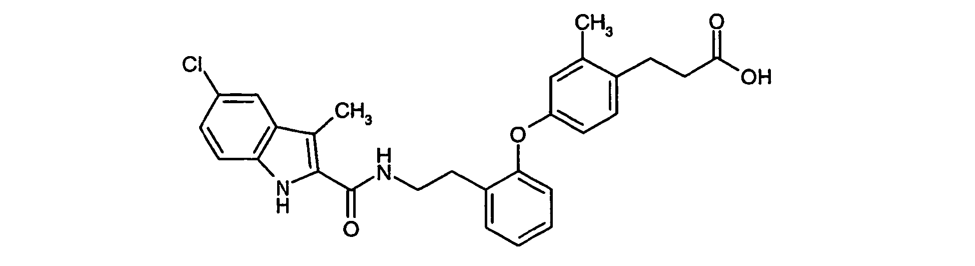

The title compound is prepared according to Example 1 utilizing 5-chloro-3-methyl-1H-indole-2-carboxylic acid (Intermediate 3) and 3-(4-[2-(2-amino-ethyl)-phenoxy]-2-methyl-phenyl}-propionic acid ethyl ester (Intermediate 28). Exact mass calcd for C₂₈H₂₈ClN₂O₄ (M+H⁺): 491.1737, found 491.1724; ¹H NMR (400 MHz, CDCl₃).

### Example 110

### [3-(3-{[(5-Chloro-1,3-dimethyl-1H-indole-2-carbonyl)-amino]-methyl}-5-fluoro-phenoxy)-phenyl]-acetic acid

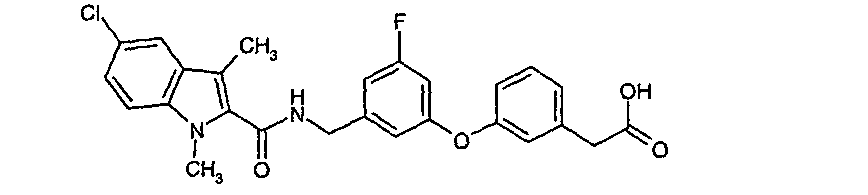

The title compound is prepared according to Example 1 utilizing 5-chloro-1,3-dimethyl-1H-indole-2-carboxylic acid (Intermediate 4) and [3-(3-aminomethyl-5-fluoro-phenoxy)-phenyl]-acetic acid methyl ester (Intermediate 48). Exact mass calcd for C₂₆H₂₃ClFN₂O₄ (M+H⁺): 481.1330, found 481.1327; ¹H NMR (400 MHz, CDCl₃).

### Example 111

### [3-(3-Fluoro-5-{[(5-fluoro-3-methyl-1H-indole-2-carbonyl)-amino]-methyl}phenoxy)-phenyl]-acetic acid

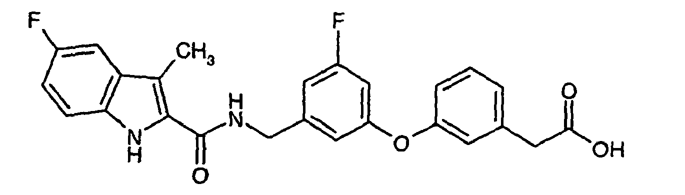

The title compound is prepared according to Example 1 utilizing 5-fluoro-3-methyl-1H-indole-2-carboxylic acid (Intermediate 1) and [3-(3-aminomethyl-5-fluoro-phenoxy)-phenyl]-acetic acid methyl (Intermediate 48). Exact mass calcd for C₂₅H₂₁F₂N₂O₄ (M+H⁺): 451.1469, found 451.1478; ¹H NMR (400 MHz, DMSO-D6).

### Example 112

### [3-(3-{[(5-Chloro-3-methyl-benzo[b]thiophene-2-carbonyl)-amino]-methyl}-5-fluoro-phenoxy)-phenyl]-acetic acid

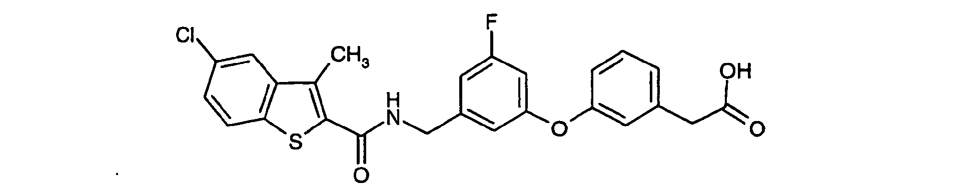

The title compound is prepared according to Example 1 utilizing 5-chloro-3-methyl-benzo[b]thiophene-2-carboxylic acid and [3-(3-aminomethyl-5-fluoro-phenoxy)-phenyl]-acetic acid methyl ester (Intermediate 48). Exact mass calcd for C₂₅H₂₀ClFNO₄S (M+H⁺): 484.0786, found 484.0778; ¹H NMR (400 MHz, CDCl₃).

### Example 113

### (3-{3-Fluoro-5-[(2-methyl-4-trifluoromethyl-benzoylamino)-methyl]-phenoxy}-phenyl)-acetic acid

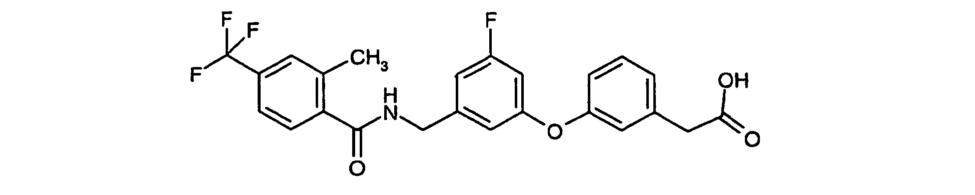

The title compound is prepared according to Example 1 utilizing 3-methyl-5-trifluoromethyl-benzoic acid (Intermediate 10) and [3-(3-aminomethyl-5-fluoro-phenoxy)-phenyl]-acetic acid methyl ester (Intermediate 48). Exact mass calcd for C₂₄H₂₀F₄NO₄ (M+H⁺): 462.1332, found 462.1328; ¹H NMR (400 MHz, DMSO-D₆).

### Example 114

### 2-[4-(3-{[(1-Ethyl-5-fluoro-3-methyl-1H-indole-2-carbonyl)-amino]-methyl}-5-fluoro-phenoxy)-2-methyl-phenoxy]-2-methyl-propionic acid

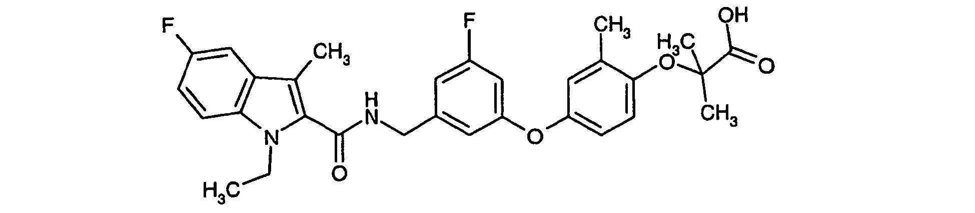

The title compound is prepared according to Example 1 utilizing 1-ethyl-5-fluoro-3-methyl-1H-indole-2-carboxylic acid (Intermediate 9) and 2-[4-(3-aminomethyl-5-fluoro-phenoxy)-2-methyl-phenoxy]-2-methyl-propionic acid (Intermediate 47). Exact mass calcd for C₃₀H₃₁F₂N₂O₅ (M+H⁺): 537.2201, found 537.2215; ¹H NMR (400 MHz, CDCl₃).

### Example 115

### 3-[4-(3-{([(1-Ethyl-5-fluoro-3-methyl-1H-indole-2-carbonyl)-amino]-methyl}-5-fluoro-phenoxy)-2-methyl-phenyl]-propionic acid

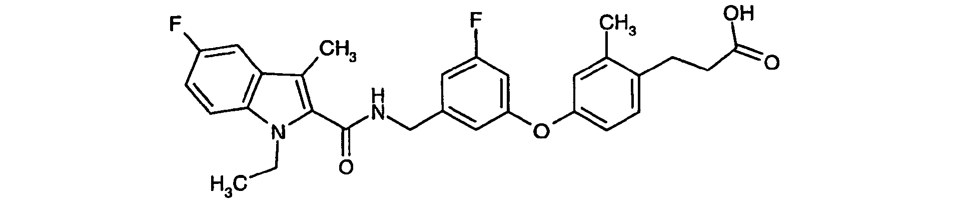

The title compound is prepared according to Example 1 utilizing 1-ethyl-5-fluoro-3-methyl-1H-indole-2-carboxylic acid (Intermediate 9) and 3-[4-(3-aminomethyl-5-fluoro-phenoxy)-2-methyl-phenyl]-propionic acid methyl ester (Intermediate 36). Exact mass calcd for C₂₉H₂₉F₂N₂O₄ (M+H⁺): 507.2095, found 507.2096; ¹H NMR (400 MHz, DMSO-D6).

### Example 116

### (R)-3-[4-(3-{1-[(5-Chloro-3-propyl-1H-indole-2-carbonyl)-amino]-ethyl}-phenoxy)-2-methyl-phenyl]-propionic acid

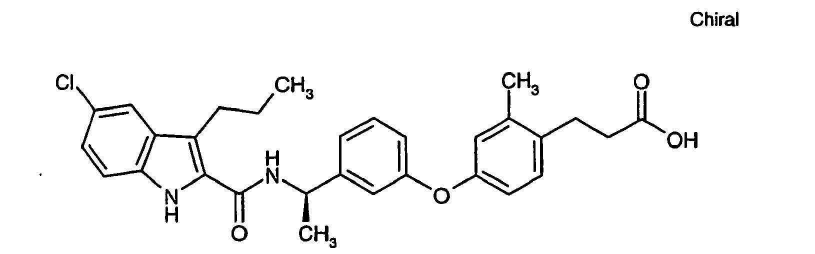

The title compound is prepared according to Example 1 utilizing 5-chloro-3-propyl-1H-indole-2-carboxylic acid (Intermediate 7) and (R)-3-{4-[3-(1-amino-ethyl)-phenoxy]-2-methyl-phenyl}-propionic acid methyl ester (Intermediate 24). Exact mass calcd for C₃₀H₃₂ClN₂O₄ (M+H⁺): 519.2051, found 519.2038; ¹H NMR (400 MHz, CDCl₃).

### Example 117

### 3-[4-(3- {[(5-Chloro-3-propyl- 1H-indole-2-carbonyl)-amino]-methyl}-5-fluoro-phenoxy)-2-methyl-phenyl]-propionic acid

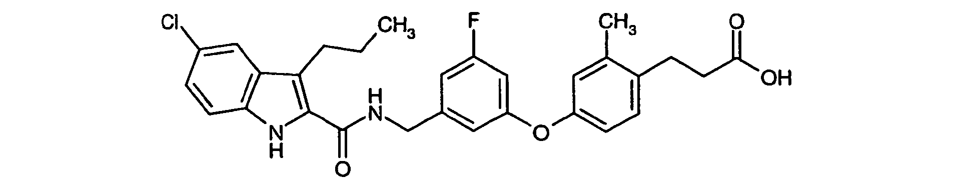

The title compound is prepared according to Example 1 utilizing 5-chloro-3-propyl-1H-indole-2-carboxylic acid (Intermediate 7) and 3-[4-(3-aminomethyl-5-fluoro-phenoxy)-2-methyl-phenyl]-propionic acid methyl ester (Intermediate 36). Exact mass calcd for C₂₉H₂₉ClFN₂O₄ (M+H⁺): 523.1800, found 523.1810; ¹H NMR (400 MHz, CDCl₃).

### Example 118

### (R)-3-(2-Ethyl-4-{3-fluoro-5-[1-(2-fluoro-4-trifluoromethyl-benzoylamino)-ethyl]-phenoxy}-phenyl)-propionic acid

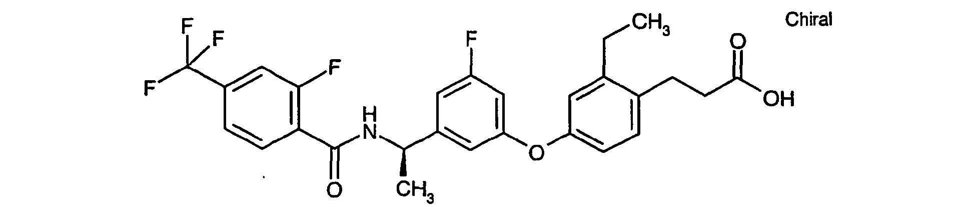

The title compound is prepared according to Example 1 utilizing 2-fluoro-5-trifluoromethyl-benzoic acid and (R)-3-{4-[3-(1-amino-ethyl)-5-fluoro-phenoxy]-2-ethyl-phenyl}-propionic acid ethyl ester (Intermediate 23). Exact mass calcd for C₂₇H₂₅F₅NO₄ (M+H⁺): 522.1703, found 522.1686; ¹H NMR (400 MHz, CDCl₃).

### Example 119

### (R)-3-[4-(3-{1-[(5-Chloro-1,3-dimethyl-1H-indole-2-carbonyl)-amino]-ethyl}-5-fluoro-phenoxy)-2-ethyl-phenyl]-propionic acid

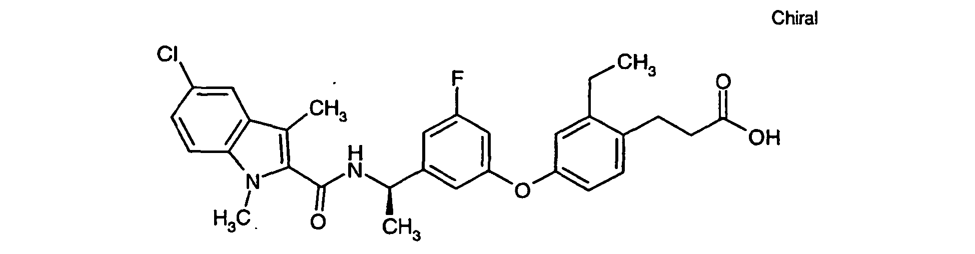

The title compound is prepared according to Example 1 utilizing 5-chloro-1,3-dimethyl-1H-indole-2-carboxylic acid (Intermediate 4) and (R)-3-{4-[3-(1-aminoethyl)-5-fluoro-phenoxy]-2-ethyl-phenyl}-propionic acid ethyl ester (Intermediate 23). Exact mass calcd for C₃₀H₃₁ClFN₂O₄ (M+H⁺): 537.1956, found 537.1956; ¹H NMR (400 MHz, DMSO-D6).

### Example 120

### (R)-3-[4-(3-{1-[(5-Chloro-1-methyl-3-propyl-1H-indole-2-carbonyl)-amino]-ethyl}-phenoxy)-2-methyl-phenyl]-propionic acid

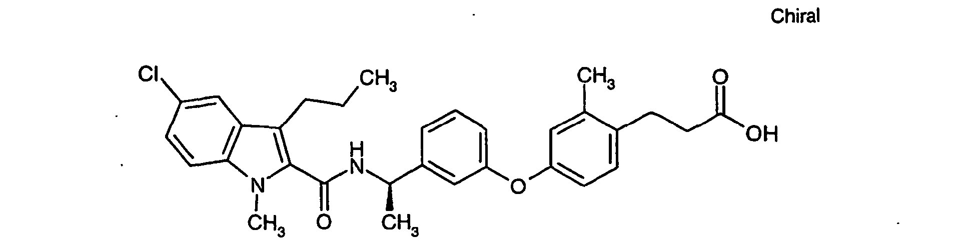

The title compound is prepared according to Example 1 utilizing 5-chloro-1-methyl-3-propyl-1H-indole-2-carboxylic acid (Intermediate 8) and (R)-3-{4-[3-(1-amino-ethyl)-phenoxy]-2-methyl-phenyl}-propionic acid methyl ester (Intermediate 24). Exact mass calcd for C₃₁H₃₄ClN₂O₄ (M+H⁺): 533.2207, found 533.2197; ¹H NMR (400 MHz, DMSO-D6).

### Example 121

### (R)-3-[2-Ethyl-4-(3-fluoro-5-{1-[(5-fluoro-1,3-dimethyl-1H-indole-2-carbonyl)-amino]-ethyl }-phenoxy)-phenyl]-propionic acid

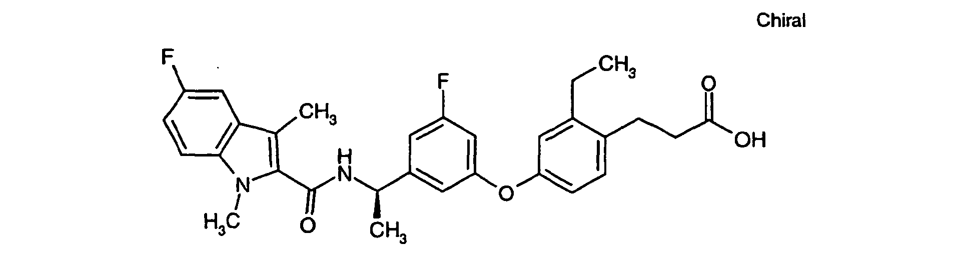

The title compound is prepared according to Example 1 utilizing 5-fluoro-1,3-dimethyl-1H-indole-2-carboxylic acid (Intermediate 2) and (R)-3-{4-[3-(1-aminoethyl)-5-fluoro-phenoxy]-2-ethyl-phenyl}-propionic acid ethyl ester (Intermediate 23). Exact mass calcd for C₃₀H₃₁F₂N₂O₄ (M+H⁺): 521.2252, found 521.2236; ¹H NMR (400 MHz, DMSO-D6).

### Example 122

### (R)-3-[2-Ethyl-4-(3-fluoro-5-{1-[(5-fluoro-3-methyl-1H-indole-2-carbonyl)-amino]-ethyl}-phenoxy)-phenyl]-propionic acid

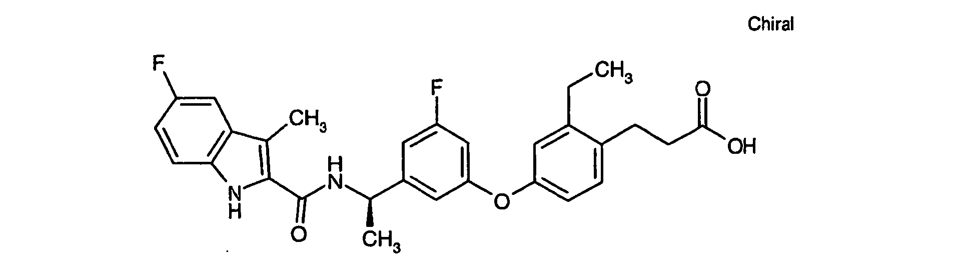

The title compound is prepared according to Example 1 utilizing 3-methyl-5-fluoro-1H-indole-2-carboxylic acid (Intermediate 1) and (R)-3-{4-[3-(1-amino-ethyl)-5-fluoro-phenoxy]-2-ethyl-phenyl)-propionic acid ethyl ester (Intermediate 23). Exact mass calcd for C₂₉H₂₉F₂N₂O₄ (M+H⁺): 507.2095, found 507.2087; ¹H NMR (400 MHz, DMSO-D6).

### Example 123

### (R)-3-[2-Ethyl-4-(3-fluoro-5-(1-[(5-fluoro-3-methyl-1H-indole-2-carbonyl)-amino]-ethyl} -phenoxy)-phenyl]-propionic acid

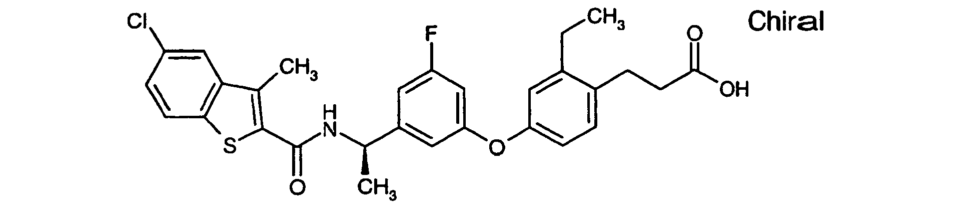

The title compound is prepared according to Example 1 utilizing 5-chloro-3-methyl-benzo[b]thiophene-2-carboxylic acid and (R)-3-{4-[3-(1-amino-ethyl)-5-fluoro-phenoxy]-2-ethyl-phenyl}-propionic acid ethyl ester (Intermediate 23). Exact mass calcd for C₂₉H₂₈ClFNO₄S (M+H⁺): 540.1412, found 540.1439; ¹H NMR (400 MHz, DMSO-D6).

### Example 124

### (R)-3-[4-(3-{1-[(5-Fluoro-1,3-dimethyl-1H-indole-2-carbonyl)-amino]-ethyl}-5-methyl-phenoxy)-2-methyl-phenyl]-propionic acid

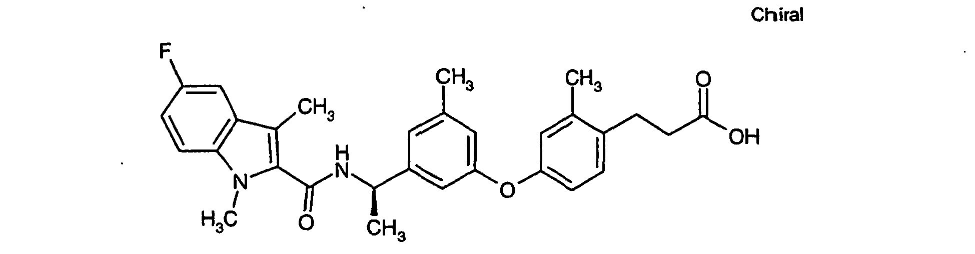

The title compound is prepared according to Example 1 utilizing 5-fluoro-1,3-dimethyl-1H-indole-2-carboxylic acid (Intermediate 2) and (R)-3-{4-[3-(1-aminoethyl)-5-methyl-phenoxy]-2-methyl-phenyl}-propionic acid methyl ester (Intermediate 26). Exact mass calcd for C₃₀H₃₂FN₂O₄ (M+H⁺): 518.1954, found 518.1956; ¹H NMR (400 MHz, DMSO-D6).

### Example 125

### (R)-3-[4-(3-{1-[(5-Fluoro-3-methyl-1H-indole-2-carbonyl)-amino]-ethyl)-5-methyl-phenoxy)-2-methyl-phenyl]-propionic acid

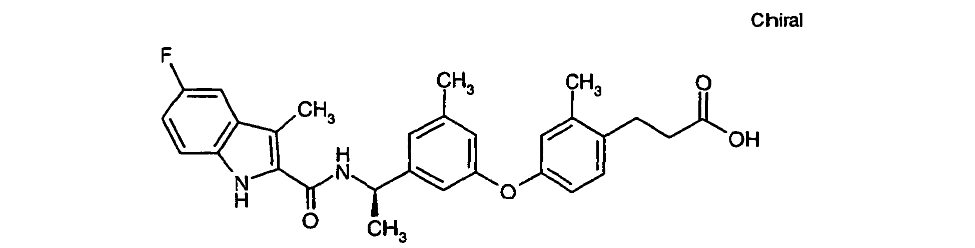

The title compound is prepared according to Example 1 utilizing 5-fluoro-3-methyl-1H-indole-2-carboxylic acid (Intermediate 1) and (R)-3-{4-[3-(1-amino-ethyl)-5-methyl-phenoxy]-2-methyl-phenyl)-propionic acid methyl ester (Intermediate 26). Exact mass calcd for C₂₉H₃₀FN₂O₄ (M+H⁺): 489.2190, found 489.2169; ¹H NMR (400 MHz, DMSO-D6).

### Example 126

### (R)-3-[4-(3-{1-[(5-Chloro-3'-methyl-benzo[b]thiophene-2-carbonyl)-amino]-ethyl}-5-methyl-phenoxy)-2-methyl-phenyl]-propionic acid

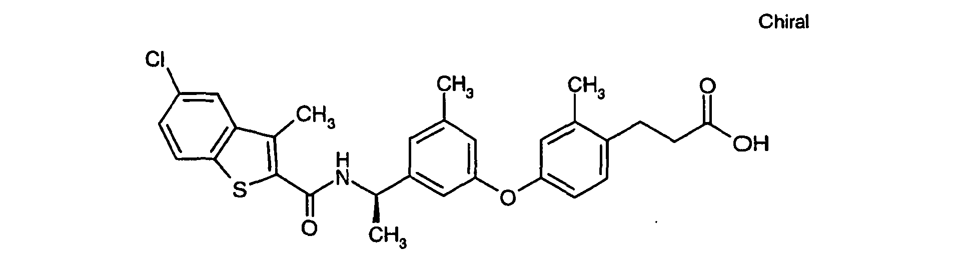

The title compound is prepared according to Example 1 utilizing 5-chloro-3-methyl-benzo[b]thiophene-2-carboxylic acid and (R)-3-{4-[3-(1-amino-ethyl)-5-methyl-phenoxy]-2-methyl-phenyl}-propionic acid methyl ester (Intermediate 26). Exact mass calcd for C₂₉H₂₉ClNO₄S (M+H⁺): 522.1506, found 522.1501; ¹H NMR (400 MHz, DMSO-D6).

### Example 127

### (R)-3-(2-Methyl-4-{3-methyl-5-[1-(2-methyl-4-trifluoromethyl-benzoylamino)-ethyl]-phenoxy} -phenyl)-propionic acid

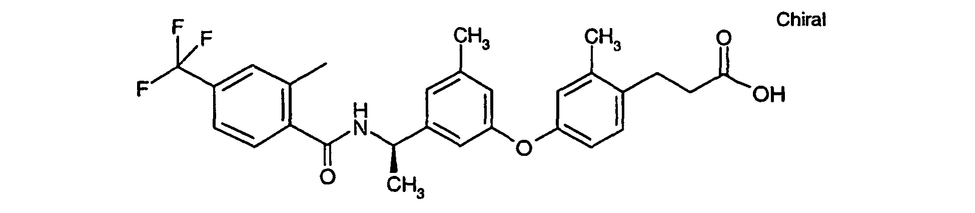

The title compound is prepared according to Example 1 utilizing 2-methyl-5-trifluoromethyl-benzoic acid (Intermediate 10) and (R)-3-{4-[3-(1-amino-ethyl)-5-methyl-phenoxy]-2-methyl-phenyl)-propionic acid methyl ester (Intermediate 26). Exact mass calcd for C₂₈H₂₉F₃NO₄ (M+H⁺): 500.2049, found 500.2016; ¹H NMR (400 MHz, CDCl₃).

### Example 128

### (R)-3-[2-Ethyl-4-(3-{1-[(5-fluoro-1,3-dimethyl-1H-indole-2-carbonyl)-amino]-ethyl}-5-methyl-phenoxy)-phenyl]-propionic acid

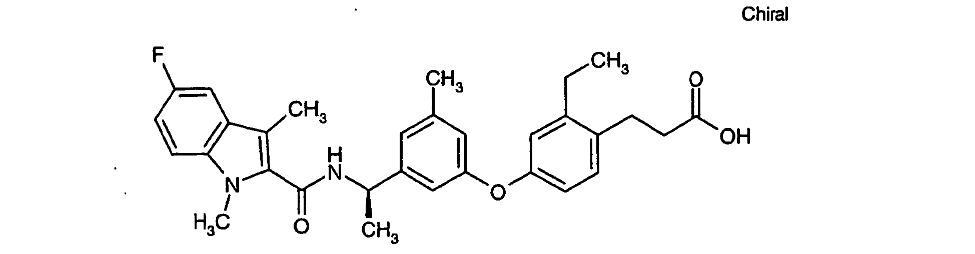

The title compound is prepared according to Example 1 utilizing 5-fluoro-1,3-dimethyl-1H-indole-2-carboxylic acid (Intermediate 2) and (R)-3-{4-[3-(1-aminoethyl)-5-methyl-phenoxy]-2-ethyl-phenyl}-propionic acid methyl ester (Intermediate 16). Exact mass calcd for C₃₁H₃₄FN₂O₄ (M+H⁺): 517.2502, found 517.2493; ¹H NMR (400 MHz, CDCl₃).

### Example 129

### (R)-3-[4-(3-{1-[(5-Chloro-1,3-dimethyl-1H-indole-2-carbonyl)-amino]-ethyl}-5-methyl-phenoxy)-2-ethyl-phenyl]-propionic acid

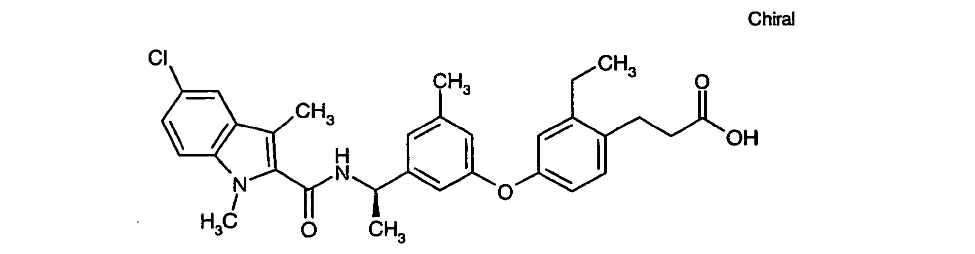

The title compound is prepared according to Example 1 utilizing 5-chloro-1,3-dimethyl-1H-indole-2-carboxylic acid (Intermediate 4) and (R)-3-{4-[3-(1-amino-ethyl)-5-methyl-phenoxy]-2-ethyl-phenyl}-propionic acid (Intermediate 16). Exact mass calcd for C₃₁H₃₄ClN₂O₄ (M+H⁺): 533.2207, found 533.2222; ¹H NMR (400 MHz, CDCl₃).

### Example 130

### 3-[2-Ethyl-4-(3-fluoro-5-{[(5-fluoro-1,3-dimethyl-1H-indole-2-carbonyl)-amino]-methyl}-phenoxy)-phenyl]-propionic acid

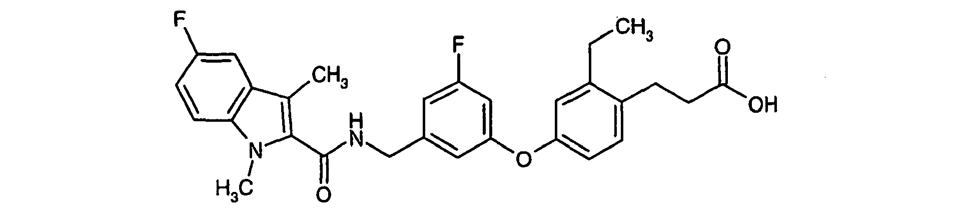

The title compound is prepared according to Example 1 utilizing 5-fluoro-1,3-dimethyl-1H-indole-2-carboxylic acid (Intermediate 2) and 3-{4-[3-(1-amino-ethyl)-5-fluoro-phenoxy]-2-ethyl-phenyl}-propionic acid ethyl ester (Intermediate 27). Exact mass calcd for C₂₉H₂₉F₂N₂O₄ (M+H⁺): 507.2095, found 507.2101; ¹H NMR (400 MHz, CDCl₃).

### Example 131

### (R)-3-[2-Ethyl-4-(3-{1-[(5-fluoro-3-methyl-1H-indole-2-carbonyl)-amino]-ethyl}-5-methyl-phenoxy)-phenyl]-propionic acid

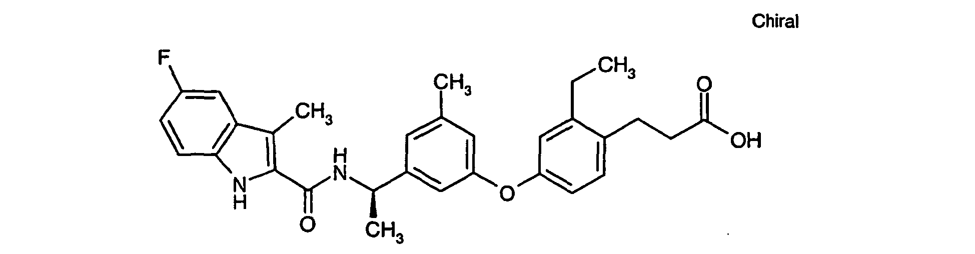

The title compound is prepared according to Example 1 utilizing 5-fluoro-3-methyl-1H-indole-2-carboxylic acid (Intermediate 1) and (R)-3-{4-[3-(1-amino-ethyl)-5-methyl-phenoxy]-2-ethyl-phenyl}-propionic acid (Intermediate 16). Exact mass calcd for C₃₀H₃₂FN₂O₄ (M+H⁺): 503.2346, found 503.2351; ¹H NMR (400 MHz, CDCl₃).

### Example 132

### 3-(2-Ethyl-4-{3-methyl-5-[(2-methyl-4-trifluoromethyl-benzoylamino)-methyl]-phenoxy}-phenyl)-propionic acid

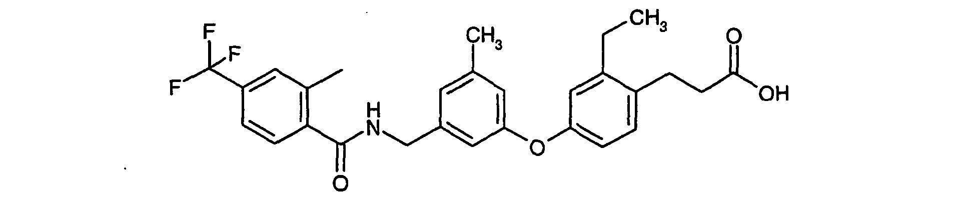

The title compound is prepared according to Example 1 utilizing 2-methyl-4-trifluoromethyl-benzoic acid (Intermediate 10) and 3-[4-(3-aminomethyl-5-methyl-phenoxy)-2-ethyl-phenyl]-propionic acid ethyl ester (Intermediate 46). Exact mass calcd for C₂₈H₂₉F₃NO₄ (M+H⁺): 500.2049, found 500.2048; ¹H NMR (400 MHz, CDCl₃).

### Example 133

### 3-[2-Ethyl-4-(3-{[(5-fluoro-3-methyl-1H-indole-2-carbonyl)-amino]-methyl}-5-methyl-phenoxy)-phenyl]-propionic acid

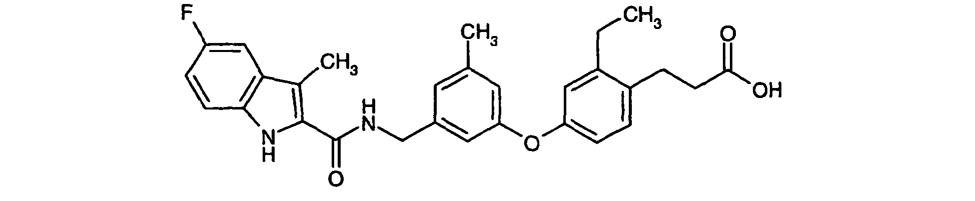

The title compound is prepared according to Example 1 utilizing 5-fluoro-3-methyl-1H-indole-2-carboxylic acid (Intermediate 1) and 3-[4-(3-aminomethyl-5-methyl-phenoxy)-2-ethyl-phenyl]-propionic acid ethyl ester (Intermediate 46). Exact mass calcd for C₂₉H₃₀FN₂O₄ (M+H⁺): 489.2190, found 489.2174; ¹H NMR (400 MHz, DMSO-D6).

### Example 134

### 3-[2-Ethyl-4-(3-{[(5-fluoro-1,3-dimethyl-1H-indole-2-carbonyl)-amino]-methyl}-5-methyl-phenoxy)-phenyl]-propionic acid

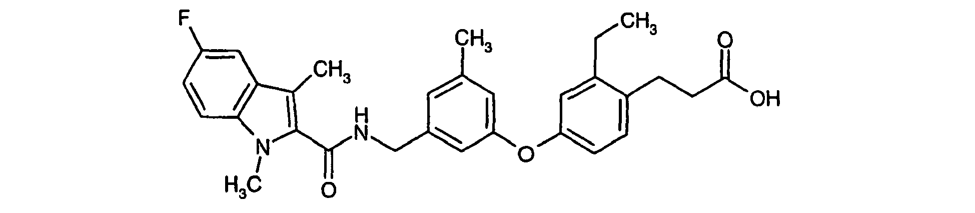

The title compound is prepared according to Example 1 utilizing 5-fluoro-1,3-dimethyl-1H-indole-2-carboxylic acid (Intermediate 2) and 3-[4-(3-aminomethyl-5-methyl-phenoxy)-2-ethyl-phenyl]-propionic acid ethyl ester (Intermediate 46). Exact mass calcd for C₃₀H₃₂FN₂O₄ (M+H⁺): 503.2643, found 503.2326; ¹H NMR (400 MHz, DMSO-D6).

### Example 135

### (R,S)-2-[4-(3-Fluoro-5-{1-[methyl-(4-trifluoromethyl-benzoyl)-amino]-ethyl}-phenoxy)-2-methyl-phenoxy]-2-methyl-propionic acid

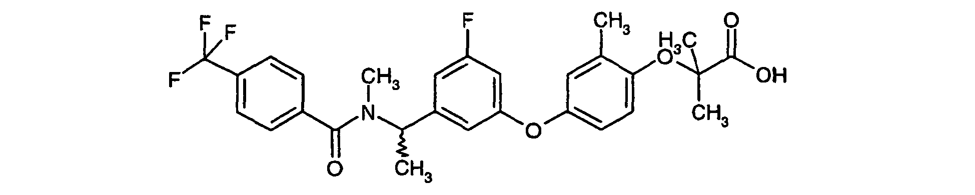

The ethyl ester of the title compound is prepared according to Example 1 Step A utilizing 4-trifluoromethyl-benzoic and (R, S)-2-{4-[3-(1-amino-ethyl)-5-fluoro-phenoxy]-2-methyl-phenoxy}-2-methyl-propionic acid ethyl ester (Intermediate 41). The ester is then reacted with sodium hydride according to Intermediate 2, Step A, followed by hydrolysis according to Example 1 Step B providing the title compound. Exact mass calcd for C₂₈H₂₈F₄NO₅ (M+H⁺): 534.1904, found 534.1910; ¹H NMR (400 MHz, DMSO-D6).

### Example 136

### 2-[4-(3-Fluoro-5-{1-[methyl-(4-trifluoromethyl-benzoyl)-amino]-ethyl}-phenoxy)-2-methyl-phenoxy]-2-methyl-propionic acid, isomer 1

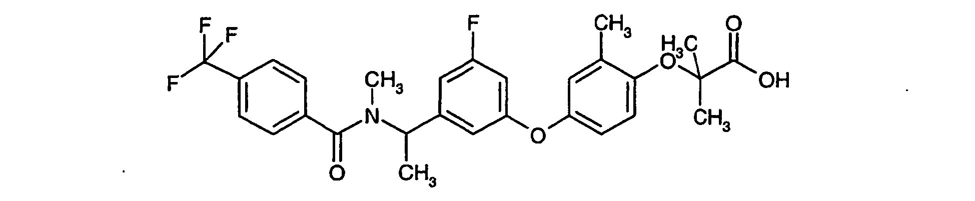

The title compound is prepared utilizing (R,S)-2-[4-(3-Fluoro-5-{1-[methyl-(4-trifluoromethyl-benzoyl)-amino]-ethyl}-phenoxy)-2-methyl-phenoxy]-2-methyl-propionic acid ethyl ester (Example 135). Chiral chromatography is used to separate the enamtiomers as the esters. The title compound is then prepared according to Example 1, Step B utilizing the pure ester enamtiomer. Exact mass calcd for C₂₈H₂₈F₄NO₅ (M+H⁺): 534.1904, found 534.1890 ; ¹H NMR (400 MHz, DMSO-D6).

### Example 137

### 2-[4-(3-Fluoro-5-{1-[methyl-(4-trifluoromethyl-benzoyl)-amino]-ethyl}-phenoxy)-2-methyl-phenoxy]-2-methyl-propionic acid, isomer 2

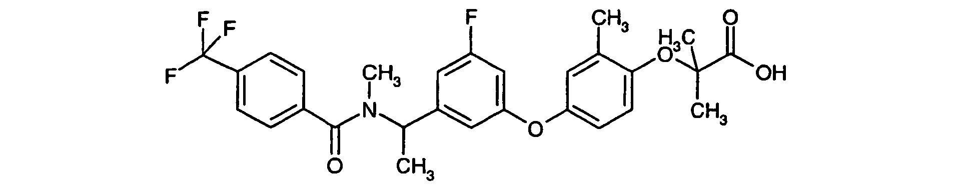

The title compound is prepared utilizing (R,S)-2-[4-(3-Fluoro-5-{1-[methyl-(4-trifluoromethyl-benzoyl)-amino]-ethyl}-phenoxy)-2-methyl-phenoxy]-2-methyl-propionic acid ethyl ester (Example 135). Chiral chromatography is used to separate the enamtiomers as the esters. The title compound is then prepared according to Example 1, Step B utilizing the pure ester enamtiomer. Exact mass calcd for C₂₈H₂₈F₄NO₅ (M+H⁺): 534.1904, found 534.1899; ¹H NMR (400 MHz, DMSO-D6).

### Example 138

### 2-(4- 3-[2-(2-Fluoro-4-trifluoromethyl-benzoylamino)-ethyl]-phenoxy}-2-methyl-phenoxy)-2-methyl-propionic acid

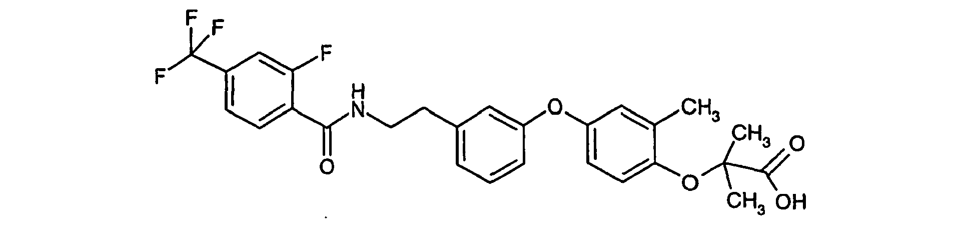

The title compound is prepared according to Example 1 utilizing 2-fluoro-4-trifluoromethyl-benzoic acid and 2-{4-[3-(2-amino-ethyl)-phenoxy]-2-methyl-phenoxy}-2-methyl-propionic acid ethyl ester (Intermediate 29). Exact mass calcd for C₂₇H₂₆F₄NO₅ (M+NH₄⁺): 537.2013, found 537.2028; ¹H NMR (400 MHz, DMSO-D6).

### Example 139

### 3-(2-Methyl-4-{3-[(4-trifluoromethyl-benzoylamino)-methyl]-phenoxy}-phenyl)-propionic acid

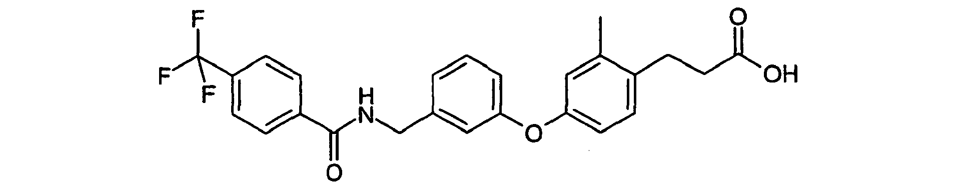

The title compound is prepared according to the general procedures described in Example 1 utilizing 4-trifluoromethyl-benzoic acid and 3-[4-(3-aminomethyl-phenoxy)-2-methyl-phenyl]-propionic acid methyl ester (Intermediate 33). Exact mass calcd for C₂₅H₂₃NO₄F₃ (M+H⁺): 458.1579; Found: 458.1574; ¹H NMR (400 MHz, CDCl₃).

### Example 140

### 2-Methyl-2-(2-methyl-4-{3-[(4-trifluoromethyl-benzoylamino)-methyl]-phenoxy}-phenoxy)-propionic acid

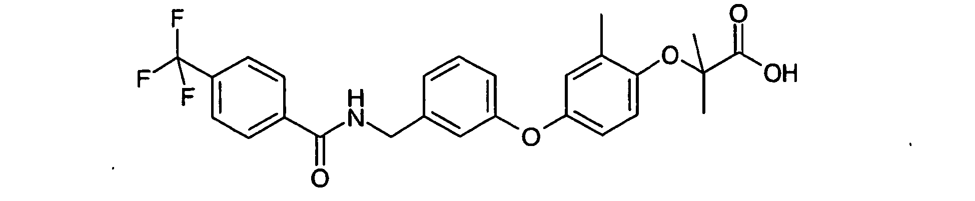

The compound of 2-[4-(3-Aminomethylphenoxy)-2-methylphenoxy]-2-methylpropionic acid ethyl ester (Intermediate 17) (53 mg, 0.15 mmol) in CH₂Cl₂ (1.5 mL) is treated sequentially at RT with N-methyl morpholine (26 mL, 0.24 mmol) and 4-trifluromethylbenzoyl chloride (36 mL, 0.24 mmol). After 16 h, tris(2-aminoethyl)amine resin (250 mg, 4.4 mmolN/g) is added. The mixture is stirred for 3 h, filtered, and concentrated. The residue is diluted in THF (0.5 mL) and MeOH (1 mL) and then treated with 2N NaOH (0.5 mL). The mixture is heated at 52 °C for 2 h, cooled, and concentrated. The residue is diluted with CH2Cl2 (1 mL) and 5 N HCl (0.8 mL) and passed through a ChemElute solid phase extraction cartridge (CH2Cl2 eluent). The crude product was purified by mass-directed HPLC to give the title compound (41 mg, 56%): MS: (ES⁺) 488.2 (M+H⁺)

### Example 141

### 2-Methyl-2-[2-methyl-4-(3-{[methyl-(4-trifluoromethyl-benzoyl)-amino]-methyl}-phenoxy)-phenoxy]-propionic acid

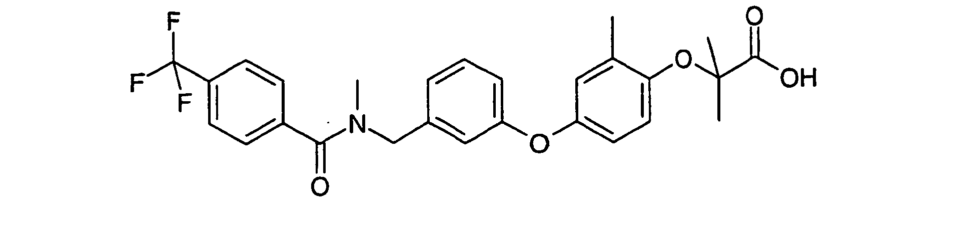

### Step A

### 2-Methyl-2-(2-methyl-4- 3-[(4-trifluoromethyl-benzoylamino)-methyl]-phenoxy}-phenoxy)-propionic acid ethyl ester

The title compound is prepared according to the general procedures described in Example 1, Step A utilizing 4-trifluoromethyl-benzoic acid and 2-[4-(3-aminomethyl-phenoxy)-2-methyl-phenoxy]-2-methyl-propionic acid ethyl ester (Intermediate 17). MS: (ES⁺) 516.3 (M+H⁺); ¹H NMR (400 MHz, CDCl₃).

### Step B

### 2-Methyl-2-[2-methyl-4-(3-{[methyl-(4-trifluoromethyl-benzoyl)-amino]-methyl}-phenoxy)-phenoxy]-propionic acid ethyl ester

To NaH (0.0090g, 0.22mmol) in dry DMF (1ml) is added the compound of Step A above (0.0800g, 0.16mmol) in dry DMF (1 ml) at rt. The mixture is stirred for 20 min, cooled to 0-5°C, and then MeI (0.02 ml, 0.38mmol) in dry DMF (1 ml) is added. The mixture is stirred at 0-5°C for 2h, and then rt overnight. The mixture is acidified with 1M HCl and diluted with EtOAc, which is then washed with brine, dried over Na₂SO₄, filtered and concentrated. Purification by chromatography by eluting with 20 % EtOAc in hexane, and 30 % EtOAc in hexane provides the title compound (0.02g) as well as methyl ester side product (0.04g) and a mixture of the ethyl ester and methyl ester (0.01g). For the title compound: MS: (ES⁺) 530.3 (M+H⁺); ¹H NMR (400 MHz, CDCl₃). For the methyl ester: MS: (ES⁺) 516.3 (M+H⁺); ¹H NMR (400 MHz, CDCl₃).

### Step C

### 2-Methyl-2-[2-methyl-4-(3-1 {[methyl-(4-trifluoromethyl-benzoyl)-amino]-methyl}-phenoxy)-phenoxy]-propionic acid

The title compound and the methyl ester from Step B above are combined (0.07g) and dissolved in dioxane (4 ml). LiOH·H₂O (0.10g, 2.4 mmol) in H₂O (2 ml) is added and stirred at rt overnight. The mixture is acidified with 1M HCl and concentrated. MS: (ES⁺) 502.3 (M+H⁺); ¹H NMR (400 MHz, CDCl₃).

### Example 142

### 2-Methyl-2-[2-methyl-4-(3-{[propyl-(4-trifluoromethyl-benzoyl)-amino]-methyl}-phenoxy)-phenoxy]-propionic acid

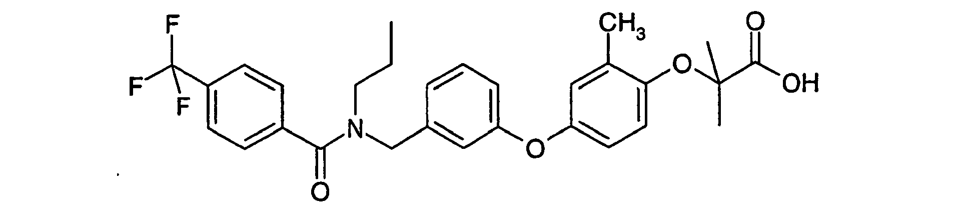

The title compound is prepared according to Example 141 utilizing 2-methyl-2-(2-methyl-4-{3-[(4-trifluoromethyl-benzoylamino)-methyl]-phenoxy}-phenoxy)-propionic acid ethyl ester (Example 141, Step A) and 1-iodo-propane. MS: (ES⁺) 530.4 (M+H⁺); ¹H NMR (400 MHz, CDCl₃).

### Example 143

### 2-[4-(3-{[(Biphenyl-4-carbonyl)-amino]-methyl)-phenoxy)-2-methyl-phenoxy]-2-methyl-propionic acid

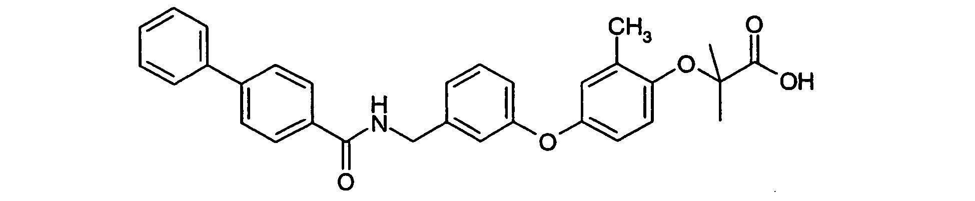

The title compound is prepared according to the general procedures described in Example 1 utilizing biphenyl-4-carboxylic acid and 2-[4-(3-aminomethyl-phenoxy)-2-methyl-phenoxy]-2-methyl-propionic acid (Intermediate 17). Exact mass calcd for C₃₁H₃₀NO₅ (M+H⁺): 496.2124, found 496.2114; ¹HNMR (400 MHz, CDCl₃).

### Example 144

### 3-(4-{3-[(4-tert-Butyl-benzoylamino)-methyl]-phenoxy}-2-methyl-phenyl)-propionic acid

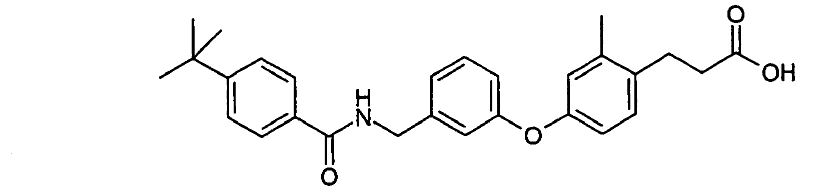

The title compound is prepared according to the general procedures described in Example 1 utilizing 4-tert-butyl-benzoic acid and 3-[4-(3-aminomethyl-phenoxy)-2-methyl-phenyl]-propionic acid methyl ester (Intermediate 33). Exact mass calcd for C₂₈H₃₂NO₄ (M+H⁺): 446.2331, found 446.2358; ¹HNMR (400 MHz, CDCl₃).

### Example 145

### 2-Methyl-2-(2-methyl-4-(3-[(2,4,6-trimethyl-benzoylamino)-methyl]-phenoxy}-phenoxy)-propionic acid

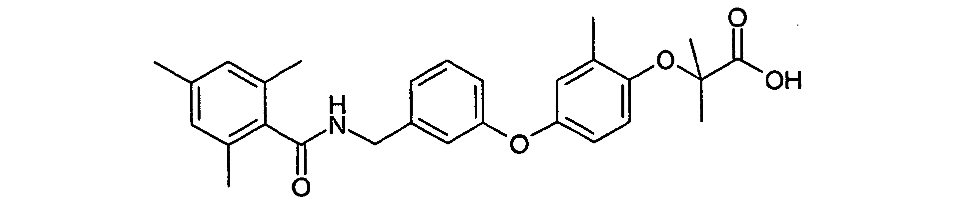

The title compound is prepared according to the general procedures described in Example 1 utilizing 2,4,6-trimethyl-benzoic acid and 2-[4-(3-aminomethyl-phenoxy)-2-methyl-phenoxy]-2-methyl-propionic acid ethyl ester (Intermediate 17). Exact mass calcd for C₂₈H₃₂NO₅ (M+H⁺): 462.2280, found 462.2295; ¹HNMR (400 MHz, CDCl₃).

### Example 146

### 2-(4-{3-[(4-Chloro-2-methyl-benzoylamino)-methyl]-phenoxy}-2-methyl-phenoxy)-2-methyl-propionic acid

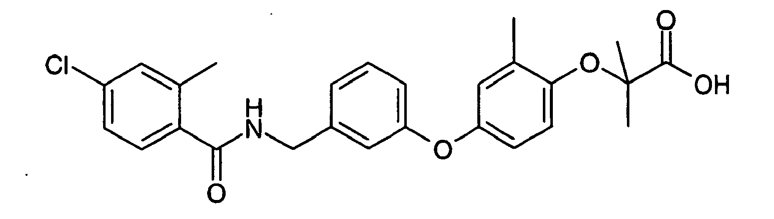

The title compound is prepared according to the general procedures described in Example 1 utilizing 4-chloro-2-methyl-benzoic acid and 2-[4-(3-aminomethyl-phenoxy)-2-methyl-phenoxy]-2-methyl-propionic acid ethyl ester (Intermediate 17). Exact mass calcd for C₂₆H₂₇NO₅Cl (M+H⁺): 468.1578, found 468.1585; ¹HNMR (400 MHz, CDCl₃).

### Example 147

### 2-Methyl-2-(2-methyl-4-{3-[(2-phenoxy-4-trifluoromethyl-benzoylamino)-methyl]-phenoxy}-phenoxy)-propionic acid

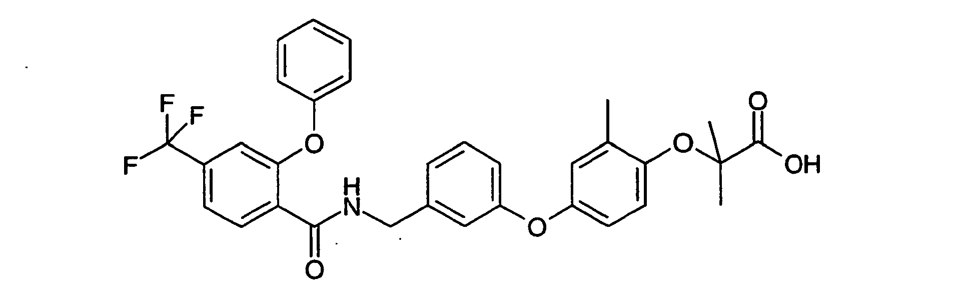

The title compound is prepared according to the general procedures described in Example 1 utilizing 2-phenoxy-4-trifluoromethyl-benzoic acid (Intermediate 31) and 2-[4-(3-aminomethyl-phenoxy)-2-methyl-phenoxy]-2-methyl-propionic acid ethyl ester (Intermediate 17). Exact mass calcd for C₃₂H₂₉NO₆F₃ (M+H⁺): 580.1947, found 580.1931; ¹HNMR (400 MHz, CDCl₃).

### Example 148

### 2-(4-{3-[(2-Ethyl-4-trifluoromethyl-benzoylamino)-methyl]-phenoxy}-2-methyl-phenoxy)-2-methyl-propionic acid

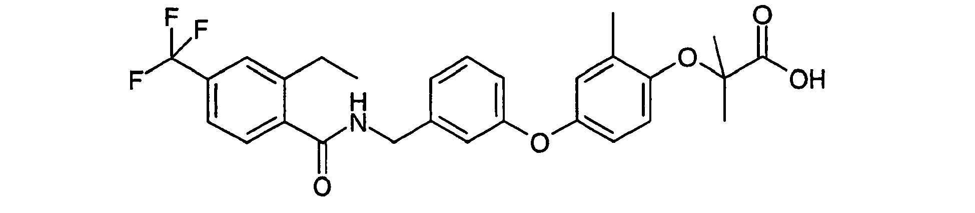

The title compound is prepared according to the general procedures described in Example 1 utilizing 2-ethyl-4-trifluoromethyl-benzoic acid (Intermediate 30) and 2-[4-(3-aminomethyl-phenoxy)-2-methyl-phenoxy]-2-methyl-propionic acid ethyl ester (Intermediate 17). Exact mass calcd for C₂₈H₂₉NO₅F₃ (M+H⁺): 516.1998, found 516.2004; ¹HNMR (400 MHz, CDCl₃).

### Example 149

### 2-Methyl-2-(2-methyl-4-{3-[(4-trifluoromethyl-benzylamino)-methyl]-phenoxy}-phenoxy)-propionic acid; compound with trifluoro-acetic acid

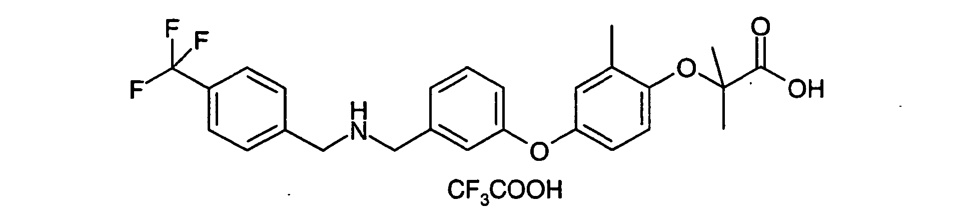

### Step A

### 2-Methyl-2-(2-methyl-4-{3-[(4-trifluoromethyl-benzylamino)-methyl]-phenoxy}-phenoxy)-propionic acid ethyl ester

The compound of 2-[4-(3-aminomethyl-phenoxy)-2-methyl-phenoxy]-2-methyl-propionic acid ethyl ester (Intermediate 17) (0.34 g, 0.99 mmol) is dissolved in MeOH (10 ml), and NaOAc (0.32g, 4.0 mmol) is added. The mixture is stirred at 0 °C. The compound of 4-(trifluoromethyl)benzaldehyde (0.12 ml, 0.89 mmol) is added and then stirred at 0 °C for 15 min. NaBH₃CN (0.09 g, 1.5 mmol) is added followed by a few drops of HOAc. The mixture is stirred at 0 °C for 1.5h and then concentrated. The residue is partitioned in EtOAc and water. The EtOAc layer is separated, washed with brine, dried over Na₂SO₄, filtered and concentrated. Purification by chromatography, eluting with 2% MeOH in CH₂Cl₂ provides the title compound (0.35g). MS: (ES⁺) 502.3 (M+H⁺); ¹H NMR (400 MHz, CDCl₃).

### Step B

### 2-Methyl-2-(2-methyl-4-{3-[(4-trifluoromethyl-benzylamino)-methyl]-phenoxy)-phenoxy)-propionic acid; compound with trifluoro-acetic acid

The title compound is prepared according to the general procedures described in Example 1, Step B utilizing the compound from Step A above and purified by reversed HPLC. Exact mass calcd for C₂₆H₂₇NO₄F₃ (M+H⁺): 474.1892, found 474.1877; ¹HNMR (400 MHz, CDCl₃).

### Example 150

### 2-[4-(3-{[Acetyl-(4-trifluoromethyl-benzyl)-amino]-methyl}-phenoxy)-2-methyl-phenoxy]-2-methyl-propionic acid

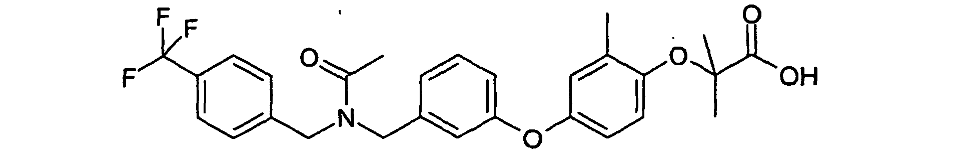

### Step A

### 2-[4-(3-{[Acetyl-(4-trifluoromethyl-benzyl)-amino]-methyl -phenoxy)-2-methyl-phenoxy]-2-methyl-propionic acid ethyl ester

The compound of 2-methyl-2-(2-methyl-4-{3-[(4-trifluoromethyl-benzylamino)-methyl]-phenoxy}-phenoxy)-propionic acid ethyl ester (Example 149, Step A, 0.10 g, 0.20 mmol) is mixed with (CH₃CO)₂O (1.5 ml) and stirred at rt for 1.5h. The mixture is concentrated at 35 °C under vacuum. The residue is dissolved in EtOAc, washed with 1M HCI, diluted NaHCO₃ and brine, dried over Na₂SO₄, filtered and concentrated. Purification by chromatography by eluting with 15% EtOAc in hexane and 25 % EtOAc in hexane provides the title compound (0.08g). MS: (ES⁺) 544.3 (M+H⁺); ¹H NMR (400 MHz, CDCl₃).

### Step B

### 2-[4-(3-{[Acetyl-(4-trifluoromethyl-benzyl)-amino]-methyl}-phenoxy)-2-methyl-phenoxy]-2-methyl-propionic acid

The title compound is prepared according to the general procedures described in Example 1, Step B, utilizing the compound of Step A above. Exact mass calcd for C₂₈H₂₉NO₅F₃ (M+H⁺): 516.1995, found 516.2004; ¹HNMR (400 MHz, CDCl₃).

### Example 151

### 2-[4-(3- {[Methanesulfonyl-(4-trifluoromethyl-benzyl)-amino]-methyl }-phenoxy)-2-methyl-phenoxy]-2-methyl-propionic acid

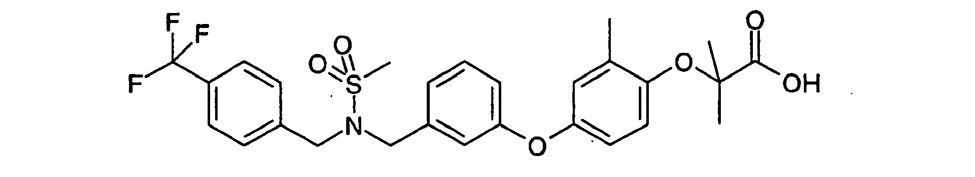

### Step A

### 2-[4-(3-{[Methanesulfonyl-(4-trifluoromethyl-benzyl)-amino]-methyl}-phenoxy)-2-methyl-phenoxy]-2-methyl-propionic acid ethyl ester

The compound of 2-methyl-2-(2-methyl-4-{3-[(4-trifluoromethyl-benzylamino)-methyl]-phenoxy}-phenoxy)-propionic acid ethyl ester (Example 149, Step A, 0.13 g, 0.26 mmol) is dissolved in CH₂Cl₂(5 ml), and Et₃N (0.05 ml, 0.39 mmol) is added. The mixture is cooled to 0°C, and MsCl (0.04 ml, 0.31 mmol) is added, which is then stirred at 0 °C to rt for 4h. The mixture is diluted with CH₂Cl₂, washed with 1MHCl, diluted NaHCO₃ and brine, dried over Na₂SO₄, filtered and concentrated. Purification by chromatography, eluting with 15% EtOAc in hexane, then 25 % EtOAc in hexane provides the title compound (0.11g). MS: (ES⁺) 597.2 (M+NH₄⁺); ¹H NMR (400 MHz, CDCl₃).

### Step B

### (2-[4-(3-{[Methanesulfonyl-(4-trifluoromethyl-benzyl)-amino]-methyl)-phenoxy)-2-methyl-phenoxy]-2-methyl-propionic acid

The title compound is prepared according to the general procedures described in Example 1, Step B utilizing the compound of Step A above. Exact mass calcd for C₂₇H₃₂N₂O₆F₃ (M+NH₄⁺): 569.1933, found 569.1926; ¹HNMR (400 MHz, CDCl₃).

### Example 152

### 2-(4-{3-[(2-Bromo-5-trifluoromethyl-benzoylamino)-methyl]-phenoxy}-2-methyl-phenoxy)-2-methyl-propionic acid

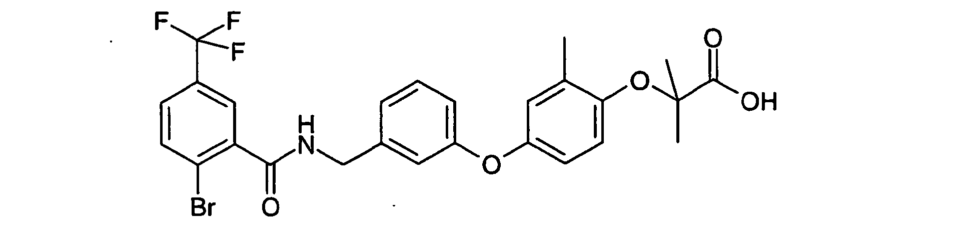

The title compound is prepared according to the general procedures described in Example 1 utilizing 2-bromo-5-trifluoromethyl-benzoic acid *(*Tetrahedron Lett., Vol.37, No.16, pp. 2767-2770, 1996) and 2-[4-(3-aminomethyl-phenoxy)-2-methyl-phenoxy]-2-methyl-propionic acid ethyl ester (Intermediate 17). Exact mass calcd for C₂₆H₂₄NO₅F₃Br (M+H⁺): 566.0790, found 566.0787; ¹HNMR (400 MHz, CDCl₃).

### Example 153

### 2-(4- {3-[(2-Bromo-5-trifluoromethyl-benzoylamino)-methyl]-phenoxy)-2-methyl-phenoxy)-2-methyl-propionic acid

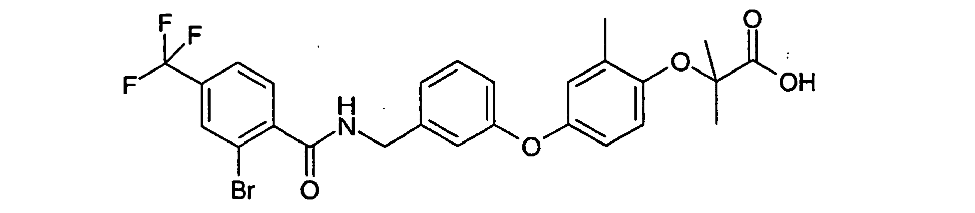

The title compound is prepared according to the general procedures described in Example 1 utilizing 2-bromo-4-trifluoromethyl-benzoic acid (Intermediate 11) and 2-[4-(3-aminomethyl-phenoxy)-2-methyl-phenoxy]-2-methyl-propionic acid ethyl ester (Intermediate 17). The final product is purified by reversed HPLC. MS: (ES⁺) 566 / 568 (M+H⁺).

### Example 154

### 2-[4-(3-{ [Acetyl-(2-phenoxy-4-trifluoromethyl-benzyl)-amino]-methyl}-phenoxy)-2-methyl-phenoxy]-2-methyl-propionic acid

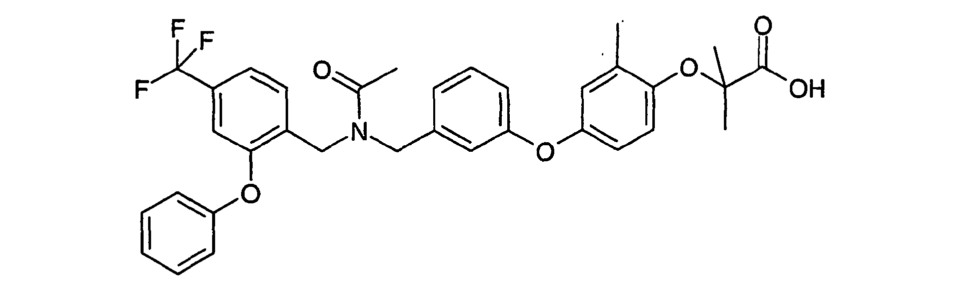

The title compound is prepared according to Example 150 utilizing 2-Phenoxy-4-trifluoromethyl-benzaldehyde (Intermediate 32) and 2-[4-(3-aminomethyl-phenoxy)-2-methyl-phenoxy]-2-methyl-propionic acid ethyl ester (Intermediate 17). Exact mass calcd for C₃₄H₃₃NO₆F₃ (M+H⁺): 608.2260, found 608.2253; ¹HNMR (400 MHz, CDCl₃).

### Example 155

### 2-[4-(3-{[Methanesulfonyl-(2-phenoxy-4-trifluoromethyl-benzyl)-amino]-methyl}-phenoxy)-2-methyl-phenoxy]-2-methyl-propionic acid

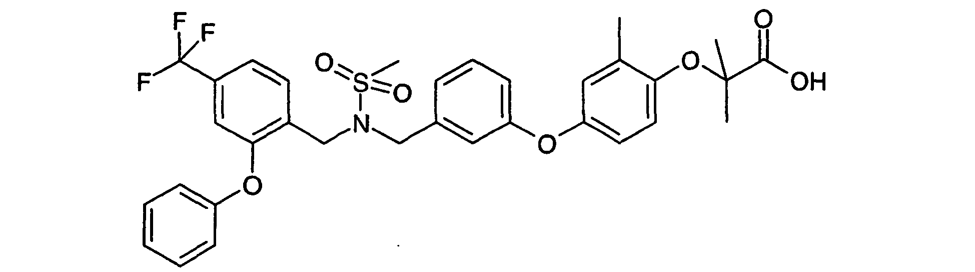

The title compound is prepared according to Example 151 utilizing 2-phenoxy-4-trifluoromethyl-benzaldehyde (Intermediate 32) and 2-[4-(3-aminomethyl-phenoxy)-2-methyl-phenoxy]-2-methyl-propionic acid ethyl ester (Intermediate 17). Exact mass calcd for C₃₃H₃₆N₂O₇F₃S (M+NH₄⁺): 661.2195, found 661.2197; ¹HNMR (400 MHz, CDCl₃).

### Example 156

### 3-[4-(3-{[(5-Chloro-1H-indole-2-carbonyl)-amino]-methyl)-phenoxy)-2-methyl-phenyl]-propionic acid

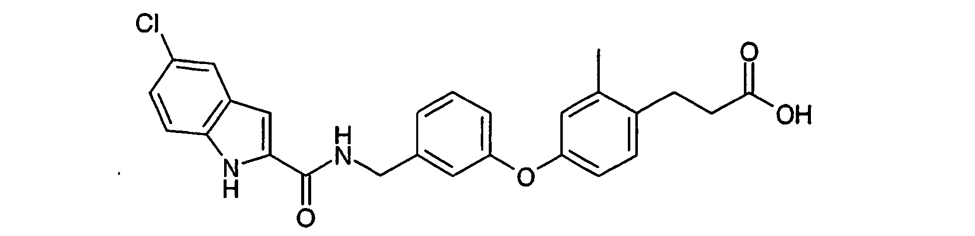

The title compound is prepared according to the general procedures described in Example 1 utilizing 5-chloro-1H-indole-2-carboxylic acid and 3-[4-(3-Aminomethyl-phenoxy)-2-methyl-phenyl]-propionic acid methyl ester (Intermediate 33). Exact mass calcd for C₂₆H₂₄N₂O₄Cl (M+H⁺): 463.1425, found 463.1402; ¹HNMR (400 MHz, DMSO-D6).

### Example 157

### 3-[4-(3-{[(5-Chloro-1-methyl-1H-indole-2-carbonyl)-amino]-methyl}-phenoxy)-2-methyl-phenyl]-propionic acid

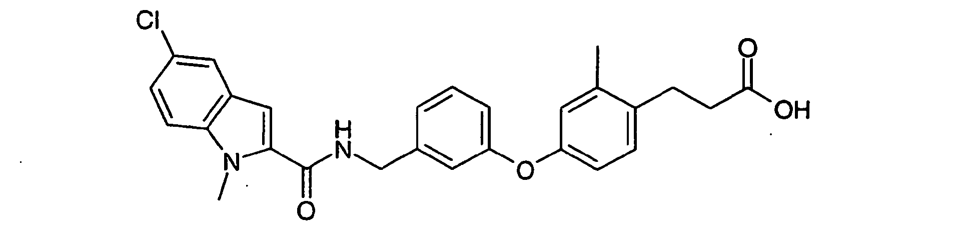

The title compound is prepared according to the general procedures described in Example 1 utilizing 5-chloro-1-methyl-1H-indole-2-carboxylic acid and 3-[4-(3-aminomethyl-phenoxy)-2-methyl-phenyl)-propionic acid methyl ester (Intermediate 33). Exact mass calcd for C₂₇H₂₆N₂O₄Cl (M+H⁺): 477.1581, found 477.1598; ¹HNMR (400 MHz, CDCl₃).

### Example 158

### 3-[4-(3-{([(5-Bromo-1H-indole-2-carbonyl)-amino]-methyl) }-phenoxy)-2-methyl-phenyl]-propionic acid

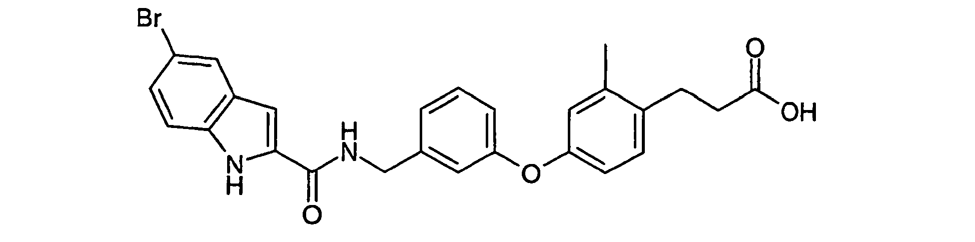

The title compound is prepared according to the general procedures described in Example 1 utilizing 5-bromo-1H-indole-2-carboxylic acid and 3-[4-(3-Aminomethyl-phenoxy)-2-methyl-phenyl]-propionic acid methyl ester (Intermediate 33). Exact mass calcd for C₂₆H₂₄N₂O₄Br (M+H⁺): 507.0919, found 507.0909; ¹HNMR (400 MHz, DMSO-D6).

### Example 159

### 3-[4-(3-{[(5-Methoxy-1H-indole-2-carbonyl)-amino]-methyl}-phenoxy)-2-methylphenyl]-propionic acid

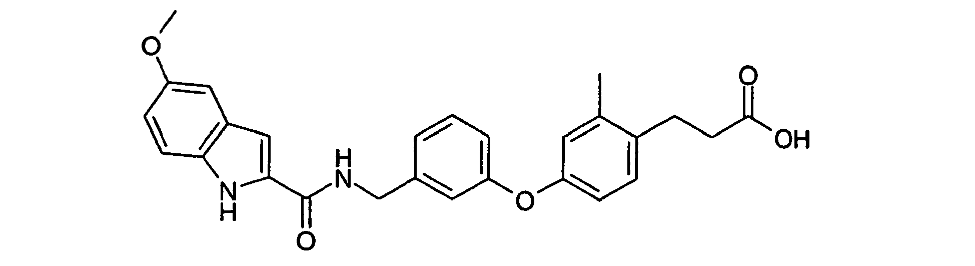

The title compound is prepared according to the general procedures described in Example 1 utilizing 5-methoxy-1H-indole-2-carboxylic acid and 3-[4-(3-Aminomethyl-phenoxy)-2-methyl-phenyl]-propionic acid methyl ester (Intermediate 33). Exact mass calcd for C₂₇H₂₇N₂O₅ (M+H⁺): 459.1920, found 459.1918; ¹HNMR (400 MHz, CDCl₃).

### Example 160

### 3-[2-Methyl-4-(3-{[(3-methyl-benzofuran-2-carbonyl)-amino]-methyl}-phenoxy)-phenyl]-propionic acid

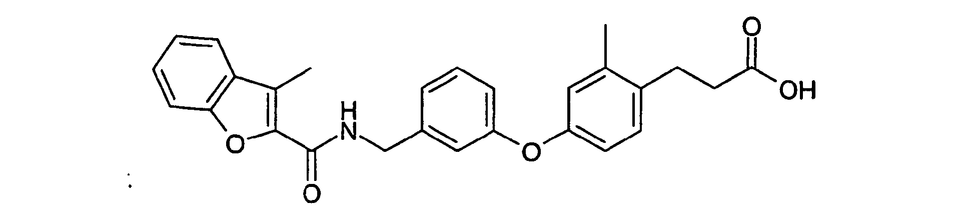

The title compound is prepared according to the general procedures described in Example 1 utilizing 3-methyl-benzofuran-2-carboxylic acid and 3-[4-(3-aminomethyl-phenoxy)-2-methyl-phenyl]-propionic acid methyl ester (Intermediate 33). Exact mass calcd for C₂₇H₂₆NO₅ (M+H⁺): 444.1811, found 444.1791; ¹HNMR (400 MHz, CDCl₃).

### Example 161

### 3-[4-(3-{[(5-Chloro-3-methyl-benzofuran-2-carbonyl)-amino]-methyl}-phenoxy)-2-methyl-phenyl]-propionic acid

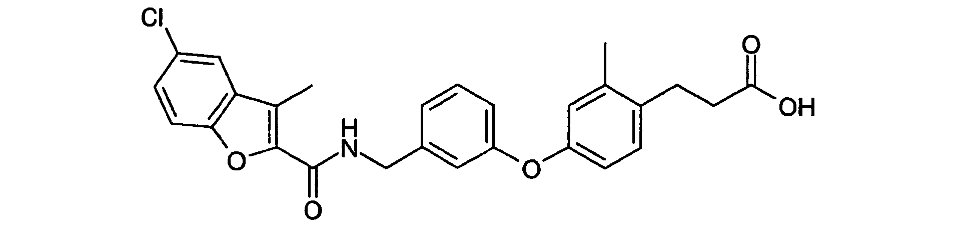

The title compound is prepared according to the general procedures described in Example 1 utilizing 5-chloro-3-methyl-benzofuran-2-carboxylic acid and 3-[4-(3-aminomethyl-phenoxy)-2-methyl-phenyl]-propionic acid methyl ester (Intermediate 33). Exact mass calcd for C₂₇H₂₅NO₅Cl (M+H⁺): 478.1421, found 478.1398; ¹HNMR (400 MHz, CDCl₃).

### Example 162

### 2-(4-{3-[(2-Chloro-4-trifluoromethyl-benzoylamino)-methyl]-phenoxy]-2-methyl-phenoxy)-2-methyl-propionic acid

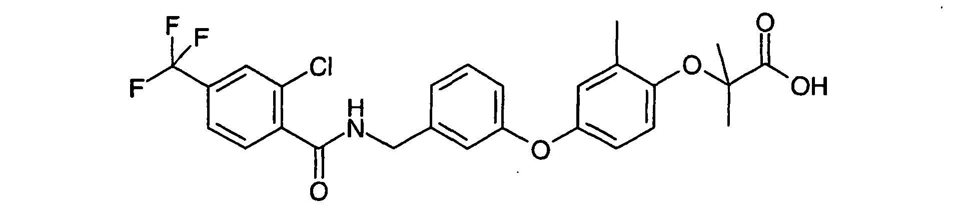

The title compound is prepared according to the general procedures described in Example 1 utilizing 2-chloro-4-trifluoromethyl-benzoic acid (Intermediate 12) and 2-[4-(3-aminomethyl-phenoxy)-2-methyl-phenoxy]-2-methyl-propionic acid ethyl ester (Intermediate 17). Exact mass calcd for C₂₆H₂₄NO₅F₃Cl (M+H⁺): 522.1295, found 522.1310; ¹HNMR (400 MHz, CDCl₃).

### Example 163

### 3-(4-{3-[(2-Chloro-4-trifluoromethyl-benzoylamino)-methyl]-phenoxy}-2-methylphenyl)-propionic acid

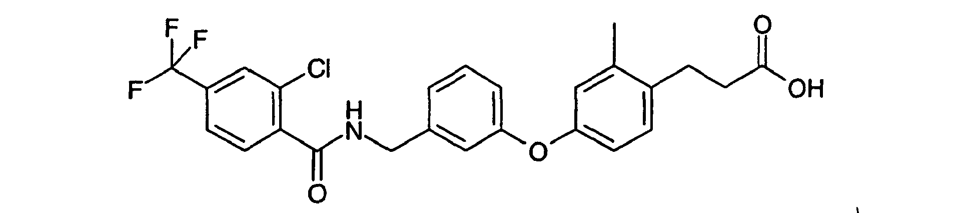

The title compound is prepared according to the general procedures described in Example 1 utilizing 2-chloro-4-trifluoromethyl-benzoic acid (Intermediate 12) and 3-[4-(3-aminomethyl-phenoxy)-2-methyl-phenyl]-propionic acid methyl ester (Intermediate 33). Exact mass calcd for C₂₅H₂₂NO₄F₃Cl (M+H⁺): 492.1189, found 492.1202; ¹HNMR (400 MHz, CDCl₃).

### Example 164

### 3-[4-(3-{[(5,6-Dichloro-1H-indole-2-carbonyl)-amino]-methyl)-phenoxy)-2-methylphenyl]-propionic acid

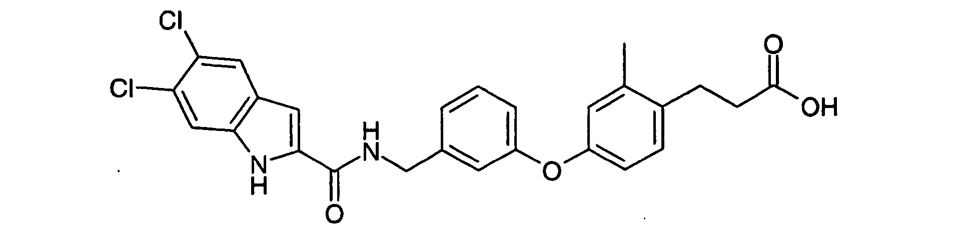

The title compound is prepared according to the general procedures described in Example 1 utilizing 5,6-dichloro-1H-indole-2-carboxylic acid (WO 9935130) and 3-[4-(3-aminomethyl-phenoxy)-2-methyl-phenyl]-propionic acid methyl ester (Intermediate 33). Exact mass calcd for C₂₆H₂₃N₂O₄Cl₂ (M+H⁺): 497.1035, found 497.1043; ¹HNMR (400 MHz, CD₃OD).

### Example 165

### 3-[4-(3-{[(1H-Benzoimidazole-2-carbonyl)-amino]-methyl}-phenoxy)-2-methyl-phenyl]-propionic acid

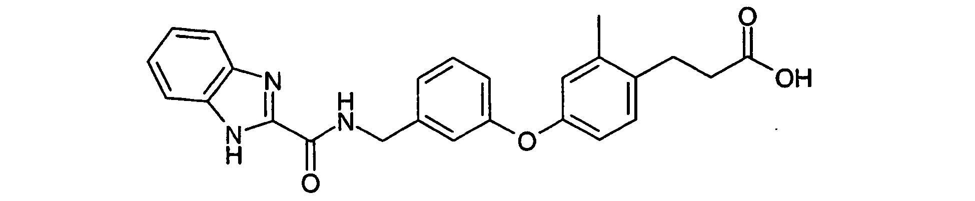

The title compound is prepared according to the general procedures described in Example 1 utilizing 1H-benzoimidazole-2-carboxylic acid and 3-[4-(3-aminomethyl-phenoxy)-2-methyl-phenyl]-propionic acid methyl ester (Intermediate 33). Exact mass calcd for C₂₅H₂₄N₃O₄ (M+H⁺): 430.1767, found 430.1772; ¹HNMR (400 MHz, DMSO-D6).

### Example 166

### (2-Methyl-4-{3-[(2-methyl-4-trifluoromethyl-benzoylamino)-methyl]-phenoxy}-phenoxy)-acetic acid

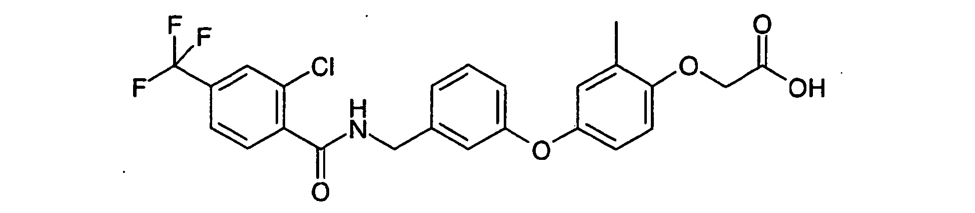

The title compound is prepared according to the general procedures described in Example 1 utilizing 2-chloro-4-trifluoromethyl-benzoic acid (Intermediate 12) and [4-(3-aminomethyl-phenoxy)-2-methyl-phenoxy]-acetic acid methyl ester (Intermediate 35). Exact mass calcd for C₂₅H₂₃NO₅F₃ (M+H⁺): 474.1528, found 474.1549; ¹HNMR (400 MHz, CDCl₃).

### Example 167

### [4-(3-{[(5-Chloro-1H-indole-2-carbonyl)-amino]-methyl}-phenoxy)-2-methyl-phenoxy]-acetic acid

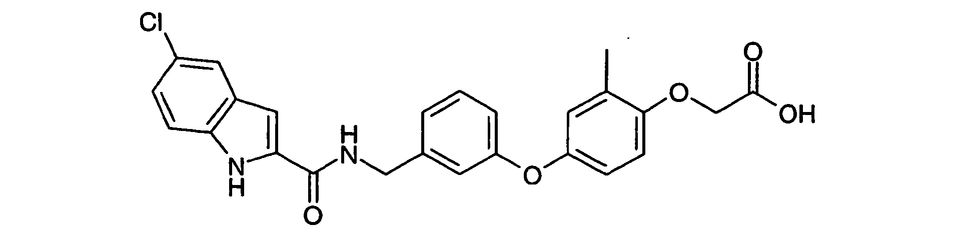

The title compound is prepared according to the general procedures described in Example 1 utilizing 5-chloro-1H-indole-2-carboxylic acid and [4-(3-aminomethyl-phenoxy)-2-methyl-phenoxy]-acetic acid methyl ester (Intermediate 35). MS: (ES⁺) 465.1 (M+H⁺); ¹H NMR (400 MHz, DMSO-D6).

### Example 168

### 2-[4-(3-{[(5-Chloro-1H-indole-2-carbonyl)-amino]-methyl}-5-fluoro-phenoxy)-2-methyl-phenoxy]-2-methyl-propionic acid

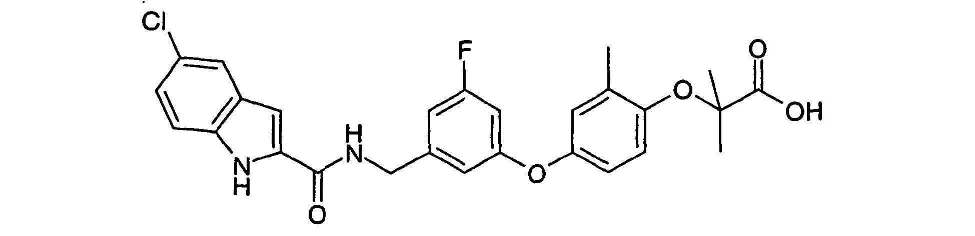

The title compound is prepared according to the general procedures described in Example 1 utilizing 5-chloro-1H-indole-2-carboxylic acid and 2-[4-(3-aminomethyl-5-fluoro-phenoxy)-2-methyl-phenoxy]-2-methyl-propionic acid ethyl ester (Intermediate 47). Exact mass.calcd for C₂₇H₂₅N₂O₅FCl (M+H⁺): 511.1436, found 511.1447; ¹HNMR (400 MHz, CDCl₃).

### Example 169

### 2-(4-{3-[(2-Chloro-4-trifluoromethyl-benzoylamino)-methyl]-5-fluoro-phenoxy}-2-methyl-phenoxy)-2-methyl-propionic acid

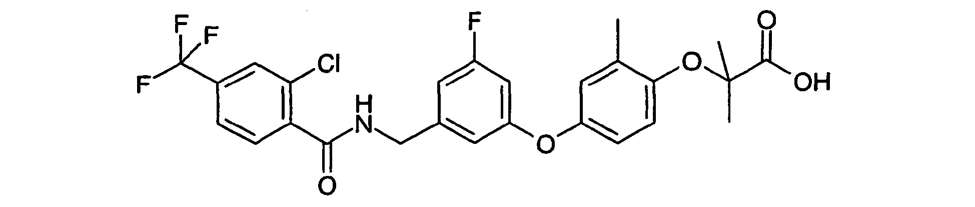

The title compound is prepared according to the general procedures described in Example 1 utilizing 2-chloro-4-trifluoromethyl-benzoic acid (Intermediate 12) and 2-[4-(3-aminomethyl-5-fluoro-phenoxy)-2-methyl-phenoxy]-2-methyl-propionic acid ethyl ester (Intermediate 47). Exact mass calcd for C₂₆H₂₃NO₅F₄Cl (M+H⁺): 540.1201, found 540.1218; ¹HNMR (400 MHz, CDCl₃).

### Example 170

### 2-(4-{3-Fluoro-5-[(2-fluoro-4-trifluoromethyl-benzoylamino)-methyl]-phenoxy}-2-methyl-phenoxy)-2-methyl-propionic acid

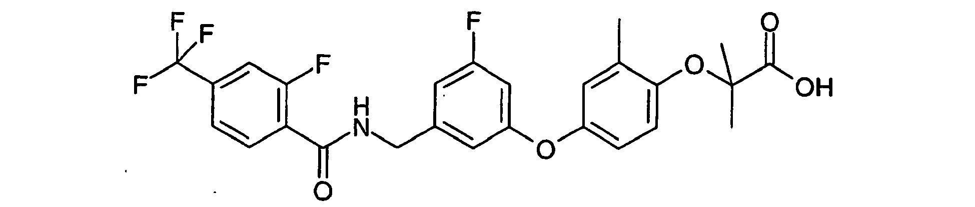

The title compound is prepared according to the general procedures described in Example 1 utilizing 2-fluoro-4-trifluoromethyl-benzoic acid and 2-[4-(3-aminomethyl-5-fluoro-phenoxy)-2-methyl-phenoxy]-2-methyl-propionic acid ethyl ester (Intermediate 47). Exact mass calcd for C₂₆H₂₃NO₅F₅ (M+H⁺): 524.1496, found 524.1512; ¹HNMR (400 MHz, CDCl₃).

### Example 171

### 3-[4-(3-{[(1H-Indole-2-carbonyl)-amino]-methyl}-phenoxy)-2-methyl-phenyl]-propionic acid

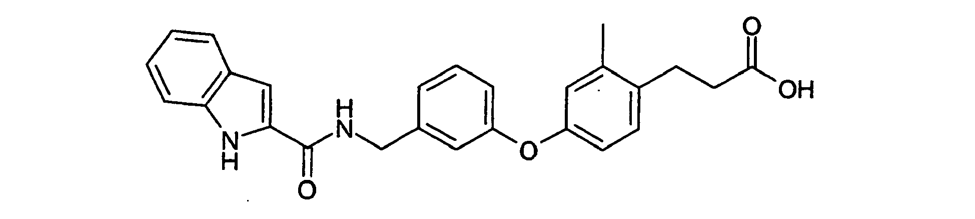

The title compound is prepared according to the general procedures described in Example 1 utilizing 1H-indole-2-carboxylic acid and 3-[4-(3-aminomethyl-phenoxy)-2-methyl-phenyl]-propionic acid methyl ester (Intermediate 33). The final product is purified by reversed HPLC. Exact mass calcd for C₂₆H₂₅N₂O₄ (M+H⁺): 429.1814, found 429.1843; ¹HNMR (400 MHz, DMSO-D6).

### Example 172

### 3-[4-(3-{[(5-Chloro-1H-indole-2-carbonyl)-amino]-methyl}-5-fluoro-phenoxy)-2-methylphenyl]-propionic acid

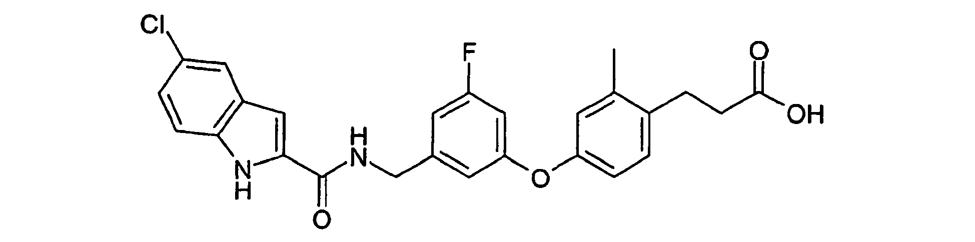

The title compound is prepared according to the general procedures described in Example 1 utilizing 5-chloro-1H-indole-2-carboxylic acid and [4-(3-aminomethyl-5-fluoro-phenoxy)-2-methyl-phenoxy]-acetic acid methyl ester (Intermediate 36). Exact mass calcd for C₂₆H₂₃N₂O₄FCl (M+H⁺): 481.1330, found 481.1357; ¹HNMR (400 MHz, DMSO-D6).

### Example 173

### 3-[4-(2-{[(5-Chloro-1H-indole-2-carbonyl)-amino]-methyl}-phenoxy)-2-methyl-phenyl]-propionic acid

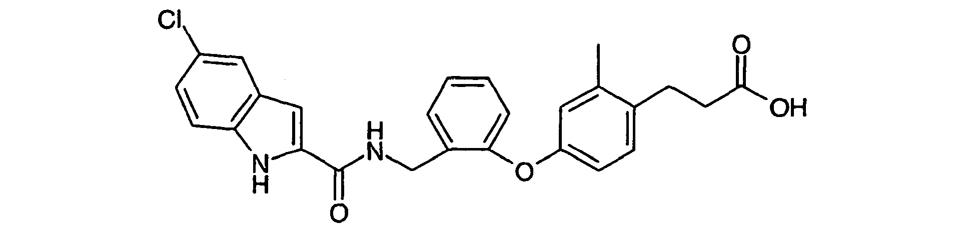

The title compound is prepared according to the general procedures described in Example 1 utilizing 5-chloro-1H-indole-2-carboxylic acid and 3-[4-(2-aminomethyl-phenoxy)-2-methyl-phenyl]-propionic acid methyl ester (Intermediate 37). Exact mass calcd for C₂₆H₂₄N₂O₄Cl (M+H⁺): 463.1424, found 463.1437; ¹HNMR (400 MHz, DMSO-D6).

### Example 174

### 3-[4-(3-{[(5-Chloro-1H-indole-2-carbonyl)-amino]-methyl}-5-trifluoromethyl-phenoxy)-2-methyl-phenyl]-propionic acid

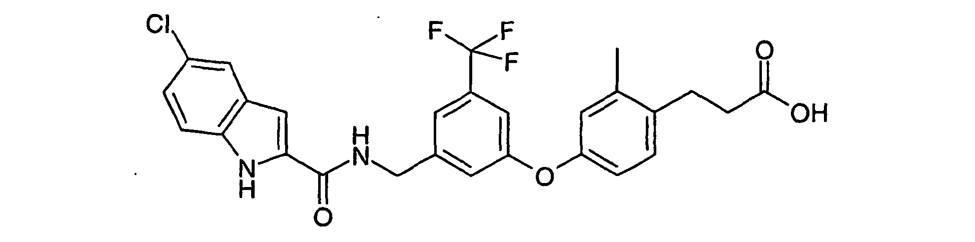

The title compound is prepared according to the general procedures described in Example 1 utilizing 5-chloro-1H-indole-2-carboxylic acid and 3-[4-(3-aminomethyl-5-trifluoromethyl-phenoxy)-2-methyl-phenyl]-propionic acid methyl ester (Intermediate 38). Exact mass calcd for C₂₇H₂₃N₂O₄ClF₃ (M+H⁺): 531.1298, found 531.1312; ¹HNMR (400 MHz, DMSO-D6).

### Example 175

### 3-[4-(5-{([(5-Chloro-1H-indole-2-carbonyl)-amino]-methyl}-2-trifluoromethyl-phenoxy)-2-methyl-phenyl]-propionic acid

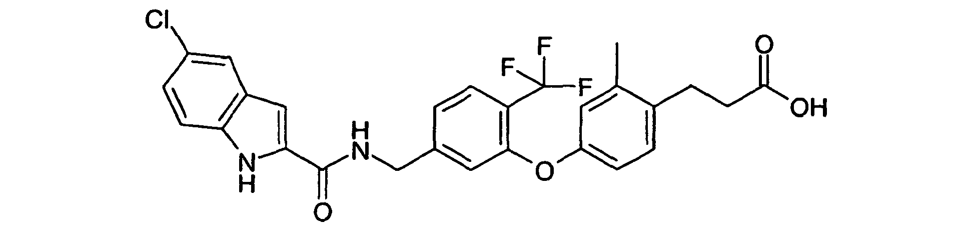

The title compound is prepared according to the general procedures described in Example 1 utilizing 5-chloro-1H-indole-2-carboxylic acid and 3-[4-(5-aminomethyl-2-trifluoromethyl-phenoxy)-2-methyl-phenyl]-propionic acid methyl ester (Intermediate 39). Exact mass calcd for C₂₇H₂₃N₂O₄ClF₃ (M+H⁺): 531.1298, found 531.1285; ¹HNMR (400 MHz, CDCl₃).

### Example 176

### 3-[4-(3-{1-[(5-Chloro-1H-indole-2-carbonyl)-amino]-ethyl}-5-fluoro-phenoxy)-2-methylphenyl]-propionic acid

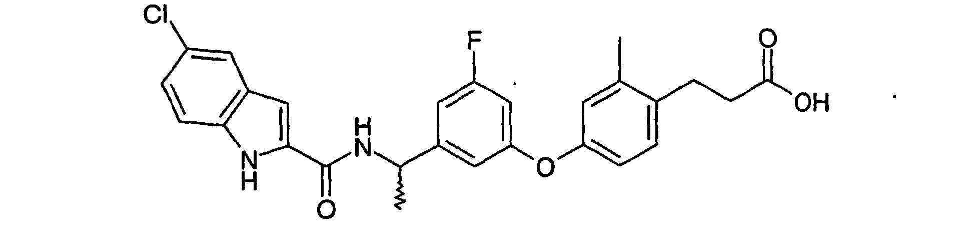

The title compound is prepared according to the general procedures described in Example 1 utilizing 5-chloro-1H-indole-2-carboxylic acid and 3-{4-[3-(1-amino-ethyl)-5-fluoro-phenoxy]-2-methyl-phenyl}-propionic acid methyl ester (Intermediate 34). Exact mass.calcd for C₂₆H₂₄N₂O₄Cl (M+H⁺): 495.1487, found 495.1471; ¹HNMR (400 MHz, CDCl₃).

The pure enantiomers are separated by chiral chromatography at the ester stage and hydrolyzed separately to provide isomer 1 (R configuration) and isomer 2 (S configuration). Isomer 1: Exact mass calcd for C₂₆H₂₄N₂O₄Cl (M+H⁺): 495.1487, found 495.1466; ¹HNMR (400 MHz, CDCl₃). Isomer 2: Exact mass calcd for C₂₆H₂₄N₂O₄Cl (M+H⁺): 495.1487, found 495.1465 ; ¹HNMR (400 MHz, CDCl₃).

### Example 177

### 2-(4-{3-Fluoro-5-[(2-methoxy-4-trifluoromethyl-benzoylamino)-methyl]-phenoxy}-2-methyl-phenoxy)-2-methyl-propionic acid

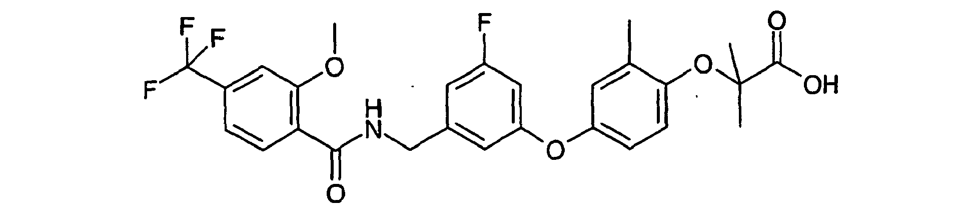

The title compound is prepared according to the general procedures described in Example 1 utilizing 2-methoxy-4-trifluoromethyl-benzoic acid *(*J.Am.Chem.Soc.; 73; 1951; 2375) and 2-[4-(3-aminomethyl-5-fluoro-phenoxy)-2-methyl-phenoxy]-2-methyl-propionic acid ethyl ester (Intermediate 47). Exact mass calcd for C₂₇H₂₆NO₆F₄ (M+H⁺): 536.1696, found 536.1706; ¹HNMR (400 MHz, CDCl₃).

### Example 178

### 2-[4-(3-{[(5-Chloro-1-methyl-1H-indole-2-carbonyl)-amino]-methyl}-5-fluoro-phenoxy)-2-methyl-phenoxy]-2-methyl-propionic acid

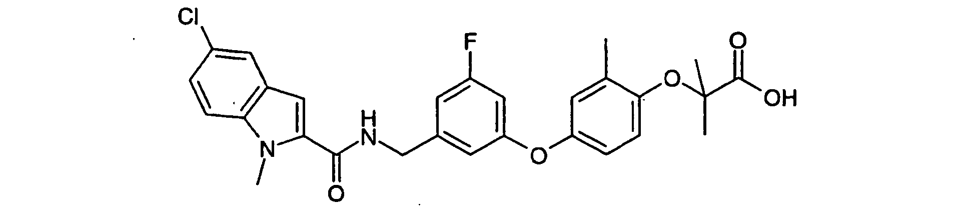

The title compound is prepared according to the general procedures described in Example 1 utilizing 5-chloro-1-methyl-1H-indole-2-carboxylic acid and 2-[4-(3-aminomethyl-5-fluoro-phenoxy)-2-methyl-phenoxy]-2-methyl-propionic acid ethyl ester (Intermediate 47). Exact mass calcd for C₂₈H₂₇N₂O₅FCl (M+H⁺): 525.1592, found 525.1592; ¹HNMR (400 MHz, CDCl₃).

### Example 179

### 3-[4-(3-{[(5-Chloro-1H-indole-2-carbonyl)-amino]-methyl}-4-trifluoromethyl-phenoxy)-2-methyl-phenyl]-propionic acid

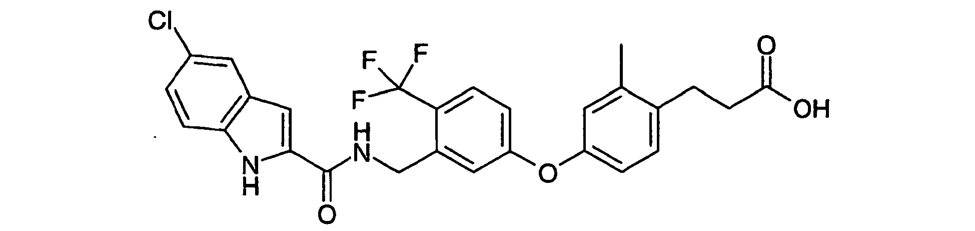

The title compound is prepared according to the general procedures described in Example 1 utilizing 5-chloro-1H-indole-2-carboxylic acid and 3-[4-(3-aminomethyl-4-trifluoromethyl-phenoxy)-2-methyl-phenyl]-propionic acid methyl ester (Intermediate 40). Exact mass calcd for C₂₇H₂₃N₂O₄ClF₃ (M+H⁺): 531.1298, found 531.1307; ¹HNMR (400 MHz, DMSO-D6).

### Example 180

### 2-[4-(3-Fluoro-5-{[(3-methyl-benzo[b]thiophene-2-carbonyl)-amino]-methyl}-phenoxy)-2-methyl-phenoxy]-2-methyl-propionic acid

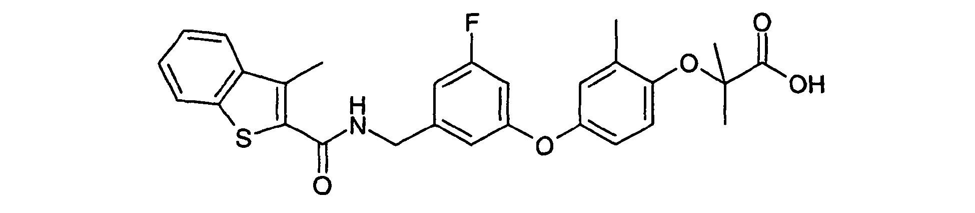

The title compound is prepared according to the general procedures described in Example 1 utilizing 3-methyl-benzo[b]thiophene-2-carboxylic acid and 2-[4-(3-aminomethyl-5-fluoro-phenoxy)-2-methyl-phenoxy]-2-methyl-propionic acid ethyl ester (Intermediate 47). Exact mass calcd for C₂₈H₂₇NO₅FS (M+H⁺): 508.1594, found 508.1588; ¹HNMR (400 MHz, CDCl₃).

### Example 181

### 2-[4-(3-{[(5-Chloro-1,3-dimethyl-1H-indole-2-carbonyl)-methyl-amino]-methyl }-5-fluoro-phenoxy)-2-methyl-phenoxy]-2-methyl-propionic acid

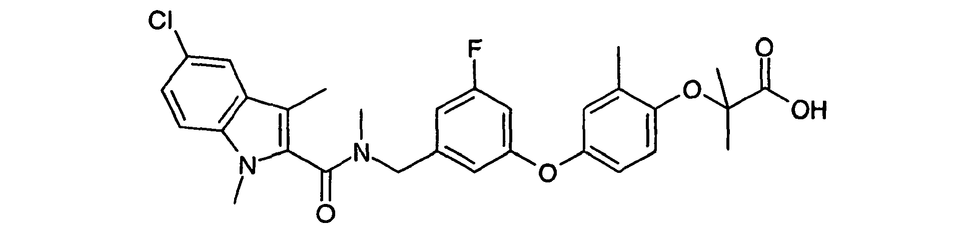

The title compound is prepared according to the procedure described in Example 141 utilizing 5-chloro-1,3-dimethyl-1H-indole-2-carboxylic acid (Intermediate 4) and 2-[4-(3-aminomethyl-5-fluoro-phenoxy)-2-methyl-phenoxy]-2-methyl-propionic acid ethyl ester (Intermediate 47). Exact mass calcd for C₃₀H₂₉N₂O₅FCl (M-H⁺): 551.1749, found 551.1747; ¹HNMR (400 MHz, CDCl₃).

### Example 182

### 2-[4-(3-Fluoro-5-1-[(3-methyl-benzo[b]thiophene-2-carbonyl)-amino]-ethyl}-phenoxy)-2-methyl-phenoxy]-2-methyl-propionic acid

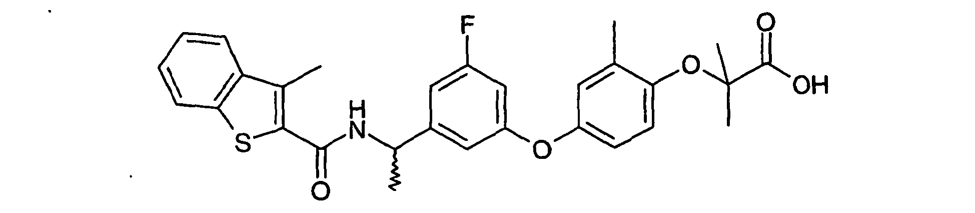

The title compound is prepared according to the general procedures described in Example 1 utilizing 3-methyl-benzo[b]thiophene-2-carboxylic acid and 2-{4-[3-(1-amino-ethyl)-5-fluoro-phenoxy]-2-methyl-phenoxy}-2-methyl-propionic acid ethyl ester (Intermediate 41). Exact mass calcd for C₂₉H₂₉NO₅FS (M+H⁺): 522.1750, found 522.1741; ¹HNMR (400 MHz, CDCl₃).

### Example 183

### 2-(4-{3-Fluoro-5-[1-(2-methyl-4-trifluoromethyl-benzoylamino)-ethyl]-phenoxy}-2-methyl-phenoxy)-2-methyl-propionic acid

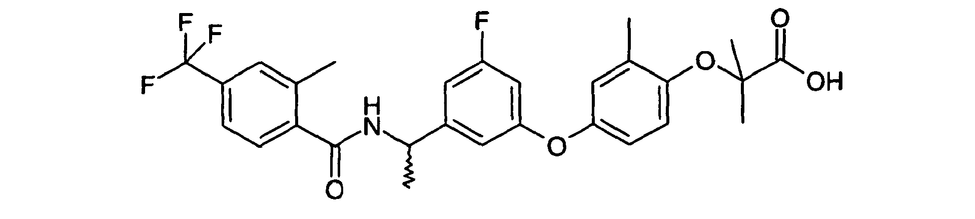

The title compound is prepared according to the general procedures described in Example 1 utilizing 2-methyl-4-trifluoromethyl-benzoic acid (Intermediate 10) and 2-{4-[3-(1-amino-ethyl)-5-fluoro-phenoxy]-2-methyl-phenoxy}-2-methyl-propionic acid ethyl ester (Intermediate 41). Exact mass calcd for C₂₈H₂₈NO₅F₄ (M+H⁺): 534.1904, found 534.1885; ¹HNMR (400 MHz, CDCl₃).

### Example 184

### 2-[4-(3-{[(5-Chloro-3-methyl-benzo[b]thiophene-2-carbonyl)-amino]-methyl}-5-methyl-phenoxy)-2-methyl-phenoxy]-2-methyl-propionic acid

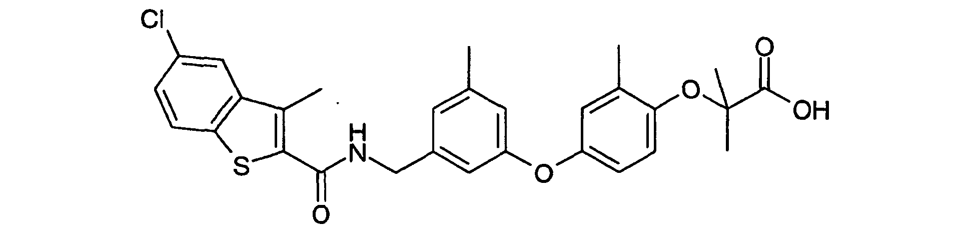

The title compound is prepared according to the general procedures described in Example 1 utilizing 5-chloro-3-methyl-benzo[b]thiophene-2-carboxylic acid and 2-[4-(3-aminomethyl-5-methyl-phenoxy)-2-methyl-phenoxy]-2-methyl-propionic acid ethyl ester (Intermediate 20). Exact mass calcd for C₂₉H₂₉ClNO₅S (M+H⁺): 538.1455, found 538.1454; ¹HNMR (400 MHz, CDCl₃).

### Example 185

### 3-[4-(3-Fluoro-5-{1-[(5-fluoro-3-methyl-1H-indole-2-carbonyl)-amino]-ethyl}-phenoxy)-2-methyl-phenyl]-propionic acid

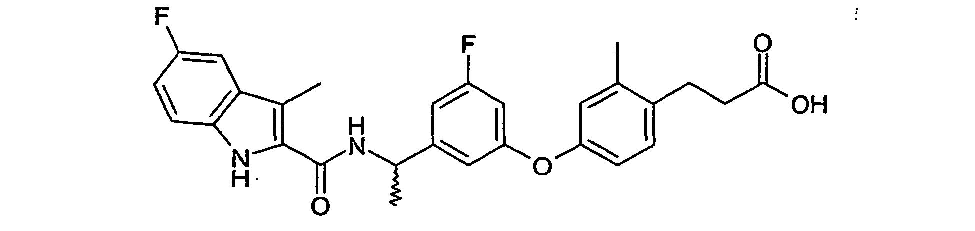

The title compound is prepared according to the general procedures described in Example 1 utilizing 5-fluoro-3-methyl-1H-indole-2-carboxylic acid (Intermediate 1) and 3-{4-[3-(1-amino-ethyl)-5-fluoro-phenoxy]-2-methyl-phenyl}-propionic acid methyl ester (Intermediate 34). Exact mass calcd for C₂₈H₂₆F₂N₂O₄ (M+H⁺): 493.1939, found 493.1950; ¹HNMR (400 MHz, CDCl₃).

### Example 186

### 3-[4-(3-Fluoro-5-{1-[(5-fluoro-1,3-dimethyl-1H-indole-2-carbonyl)-amino]-ethyl}-phenoxy)-2-methyl-phenyl]-propionic acid

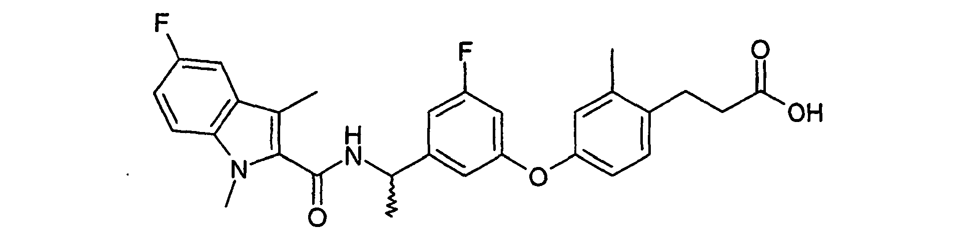

The title compound is prepared according to the general procedures described in Example 1 utilizing 5-fluoro-1,3-dimethyl-1H-indole-2-carboxylic acid (Intermediate 2) and 3-{4-[3-(1-amino-ethyl)-5-fluoro-phenoxy]-2-methyl-phenyl}-propionic acid methyl ester (Intermediate 34). Exact mass calcd for C₂₉H₂₉F₂N₂O₄ (M+H⁺): 507.2095, found 507.2097; ¹HNMR (400 MHz, CDCl₃).

### Example 187

### 3-[4-(3-{1-[(5-Chloro-1,3-dimethyl-1H-indole-2-carbonyl)-amino]-ethyl}-5-fluoro-phenoxy)-2-methyl-phenyl]-propionic acid

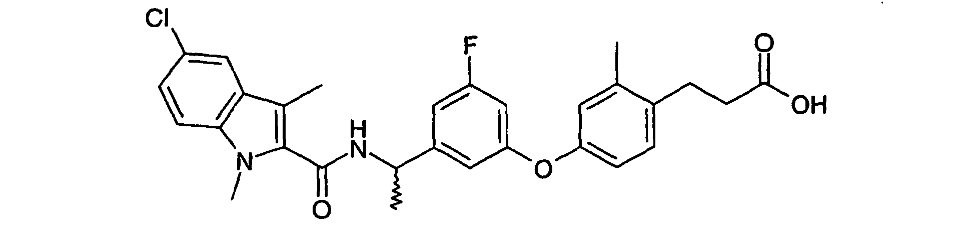

The title compound is prepared according to the general procedures described in Example 1 utilizing 5-chloro-1,3-dimethyl-1H-indole-2-carboxylic acid (Intermediate 4) and 3-{4-[3-(1-amino-ethyl)-5-fluoro-phenoxy]-2-methy]-phenyl}-propionic acid methyl ester (Intermediate 34). Exact mass calcd for C₂₉H₂₉ClFN₂O₄ (M+H⁺): 523.1800, found 523.1782; ¹HNMR (400 MHz, CDCl₃).

### Example 188

### (R) -3-[4-(3-Fluoro-5-{ 1-[(5-fluoro-3-methyl-1H-indole-2-carbonyl)-amino]-ethyl}-phenoxy)-2-methyl-phenyl]-propionic acid

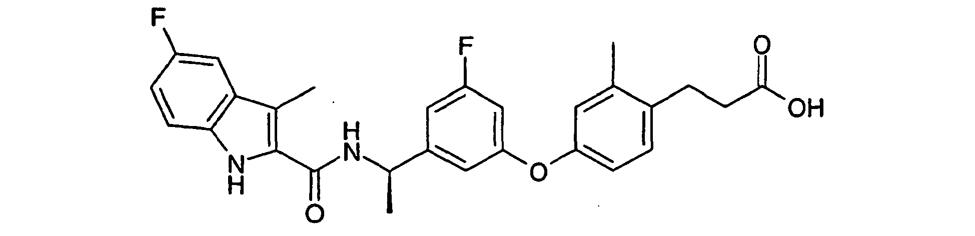

The title compound is prepared according to the procedure described in Example 176 from 3-[4-(3-{1-[(5-fluoro-3-methyl-1H-indole-2-carbonyl)-amino]-ethyl}-5-methyl-phenoxy)-2-methyl-phenyl]-propionic acid methyl ester (Example 185). Exact mass calcd for C₂₈H₂₇F₂N₂O₄ (M+H⁺): 493.1939, found 493.1946; ¹HNMR (400 MHz, CDCl₃).

### Example 189

### (S)-3-[4-(3-Fluoro-5-{1-[(5-fluoro-3-methyl-1H-indole-2-carbonyl)-amino]-ethyl }-phenoxy)-2-methyl-phenyl]-propionic acid

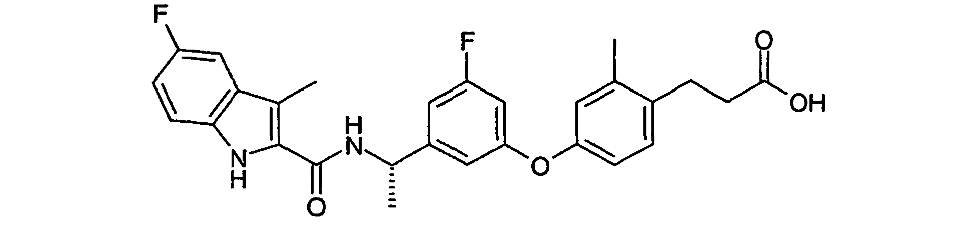

The title compound is prepared according to the procedure described in Example 176 from 3-[4-(3-{1-[(5-fluoro-3-methyl-1H-indole-2-carbonyl)-amino]-ethyl}-5-methyl-phenoxy)-2-methyl-phenyl]-propionic acid methyl ester (Example 185). Exact mass calcd for C₂₈H₂₇F₂N₂O₄ (M+H⁺): 493.1939, found 493.1930; ¹HNMR (400 MHz, CDCl₃).

### Example 190

### (R)-3-[4-(3-{1-[(5-Chloro-1,3-dimethyl-1H-indole-2-carbonyl)-amino]-ethyl}-5-fluoro-phenoxy)-2-methyl-phenyl]-propionic acid

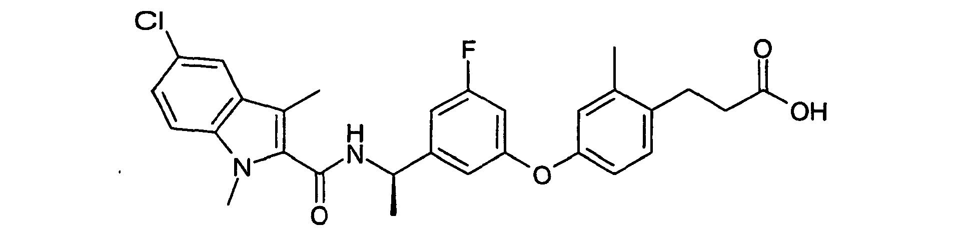

The title compound is prepared according to the procedure described in Example 176 from 3-[4-(3-{1-[(5-chloro-1,3-dimethyl-1H-indole-2-carbonyl)-amino]-ethyl}-5-fluoro-phenoxy)-2-methyl-phenyl]-propionic acid methyl ester (Example 187). Exact mass calcd for C₂₉H₂₉ClFN₂O₄ (M+H⁺): 523.1800, found 523.1789; ¹HNMR (400 MHz, CDCl₃).

### Example 191

### (S)-3-[4-(3-{1-[(5-Chloro-1,3-dimethyl-1H-indole-2-carbonyl)-amino]-ethyl)-5-fluoro-phenoxy)-2-methyl-phenyl]-propionic acid

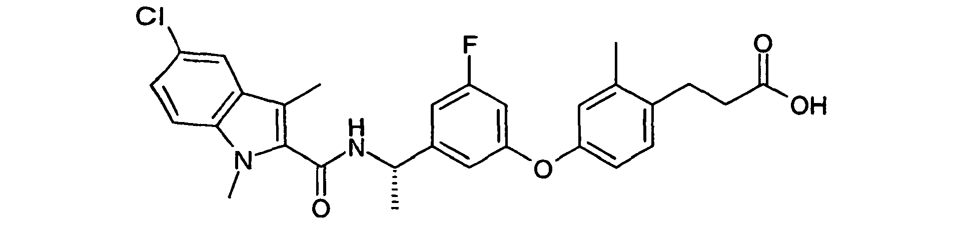

The title compound is prepared according to the procedure described in Example 176 from 3-[4-(3-{1-[(5-chloro-1,3-dimethyl-1H-indole-2-carbonyl)-amino]-ethyl}-5-fluoro-phenoxy)-2-methyl-phenyl]-propionic acid methyl ester (Example 187). Exact mass calcd for C₂₉H₂₉ClFN₂O₄ (M+H⁺): 523.1800, found 523.1794; ¹HNMR (400 MHz, CDCl₃).

### Example 192

### 3-(4-{3-Fluoro-5-[(2-methyl-4-trifluoromethyl-benzoylamino)-methyl]-phenoxy}-2-methyl-phenyl)-2,2-dimethyl-propionic acid

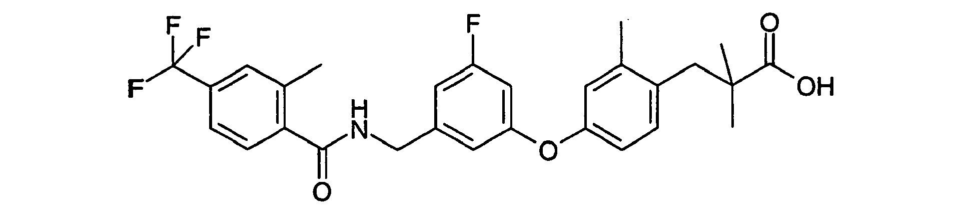

The title compound is prepared according to the general procedures described in Example 1 utilizing 2-methyl-4-trifluoromethyl-benzoic acid (Intermediate 10) and 3-[4-(3-aminomethyl-5-fluoro-phenoxy)-2-methyl-phenyl]-2,2-dimethyl-propionic acid methyl ester (Intermediate 42). Exact mass calcd for C₂₈H₂₈NO₄F₄ (M+H⁺): 518.1954, found 518.1943; ¹HNMR (400 MHz, CDCl₃).

### Example 193

### 3-[4-(3-Fluoro-5-{1-[(5-fluoro-3-methyl-1H-indole-2-carbonyl)-amino]-propyl)-phenoxy)-2-methyl-phenyl]-propionic acid

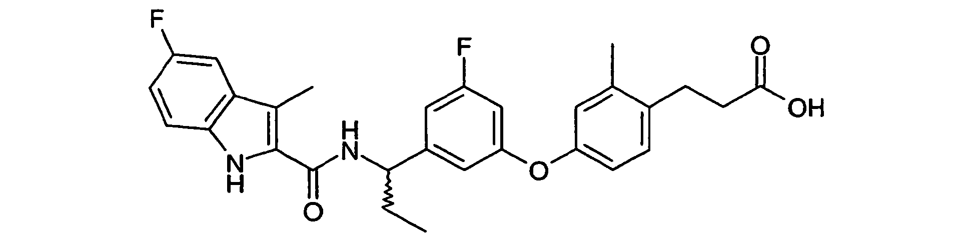

The title compound is prepared according to the general procedures described in Example 1 utilizing 5-fluoro-3-methyl-1H-indole-2-carboxylic acid (Intermediate 1) and 3-{4-[3-(1-amino-propyl)-5-fluoro-phenoxy]-2-methyl-phenyl)-propionic acid methyl ester (Intermediate 43). Exact mass calcd for C₂₉H₂₉F₂N₂O₄ (M+H⁺): 507.2095, found 507.2085; ¹HNMR (400 MHz, CDCl₃).

### Example 194

### 3-[4-(3-{1-[(5-Chloro-1,3-dimethyt-1H-indole-2-carbonyl)-amino]-propyl}-5-fluoro-phenoxy)-2-methyl-phenyl]-propionic acid

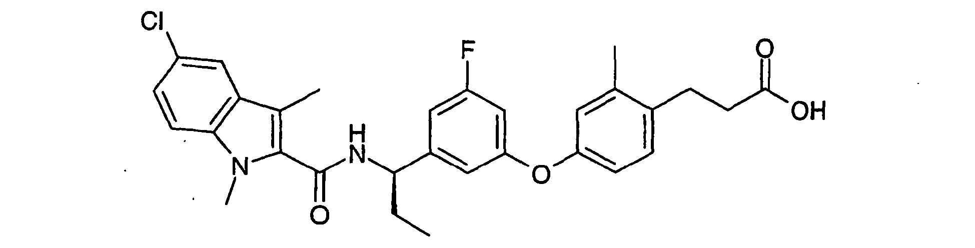

The title compound is prepared according to the general procedures described in Example 1 utilizing 5-chloro-1,3-dimethyl-1H-indole-2-carboxylic acid (Intermediate 4) and 3-{4-[3-(1-amino-propyl)-5-fluoro-phenoxy]-2-methyl-phenyl)-propionic acid methyl ester (Intermediate 44). Exact mass calcd for C₃₀H₃₁ClFN₂O₄ (M+H⁺): 537.1956, found 537.1942; ¹HNMR (400 MHz, CDCl₃).

### Example 195

### 3-[4-(3-Fluoro-5-{1-[(5-fluoro-3-methyl-1H-indole-2-carbonyl)-amino]-propyl}-phenoxy)-2-methyl-phenyl]-propionic acid

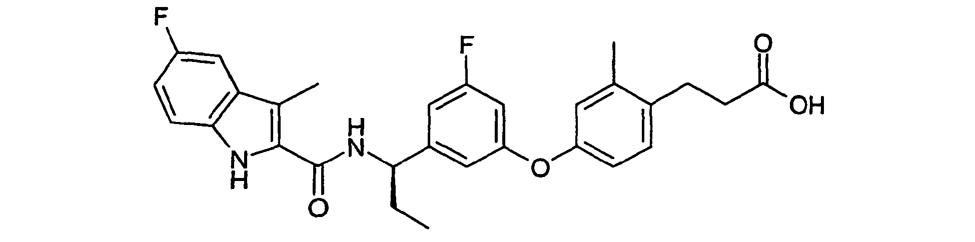

The title compound is prepared according to the general procedures described in Example 1 utilizing 5-fluoro-3-methyl-1H-indole-2-carboxylic acid (Intermediate 1) and 3-{4-[3-(1-amino-propyl)-5-fluoro-phenoxy]-2-methyl-phenyl}-propionic acid methyl ester (Intermediate 44). Exact mass calcd for C₂₉H₂₉F₂N₂O₄ (M+H⁺): 507.2095, found 507.2083; ¹HNMR (400 MHz, CDCl₃).

### Example 196

### 3-[4-(3-Fluoro-5-(1-[(5-fluoro-1,3-dimethyl-1H-indole-2-carbonyl)-amino]-propyl)-phenoxy)-2-methyl-phenyl]-propionic acid

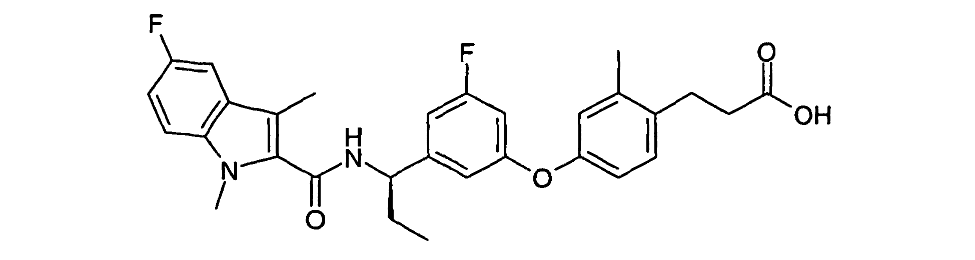

The title compound is prepared according to the general procedures described in Example 1 utilizing 5-fluoro-1,3-dimethyl-1H-indole-2-carboxylic acid (Intermediate 2) and 3-{4-[3-(1-amino-propyl)-5-fluoro-phenoxy]-2-methyl-phenyl}-propionic acid methyl ester (Intermediate 44). Exact mass calcd for C₃₀H₃₁F₂N₂O₄ (M+H⁺): 521.2252, found 521.2243; ¹HNMR (400 MHz, CDCl₃).

### Example 197

### 3-(4-{3-[1-(2-Fluoro-4-trifluoromethyl-benzoylamino)-ethyl]-phenoxy}-2-methylphenyl)-propionic acid

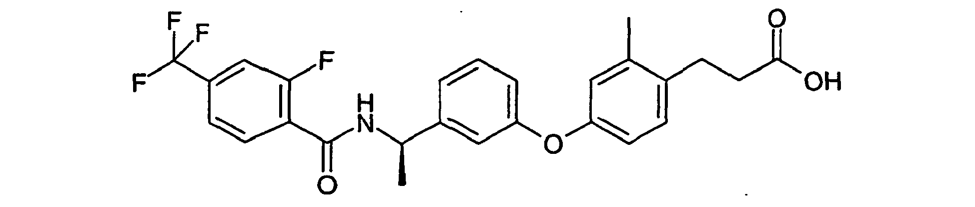

The title compound is prepared according to the general procedures described in Example 1 utilizing 2-fluoro-4-trifluoromethyl-benzoic acid and 3-{4-[3-(1-amino-ethyl)-phenoxy]-2-methyl-phenyl}-propionic acid methyl ester (Intermediate 24). Exact mass calcd for C₂₆H₂₃NO₄F₄(M+H⁺): 490.1641, found 490.1625; ¹HNMR (400 MHz, CDCl₃).

### Example 198

### 3-(4-{3-[1-(2-Methoxy-4-trifluoromethyl-benzoylamino)-ethyl]-phenoxy}-2-methylphenyl)-propionic acid

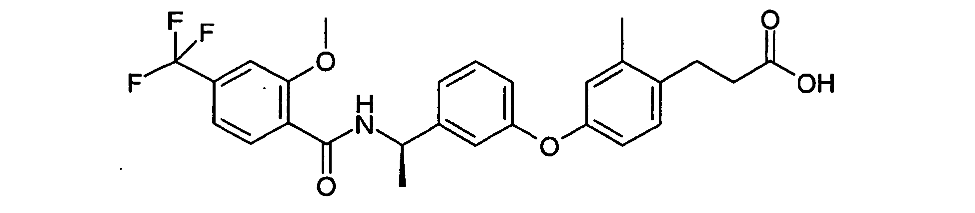

The title compound is prepared according to the general procedures described in Example 1 utilizing 2-methoxy-4-trifluoromethyl-benzoic acid *(*J.Am.Chem.Soc.; 73; 1951; 2375) and 3-{4-[3-(1-amino-ethyl)-phenoxy]-2-methylphenyl}-propionic acid methyl ester (Intermediate 24). Exact mass calcd for C₂₇H₂₇NO₅F₃ (M+H⁺): 502.1841, found 502.1827; ¹HNMR (400 MHz, CDCl₃).

### Example 199

### 3-[4-(3-{[(5-Chloro-1,3-dimethyl-1H-indole-2-carbonyl)-amino]-methyl}-5-methyl-phenoxy)-2-methyl-phenyl]-propionic acid

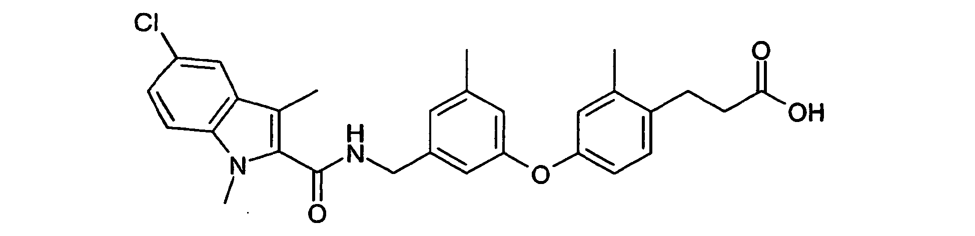

The title compound is prepared according to the general procedures described in Example 1 utilizing 5-chloro-1,3-dimethyl-1H-indole-2-carboxylic acid (Intermediate 4) and 3-[4-(3-aminomethyl-5-methyl-phenoxy)-2-methyl-phenyl]-propionic acid methyl ester (Intermediate 45). Exact mass calcd for C₂₉H₃₀CIN₂O₄ (M+H⁺): 505.1894, found 505.1882; ¹HNNM (400 MHz, CDCl₃).

### Example 200

### 3-[4-(3-{[(5-Chloro-3-methyl-benzo[b]thiophene-2-carbonyl)-amino]-methyl)-5-methyl-phenoxy)-2-methyl-phenyl]-propionic acid

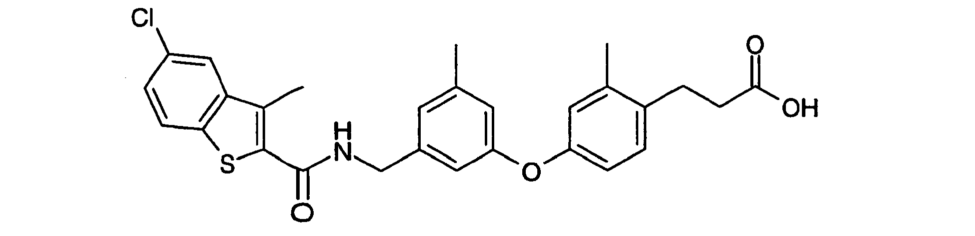

The title compound is prepared according to the general procedures described in Example 1 utilizing 5-chloro-3-methyl-benzo[b]thiophene-2-carboxylic acid and 3-[4-(3-aminomethyl-5-methyl-phenoxy)-2-methyl-phenyl]-propionic acid methyl ester (Intermediate 45). Exact mass calcd for C₂₈H₂₇ClNO₄S (M+H⁺): 508.1349, found 508.1346; ¹HNMR (400 MHz, CDCl₃).

### Example 201

### 3-(4-{3-[(2-Fluoro-4-trifluoromethyl-benzoylamino)-methyl]-5-methyl-phenoxy}-2-methyl-phenyl)-propionic acid

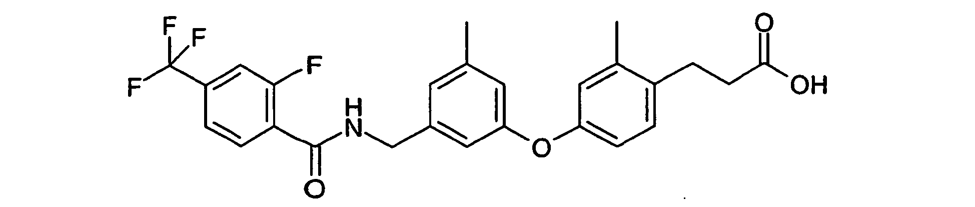

The title compound is prepared according to the general procedures described in Example 1 utilizing 2-fluoro-4-trifluoromethyl-benzoic acid and 3-[4-(3-aminomethyl-5-methyl-phenoxy)-2-methyl-phenyl]-propionic acid methyl ester (Intermediate 45). Exact mass calcd for C₂₆H₂₄F₄NO₄ (M+H⁺): 490.1641, found 490.1645; ¹HNMR (400 MHz, CDCl₃).

### Example 202

### 3-(2-Ethyl-4-{3-fluoro-5-[(2-methyl-4-trifluoromethyl-benzoylamino)-methyl]-phenoxy}-phenyl)-propionic acid

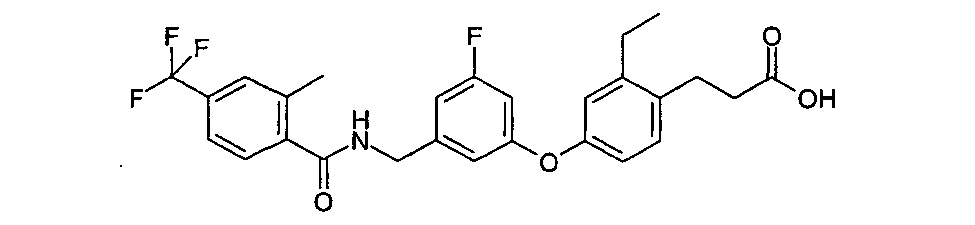

The title compound is prepared according to the general procedures described in Example 1 utilizing 2-methyl-4-trifluoromethyl-benzoic acid (Intermediate 10) and 3-[4-(3-aminomethyl-5-fluoro-phenoxy)-2-ethyl-phenyl]-propionic acid ethyl ester (Intermediate 27). Exact mass calcd for C₂₇H₂₆NO₄F₄ (M+H⁺): 504.1798, found 504.1791; ¹HNMR (400 MHz, CDCl₃).

### Example 203

### 3-[4-(3-{1-[(5-Chloro-1,3-dimethyl-1H-indole-2-carbonyl)-amino]-ethyl}-5-methyl-phenoxy)-2-methyl-phenyl]-propionic acid

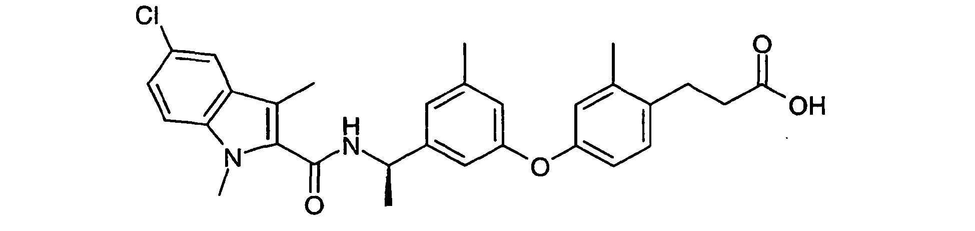

The title compound is prepared according to the general procedures described in Example 1 utilizing 5-chloro-1,3-dimethyl-1H-indole-2-carboxylic acid (Intermediate 4) and 3-{4-[3-(1-amino-ethyl)-5-methyl-phenoxy]-2-methyl-phenyl}-propionic acid methyl ester (Intermediate 26). Exact mass calcd for C₃₀H₃₂ClN₂O₄ (M+H⁺): 519.2051, found 519.2045; ¹HNNM (400 MHz, CDCl₃).

### Example 204

### 2-{4-[3-Fluoro-5-(2-fluoro-4-trifluoromethyl-benzoylamino)-phenoxy]-2-methyl-phenoxy}-2-methyl-propionic acid

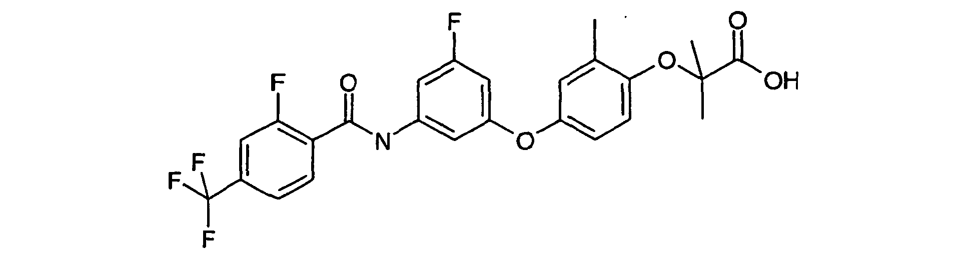

The title compound is prepared according to the general procedures described in Example 1 utilizing 2-fluoro-4-trifluoromethyl-benzoic acid and 2-[4-(3-amino-5-fluoro-phenoxy)-2-methyl-phenoxy]-2-methyl-propionic acid ethyl ester (Intermediate 47). Exact mass calcd for C₂₅H₂₁F₅NO₅ (M+H⁺): 510.1340, found 510.1328; ¹HNMR (400 MHz, CDCl₃).

### Example 205

### 3-[4-(3-{1-[(5-Chloro-3-methyl-benzo[b]thiophene-2-carbonyl)-amino]-ethyl}-5-methyl-phenoxy)-2-ethyl-phenyl]-propionic acid

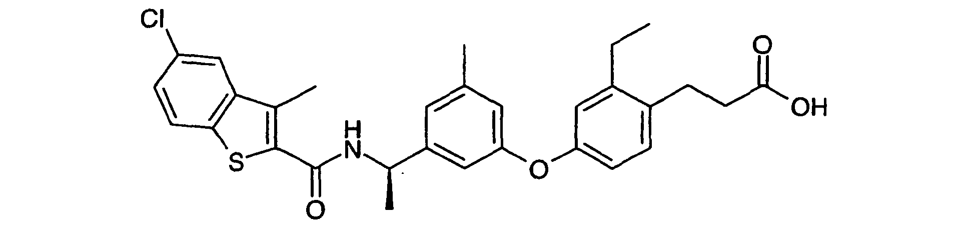

The title compound is prepared according to the general procedures described in Example 1 utilizing 5-chloro-3-methyl-benzo[b]thiophene-2-carboxylic acid and 3-{4-(3-(1-amino-ethyl)-5-methyl-phenoxy]-2-ethyl-phenyl}-propionic acid ethyl ester (Intermediate 16). Exact mass calcd for C₃₀H₃₁ClNO₄S (M+H⁺): 536.1662, found 536.1648; ¹HNMR (400 MHz, CDCl₃).

### Example 206

### 3-(2-Ethyl-4-{3-[1-(2-fluoro-4-trifluoromethyl-benzoylamino)-ethyl]-5-methyl-phenoxy}-phenyl)-propionic acid

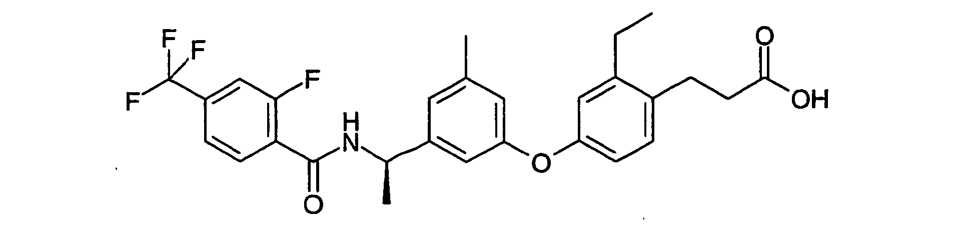

The title compound is prepared according to the general procedures described in Example 1 utilizing 2-fluoro-4-trifluoromethyl-benzoic acid and 3-{4-[3-(1-amino-ethyl)-5-methyl-phenoxy]-2-ethyl-phenyl -propionic acid ethyl ester (Intermediate 16). Exact mass calcd for C₂₈H₂₈F₄NO₄ (M+H⁺): 518.1954, found 518.1937; ¹HNMR (400 MHz, CDCl₃).

### Example 207

### 3-[4-(3-{[(5-Chloro-1,3-dimethyl-1H-indole-2-carbonyl)-amino]-methyl}-5-methyl-phenoxy)-2-ethyl-phenyl]-propionic acid

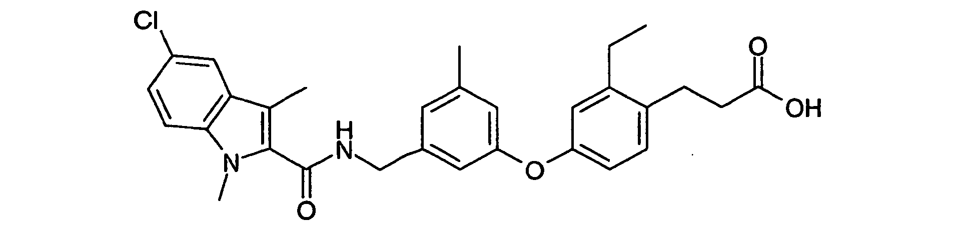

The title compound is prepared according to the general procedures described in Example 1 utilizing 5-chloro-1,3-dimethyl-1H-indole-2-carboxylic acid (Intermediate 4) and -[4-(3-aminomethyl-5-methyl-phenoxy)-2-ethyl-phenyl]-propionic acid ethyl ester (Intermediate 46). Exact mass calcd for C₃₀H₃₂CIN₂O₄ (M+H⁺): 519.2051, found 519.2032; ¹HNMR (400 MHz, CDCl₃).

### Example 208

### 3-[4-(3-{[(5-Chloro-3-methyl-benzo[b]thiophene-2-carbonyl)-amino]-methyl}-5-methyl-phenoxy)-2-ethyl-phenyl]-propionic acid

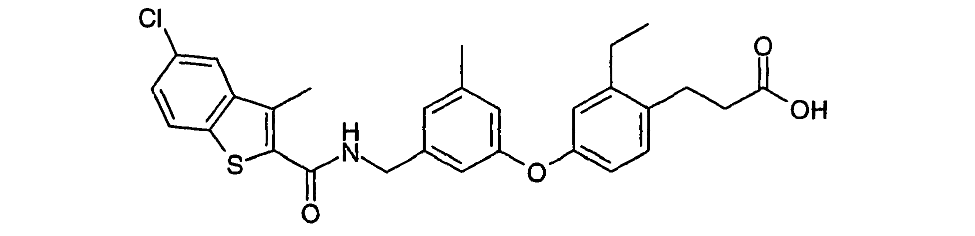

The title compound is prepared according to the general procedures described in Example 1 utilizing 5-chloro-3-methyl-benzo[b]thiophene-2-carboxylic acid and -[4-(3-aminomethyl-5-methyl-phenoxy)-2-ethyl-phenyl]-propionic acid ethyl ester (Intermediate 46). Exact mass calcd for C₂₉H₂₉ClNO₄S (M+H⁺): 522.1506, found 522.1502; ¹HNMR (400 MHz, CDCl₃).

### Example 209

### 3-[4-(3-{[(5-Fluoro-1,3-dimethyl-1H-indole-2-carbonyl)-amino]-methyl}-5-methyl-phenoxy)-2-methyl-phenyl]-propionic acid

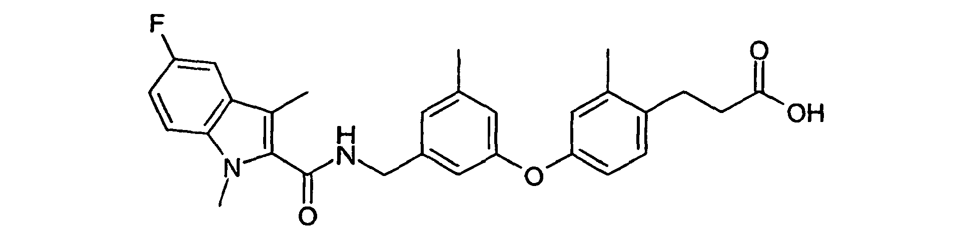

The title compound is prepared according to the general procedures described in Example 1 utilizing 5-fluoro-1,3-dimethyl-1H-indole-2-carboxylic acid (Intermediate 2) and 3-[4-(3-aminomethyl-5-methyl-phenoxy)-2-methyl-phenyl]-propionic acid methyl ester (Intermediate 45). Exact mass calcd for C₂₉H₃₀FN₂O₄ (M+H⁺): 489.2190, found 489.2188; ¹HNMR (400 MHz, CDCl₃).

### Example 210

### 3-[4-(3-{[(5-Fluoro-3-methyl-1H-indole-2-carbonyl)-amino]-methyl}-5-methyl-phenoxy)-2-methyl-phenyl]-propionic acid

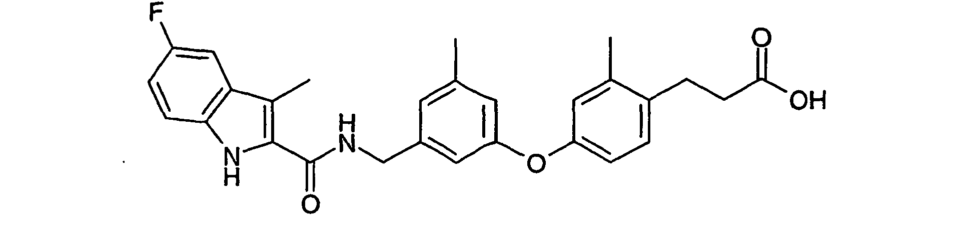

The title compound is prepared according to the general procedures described in Example 1 utilizing 5-fluoro-3-methyl-1H-indole-2-carboxylic acid (Intermediate 1) and 3-[4-(3-aminomethyl-5-methyl-phenoxy)-2-methyl-phenyl)-propionic acid methyl ester (Intermediate 45). Exact mass calcd for C₂₈H₂₈FN₂O₄ (M+H⁺): 475.2033, found 475.2020; ¹HNMR (400 MHz, CDCl₃).

### Example 211

### 3-[2-Methyl-4-(3-[(3-methyl-5-trifluoromethyl-benzo[b]thiophene-2-carbonyl)-amino]-methyl}-phenoxy)-phenyl]-propionic acid

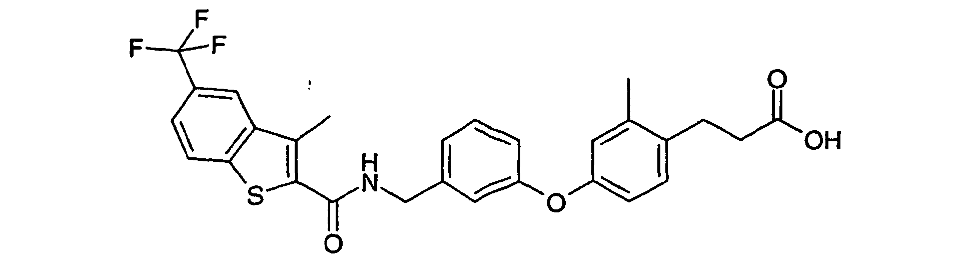

The title compound is prepared according to the general procedures described in Example 1 utilizing 3-methyl-5-trifluoromethyl-benzo[b]thiophene-2-carboxylic acid (Intermediate 50) and 3-[4-(3-aminomethyl-phenoxy)-2-methyl-phenyl]-propionic acid methyl ester (Intermediate 33).

### Example 212

### 2-Methyl-2-(2-methyl-4-{3-[(4-trifluoromethyl-benzenesulfonylamino)-methyl]-phenoxy}-phenoxy)-propionic acid

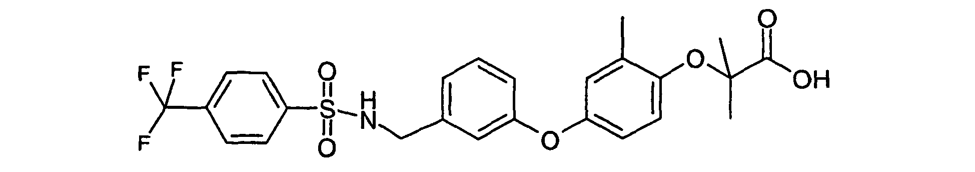

The title compound is prepared according to the general procedures described in Example 140 utilizing 4-trifluoromethylbenzenesulfonyl chloride and 2-[4-(3-aminomethylphenoxy)-2-methylphenoxy]-2-methylpropionic acid ethyl ester (Intermediate 17). Mass (ES⁺): 522.2 (M+H⁺).

### Example 213

### 2-{ 4-[3-(Isopropoxycarbonylamino-methyl)-phenoxy]-2-methyl-phenoxy}-2-methyl-propionic acid

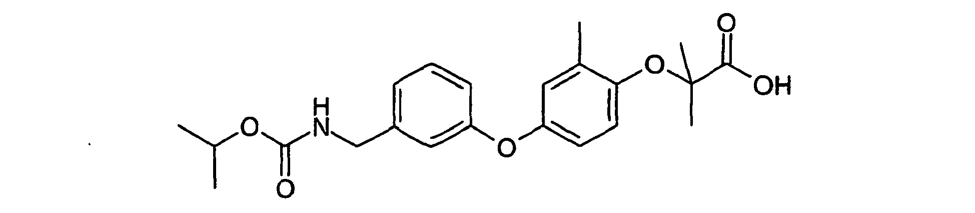

The title compound is prepared according to the general procedures described in Example 140 utilizing isopropyl chloroformate and 2-[4-(3-aminomethylphenoxy)-2-methylphenoxy]-2-methylpropionic acid ethyl ester (Intermediate 17). Mass (ES⁺): 402.3 (M+H⁺).

### Example 214

### {2-Methyl-4-[3-(4-trifluoromethylbenzylcarbamoyl)phenoxy]phenoxy}acetic acid

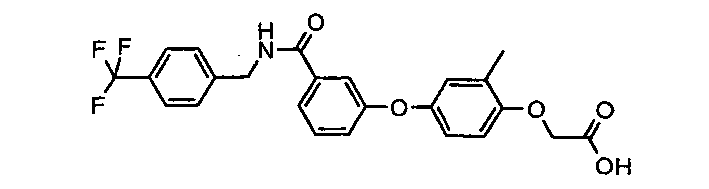

### Step A

### 3-(4-Benzyloxy-3-methylphenoxy)benzonitrile

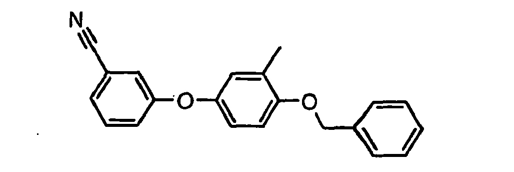

A mixture of 4-benzyloxy-3-methylphenol (2.19 g, 10.2 mmol) (prepared substantially the similar way as described in WO9723216), 3-fluorobenzonitrile (1.09 mL, 10.2 mmol), 18-Crown-6 (0.27 g, 1.02 mmol) and 37% potassium fluoride-alumina (3.8 g) (prepared substantially the similar way as described in Tet Lett, 32, 7207, (1997)) is stirred in DMSO (25 mL) at 145 °C for about 8 h. The reaction is diluted in 400 mL ether, and the insoluble material is filtered away. The filtrate is washed with brine (3 x 200 mL), dried with Na₂SO₄ and concentrated to give the title compound as a dark oil (3.1 g, 96%). MS (ES) *mle* 314 (M-1).

### Step B

### 3-(4-Benzyloxy-3-methylphenoxy)benzoic acid

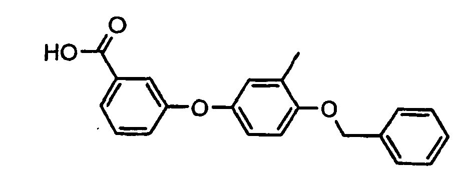

A solution of 3-(4-benzyloxy-3-methylphenoxy)benzonitrile (3.1 g, 9.8 mmol) in methanol (7 mL) and ethanol (10 mL) is treated with KOH pellets (11.0 g, 196.0 mmol). To this slurry is added 30% H₂O₂, and the mixture is heated to 100 °C for 2 h. After cooling, the solution is treated with 5N NaOH (30 mL), ether (30mL), and acidified to pH 1 with conc. HCl (20 mL). The residue is diluted with EtOAc (100 mL) and washed with brine. The organic layer is dried with Na₂SO₄ and concentrated to give the title compound as a tan solid (3.17 g, 96%). MS (ES) *m*/*e* 335 (M+1).

### Step C

### 3-(4-Hydroxy-3-methylphenoxy)benzoic acid

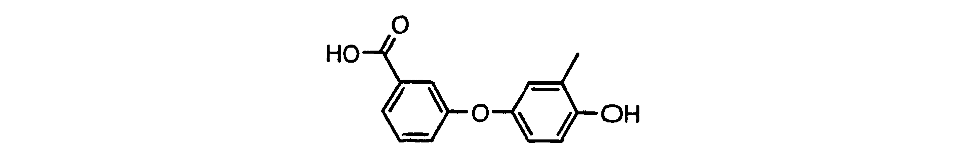

To a solution of 3-(4-benzyloxy-3-methylphenoxy)benzoic acid (3.15 g (9.42 mmol) dissolved in THF (10 mL) and EtOH (50 mL) is treated with 5% Pd/C (0.60 g) and H₂ gas (1 atm) for about 16 h. The catalyst is filtered away, and the filtrate is concentrated and redissolved in MeCl₂ (100 mL). The mixture is washed with brine, dried with Na₂SO₄ and concentrated to give the title compound as a yellow solid (2.17 g, 94%). MS (ES) *mle* 243 (M-1).

### Step D

### 3-(4-Hydroxy-3-methylphenoxy)benzoic acid benzyl ester

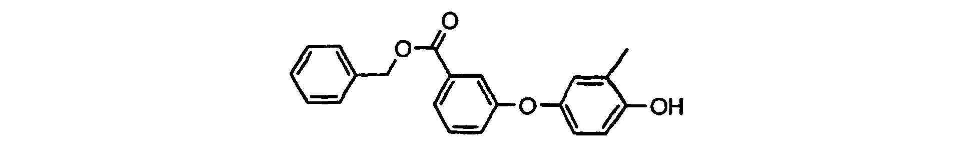

A mixture of 3-(4-hydroxy-3-methylphenoxy)benzoic acid (2.13 g, 8.72 mmol), benzyl bromide (1.35 mL, 11.3 mmol) and cesium carbonate (1.42 g, 4.36 mmol) in DMF (8 mL) is stirred at RT for 16 h. The reaction is concentrated, and the residue is taken up in EtOAc (100 mL), which is then washed with brine, dried with Na₂SO₄, concentrated and purified (radial chromatography, 4 mm plate, 5:95 to 25:75 EtOAc:hex) to give the title compound as a yellow solid (1.70 g, 58%). MS (ES) *m*/*e* 333 (M-1).

### Step E

### 3-(4-Ethoxycarbonylmethoxy-3-methylphenoxy)benzoic acid benzyl ester

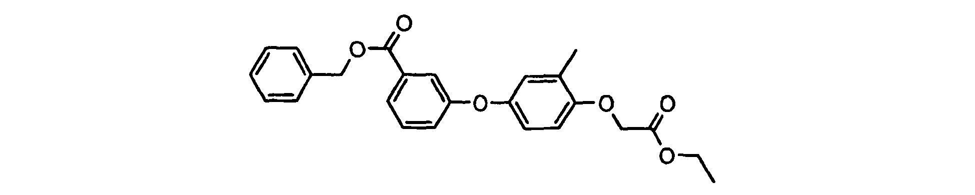

A mixture of 3-(4-hydroxy-3-methylphenoxy)benzoic acid benzyl ester (0.85 g, 2.54 mmol), ethylbromoacetate (0.65 mL, 5.08 mmol) and cesium carbonate (1.65 g, 5.08 mmol) in DMF (6 mL) is stirred at RT for 2 h. The reaction is concentrated, and the residue is taken up in EtOAc (100 mL), which is then washed with brine, dried with Na₂SO₄, concentrated and purified (radial chromatography, 2 mm plate, 5:95 to 15:85 EtOAc:hex) to give the title compound as a colorless oil (0.82 g, 76%). MS (ES) *mle* 438 (M+NH₄).

### Step F

### 3-(4-Ethoxycarbonylmethoxy-3-methylphenoxy)benzoic acid

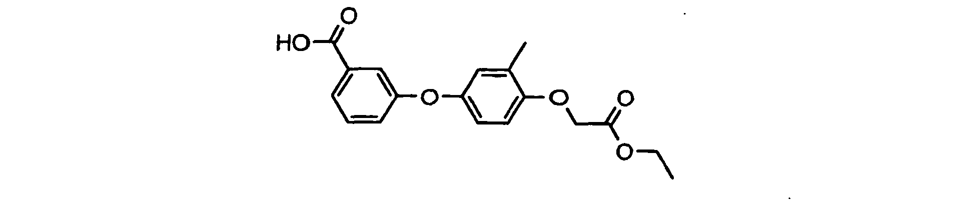

A mixture of 3-(4-ethoxycarbonylmethoxy-3-methylphenoxy)benzoic acid benzyl ester (0.80 g, 1.90 mmol) in EtOH (200 mL) is treated with 5% Pd/C (0.19 g) and H₂ gas (60 psi) at RT for about 4.5 h. The catalyst is filtered, and the filtrate is concentrated to give the title compound as a white solid (0.61 g, 96%). MS (ES) *m*/*e* 348 (M+NH₄).

### Step G

### [4-(3-Chlorocarbonylphenoxy)-2-methylphenoxy]acetic acid ethyl ester

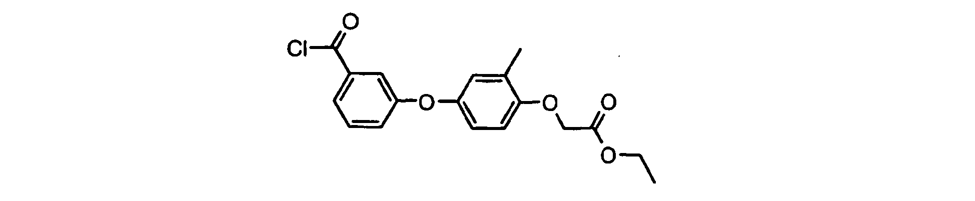

To a solution of 3-(4-ethoxycarbonylmethoxy-3-methylphenoxy)benzoic acid (0.61 g, 1.84 mmol) in MeCl₂ (30 mL) is added oxalyl chloride (0.95 mL, 11.1 mmol) and one drop of DMF. The solution is stirred for 4 h at RT and concentrated to give the title compound as a pale yellow oil (0.61 g, 95%), which is used directly in the next step.

### Step H-I

### {2-Methyl-4-[3-(4-trifluoromethylbenzylcarbamoyl)phenoxy]phenoxy} acetic acid

A solution of 4-trifluoromethylbenzylamine (0.031 mL, 0.24 mmol) and Et₃N (0.033 mL, 0.24 mmol) in MeCl₂ (1 mL) is treated with [4-(3-chlorocarbonyl-phenoxy)-2-methylphenoxy]acetic acid ethyl ester (0.040 g, 0.11 mmol) in 1 mL MeCl₂. The reaction is stirred at RT for 2.5 h and concentrated. The residue is treated with EtOH (1 mL), THF (0.5 mL) and 2N NaOH (0.30 mL, 0.60 mmol) and heated at 50 °C for 16 h. After cooling, the reaction is concentrated and diluted with 20 mL MeCl₂ and 20 mL water and acidified to pH 1 using 1N HCl. The organic layer is washed with brine and concentrated. The crude residue is purified using mass-directed reverse phase HPLC to give the title compound as a white solid (0.013 g, 25%. MS (ES) *m*/*e* 460 (M+1).

Examples 215 to 219 are prepared in the substantially the similar way as described in Example 214.

### Example 215

### [4-(3-Diphenethylcarbamoylphenoxy)-2-methylphenoxy]acetic acid

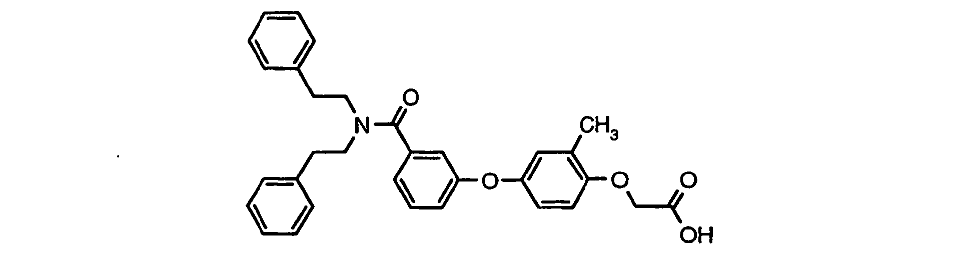

The title compound is prepared by using diphenethylamine, which is prepared substantially the similar way as described in Example 258, Steps A and B. MS (ES) *m*/*e* 510 (M+1).

### Example 216

### [4-(3-{[2-(4-Methoxyphenyl)ethyl]phenethylcarbamoyl}phenoxy)-2-methylphenoxy] acetic acid

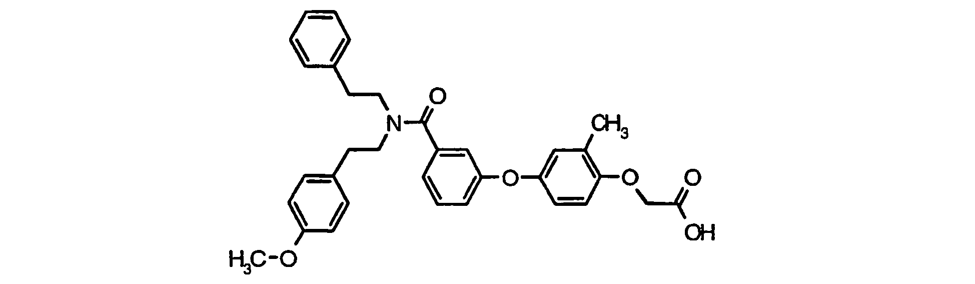

The title compound is prepared by using [2-(4-methoxyphenyl)ethyl] phenethylamine, which is prepared substantially the similar way as described in Example 258, Steps A and B. MS (ES) m/e 540 (M+1).

### Example 217

### (4-[3-(Benzylphenethylcarbamoyl)phenoxy]-2-methylphenoxy}acetic acid

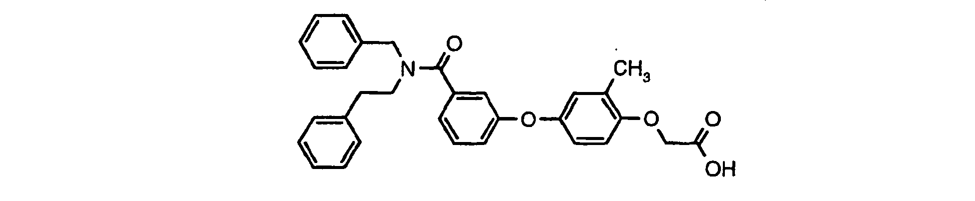

The title compound is prepared by using commercially available benzylphenethylamine. MS (ES) *m*/*e* 496 (M+1).

### Example 218

### {4-[3-(4-Methoxybenzylcarbamoyl)phenoxy]-2-methylphenoxy} acetic acid

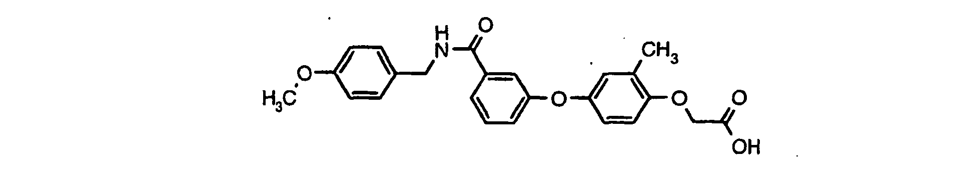

The title compound is prepared by using commercially available 4-methoxybenzylamine. MS (ES) *m*/*e* 422 (M+1).

### Example 219

### [4-(3-Hexylcarbamoylphenoxy)-2-methylphenoxy]acetic acid

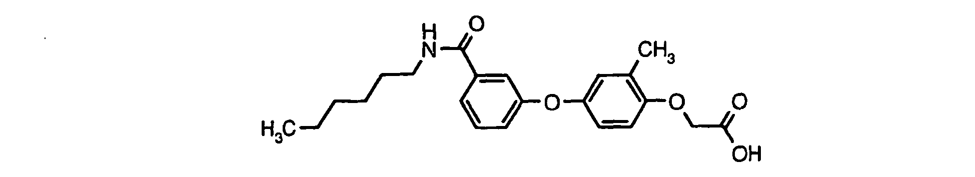

The title compound is prepared by using commercially available n-hexylamine. MS (ES) *m*/*e* 386 (M+1).

Example 220 to 254 are prepared substantially the similar way as described in Example 214 except that ethyl 2-bromoisobutyrate is used in Example 214, Step E.

### Example 220

### 2-[4-(3-Diphenethylcarbamoylphenoxy)-2-methylphenoxyl-2-methylpropionic acid

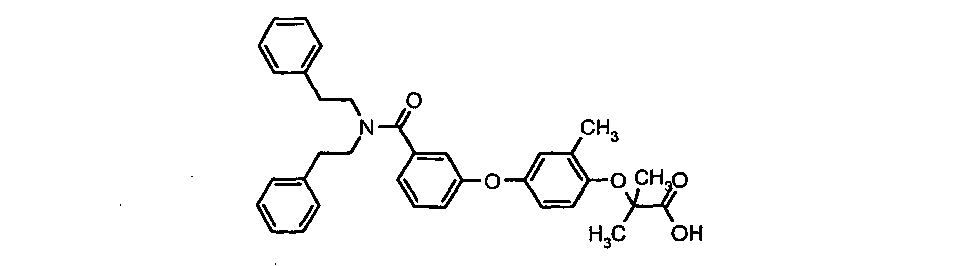

The title compound is prepared by using diphenythylamine, which is prepared substantially the similar way as described in Example 258, Steps A and B. MS (ES) *m*/*e* 538 (M+1).

### Example 221

### 2-[4-(3-{[2-(4-Methoxyphenyl)ethyl]phenethylcarbamoyl}phenoxy)-2-methyphenoxy]-2-methylpropionic acid

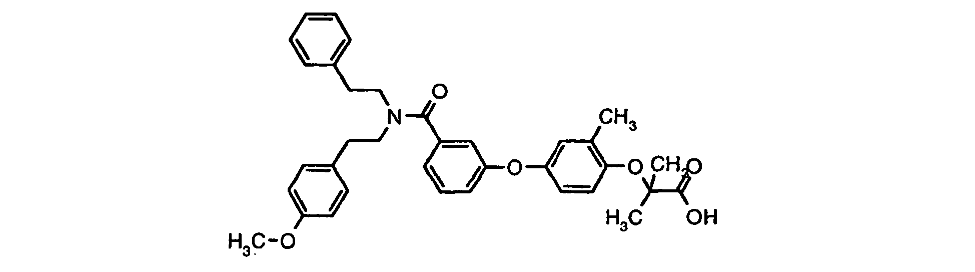

The title compound is prepared by using [2-(4-methoxyphenyl)ethyl] phenethylamine which is prepared substantially the similar way as described in Example 258, Steps A and B. MS (ES) *m*/*e* 554 (M+1).

### Example 222

### 2-{4-[3-(Benzylphenethylcarbamoyl)phenoxy]-2-methylphenoxy}-2-methylpropionic acid

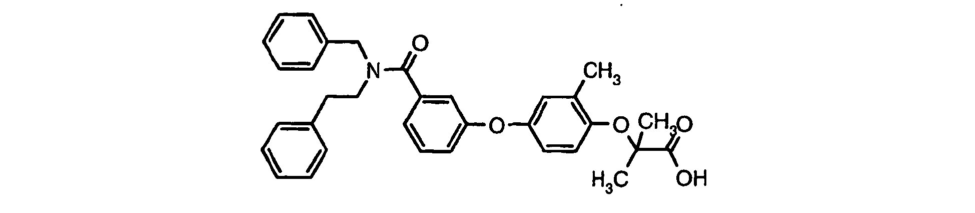

The title compound is prepared by using the commercially available benzylphenethylamine. MS (ES) *m*/*e* 524 (M+1).

### Example 223

### 2-Methyl-2-{2-methyl-4-[3-(4-trifluoromethylbenzylcarbamoyl)phenoxy]phenoxy} propionic acid

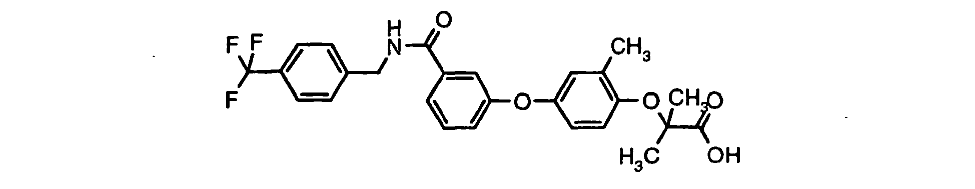

The title compound is prepared by using the commercially available 4-trifluoromethylbenzylamine. MS (ES) *m*/*e* 488 (M+1).

### Example 224

### 2-{4-[3-(4-Methoxybenzylcarbamoyl)phenoxy]-2-methylphenoxy}-2-methylpropionic acid,

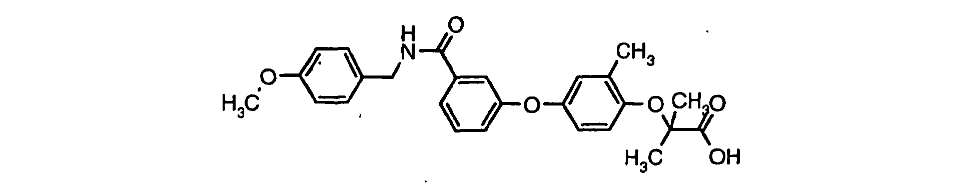

The title compound is prepared by using the commercially available 4-methoxybenzylamine. MS (ES) *m*/*e* 450 (M+1).

### Example 225

### 2-[4-(3-Hexylcarbamoylphenoxy)-2-methylphenoxy]-2-methylpropionic acid

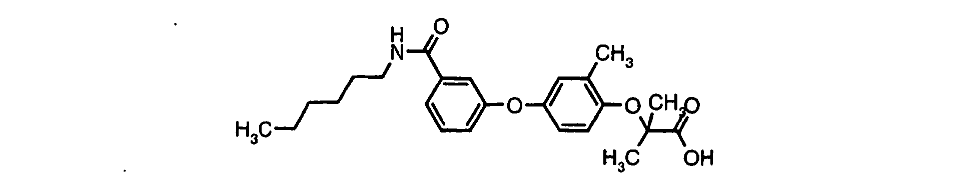

The title compound is prepared by using the commercially available n-hexylamine. MS (ES) *mle* 414 (M+1).

### Example 226

### 2-{4-[3-(3,5-Bistrifluoromethylbenzylcarbamoyl)phenoxy]-2-methylphenoxy}-2-methylpropionic acid

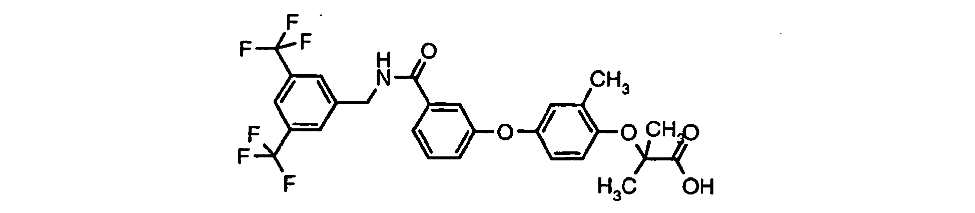

The title compound is prepared by using the commercially available 3,5-trifluoromethylbenzylamine. MS (ES) *m*/*e* 556 (M+1).

### Example 227

### 2-[4-(3-{[2-(3,4-Dimethoxyphenyl)ethyl]hexylcarbamoyl}phenoxy)-2-methylphenoxy]-2-methylpropionic acid

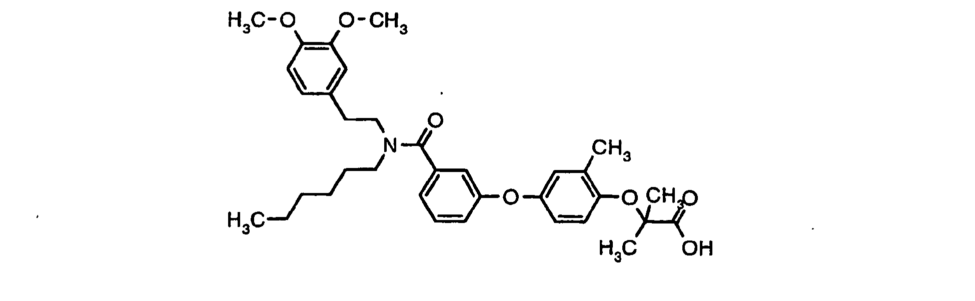

The title compound is prepared by using [2-(3,4-dimethoxyphenyl) ethyl]hexylamine which may be prepared essentially as described in Example 258, Steps A and B. MS (ES) *m*/*e* 578 (M+1).

### Example 228

### 2-[4-(3-{[2-(3,4-Dimethoxyphenyl)ethyl]heptylcarbamoyl}phenoxy)-2-methylphenoxy]-2-methylpropionic acid

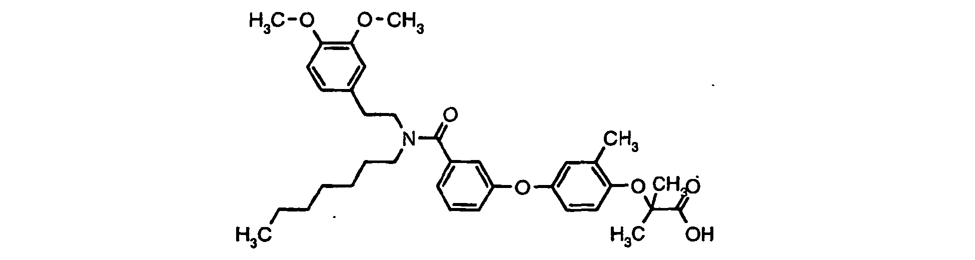

The title compound is prepared by using [2-(3,4-dimethoxyphenyl)ethyl] heptylamine which is prepared substantially the similar way as described in Example 258, Steps A and B. MS (ES) *m*/*e* 592 (M+1).

### Example 229

### 2-[4-(3-{[2-(3,5-Bistrifluoromethylphenyl)ethyl]phenethylcarbamoyl}phenoxy)-2-methylphenoxy]-2-methylpropionic acid

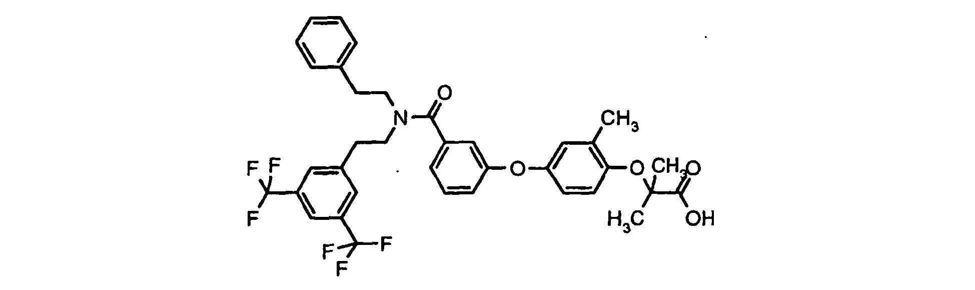

The title compound is prepared by using [2-(3,5-bistrifluoromethylphenyl) ethyl]phenethylamine which is prepared substantially the similar way described in Example 258, Steps A and B. MS (ES) *m*/*e* 674 (M+1).

### Example 230

### 2-Methyl-2-[2-methyl-4-(3-{phenethyl-[2-(4-trifluoromethylphenyl)ethyl]carbamoyl} phenoxy)phenoxy]propionic acid

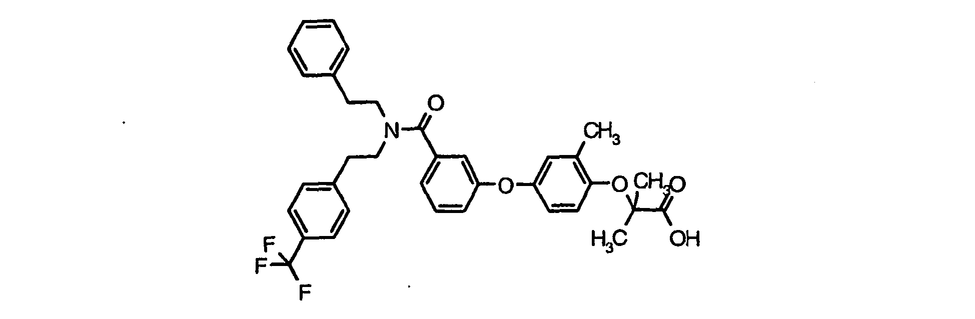

The title compound is prepared by using [2-(4-trifluoromethylphenyl) ethyl]phenethylamine, which is prepared substantially the similar way as described in Example 258, Steps A and B. MS (ES) *m*/*e* 606 (M+1).

### Example 231

### 2-{4-[3-(6-Methoxy-3,4-dihydro-1H-isoquinoline-2-carbonyl)phenoxy]-2-methylphenoxy}-2-methylpropionic acid

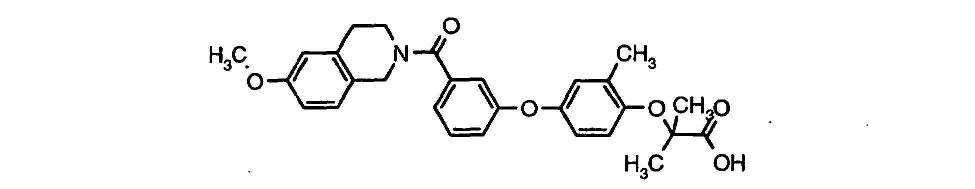

The title compound is prepared by using 6-methoxy-1,2,3,4-tetrahydoisoquinoline which is prepared substantially the similar way as described in JACS, 56, 1769-1771 (1934). MS (ES) *m*/*e* 476 (M+1).

### Example 232

### 2-Methyl-2-{2-methyl-4-[3-(1-methyl-3,4-dihydro-1H-isoquinoline-2-carbonyl)phenoxy] phenoxy}propionic acid

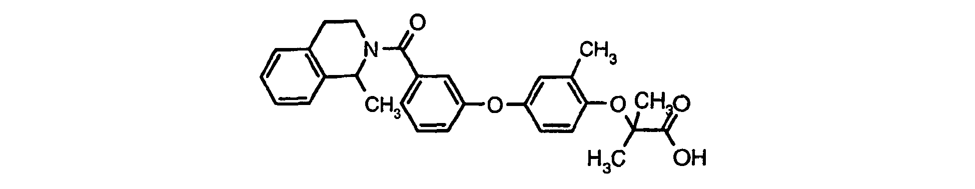

The title compound is prepared by using 1-methyl-1,2,3,4-tetrahydroisoquinoline, which is prepared substantially the similar way as described in J. Chem. Soc. Perkin Trans. 1, 9, 955-977 (2001). MS (ES) *m*/*e* 460 (M+1).

### Example 233

### 2-{4-[3-(6,7-Dichloro-3,4-dihydro-1H-isoquinoline-2-carbonyl)phenoxy]-2-methylphenoxy}-2-methylpropionic acid

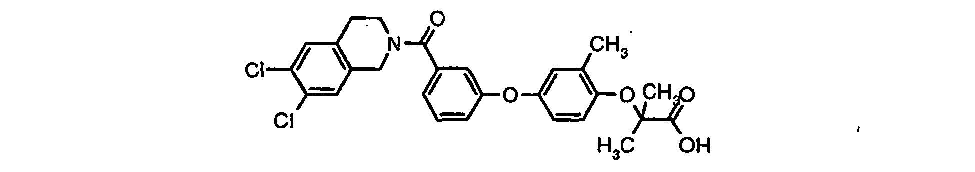

The title compound is prepared by using 6,7-dichloro-1,2,3,4-tetrahydoisoquinoline, which is prepared substantially the similar way as described in Tet. Lett., 21 1393-1396 (1980). MS (ES) *m*/*e* 515 (M+1).

### Example 234

### 2-Methyl-2-{2-methyl-4-[3-(1-phenyl-3,4-dihydro-1H-isoquinoline-2-carbonyl)phenoxy] phenoxy}propionic acid

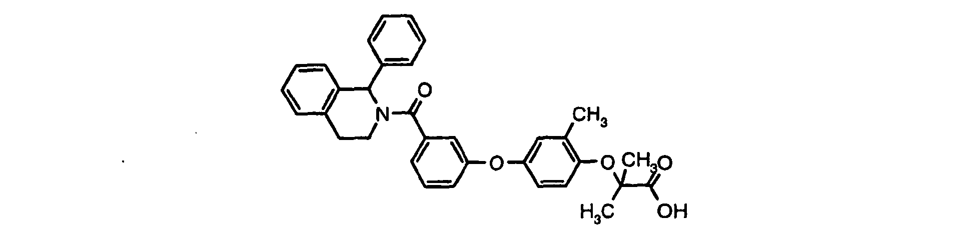

The title compound is prepared by using 1-phenyl-1,2,3,4-tetrahydroisoquinolin, which is prepared substantially the similar way as described in Chem. Ber., 91, 1133-1138 (1958). MS (ES) *m*/*e* 522 (M+1).

### Example 235

### 2-Methyl-2-{2-methyl-4-[3-(1-methyl-3,4-dihydro-1H-isoquinoline-2-carbonyl)phenoxy] phenoxy}propionic acid

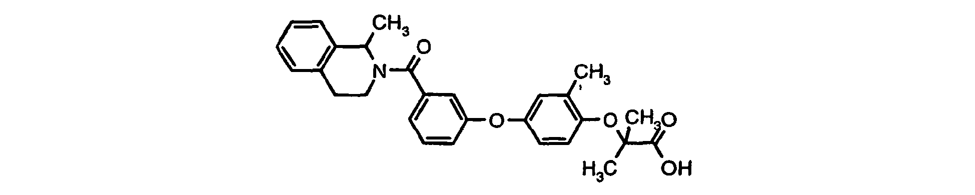

The title compound is prepared by using 1-methyl-1,2,3,4-tetrahydroisoquinoline, which is prepared substantially the similar way as described in *J.* Chem. Soc. Perkin Trans. 1, 9, 955-977 (2001). MS (ES) *m*/*e* 460 (M+1).

### Example 236

### 2-{4-[3-(3,4-Dihydro-1H-isoquinoline-2-carbonyl)phenoxy]-2-methylphenoxy}-2-methylpropionic acid

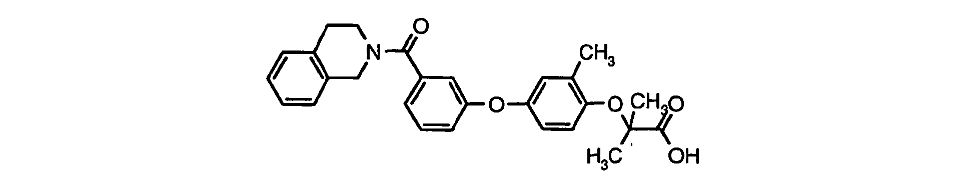

The title compound is prepared by using the commercially available 1,2,3,4-tetrahydroisoquinoline. MS (ES) *mle* 446 (M+1).

### Example 237

### 2-Methyl-2-(2-methyl-4-{3-[2-(4-trifluoromethylphenyl)ethylcarbamoyl] phenoxy}phenoxy)propionic acid

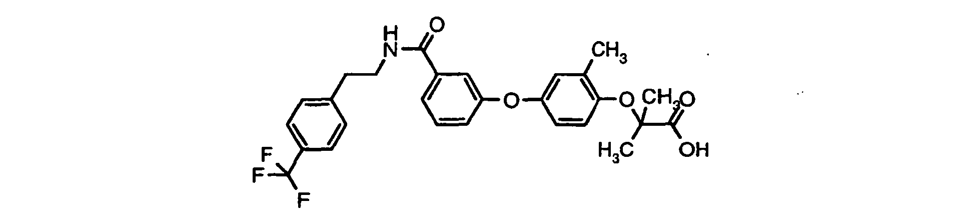

The title compound is prepared by using the commercially available 4-trifluoromethylphenethylamine. MS (ES) *m*/*e* 502 (M+1).

### Example 238

### 2-Methyl-2-(2-methyl-4-{3-[2-(3-trifluoromethylphenyl)ethylcarbamoyl]phenoxy} phenoxy) propionic acid

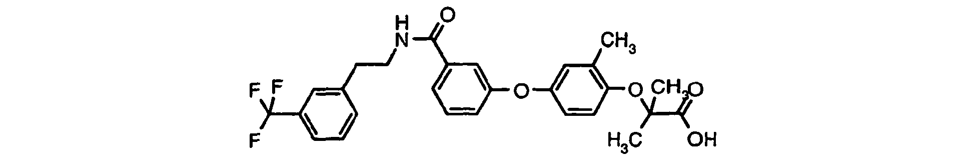

The title compound is prepared by using the commercially available 3-trifluoromethylphenethylamine. MS (ES) *m*/*e* 502 (M+1).

### Example 239

### 2-Methyl-2-[2-methyl-4-(3-{methyl-[2-(3-trifluoromethylphenyl)ethyl]carbamoyl} phenoxy) phenoxy]propionic acid

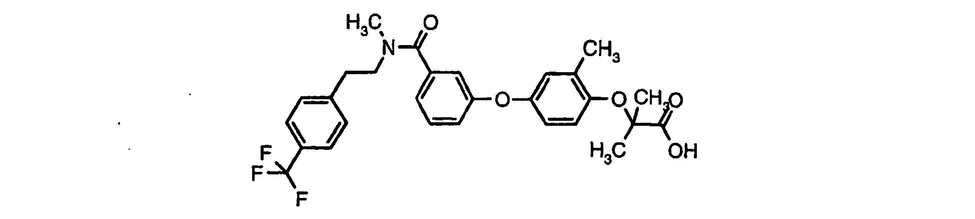

The title compound is prepared by using methyl[2-(4-trifluoromethylphenyl) ethyl]amine, which is prepared substantially the similar way as described in Example 258, Steps A and B. MS (ES) *m*/*e* 516 (M+1).

### Example 240

### 2-{4-[3-(6,7-Dimethoxy-3,4-dihydro-1H-isoquinoline-2-carbonyl)phenoxy]-2-methylphenoxy}-2-methylpropionic acid

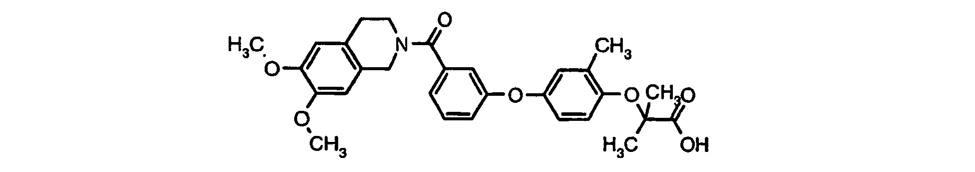

The title compound is prepared by using 6,7-dimethoxy-1,2,3,4-tetrahydoisoquinoline, which is prepared substantially the similar way as described in JACS, 56, 1769-1771 (1934), MS (ES), *mle* 506 (M+1).

### Example 241

### 2-Methyl-2-(2-methyl-4-{3-[methyl-(4-trifluoromethylbenzyl)carbamoyl]phenoxy) phenoxy)propionic acid

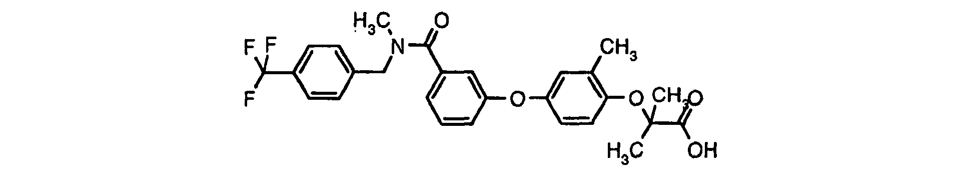

The title compound is prepared by using methyl(4-trifluoromethylbenzyl)amine which is prepared substantially the similar way as described in Example 258, Steps A and B. MS (ES) *m*/*e* 502 (M+1).

### Example 242

### 2-Methyl-2-{2-methyl-4-[3-(6-trifluoromethyl-3,4-dihydro-1H-isoquinoline-2-carbonyl)phenoxy]phenoxy}propionic acid

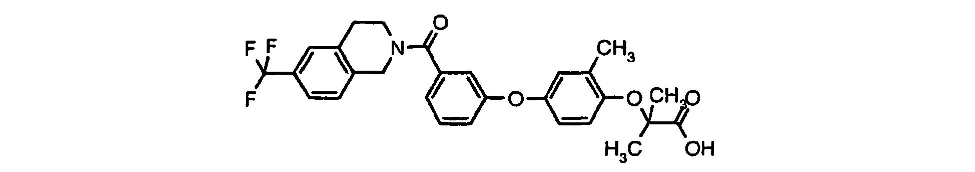

The title compound is prepared by using 6-trifluoromethyl-1,2,3,4-tetrahydroisoquinoline, which is prepared substantially the similar way as described in WO 9850364. MS (ES) *m*/*e* 515 (M+1).

### Example 243

### 2-{4-[3-(6-Methoxy-3,4-dihydro-1H-isoquinoline-2-carbonyl)phenoxy]-2-methylphenoxy}-2-methylpropionic acid

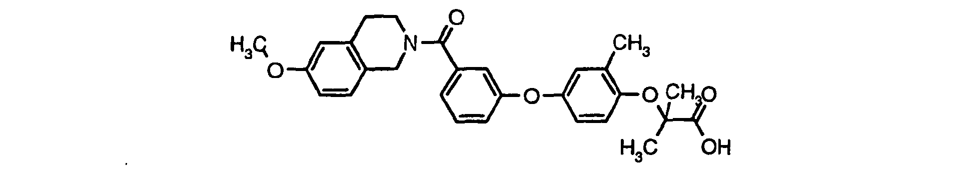

The title compound is prepared by using 6-methoxy-1,2,3,4-tetrahydroisoquinoline, which is prepared substantially the similar way as described in JACS, 56, 1769-1771 (1934). MS (ES) *mle* 476 (M+1).

### Example 244

### 2-Methyl-2-{2-methyl-4-[3-(1-methyl-3,4-dihydro-1H-isoquinoline-2-carbonyl)phenoxy] phenoxy}propionic acid

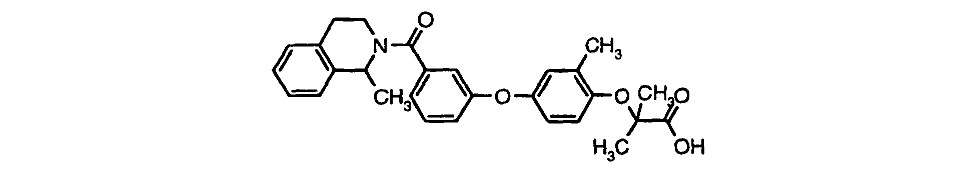

The title compound is prepared by using 1-methyl-1,2,3,4-tetrahydroisoquinoline, which is prepared substantially the similar way as described in J. Chem. Soc. Perkin Trans. 1, 9, 955-977 (2001). MS (ES) *m*/*e* 460 (M+1).

### Example 245

### 2-{4-[3-(6,7-Dichloro-3,4-dihydro-1H-isoquinoline-2-carbonyl)phenoxy]-2-methylphenoxy}-2-methylpropionic acid

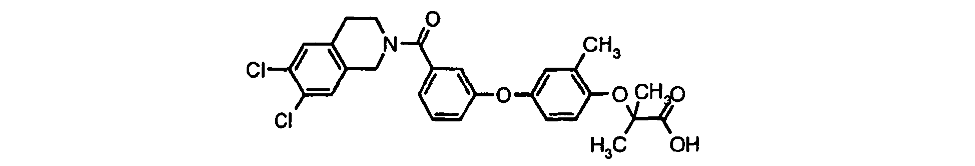

The title compound is prepared by using 6,7-dichloro-1,2,3,4-tetrahydoisoquinoline, which is prepared substantially the similar way as described in Tet. Lett., 21, 1393-1396 (1980). MS (ES) *m*/*e* 515 (M+1).

### Example 246

### 2-Methyl-2- 2-methyl-4-[3-(1-phenyl-3,4-dihydro-1H-isoquinoline-2-carbonyl)phenoxy] phenoxy}propionic acid

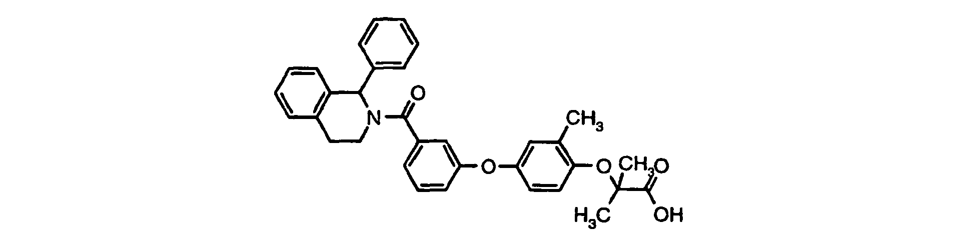

The title compound is prepared by using 1-phenyl-1,2,3,4-tetrahydroisoquinoline, which is prepared substantially the similar way as described in Chem. Ber., 91, 1133-1138 (1958). MS (ES) *m*/*e* 522 (M+1).

### Example 247

### 2-Methyl-2-{2-methyl-4-[3-(1-methyl-3,4-dihydro-1H-isoquinoline-2-carbonyl)phenoxy]phenoxy}propionic acid

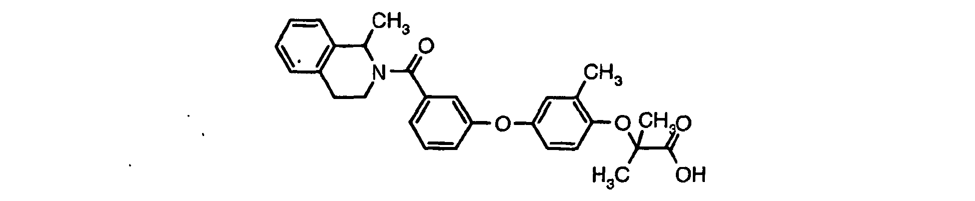

The title compound is prepared by using 1-methyl-1,2,3,4-tetrahydroisoquinoline, which is prepared substantially the similar way as described in J. Chem. Soc. Perkin Trans. 1, 9, 955-977 (2001). MS (ES) *m*/*e* 460 (M+1).

### Example 248

### 2-{4-[3-(3,4-Dihydro-1H-isoquinoline-2-carbonyl)phenoxy]-2-methylphenoxy}-2-methylpropionic acid

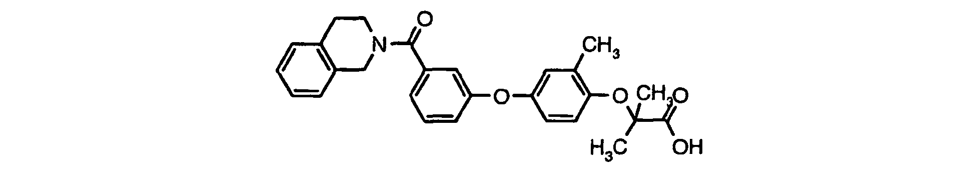

The title compound is prepared by using the commercially available 1,2,3,4-tetrahydroisoquinoline. MS (ES) *mle* 446 (M+1).

### Example 249

### 2-Methyl-2-(2-methyl-4-(3-[2-(4-trifluoromethylphenyl)ethylcarbamoyl]phenoxy} phenoxy)propionic acid

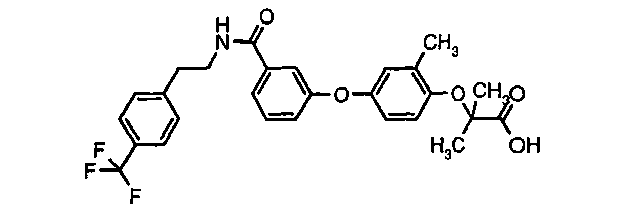

The title compound is prepared by using the commercially available 4-trifluoromethylphenethylamine. MS (ES) *m*/*e* 502 (M+1).

### Example 250

### 2-Methyl-2-(2-methyl-4- {3-[2-(3-trifluoromethylphenyl)ethylcarbamoyl]phenoxy} phenoxy)propionic acid

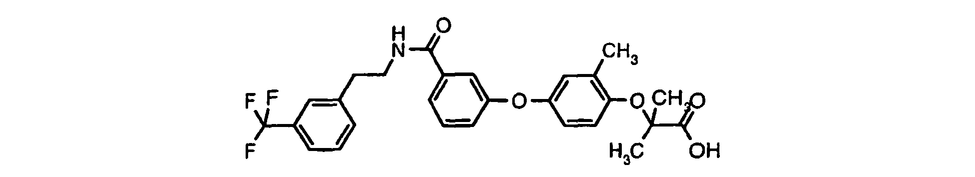

The title compound is prepared by using the commercially available 3-trifluoromethylphenethylamine. MS (ES) *m*/*e* 502 (M+1).

### Example 251

### 2-Methyl-2-[2-methyl-4-(3-{ methyl-[2-(4-trifluoromethylphenyl)ethyl]carbamoyl } phenoxy)phenoxy]propionic acid

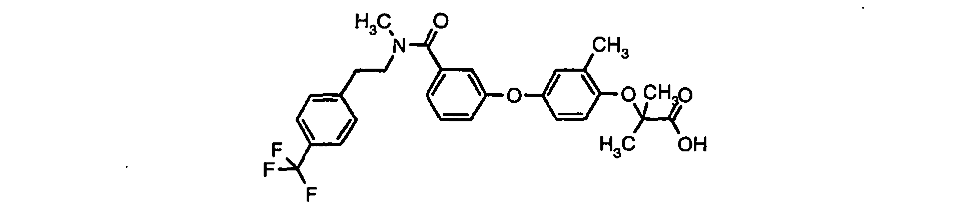

The title compound is prepared by using methyl(4-trifluoromethylphenyl) ethylamine, which is prepared substantially the similar way as described in Example 258, Steps A and B. MS (ES) *m*/*e* 516 (M+1).

### Example 252

### 2-Methyl-2-[2-methyl-4-(3-{methyl-[2-(3-trifluoromethylphenyl)ethyl]carbamoyl} phenoxy)phenoxy]propionic acid

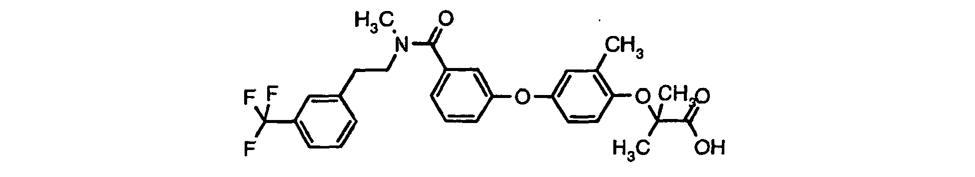

The title compound is prepared by using methyl(3-trifluoromethylphenyl) ethylamine, which is prepared substantially the similar way as described in Example 258, Steps A and B. MS (ES) *m*/*e* 516 (M+1).

### Example 253

### 2-{4-[3-(6,7-Dimethoxy-3,4-dihydro-1H-isoquinoline-2-carbonyl)phenoxy]-2-methylphenoxy}-2-methylpropionic acid

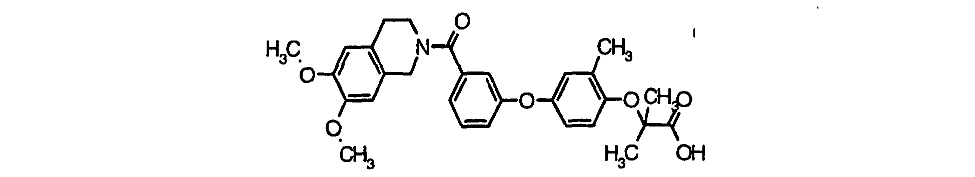

The title compound is prepared by using 6,7-dimethoxy-1,2,3,4-tetrahydoisoquinoline, which is prepared substantially the similar way as described in JACS, 56, 1769-1771 (1934). MS (ES) *m*/*e* 506 (M+1).

### Example 254

### 2-Methyl-2-(2-methyl-4-{3-[methyl-(4-trifluoromethylbenzyl)carbamoyl]phenoxy} phenoxy)propionic acid

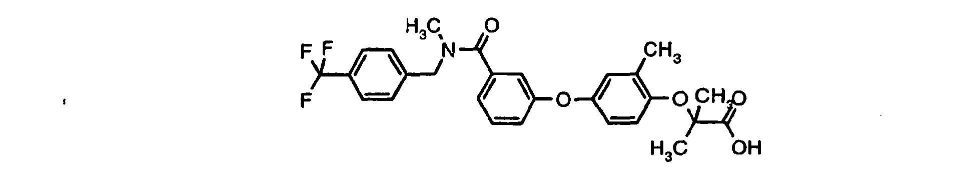

The title compound is prepared by using methyl(4-trifluoromethylbenzyl)amine, which is prepared substantially the similar way as described in Example 258, Steps A and B. MS (ES) *m*/*e* 502 (M+1).

### Example 255

### 2-Methyl-2-{4-[4-(4-trifluoromethylbenzylcarbamoyl)phenoxy]phenoxy}propionic acid

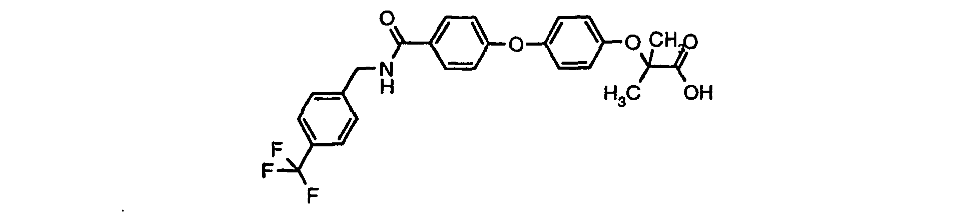

The title compound is prepared by using the commercially available 4-trifluoromethylbenzylamine and following the procedure described in Example 214 except that the synthesis begins with Example 214, Step C using the commercially available 4-(4-hydoxyphenoxy)benzoic acid and ethyl 2-bromoisobutyrate in Example 214, Step E. MS (ES) *m*/*e* 474 (M+1).

Examples 256 to 257 are prepared as described in Example 214 except that 2-fluorobenzonitrile is used in Example 214, Step A and ethyl 2-bromoisobutyrate is used in Example 214, Step E.

### Example 256

### 2-Methyl-2-{4-[2-(6-trifluoromethyl-3,4-dihydro-1H-isoquinoline-2-carbonyl)phenoxy]phenoxy}propionic acid

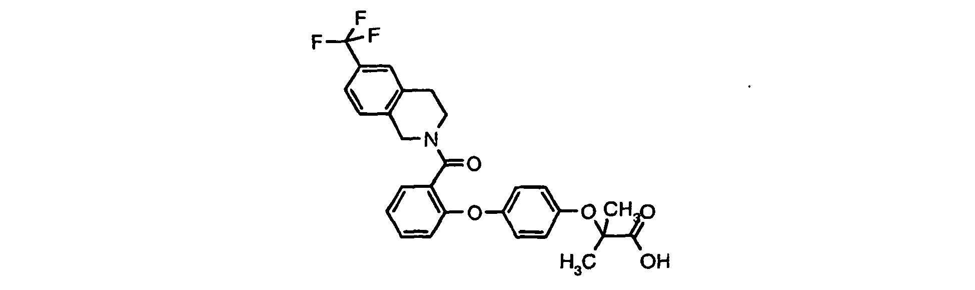

The title compound is prepared by using 6-trifluoromethyl-1,2,3,4-tetrahydroisoquinoline, which is prepared substantially the similar way as described in WO 9850364. MS (ES) *m*/*e* 500 (M+1).

### Example 257

### 2-Methyl-2-(4-[2-(4-trifluoromethylbenzylcarbamoyl)phenoxy]phenoxy)propionic acid

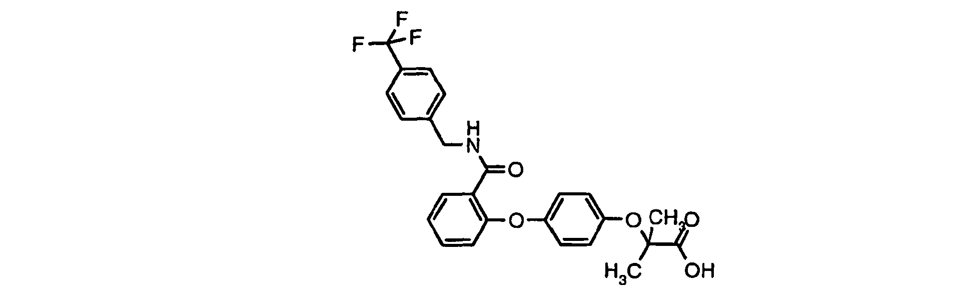

The title compound is prepared by using the commercially available 4-trifluoromethylbenzylamine. MS (ES) *m*/*e* 474 (M+1).

### Example 258

### Phenethyl[2-(4-trifluoromethylphenyl)ethyl]amine

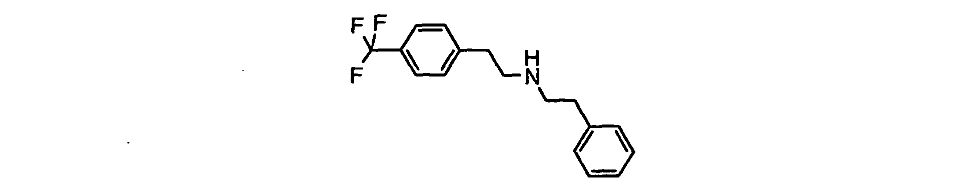

### Step A

### N-Phenethyl-2-(4-trifluoromethylphenyl)acetamide

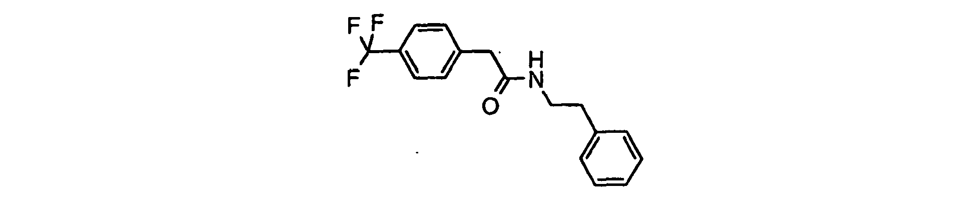

To a solution of 4-trifluoromethylphenylacetic acid (3.0 g, 14.7 mmol) in MeCl₂ (30 mL) is added oxalyl chloride (7.6 mL, 88.2 mmol) with 3 drops of DMF, and the mixture is stirred at RT for 5 h. The reaction is concentrated, and the crude acid chloride is redissolved in MeCl₂ (15 mL) and added dropwise to an ice bath cooled solution of phenethylamine (2.3 mL, 18.4 mmol) and Et₃N (4.1 mL, 30 mmol) in MeCl₂ (15 mL). The reaction is stirred at RT for about 16 h and diluted with 50 mL MeCl₂ and 100 mL water, which is then acidified to pH 1 using 1N HCl. The organic layer is washed with brine, dried with Na₂SO₄, concentrated, and purified (radial chromatography, 4 mm plate, 15:85 to 25:75 EtOAc:hex) to give the title compound as a pale yellow solid (2.57 g, 56% over 2 steps). MS (ES) *m*/*e* 308 (M+1).

### Step B

### Phenethyl[2-(4-trifluoromethylphenyl)ethyl]amine

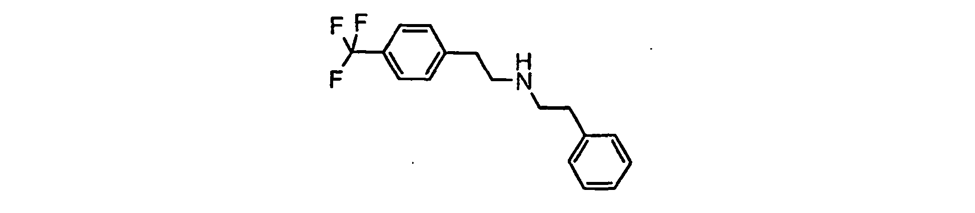

To a mixture of lithium borohydride (0.73 g, 33.5 mmol) and trimethylsilyl chloride (8.1 mL, 64.4 mmol) in THF (15 mL) is added a solution of N-phenethyl-2-(4-trifluoromethylphenyl)acetamide (2.54 g, 6.77 mmol) in THF (15 mL). The reaction is stirred for about 56 h (40 h RT, 16 h at 50 °C). After cooling, the reaction is treated slowly with 20 mL MeOH and concentrated. The residue is taken up in MeCl₂ (150 ml) and washed with water (100 mL), and then treated with 5N NaOH to bring the pH to about 12. The organic layer is washed with water, dried with Na₂SO₄, and concentrated to give the title compound as a yellow oil. MS(ES) *m*/*e* 294 (M+1).

### Example 259

### 3-{2-Methyl-4-[3-(4-trifluoromethylbenzylcarbamoyl)phenoxy]phenyl}propionic acid

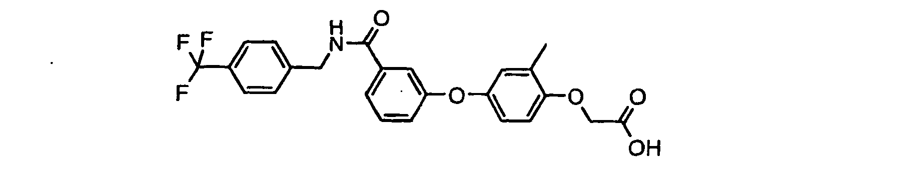

### Step A

### 3-(4-Bromo-3-methylphenoxy)benzonitrile

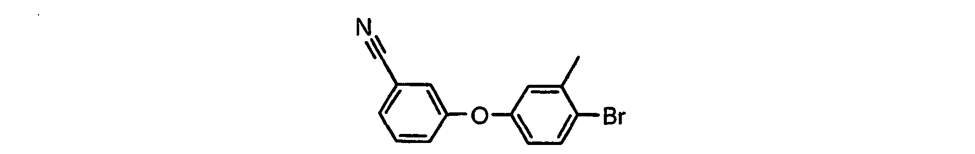

This compound is prepared following the procedure of Example 214, Step A except that commercially available 4-bromo-3-methylphenol is used. MS (ES) *m*/*e* 306 (M+NH4).

### Step B

### 3-(4-Bromo-3-methylphenoxy)benzoic acid

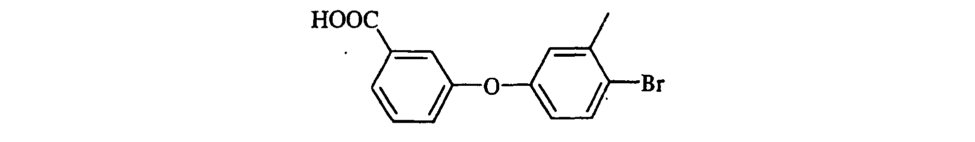

This compound is prepared as described in Example 214, Step B. MS (ES) *m*/*e* 305 (M-1).

### Step C

### 3-(4-Bromo-3-methylphenoxy)benzoic acid benzyl ester

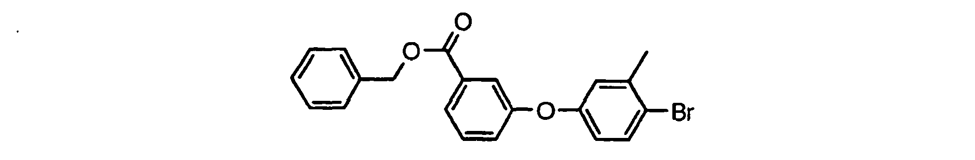

This compound is prepared as described in Example 214, Step D. MS (ES) *m*/*e* 397 (M+1).

### Steps D-E

### 3-[4-(2-Methoxycarbonylethyl)-3-methylphenoxy]benzoic acid

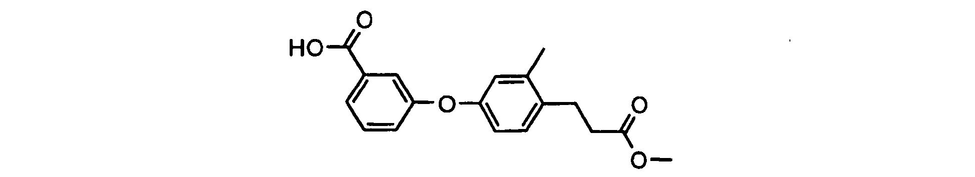

The compound of 3-(4-bromo-3-methylphenoxy)benzoic acid benzyl ester (10.4 g, 26.1 mmol) in proprionitrile (200 mL) is treated with DIPEA (10.5 mL, 60.3 mmol). The mixture is degassed (3x vacuum/N₂ purge), and methyl acrylate (11 mL, 120 mmol) is added. The mixture is degassed (1x), and tri-o-tolylphosphine (3.65 g, 12.0 mmol) and Pd(OAc)₂ are added. The mixture is degassed (2x), heated at 110 °C for 2.5 h, cooled, filtered, and concentrated. The crude product is purified (silica gel chromatography, hex:EtOAc 100:0 to 80:20) to give 3-[4-(2-methoxycarbonylvinyl)-3-methylphenoxy]benzoic acid benzyl ester as a yellow oil (9.76 g).

The oily material is dissolved in MeOH (200 mL) and treated with 5% Pd/C (1.25 g) and H₂ gas (60 psi) at RT overnight. The mixture is filtered and concentrated. The product mixture is purified (silica gel chromatography, hex:EtOAc:HOAc 3:1:0 to 1:1:0.02) to give 3-[4-(2-methoxycarbonylvinyl)-3-methylphenoxy]benzoic acid. This material is dissolved in MeOH (125 mL) and treated with 5% Pd/C (5 g) and H₂ gas (60 psi) at RT 16 h. The mixture is filtered and concentrated. The crude product is purified (silica gel chromatography, hex:EtOAc:HOAc 3:1:0 to 1:1:0.02) to give the title compound (1.8 g, 22%). MS (ES) *m*/*e* 313 (M-1).

### Step F

### 3-[4-(3-Chlorocarbonylphenoxy)-2-methylphenyl]propionic acid methyl ester

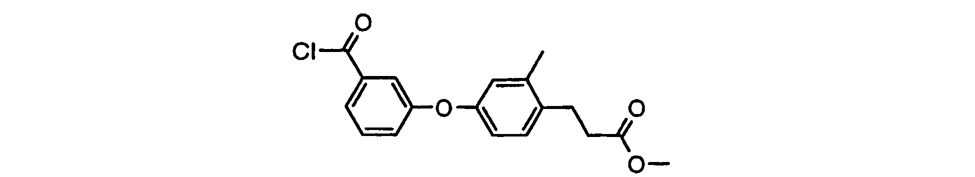

The title compound is prepared according to the procedure described in Example 214, Step G.

### Steps G-H

### 3-{2-Methyl-4-[3-(4-trifluoromethylbenzylcarbamoyl)phenoxy]phenyl}propionic acid

The title compound is prepared according to the procedure described in Example 214, Steps H-I. MS(ES) *m*/*e* 458 (M+1).

Examples 260 and 281 are prepared substantially the similar way as described in Example 259.

### Example 260

### 3-[4-(3-Cyclohexylcarbamoylphenoxy)-2-methylphenyl]propionic acid

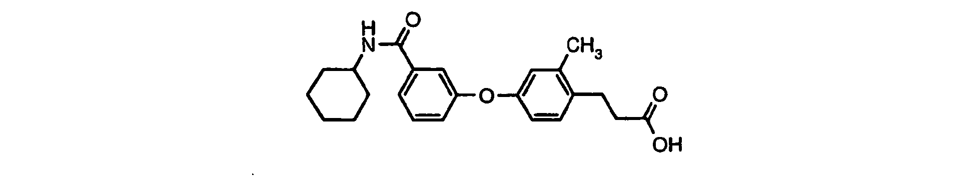

The title compound is prepared by using the commercially available cyclohexylamine. MS (ES) *m*/*e* 382 (M+1).

### Example 261

### 3-[4-(3-Benzylcarbamoylphenoxy)-2-methylphenyl]propionic acid

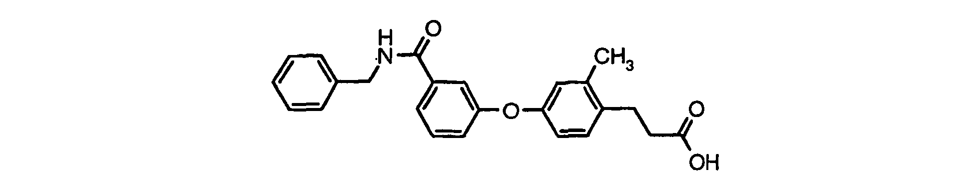

The title compound is prepared by using the commercially available benzylamine. MS (ES) *mle* 390 (M+1).

### Example 262

### 3-[2-Methyl-4-(3-phenethylcarbamoylphenoxy)phenyl]propionic acid

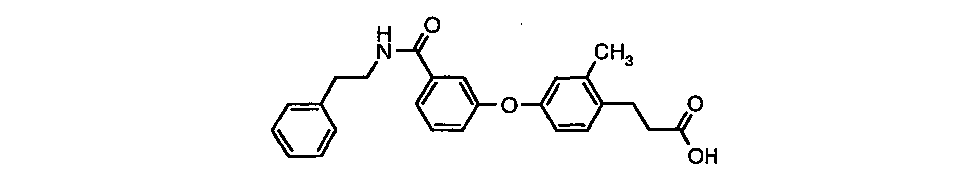

The title compound is prepared by using commercially available phenethylamine. MS (ES) *m*/*e* 404 (M+1).

### Example 263

### 3-[2-Methyl-4-(3-phenethylcarbamoylphenoxy)phenyl]propionic acid

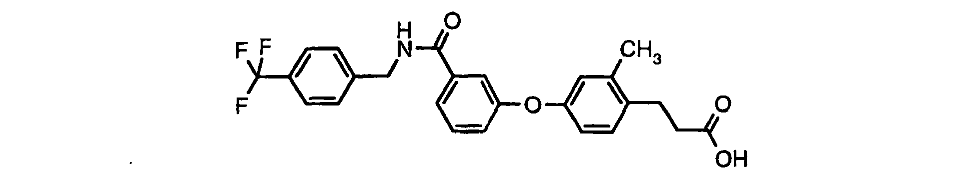

The title compound is prepared by using commercially available 4-trifluoromethylbenzylamine. MS (ES) *mle* 458 (M+1).

### Example 264

### 3-{4-[3-(4-Methoxybenzylcarbamoyl)phenoxy]-2-methylphenyl}propionic acid

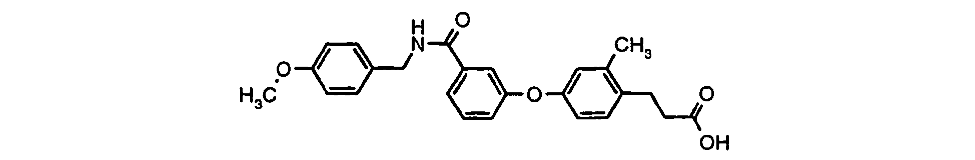

The title compound is prepared by using commercially available 4-methoxybenzylamine. MS (ES) *m*/*e* 420 (M+1).

### Example 265

### 3-(4-{3-[(Biphenyl-3-ylmethyl)carbamoyl]phenoxy)-2-methylphenyl)propionic acid

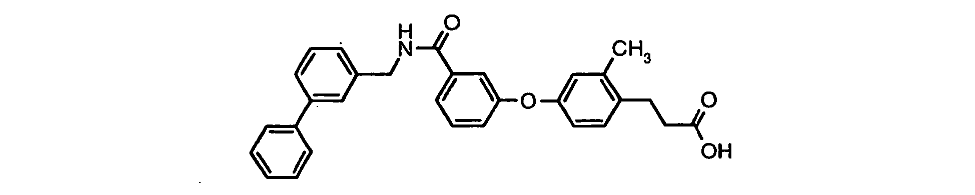

The title compound is prepared by using commercially available biphenyl-3-ylmethylamine. MS (ES) *mle* 466 (M+1).

### Example 266

### 3- {4-[3-(Benzylphenethylcarbamoyl)phenoxy]-2-methylphenyl}propionic acid

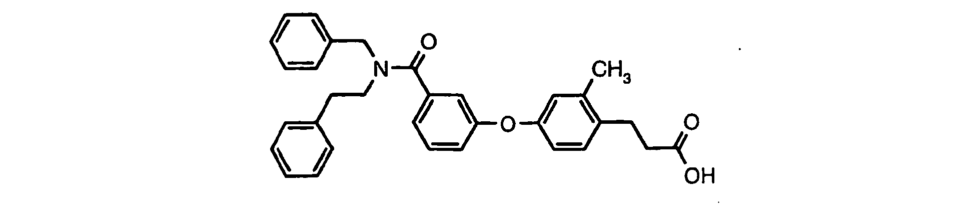

The title compound is prepared by using commercially available benzylphenethylamine. MS (ES) *m*/*e* 494 (M+1).

### Example 267

### 3-[4-(3-Diphenethylcarbamoylphenoxy)-2-methylphenyl]propionic acid

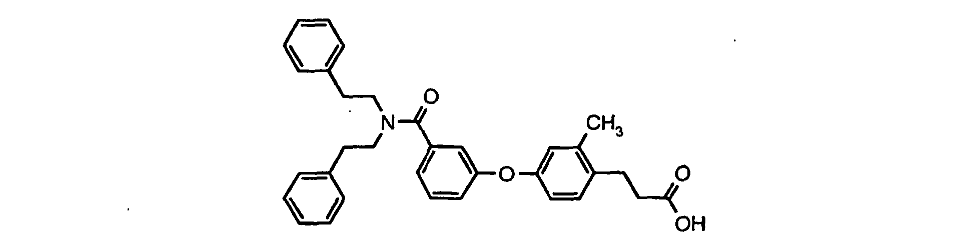

The title compound is prepared by using diphenethylamine, which is prepared substantially the similar way as described in Example 258, Steps A and B. MS (ES) *m*/*e* 508 (M+1).

### Example 268

### 3- [4-(3- (2-(3,4-Dimethoxyphenyl)ethyl]hexylcarbamoyl}phenoxy)-2-methylphenylJ propionic acid

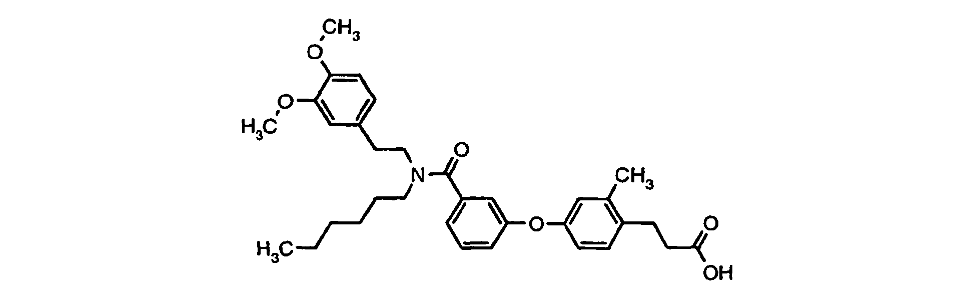

The title compound is prepared by using [2-(3,4-dimethoxyphenyl) ethyl]heptylamine, which is prepared substantially the similar way as described in Example 258, Steps A and B. MS (ES) *mle* 548 (M+1).

### Example 269

### 3-[4-(3-{[2-(3,5-Bis-trifluoromethylphenyl)ethyl]phenethylcarbamoyl}phenoxy)-2-methylphenyl]propionic acid

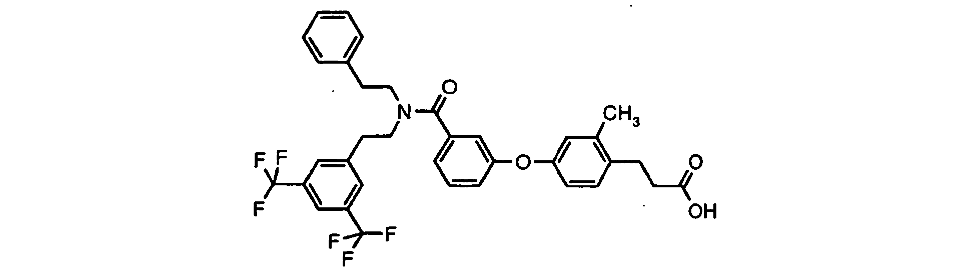

The title compound is prepared by using [2-(3,5-bistrifluoromethylphenyl)ethyl] phenethylamine which is prepared substantially the similar way as described in Example 258, Steps A and B. MS (ES) *m*/*e* 644 (M+1).

### Example 270

### 3-[2-Methyl-4-(3-{phenethyl[2-(4-trifluoromethylphenyl)ethyl]carbamoyl}phenoxy) phenyl] propionic acid

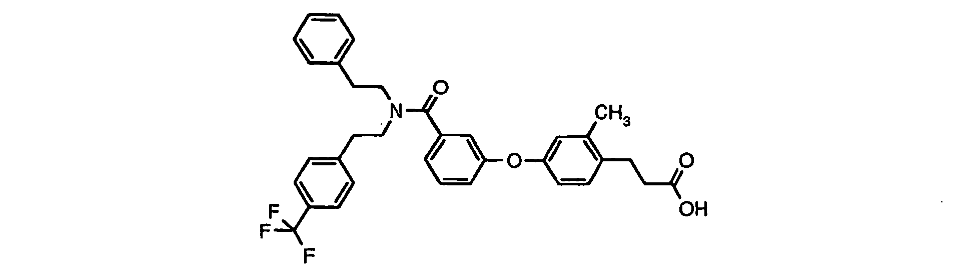

The title compound is prepared by using [2-(4-trifluoromethylphenyl)ethyl] phenethylamine, which is prepared substantially the similar way as described in Example 258, Steps A and B. MS (ES) *m*/*e* 576 (M+1).

### Example 271

### 3-{2-Methyl-4-[3-(4-trifluoromethoxybenzylcarbamoyl)phenoxy]phenyl}propionic acid

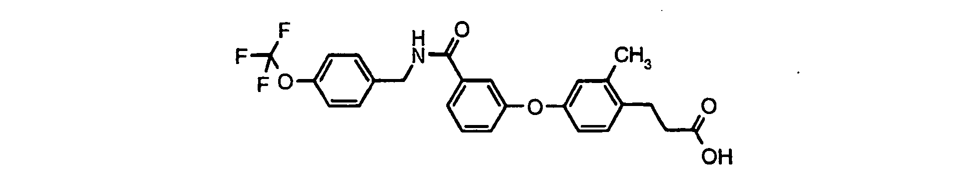

The title compound is prepared by using the commercially available 4-trifluoromethoxybenzylamine. MS (ES) *m*/*e* 474 (M+1).

### Example 272

### 3-(2-Methyl-4-{3-[2-(4-trifluoromethylphenyl)ethylcarbamoyl]phenoxy}phenyl)propionic acid

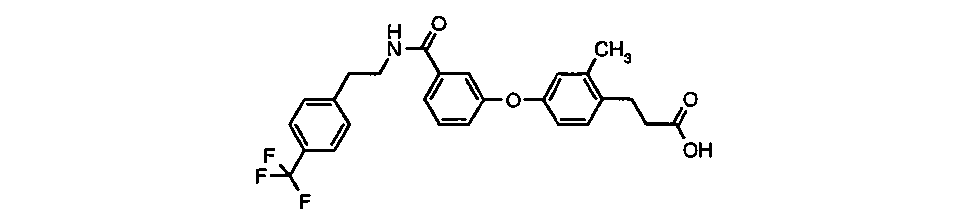

The title compound is prepared by using the commercially available 4-trifluoromethylphenethylamine. MS (ES) *m*/*e* 472 (M+1).

### Example 273

### 3-(2-Methyl-4-{3-[1-(4-trifluoromethylphenyl)ethylcarbamoyl]phenoxy}phenyl) propionic acid

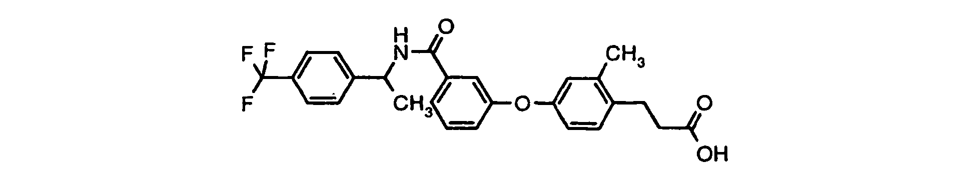

The title compound is prepared by using 1-(4-trifluoromethylphenyl)ethylamine, which is prepared substantially the similar way as described in J. Med. Chem., 10, 873-840 (1967). MS (ES) *mle* 472 (M+1).

### Example 274

### 3-[2-Methyl-4-(3-{methyl-[2-(4-trifluoromethylphenyl)ethyl)carbamoyl }phenoxy) phenyl]propionic acid

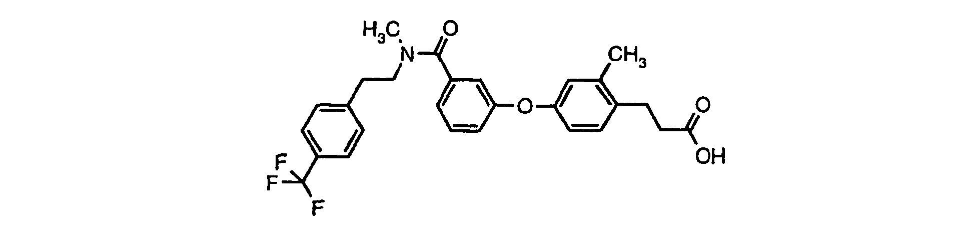

The title compound is prepared by using methyl(4-trifluoromethylphenyl) ethylamine, which is prepared substantially the similar way as described in Example 258, Steps A and B. MS (ES) *m*/*e* 486 (M+1).

### Example 275

### 3-{2-Methyl-4-[3-(6-trifluoromethyl-3,4-dihydro-1H-isoquinoline-2-carbonyl)phenoxy] phenylpropionic acid

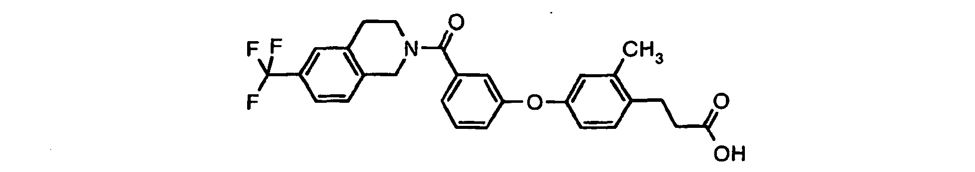

The title compound is prepared by using 6-trifluoromethyl-1,2,3,4-tetrahydroisoquinoline, which is prepared substantially the similar way as described in WO 9850364. MS (ES) *m*/*e* 617 (M+1).

### Example 276

### 3-(2-Methyl-4-{3-[3-(3-trifluoromethylphenyl)piperidine-1-carbonyl]phenoxy}phenyl) propionic acid

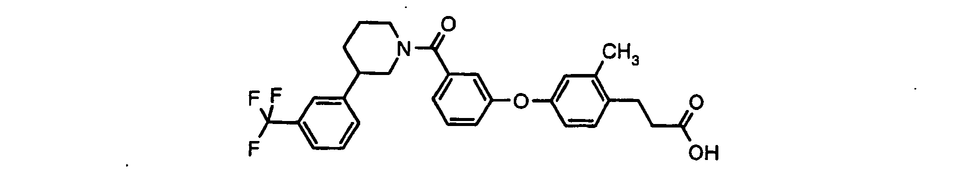

The title compound is prepared by using 3-(3-trifluoromethylphenyl)piperidine, which is prepared substantially the similar way as described in J. Med. Chem., 30, 2169-2174 (1987). MS (ES) *m*/*e* 512 (M+1).

### Example 277

### 3-(2-Methyl-4-{3-[2-(3-trifluoromethylphenyl)ethylcarbamoyl]phenoxy}phenyl) propionic acid

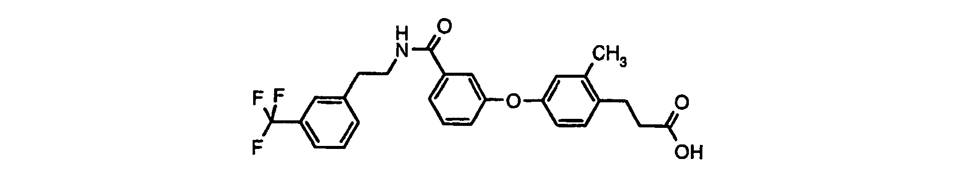

The title compound is prepared by using the commercially available 3-trifluoromethylphenethylamine. MS (ES) *mle* 472 (M+1).

### Example 278

### 3-(4- {3-[Cyclopropyl-(3-trifluoromethylbenzyl)carbamoyl]phenoxy}-2-methylphenyl) propionic acid

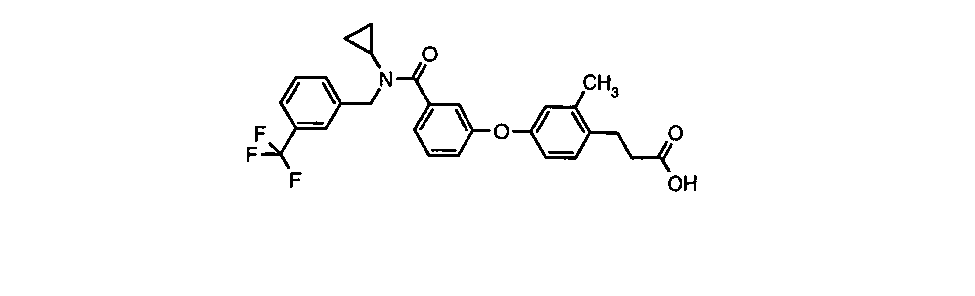

The title compound is prepared by using cyclopropyl(3-trifluoromethylbenzyl)amine which is prepared substantially the similar way as described in Example 258, Steps A and B. MS (ES) *m*/*e* 498 (M+1).

### Example 279

### 3-[4-(3-{Benzyl-[2-oxo-2-(4-trifluoromethylphenyl)ethyl]carbamoyl}phenoxy)-2-methylphenyl]propionic acid

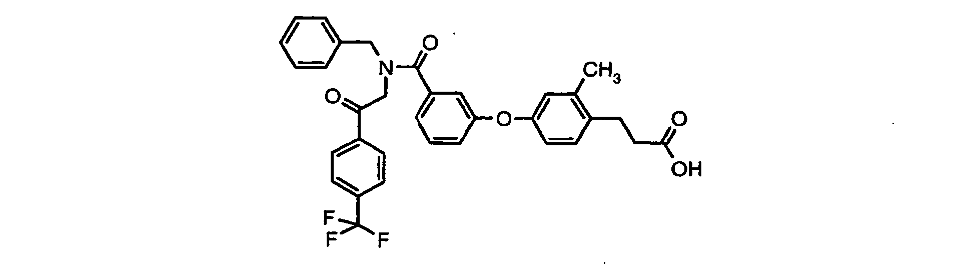

The title compound is prepared by using 2-benzylamino-1-(4-trifluoromethylphenyl)ethanone which is prepared substantially the similar way as described in Example 258, Step A. MS (ES) *m*/*e* 576 (M+1).

### Example 280

### 3-{4-[3-(6-Methoxy-3,4-dihydro-1H-isoquinoline-2-carbonyl)phenoxy]-2-methylphenyl}propionic acid

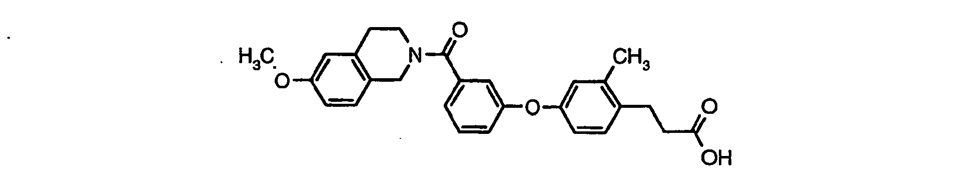

The title compound is prepared by using 6-methoxy-1,2,3,4-tetrahydroisoquinoline, which is prepared substantially the similar way as described in JACS, 56, 1769-1771 (1934). MS (ES) *m*/*e* 446 (M+1).

### Example 281

### 3-(2-Methyl-4-[3-(1-methyl-3,4-dihydro-1H-isoquinoline-2-carbonyl)phenoxy] phenyl}propionic acid

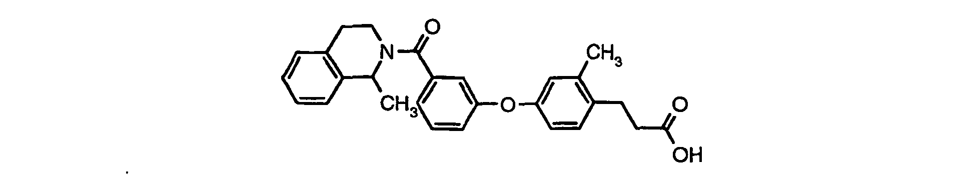

The title compound is prepared by using 1-methyl-1,2,3,4-tetrahydroisoquinoline, which is prepared substantially the similar way as described in J. Chem. Soc. Perkin Trans. 1, 9, 955-977 (2001). MS (ES) *m*/*e* 430 (M+1).

Examples 282 to 292 are prepared according to the procedure described in Example 259 except that 3-fluoro-2-methylbenzonitrile is used in Example 259, Step A. The compound of 3-fluoro-2-methylbenzonitrile is prepared substantially the similar way as described in US 6,063,789

### Example 282

### 3- {2-Methyl-4-[2-methyl-3-(4-trifluoromethoxybenzylcarbamoyl)phenoxy]phenyl} propionic acid

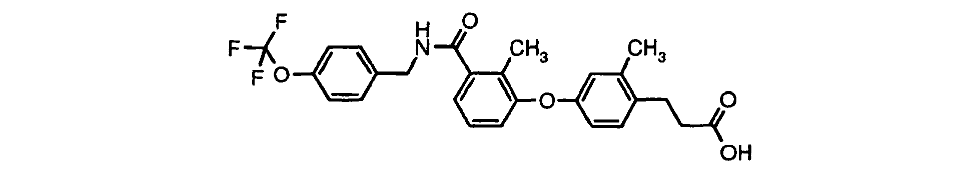

The title compound is prepared by using the commercially available trifluoromethoxybenzylamine. MS (ES) *m*/*e* 488 (M+1).

### Example 283

### 3-(2-Methyl-4-{2-methyl-3-[2-(4-trifluoromethylphenyl)ethylcarbamoyl]phenoxy} phenyl)propionic acid

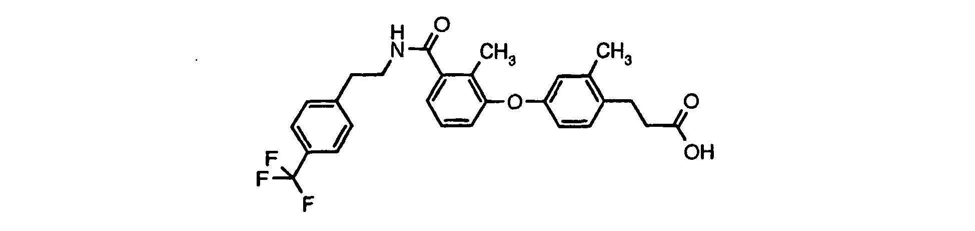

The title compound is prepared by using the commercially available 4-trifluoromethylphenethylamine. MS (ES) *m*/*e* 486 (M+1).

### Example 284

### 3-[4-(3-Benzylcarbamoyl-2-methylphenoxy)-2-methylphenyl]propionic acid

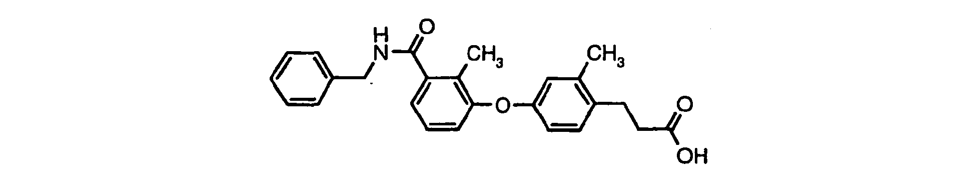

The title compound is prepared by using the commercially available benzylamine. MS (ES) *m*/*e* 404 (M+1).

### Example 285

### 3-{2-Methyl-4-[2-methyl-3-(4-trifluoromethylbenzylcarbamoyl)phenoxy]phenyl} propionic acid

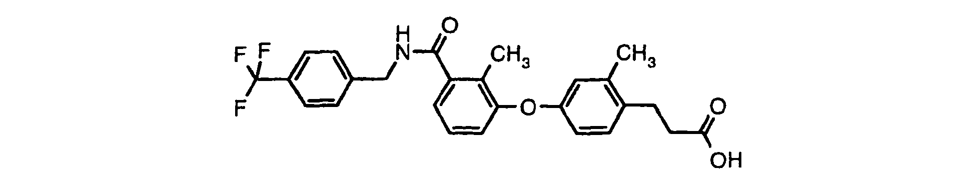

The title compound is prepared by using the commercially available 4-trifluoromethylbenzylamine. MS (ES) *m*/*e* 472 (M+1).

### Example 286

### 3-[2-Methyl-4-(2-methyl-3-phenethylcarbamoylphenoxy)phenyl]propionic acid

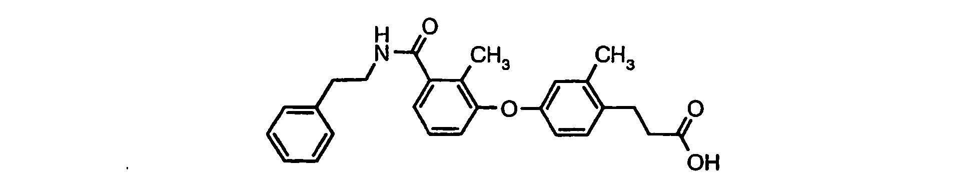

The title compound is prepared by using the commercially available phenethylamine. MS (ES) *m*/*e* 418 (M+1).

### Example 287

### 3-{4-[3-(4-Methoxybenzylcarbamoyl)-2-methylphenoxy]-2-methylphenyl}propionic acid

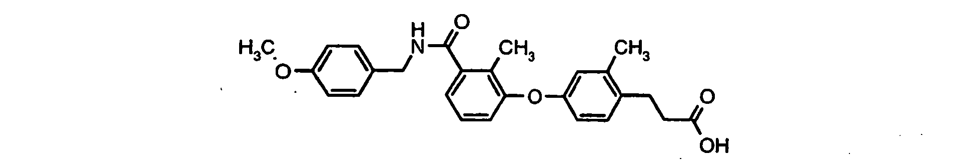

The title compound is prepared by using the commercially available 4-methoxybenzylamine. MS (ES) *mle* 434 (M+1).

### Example 288

### 3-[2-Methyl-4-(2-methyl-3-{methyl-[2-(4-trifluoromethylphenyl)ethyl]carbamoyl} phenoxy)phenyl]propionic acid

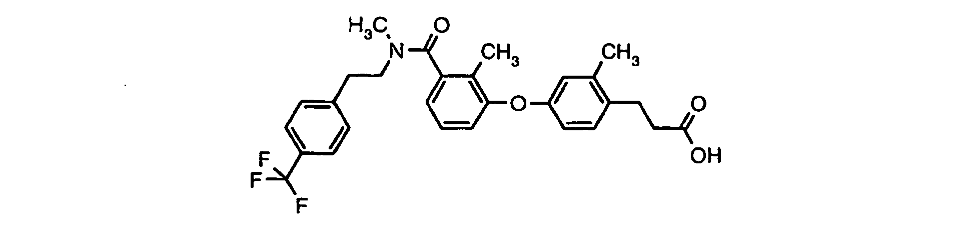

The title compound is prepared by using methyl(4-trifluoromethylphenyl) ethylamine which is prepared substantially the similar way as described in Example 258, Steps A and B. MS (ES) *m*/*e* 500 (M+1).

### Example 289

### 3-{4-[3-(6,7-Dichloro-3,4-dihydro-1H-isoquinoline-2-carbonyl)-2-methylphenoxy]-2-methylphenyl}propionic acid

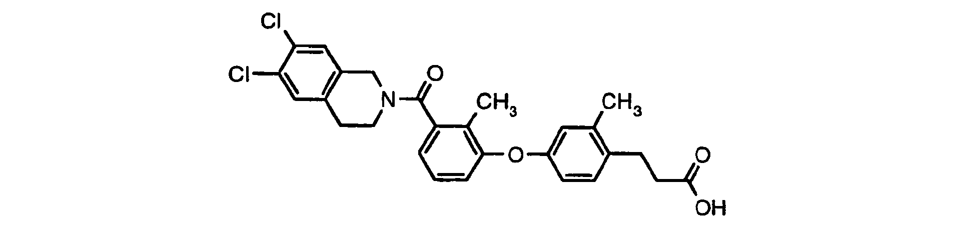

The title compound is prepared by using 6,7-dichloro-1,2,3,4-tetrahydoisoquinoline which is prepared substantially the similar way as described in Tet. Lett., 21, 1393-1396 (1980). MS (ES) *m*/*e* 499 (M+1).

### Example 290

### 3-{2-Methyl-4-[2-methyl-3-(3-methyl-3,4-dihydro-1H-isoquinoline-2-carbonyl)phenoxy] phenyl}propionic acid

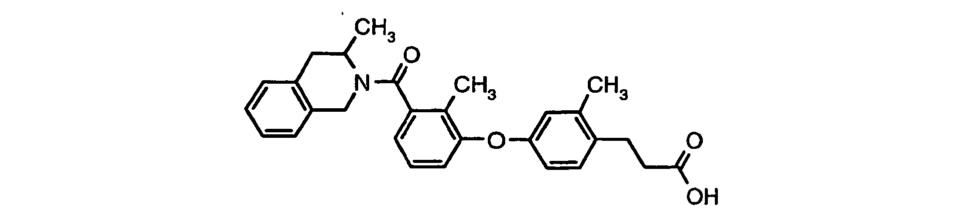

The title compound is prepared by using 3-methyl-1,2,3,4-tetrahydroisoquinoline, which is prepared substantially the similar way as described in J. Chem. Soc. Perkin Trans. 1, 9, 955-977 (2001). MS (ES) *m*/*e* 444 (M+1).

### Example 291

### 3-[4-(3-Diphenethylcarbamoyl-2-methylphenoxy)-2-methylphenyl]propionic acid

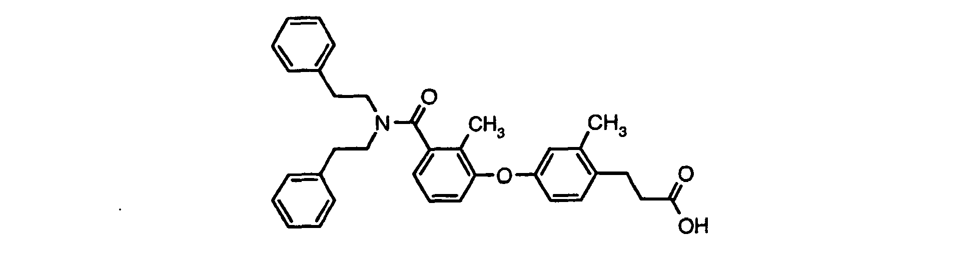

The title compound is prepared by using diphenethylamine, which is prepared substantially the similar way as described in Example 258, Steps A and B. MS (ES) *m*/*e* 522 (M+1).

### Example 292

### 3-{2-Methyl-4-[2-methyl-3-(6-trifluoromethyl-3,4-dihydro-1H-isoquinoline-2-carbonyl) phenoxy]phenyl}propionicacid

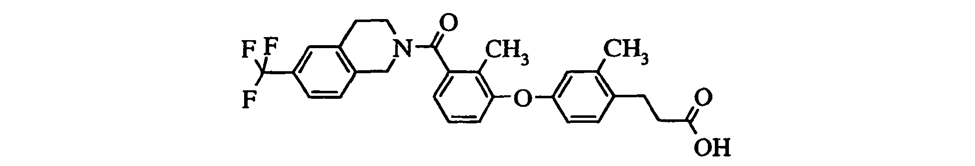

The title compound is prepared by using 6-trifluoromethyl-1,2,3,4-tetrahydroisoquinoline, which is prepared substantially the similar way as described in WO 9850364. MS (ES) *mle* 498 (M+1).

### Example 293

### 3-[4-(3-Chloro-5-{[(5-chloro-1,3-dimethyl-1H-indole-2-carbonyl)-amino]-methyl}-phenoxy)-2-methyl-phenyl]-propionic acid

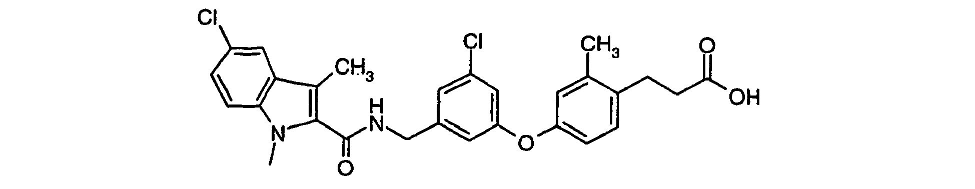

The title compound is prepared according to Example 1 utilizing 5-chloro-1,3-dimethyl-1H-indole-2-carboxylic acid (Intermediate 4) and 3-[4-(3-aminomethyl-5-chloro-phenoxy)-2-methyl-phenyl]-propionic acid methyl ester (Intermediate 61). Exact mass calcd for C₂₈H₂₇Cl₂N₂O₄ (M+H⁺): 525.1348, found 525.1332; ¹H NMR (400 MHz, DMSO-D6).

### Example 294

### 3-(2-Ethyl-4-{3-[(2-methoxy-4-trifluoromethyl-benzoylamino)-methyl]-phenoxy)-phenyl)-propionic acid

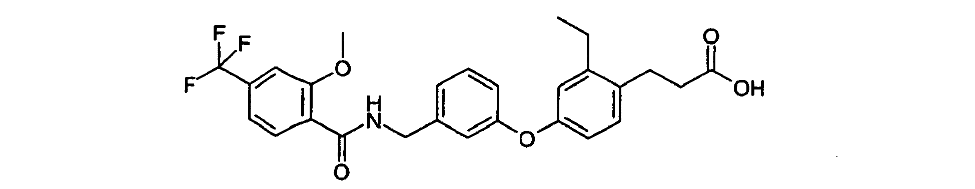

### Step A

### 3-[4-(3-Cyano-phenoxy)-2-ethyl-phenyl]-propionic acid methyl ester

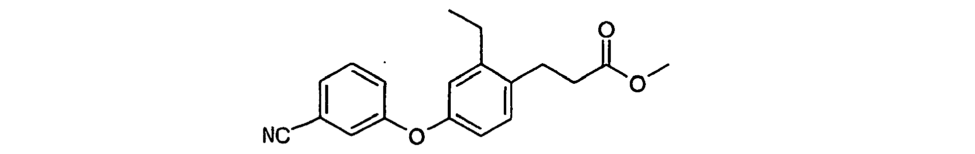

A mixture of 3-bromobenzonitrile (1.3 g, 7.2 mmol), 3-(2-ethyl-4-hydroxy-phenyl)-propionic acid methyl ester (0.5 g, 2.4 mmol), copper (I) chloride (0.12 g, 1.2 mmol), cesium carbonate (1.6 g, 4.8 mmol), and 2,2,6,6-tetramethyl-3,5-heptanedione (0.12 mL, 0.6 mmol) in NMP (10 mL) is purged with nitrogen. The reaction is heated to 120 °C and stirred overnight. The reaction is cooled to room temperature and filtered through celite. The filtrate is quenched with 1N aqueous HCl and extracted with diethyl ether. The organic is washed with brine, dried over sodium sulfate, filtered and the solvent is removed. The crude product is purified by silica gel column chromatography using 4/1 Hexanes/Ethyl acetate to elute the pure product. The solvent is removed to afford about 0.375 g (50%) of desired product. ¹H NMR (400 MHz, CDCl₃); MS (ES⁺) *mlz* mass calcd for C₁₉H₁₉NO₃ 309, found 310 (M + 1).

### Step B

### 3-[4-(3-Aminomethyl-phenoxy)-2-ethyl-phenyl]-propionic acid methyl ester

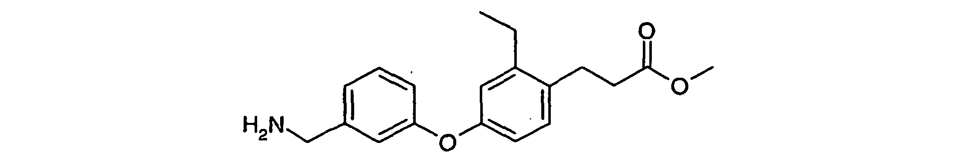

A solution of 3-[4-(3-cyano-phenoxy)-2-ethyl-phenyl]-propionic acid methyl ester (0.38 g, 1.2 mmol) and 5% palladium on carbon (40 mg) in acetic acid (50 mL) is purged with hydrogen (60 psi). The reaction stirred at room temperature overnight. The reaction is filtered through celite and the filtrate is concentrated to one-quarter volume. The solution is quenched with saturate aqueous sodium bicarbonate solution. The aqueous is extracted with diethyl ether. The organic is washed with brine, dried over sodium sulfate, filtered and the solvent is removed to afford about 0.28 g (74%) of desired product. ¹H NMR (400 MHz, CDCl₃); MS (ES⁺) *m*/*z* mass calcd for C₁₉H₂₃NO₃ 313, found 314 (M + 1, 100%).

### Step C

### 3-(2-Ethyl-4-{3-[(2-fluoro-4-trifluoromethyl-benzoylamino)-methyl]-phenoxy}-phenyl)-propionic acid methyl ester

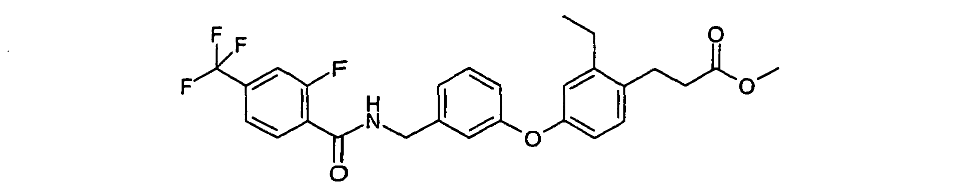

A solution of 3-[4-(3-aminomethyl-phenoxy)-2-ethyl-phenyl]-propionic acid methyl ester (0.28 g, 0.9 mmol), 2-fluoro-4-(trifluoromethyl)benzoic acid (0.22 g, 1.07 mmol), 1-hydroxybenzotriazole hydrate (0.15 g, 1.07 mmol), 1-(3-dimethylaminopropyl)-3-ethylcarbodiimide hydrochloride (0.21 g, 1.07 mmol) and N,N-diisopropylethylamine (0.16 mL, 0.9 mmol) is is combined in THF (10 mL). The reaction stirred overnight at room temperature. The reaction is quenched with 1N aqueous HCl and extracted with diethyl ether. The organic is washed with brine, dried over sodium sulfate, filtered and the solvent is removed. The crude is purified by silica gel column chromatography using 4/1 (Hexanes/Ethyl acetate) to elute the pure product. The solvent is removed to afford about 0.24 g (53%) of desired product. ¹H NMR (400 MHz, CDCl₃); MS (ES⁺) *m*/*z* mass calcd for C₂₇H₂₅F₄NO₄ 503, found 504 (M + 1, 100%).

### Step D

### 3-(2-Ethyl-4-{3-[(2-methoxy-4-trifluoromethyl-benzoylamino)-methyl]-phenoxy}-phenyl)-propionic acid

A solution of 3-(2-ethyl-4-{3-[(2-fluoro-4-trifluoromethyl-benzoylamino)-methyl]-phenoxy}-phenyl)-propionic acid methyl ester (0.24 g, 0.5 mmol) in methanol (10 mL) is treated with 5N aqueous sodium hydroxide (0.9 mL). The reaction is heated to reflux and stirred for 1 hour. The reaction is quenched with 1N aqueous HCl and extracted with diethyl ether. The organic is washed with brine, dried over sodium sulfate, and filtered. The solvent is removed to afford about 0.2 g (83%) of desired product. ¹H NMR (400 MHz, CDCl₃); MS (ES⁺) m/z mass calcd for C₂₇H₂₆F₃NO₅ 501, found 502 (M + 1, 100%).

### Example 295

### 3-(2-Ethyl-4-{3-[(2-fluoro-4-trifluoromethyl-benzoylamino)-methyl]-phenoxy-phenyl)-propionic acid

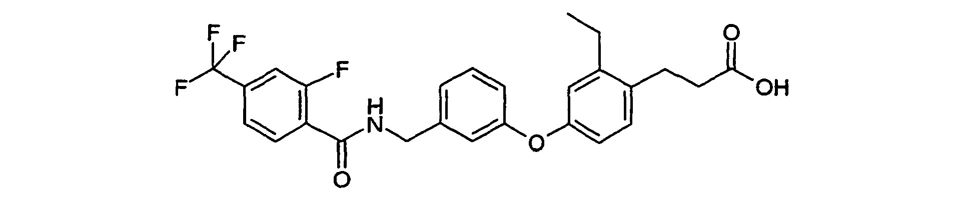

A solution of 3-(2-ethyl-4-{3-[(2-fluoro-4-trifluoromethyl-benzoylamino)-methyl]-phenoxy}-phenyl)-propionic acid methyl ester (0.81 g, 1.6 mmol) in dioxane (10 mL) is treated with 1N aqueous lithium hydroxide (16 mL). The reaction is stirred overnight at room temperature. The reaction is quenched with 1N aqueous HCl and extracted with diethyl ether. The organic is washed with brine, dried over sodium sulfate, and filtered. The solvent is removed to afford about 0.65 g (82%) of desired product. ¹H NMR (400 MHz, CDCl₃); MS (ES⁺) *m*/*z* mass calcd for C₂₆H₂₃F₄O₄ 489, found 490 (M + 1,100%).

### Example 296

### (R)-3-(2-Ethyl-4-{3-[1-(2-fluoro-4-trifluoromethyl-benzoylamino)-ethyl]-phenoxy}-phenyl)-propionic acid

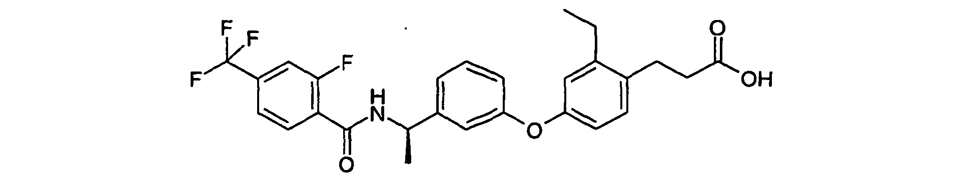

### Step A

### (R)-3-(2-Ethyl-4-{3-[1-(2-fluoro-4-trifluoromethyl-benzoylamino)-ethyl]-phenoxy}-phenyl)-propionic acid ethyl ester

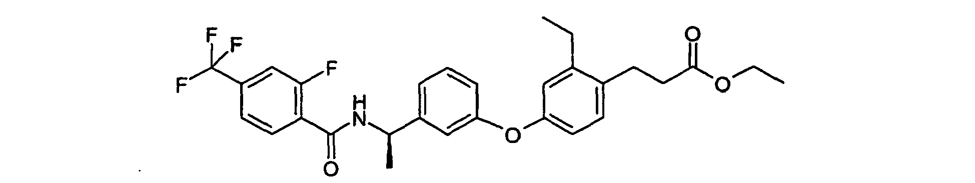

The procedure from Example 294, Step C is utilized with (R)-3-{4-[3-(1-amino-ethyl)-phenoxy]-2-ethyl-phenyl}-propionic acid ethyl ester to afford about 0.43 g (80%) of desired product. ¹H NMR (400 MHz, CDCl₃); MS (ES⁺) m/z mass calcd for C₂₉H₂₉F₄NO₄ 531, found 532 (M + 1, 100%).

### Step B

### (R)-3-(2-Ethyl-4-{3-[1-(2-fluoro-4-trifluoromethyl-benzoylamino)-ethyl]-phenoxy}-phenyl)-propionic acid

The procedure from Example 295 is utilized with (R)-3-(2-ethyl-4-{3-[1-(2-fluoro-4-trifluoromethyl-benzoylamino)-ethyl]-phenoxy}-phenyl)-propionic acid ethyl ester to afford about 0.23 g (57%) of desired product. ¹H NMR (400 MHz, CDCl₃); MS (ES⁺) m/z mass calcd for C₂₇H₂₅F₄NO₄ 503, found 504 (M + 1,100%).

### Example 297

### (R)-3-(4-{3-Fluoro-S-[1-(2-methyl-4-trifluoromethyl-benzoylamino)-ethyl]-phenoxy}-2-methyl-phenyl)-propionic acid

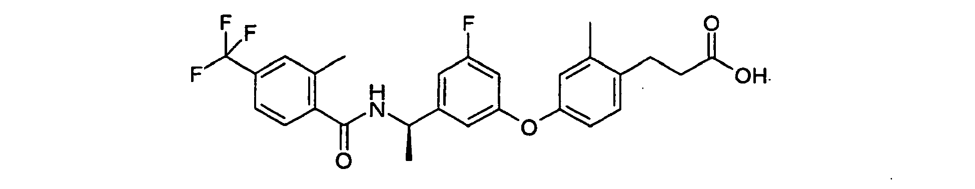

### Step A

### (R)-3-(4-{3-Fluoro-5-[1-(2-methyl-4-trifluoromethyl-benzoylamino)-ethyl]-phenoxy}-2-methyl-phenyl)-propionic acid methyl ester

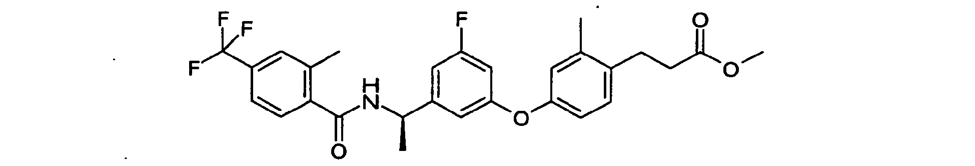

The procedure from Example 294, Step C is utilized with 3-{4-[3-(1-amino-ethyl)-5-fluoro-phenoxy]-2-methyl-phenyl}-propionic acid methyl ester and 2-methyl-4-trifluoromethyl-benzoic acid to afford about 0.12 g (50%) of desired product. ¹H NMR (400 MHz, CDCl₃); MS (ES⁺) *m*/*z* mass calcd for C₂₈H₂₇F₄NO₄ 517, found 518 (M + 1, 100%).

### Step B

### (R)-3-(4-{3-Fluoro-5-[1-(2-methyl-4-trifluoromethyl-benzoylamino)-ethyl]-phenoxy}-2-methyl-phenyl)-propionic acid

The procedure from Example 295 is utilized with (R)-3-(4-{3-fluoro-5-[1-(2-methyl-4-trifluoromethyl-benzoylamino)-ethyl]-phenoxy -2-methyl-phenyl)-propionic acid methyl ester to afford about 0.01 g (87%) of desired product. ¹H NMR (400 MHz, CDCl₃); MS (ES⁺) m/z mass calcd for C₂₇H₂₅F₄NO₄ 503, found 504 (M + 1, 100%).

### Example 298

### (R)-3-(4-{3-Fluoro-5-[1-(2-chloro-4-trifluoromethyl-benzoylamino)-ethyl]-phenoxy}-2-methyl-phenyl)-propionic acid

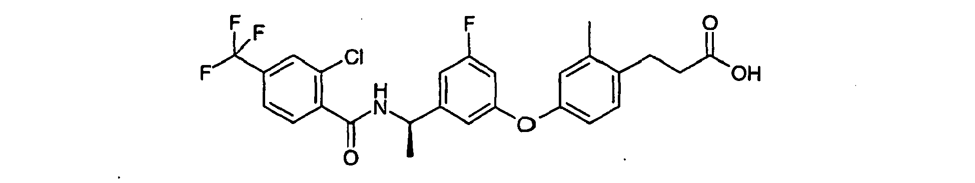

### Step A

### (R)-3-(4-{ 3-Fluoro-5-[1-(2-chloro-4-trifluoromethyl-benzoylamino)-ethyl]-phenoxy}-2-methyl-phenyl)-propionic acid methyl ester

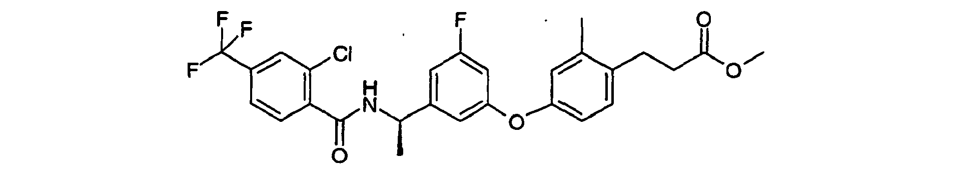

The procedure from Example 294, Step C is utilized with 3-{4-[3-(1-amino-ethyl)-5-fluoro-phenoxy]-2-methyl-phenyl}-propionic acid methyl ester and 2-chloro-4-trifluoromethyl-benzoic acid to afford about 0.13 g (54%) of desired product. ¹H NMR (400 MHz, CDCl₃); MS (ES⁺) *m*/*z* mass calcd for C₂₇H₂₄ClF₄NO₄ 537, found 538 (M+1, 100%).

### Step B

### (R)-3-(4-{3-Fluoro-5-[1-(2-chloro-4-trifluoromethyl-benzoylamino)-ethyl]-phenoxy}-2-methyl-phenyl)-propionic acid

The procedure from Example 295 is utilized with (R)-3-(4-{3-fluoro-5-[1-(2-chloro-4-trifluoromethyl-benzoylamino)-ethyl]-phenoxy}-2-methyl-phenyl)-propionic acid methyl ester to afford about 0.01 g (84%) of desired product. ¹H NMR (400 MHz, CDCl₃); MS (ES⁺) *m*/*z* mass calcd for C₂₆H₂₂CIF₄NO₄ 523, found 524 (M+1, 100%).

### Example 299

### (R)-3-(4-{3-Fluoro-5-[1-(2-methoxy-4-trifluoromethyl-benzoylamino)-ethyl]-phenoxy]-2-methyl-phenyl)-propionic acid

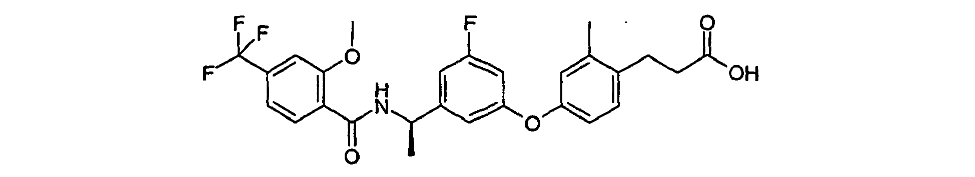

### Step A

### (R)-3-(4-{3-Fluoro-5-[1-(2-methoxy-4-trifluoromethyl-benzoylamino)-ethyl]-phenoxy}-2-methyl-phenyl)-propionic acid methyl ester

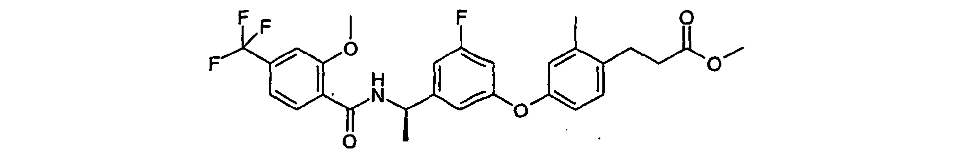

The procedure from Example 294, Step C is utilized with 3-{4-[3-(1-amino-ethyl)-5-fluoro-phenoxy]-2-methyl-phenyl}-propionic acid methyl ester and 2-methoxy-4-trifluoromethyl-benzoic acid to afford about 0.12 g (51%) of desired product. ¹H NMR (400 MHz, CDCl₃); MS (ES⁺) *m*/*z* mass calcd for C₂₈H₂₇F₄NO₅ 533, found 534 (M+1, 100%).

### Step B

### (R)-3-(4-{3-Fluoro-5-[1-(2-methoxy-4-trifluoromethyl-benzoylamino)-ethyl]-phenoxy}-2-methyl-phenyl)-propionic acid

The procedure from Example 295 is utilized with (R)-3-(4-{3-fluoro-5-[1-(2-methoxy-4-trifluoromethyl-benzoylamino)-ethyl]-phenoxy }-2-methyl-phenyl)-propionic acid methyl ester to afford about 0.11 g (94%) of desired product. ¹H NMR (400 MHz, CDCl₃); MS (ES⁺) *m*/*z* mass calcd for C₂₇H₂₅F₄NO₅ 519, found 520 (M + 1, 100%).

### Example 300

### 3-(4-{3-[(2-Chloro-4-trifluoromethyl-benzoylamino)-methyl]-5-methyl-phenoxy}-2-methyl-phenyl)-propionic acid

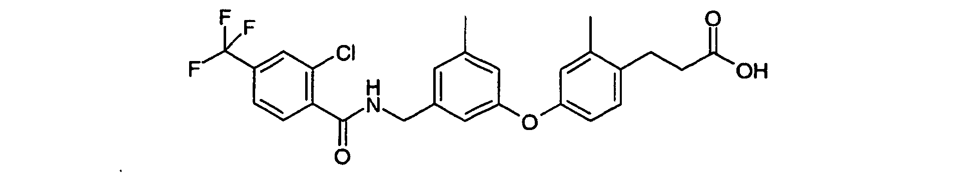

### Step A

### 3-(4-{3-[(2-Chloro-4-trifluoromethyl-benzoylamino)-methyl]-5-methyl-phenoxy}-2-methyl-phenyl)-propionic acid methyl ester

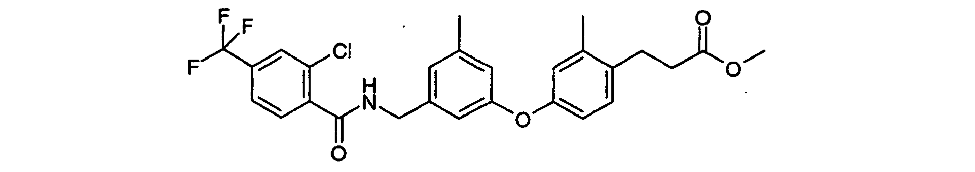

The procedure from Example 294, Step C is utilized with 3-[4-(3-aminomethyl-5-methyl-phenoxy)-2-methyl-phenyll-propionic acid methyl ester and 2-chloro-4-trifluoromethyl-benzoic acid to afford about 0.23 g (42%) of desired product. ¹H NMR (400 MHz, CDCl₃); MS (ES⁺) *m*/*z* mass calcd for C₂₇H₂₅CIF₃NO₄ 519, found 520 (M + 1, 100%).

### Step B

### 3-(4-{3-[(2-Chloro-4-trifluoromethyl-benzoylamino)-methyl]-5-methyl-phenoxy}-2-methyl-phenyl)-propionic acid

The procedure from Example 295 is utilized with 3-(4-{3-[(2-chloro-4-trifluoromethyl-benzoylamino)-methyl]-5-methyl-phenoxy}-2-methyl-phenyl)-propionic acid methyl ester to afford about 0.2 g (93%) of desired product. ¹H NMR (400 MHz, CDCl₃); MS (ES⁺) *m*/*z* mass calcd for C₂₆H₂₃ClF₃NO₄ 505, found 506 (M + 1, 100%).

### Example 301

### 3-(2-Ethyl-4-{3-fluoro-5-[1-(2-methoxy-4-trifluoromethyl-benzoylamino)-ethyl]-phenoxy}-phenyl)-propionic acid

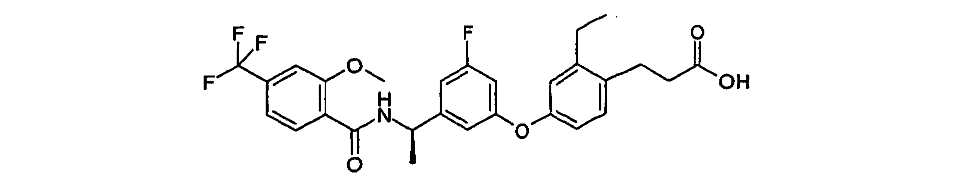

The title compound is prepared according to Example 2 by using 2-methoxy-4-trifluoromethyl-benzoic acid and 3-{4-[3-(1-amino-ethyl)-5-fluoro-phenoxy]-2-ethyl-phenyl}-propionic acid ethyl ester to afford about 143 mg (81 %). ¹H NMR (400 MHz, CDCl₃); MS (ES⁺) *m*/*z* mass calcd for C₂₈H₂₇O₅NF₄ 533, found 534 (M + 1, 100%).

### Example 302

### 3-(4-{3-[1-(2,4-Bis-trifluoromethyl-benzoylamino)-ethyl]-5-fluoro-phenoxy}-2-ethylphenyl)-propionic acid

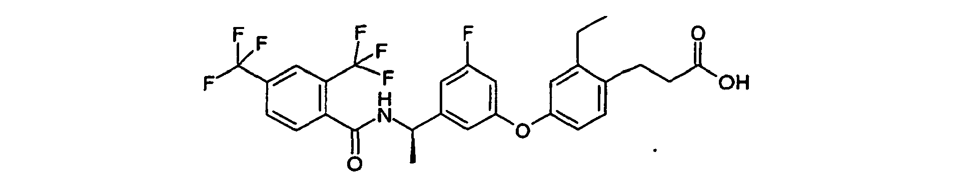

The title compound is prepared according to Example 2 by using 2,4-bis-trifluoromethyl-benzoic acid and 3-{4-[3-(1-amino-ethyl)-5-fluoro-phenoxy]-2-ethylphenyl}-propionic acid ethyl ester to afford about 38 mg (90%). ¹H NMR (400 MHz, CDCl₃); MS (ES⁺) m/z mass calcd for C₂₈H₂₄O₄NF₇ 571, found 572 (M + 1, 100%).

### Example 303

### 3-(2-Ethyl-4-{3-methyl-5-[1-(2-methyl-4-trifluoromethyl-benzoylamino)-ethyl]-phenoxy}-phenyl)-propionic acid

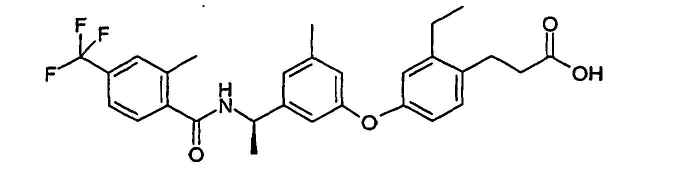

The title compound is prepared according to Example 131 by using 2-methyl-4-trifluoromethyl-benzoic acid and 3-{4-[3-(1-amino-ethyl)-5-methyl-phenoxy]-2-ethyl-phenyl)-propionic acid ethyl ester to afford about 83 mg (70%). ¹H NMR (400 MHz, CDCl₃); MS (ES⁺) *m*/*z* mass calcd for C₂₉H₃₀O₄NF₃ 513, found 514 (M + 1, 100%).

### Example 304

### 3-(2-Ethyl-4-{3-[1-(2-methoxy-4-trifluoromethyl-benzoylamino)-ethyl]-5-methyl-phenoxy}-phenyl)-propionic acid

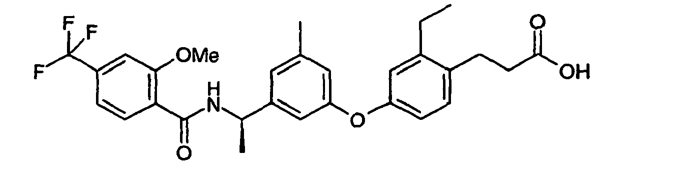

The title compound is prepared according to Example 131 by using 2-methoxy-4-trifluoromethyl-benzoic acid and 3-{4-[3-(1-amino-ethyl)-5-methyl-phenoxy]-2-ethylphenyl}-propionic acid ethyl ester to afford about 232 mg (82%). ¹H NMR (400 MHz, CDCl₃); MS (ES⁺) m/z mass calcd for C₂₉H₃₀O₅NF₃ 529, found 530 (M + 1, 100%).

### Example 305

### 3-(4-(3-[(2-Chloro-4-trifluoromethyl-benzoylamino)-methyl]-5-methyl-phenoxy}-2-ethyl-phenyl)-propionic acid

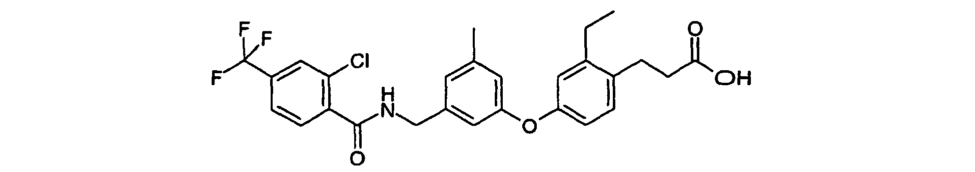

The title compound is prepared according to Example 132 by using 2-chloro-4-trifluoromethyl-benzoic acid and 3-[4-(3-aminomethyl-5-methyl-phenoxy)-2-ethyl-phenyl]-propionic acid ethyl ester to afford about 217 mg (60%). ¹H NMR (400 MHz, CDCl₃); MS (ES⁺) *m*/*z* mass calcd for C₂₇H₂₅O₄NF₃Cl 519, found 520 and 522 (M + 1 and M + 3, 100%).

### Example 306

### 3-(2-Ethyl-4-{3-[(2-methoxy-4-trifluoromethyl-benzoylamino)-methyl]-5-methyl-phenoxy}-phenyl)-propionic acid

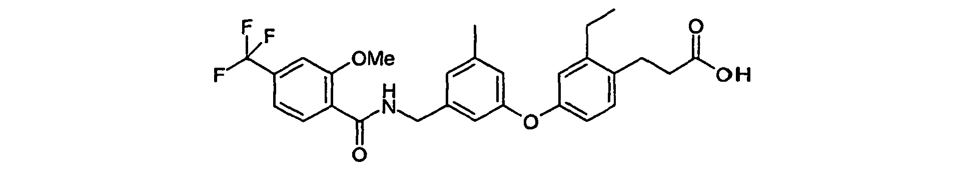

The title compound is prepared according to Example 1 32 by using 2-methoxy-4-trifluoromethyl-benzoic acid and 3-[4-(3-aminomethyl-5-methyl-phenoxy)-2-ethyl-phenyl]-propionic acid ethyl ester to afford about 115 mg (79%). ¹H NMR (400 MHz, CDCl₃); MS (ES⁺) m/z mass calcd for C₂₈H₂₈O₅NF₃ 515, found 516 (M + 1, 100%).

### Example 307

### 3-(4-{3-[1-(2-Chloro-4-trifluoromethyl-benzoylamino)-ethyl]-5-methyl-phenoxy}-2-ethyl-phenyl)-propionic acid

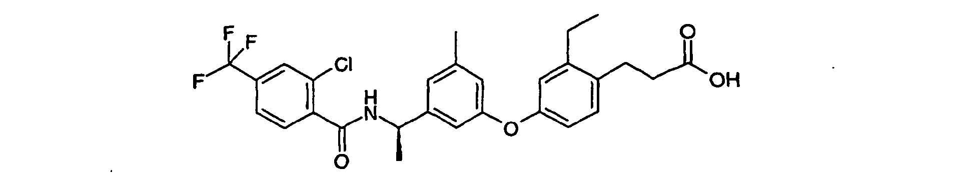

The title compound is prepared according to Example 131 by using 2-chloro-4-trifluoromethyl-benzoic acid and 3-{4-[3-(1-amino-ethyl)-5-methyl-phenoxy]-2-ethyl-phenyl}-propionic acid ethyl ester to afford about 67 mg (33%). ¹H NMR (400 MHz, CDCl₃); MS (ES⁺) *m*/*z* mass calcd for C₂₈H₂₇O₄NF₃Cl 533, found 534 and 536 (M + 1 and M + 3, 100%).

### Example 308

### 3-(4-{3-[1-(2-Chloro-4-trifluoromethyl-benzoylamino)-ethyl]-5-methyl-phenoxy}-2-methyl-phenyl)-propionic acid

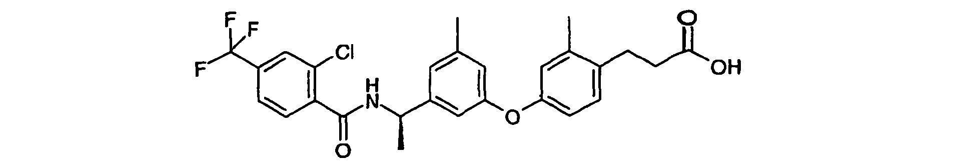

The title compound is prepared according to Example 124 by using 2-chloro-4-trifluoromethyl-benzoic acid and 3-{4-[3-(1-amino-ethyl)-5-methyl-phenoxy]-2-methyl-phenyl}-propionic acid methyl ester to afford about 132 mg (80%). ¹H NMR (400 MHz, CDCl₃); MS (ES⁺) m/z mass calcd for C₂₇H₂₅O₄NF₃Cl 519, found 520 and 522 (M + 1 and M + 3, 100%).

### Example 309

### 3-(4-{3-[1-(2-Chloro-4-trifluoromethyl-benzoylamino)-ethyl]-phenoxy]-2-methylphenyl)-propionic acid

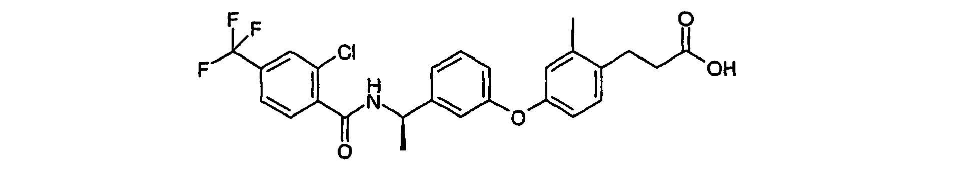

The title compound is prepared according to Example 100 by using 2-chloro-4-trifluoromethyl-benzoic acid and 3-{4-[3-(1-amino-ethyl)-phenoxy]-2-methylphenyl}-propionic acid methyl ester to afford about 638 mg (69%). ¹H NMR (400 MHz, CDCl₃); MS (ES⁺) *m*/*z* mass calcd for C₂₆H₂₃O₄NF₃Cl 505, found 506 and 508 (M + 1 and M + 3, 100%).

### Example 310

### 3-(4-{3-[1-(2-Chloro-4-trifluoromethyl-benzoylamino)-ethyl]-phenoxy}-2-methylphenyl)-propionic acid

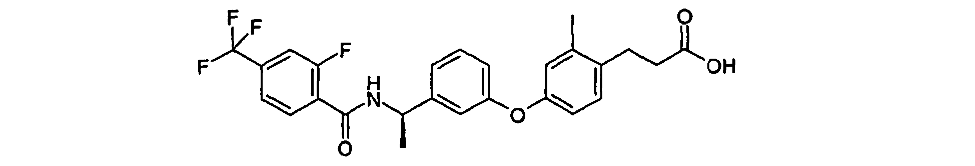

The title compound is prepared according to Example 100 by using 2-fluoro-4-trifluoromethyl-benzoic acid and 3-(4-{3-[1-(2-fluoro-4-trifluoromethyl-benzoylanuno)-ethyl]-phenoxy}-2-methyl-phenyl)-propionic acid to afford about 662 mg (74%). ¹H NMR (400 MHz, CDCl₃); MS (ES⁺) *m*/*z* mass calcd for C₂₆H₂₃O₄NF₄ 489, found 490 (M + 1, 100%).

### Example 311

### 3-(2-Methyl-4-{3-[1-(2-methyl-4-trifluoromethyl-benzoylamino)-ethyl]-phenoxy}-phenyl)-propionic acid

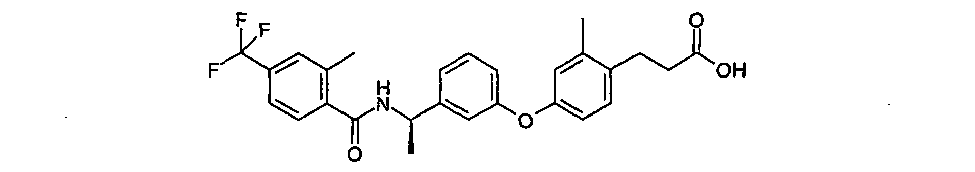

The title compound is prepared according to Example 100 by using 2-methyl-4-trifluoromethyl-benzoic acid and 3-(4-{3-[1-(2-fluoro-4-trifluoromethyl-benzoylamino)-ethyl]-phenoxy}-2-methyl-phenyl)-propionic acid to afford about 486 mg (55%). ¹H NMR (400 MHz, CDCl₃); MS (ES⁺) *m*/*z* mass calcd for C₂₇H₂₆O₄NF₃ 485, found 486 (M + 1, 100%).

### Example 312

### 3-(4-{3-[1-(2-Chloro-4-trifluoromethyl-benzoylamino)-ethyl]-5-fluoro-phenoxy}-2-ethylphenyl)-propionic acid

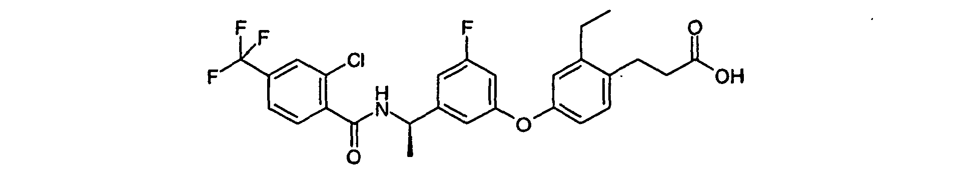

The title compound is prepared according to Example 2 by using 2-chloro-4-trifluoromethyl-benzoic acid and 3-{4-[3-(1-amino-ethyl)-5-fluoro-phenoxy]-2-ethylphenyl }-propionic acid ethyl ester to afford about 166 mg (28%). ¹H NMR (400 MHz, CDCl₃); MS (ES⁺) *m*/*z* mass calcd for C₂₇H₂₄O₄NF₄Cl 537, found 538 and 540 (M + 1 and M + 3, 100%).

### Example 313

### 3-(4-{3-[1-(2-Chloro-4-trifluoromethyl-benzoylamino)-ethyl]-phenoxy}-2-ethyl-phenyl)-propionic acid

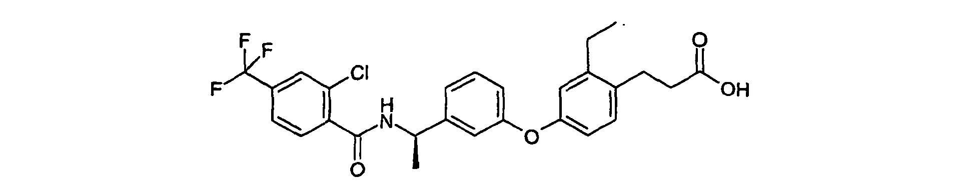

The title compound is prepared according to Example 296 by using 2-chloro-4-trifluoromethyl-benzoic acid and 3-{4-[3-(1-amino-ethyl)-phenoxy]-2-ethylphenyl}-propionic acid ethyl ester to afford about 456 mg (86%). ¹H NMR (400 MHz, CDCl₃); MS (ES⁺) *m*/*z* mass calcd for C₂₇H₂₅O₄NF₃Cl 519, found 520 and 522 (M + 1 and M + 3, 100%).

### Example 314

### 3-(2-Ethyl-4-{ 3-[1-(2-methyl-4-trifluoromethyl-benzoylantino)-ethyl]-phenoxy }-phenyl)-propionic acid

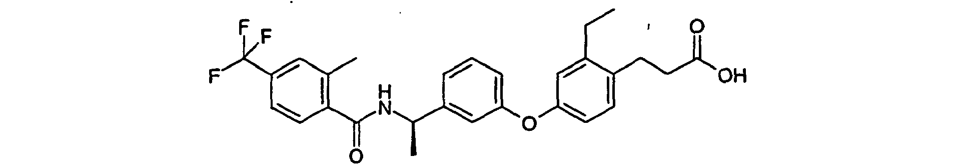

The title compound is prepared according to Example 296 by using 2-methyl-4-trifluoromethyl-benzoic acid and 3-{4-[3-(1-amino-ethyl)-phenoxy]-2-ethylphenyl}-propionic acid ethyl ester to afford about 401 mg (79%). ¹H NMR (400 MHz, CDCl₃); MS (ES⁺) *m*/*z* mass calcd for C₂₈H₂₈O₄NF₃ 499, found 500 (M + 1,100%).

### Example 315

### 3-(2-Ethyl-4-{3-[1-(2-methoxy-4-trifluoromethyl-benzoylamino)-ethyl]-phenoxy}-phenyl)-propionic acid

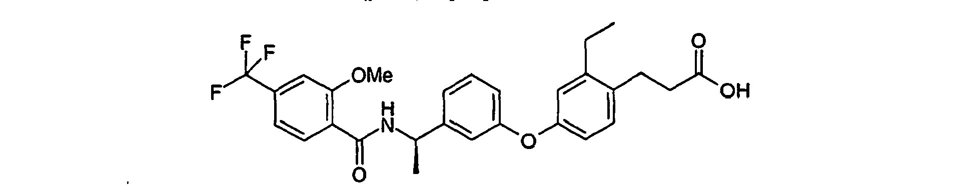

The title compound is prepared according to Example 296 by using 2-methyl-4-trifluoromethyl-benzoic acid and 3-{4-[3-(1-amino-ethyl)-phenoxy]-2-ethylphenyl}-propionic acid ethyl ester to afford about 450 mg (86%). ¹H NMR (400 MHz, CDCl₃); MS (ES⁺) *m*/*z* mass calcd for C₂₈H₂₈O₅NF₃ 515, found 516 (M + 1, 100%).

### Example 316

### 3-[4-(3-{1-[(5-Chloro-1,3-dimethyl-1H-indole-2-carbonyl)-amino]-ethyl}-5-fluoro-phenoxy)-2-ethyl-phenyl]-propionic acid

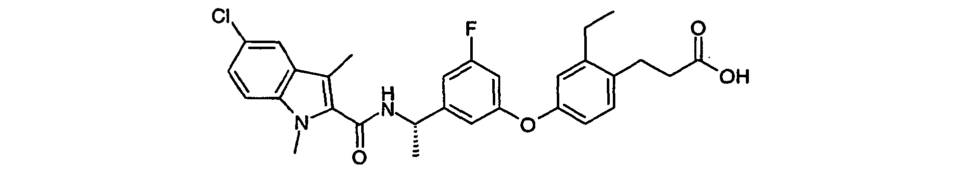

The title compound is prepared according to Example 2 by using 5-chloro-1,3-dimethyl-1H-indole-2-carboxylic acid and 3-{4-[3-(1-amino-ethyl)-5-fluoro-phenoxy]-2-ethyl-phenyl}-propionic acid ethyl ester to afford about 76 mg (100%). ¹H NMR (400 MHz, CDCl₃); MS (ES⁺) *m*/*z* mass calcd for C₃₀H₃₀O₄N₂FCl 536, found 537 and 539 (M + 1 and M + 3, 100%).

### Example 317

### 3-[4-(3-{1-[(5-Chloro-1,3-dimethyl-1H-indole-2-carbonyl)-amino]-ethyl}-5-fluoro-phenoxy)-2-ethyl-phenyl]-propionic acid

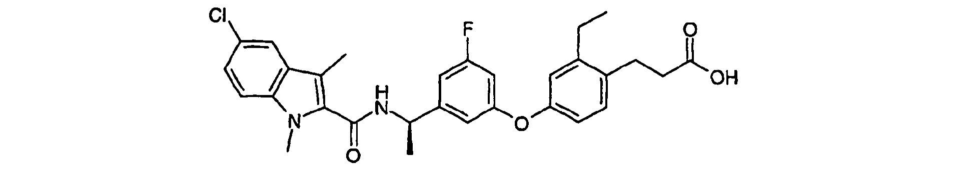

The title compound is prepared according to Example 2 by using 5-chloro-1,3-dimethyl-1H-indole-2-carboxylic acid and 3-{4-[3-(1-amino-ethyl)-5-fluoro-phenoxy]-2-ethyl-phenyl}-propionic acid ethyl ester to afford 2.48 g (45%). ¹H NMR (400 MHz, CDCl₃); MS (ES⁺) m/z mass calcd for C₃₀H₃₀O₄N₂FCl 536, found 537 and 539 (M + 1 and M + 3, 100%).

### Example 318

### 3-[4-(3-{1-[(5-Chloro-3-methyl-1H-indole-2-carbonyl)-amino]-ethyl} -5-fluoro-phenoxy)-2-ethyl-phenyl]-propionic acid

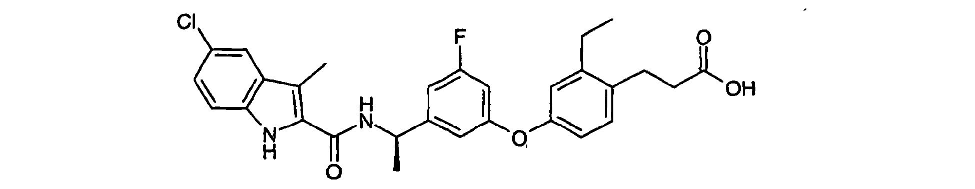

The title compound is prepared according to Example 2 by using 5-chloro-3-methyl-1H-indole-2-carboxylic acid and 3-(4-[3-(1-amino-ethyl)-5-fluoro-phenoxy]-2-ethyl-phenyl}-propionic acid ethyl ester to afford about 240 mg (40%). ¹H NMR (400 MHz, CDCl₃); MS (ES⁺) *m*/*z* mass calcd for C₂₉H₂₈O₄N₂FCl 522, found 523 and 525 (M + 1 and M + 3, 100%).

### Example 319

### 2-(4-{3-Fluoro-5-[(4-trifluoromethyl-benzoylamino)-methyl]-phenoxy}-2-methyl-phenoxy)-2-methyl-propionic acid

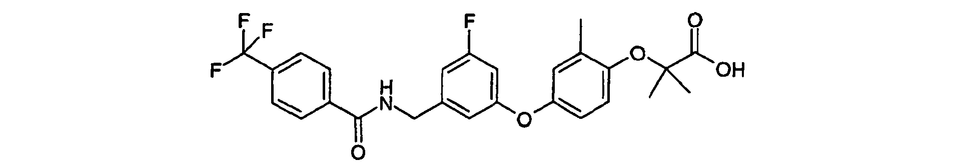

The title compound is prepared according to Example 48 by using 4-trifluoromethyl-benzoic acid and 2-[4-(3-aminomethyl-5-fluoro-phenoxy)-2-methyl-phenoxy]-2-methyl-propionic, acid ethyl ester to afford about 111 mg (93%). ¹H NMR (400 MHz, CDCl₃); MS (ES⁺) *m*/*z* mass calcd for C₂₆H₂₃O₅NF₄ 505, found 506 (M + 1, 100%).

### Example 320

### 3-[4-(3-Chloro-5-{[(5-chloro-1,3-dimethyl-1H-indole-2-carbonyl)-amino]-methyl}-phenoxy)-2-methyl-phenyl]-propionic acid

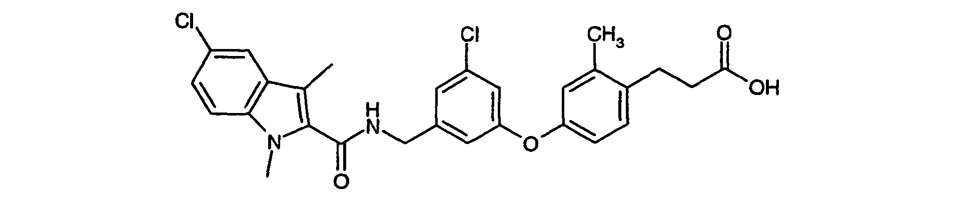

### Preparation of Intermediate

### 3-[4-(3-Aminomethyl-5-chloro-phenoxy)-2-methyl-phenyl]-propionic acid methyl ester

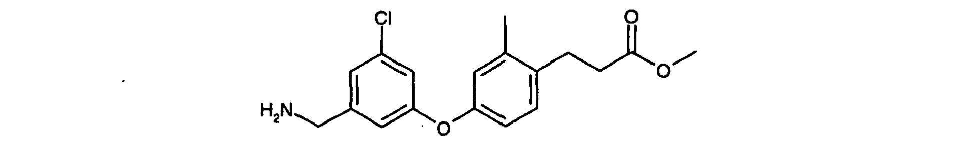

### Step A

### 3-[4-(3-Chloro-5-cyano-phenoxy)-2-methyl-phenyl]-propionic acid methyl ester

The compounds of 3-chloro-5-fluoro-benzonitrile (2.0 g, 134.9 mmol), 3-(4-hydroxy-2-methyl-phenyl)-propionic acid methyl ester (2.7 g, 13.5 mmol) (J.Chem.Soc.Perkin Trans.1; 4; 1990; 1041-1045) and cesium carbonate (8.4 g, 25.7 mmol) are added to dimethylformamide (20 ml) and heated overnight at 85 °C under nitrogen. After cooling, water is added and extracted 3 times with ethyl acetate, washed with brine, dried over sodium sulfate and concentrated under reduced pressure. Purification by flash chromatography, eluting with 10 % EtOAc in hexane then 15 % EtOAc in hexane provides the title compound (2.8 g, 65 %). MS(ES⁺): 347 (M+NH₄⁺); ¹HNMR (400 MHz, CDCl₃).

### Step B

### 3-[4-(3-Aminomethyl-5-chloro-phenoxy)-2-methyl-phenyl]-propionic acid methyl ester

Compound obtained from Step A (208.0. mg, 0.7 mmol) is reacted with platinum oxide (41.0 mg) in methanol (100 mL) and acetic acid (0.5 mL) at room temperature with 60 psi hydrogen for abut 3 hours in a Parr shaker. Reaction mixture is filtered and concentrated under reduced pressure with rt bath. Purification by SCX column, eluting with 10 % ammonia (2.0 M in methanol) in dichloromethane affords the title compound (81.0 mg, 38%) that is utilized without purification. MS(ES⁺): 334 (M+H⁺); ¹HNMR (400 MHz, CDCl₃).

The final compound of 3-[4-(3-chloro-5-{[(5-chloro-1,3-dimethyl-1H-indole-2-carbonyl)-amino]-methyl}-phenoxy)-2-methyl-phenyl]-propionic acid is prepared according to Example 1 utilizing 5-chloro-1,3-dimethyl-1H-indole-2-carboxylic acid (Intermediate 4) and 3-[4-(3-aminomethyl-5-chloro-phenoxy)-2-methylphenyl]-propionic acid methyl ester. Exact mass calcd for C₂₈H₂₆Cl₂N₂O₄ (M+H⁺): 525.1348, found 525.1332; ¹H NMR (400 MHz, CDCl₃).

### Example 321

### 2-Methyl-2-(2-methyl-4-{3-[1-(2-methyl-4-trifluoromethyl-benzoylamino)-ethyl]-phenoxy}-phenoxy)-propionic acid

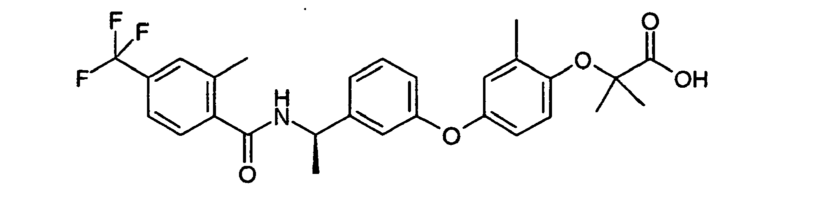

### Preparation of intermediate

### 2-(4-[3-(1-amino-ethyl)-phenoxy]-2-methyl-phenoxy)-2-methyl-propionic acid ethyl ester

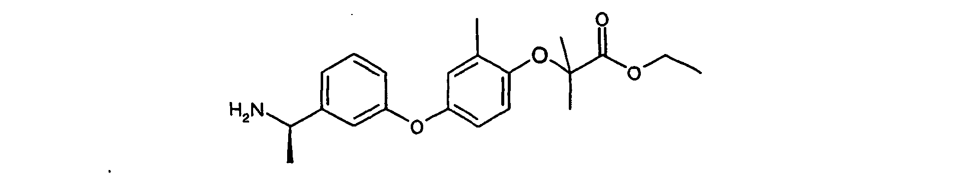

2- {4-[3-(1-Tert-butoxycarbonylamino-ethyl)-phenoxy]-2-methyl-phenoxy}-2-methyl-propionic acid ethyl ester (0.5 g, 21.9 mmol) is dissolved in 4M HCl in dioxane (80 mL) with an ice bath in place. Bath is removed in 20 minutes and the reaction is stirred for about 1 hour. The reaction is concentrated under reduced pressure which providees the HCl salt of the title compound (0.42 g, 92%). MS(ES⁺): 376 (M+H⁺); ¹HNMR (400 MHz, CDCl₃).

The title compound of 2-methyl-2-(2-methyl-4-{3-[1-(2-methyl-4-trifluoromethyl-benzoylamino)-ethyl]-phenoxy}-phenoxy)-propionic acid is prepared according to Example 1 by using 4-trifluoromethyl-benzoic acid (Example 10) and 2-{4-[3-(1-amino-ethyl)-phenoxy]-2-methyl-phenoxy}-2-methyl-propionic acid ethyl ester. ¹H NMR (400 MHz, CDCl₃); MS (ES⁺) *m*/*z* mass calcd for C₂₈H₂₈ F₃ N O₅ 516.1998, found 516.1987 (M + 1, 100%).

### Example 322

### 2-Methyl-2-(2-methyl-4-{3-[1-(4-trifluoromethyl-benzoylamino)-ethyl]-phenoxy}-phenoxy)-propionic acid

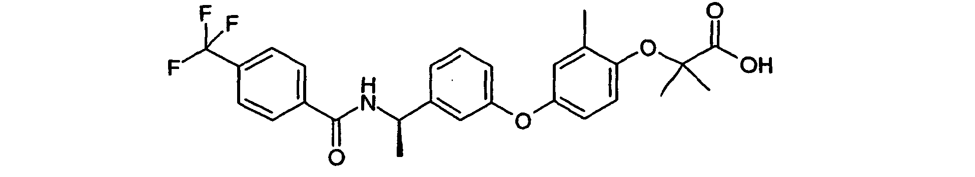

The title compound is prepared according to Example 321 by using 4-trifluoromethyl-benzoic acid and 2-{4-[3-(1-amino-ethyl)-phenoxy]-2-methyl-phenoxy}-2-methyl-propionic acid ethyl ester to afford about 48 mg (64%). ¹H NMR (400 MHz, CDCl₃); MS (ES⁺) m/z mass calcd for C₂₇H₂₆O₅NF₃ 501, found 502 (M + 1, 100%).

## Claims

1. A compound having a formula I, 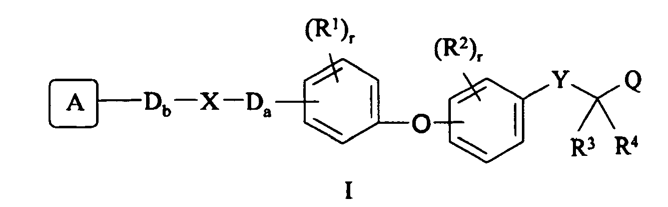 or a pharmaceutically acceptable salt or stereoisomer thereof, wherein:
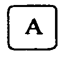 is : a) aryl,
b) a 5- to 10-membered heteroaryl wherein the heteroaryl containing at least one heteroatom selected from N, O or S,
c) C₃-C₈ cycloalkyl,
d) aliphatic group, or
e) heterocyclyl,
wherein aryl, heteroaryl, cycloalkyl, heterocyclyl and aliphatic group being optionally substituted with one or more groups independently selected from R⁸;
Dₐ and D_{b} are each independently:
a bond or
-[C(R^{c})(R^{d})]ₙ, wherein R^{c} and R^{d} are each independently hydrogen, C₁-C₆ alkyl or aryl;
Q is: -C(O)OR⁵ or R^{5A};
X is: NR⁶C[O]ₚ,
NR⁶S(O)a,
C[O]ₚ,NR⁶,
S(O)₂NR⁶ or
NR⁷;
Y is: a bond, CH₂, S or O; 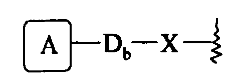
is: 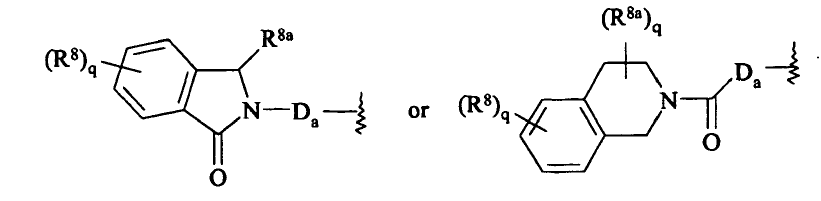
n and r are each independently: 1, 2, 3 or 4;
q is: 1, 2, 3, 4 or 5;
p is: 1 or 2;
R¹ and R² are each independently: hydrogen, C₁-C₆ alkyl, halo or haloalkyl;
R³ and R⁴ are each independently:
hydrogen,
halo,
C₁-C₆ alkyl,
C₁-C₆ alkoxy or
aryloxy;
R³ and R⁴ are together a 3- to 6- membered carbocyclyl or heterocyclyl;
R⁵ is: hydrogen, C₁-C-₆alkyl or aminoalkyl;
R^{5A} is: carboxamide, sulfonamide, acylsulfonamide, tetrazole, 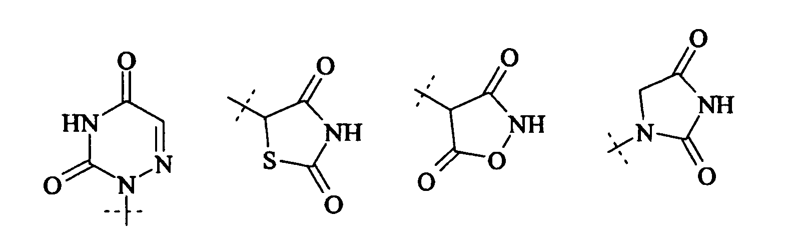 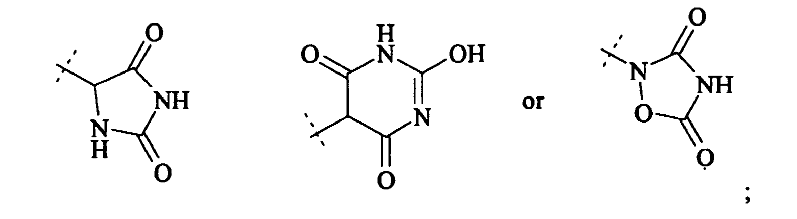
R⁶ is each independently:
hydrogen,
C₁-C₁₂alkyl,
arylalkyl,
C₃-C₈ cycloalkyl, or
(CH₂)ₙC(O)aryl,
wherein alkyl, arylalkyl and cycloalkyl group being optionally substituted with one or more groups independently selected from R⁸;
R⁷ is: hydrogen,
acyl, or
sulfonyl;
R⁸ and R^{8a} are each independently:
hydrogen,
C₁-C₆alkyl,
C₁-C₆ alkoxy,
nitro,
cyano,
halo,
haloalkyl,
haloalkyloxy,
aryl,
heteroaryl,
benzyl,
aryloxy,
SR⁹,
S[O]pR⁹ or
C[O]ₚR⁹; and
R⁹ is: hydrogen, C₁-C₆ alkyl, or C₃-C₈ cycloalkyl.

2. A compound of Claim 1, wherein aryl or heteroaryl are selected from the group consisting of phenyl, naphthyl, indolyl, isoindolyl, benzoimidazolyl, quinolinyl, isoquinolinyl, pyridyl, benzothiophenyl and benzofuranyl.

3. A compound of Claim 2, having a structural formula II, 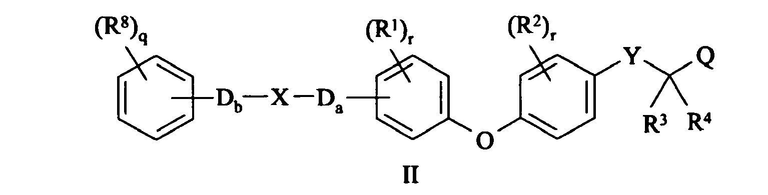 or a pharmaceutically acceptable salt or stereoisomer thereof, wherein:
q is 1,2,3,4,or 5.

4. A compound of Claim 3, wherein R⁸ is disubtituted in 2 and 4 positions, or trisubstituted in 2, 4, and 6 positions of phenyl ring relative to -D_{b}-.

5. A compound of Claim 3, having a structural formula III, 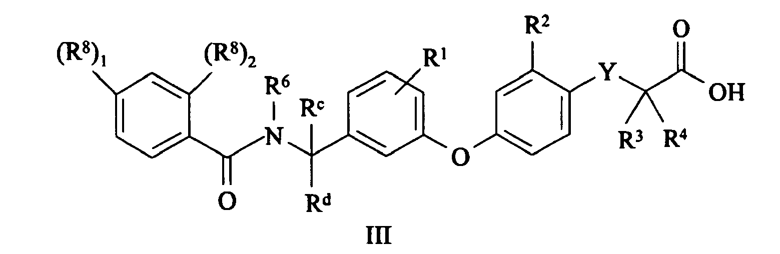 or a pharmaceutically acceptable salt or stereoisomer thereof, wherein:
Y is: O or CH₂;
R¹ is: hydrogen, halo or C₁-C₄alkyl;
R², R³ and R⁴, R⁶, R^{c} and R^{d} are each independently: hydrogen or C₁-C₄ alkyl;
(R⁸)₁ and (R⁸)₂ are each independently: hydrogen, halo, haloalkyl or haloalkyloxy, cyano, nitro, C₁-C₆ alkyl, C₁-C₆ alkoxy or SR⁹;
R⁶ is: hydrogen or C₁-C₄ alkyl; and
R⁹ is: hydrogen or C₁-C₄ alkyl or C₃-C₆ cycloalkyl

6. A compound of Claim 5, having a structural formula IV, 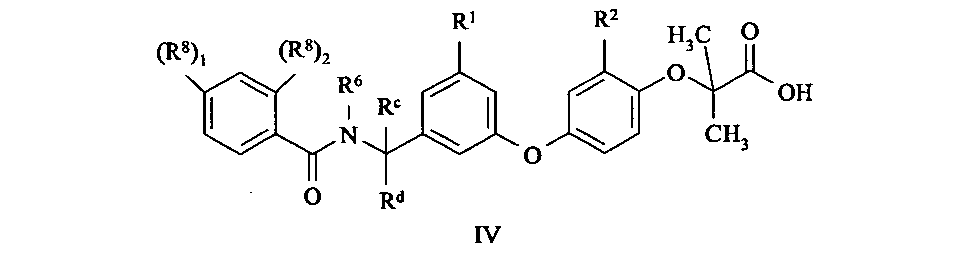 or a pharmaceutically acceptable salt or stereoisomer thereof, wherein:
R¹ and R² are each independently: hydrogen, halo or C₁-C₄ alkyl;
R^{c}, R^{d} and R⁶ are each independently: hydrogen or methyl; and
(R⁸)₁ and (R⁸)₂ are each independently:
hydrogen, F, Cl, Br, OMe, CF₃, OCF₃, SCH₃, NO₂, cyano, methyl, ethyl, isobutyl, isopropyl or *tert-*butyl*.*

7. A compound of Claim 6, having a structural formula V, 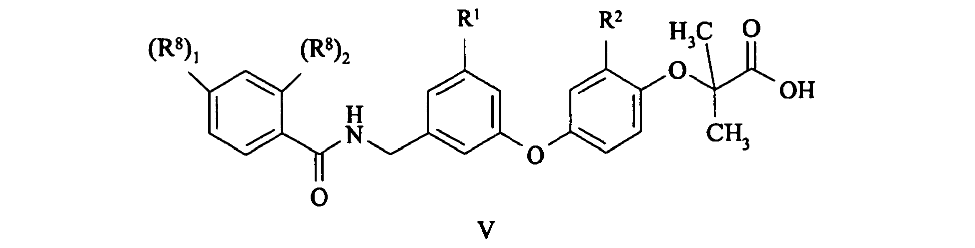 or a pharmaceutically acceptable salt or stereoisomer thereof, wherein:
R¹ and R² are each independently: hydrogen, methyl, ethyl or fluoro; and
(R⁸)₁ and (R⁸)₂ are each independently:
hydrogen, F, Cl, Br, OMe, CF₃, OCF₃, SCH₃, NO₂, cyano, methyl, ethyl, isobutyl, isopropyl or *tert*-butyl.

8. A compound of Claim 3, having a structural formula VII, 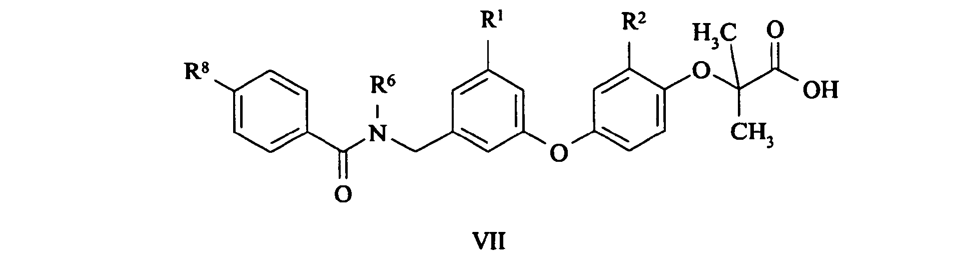 or a pharmaceutically acceptable salt or stereoisomer thereof, wherein:
R¹ and R² are each independently: hydrogen, halo or C₁-C₄ alkyl;
R⁶ is: hydrogen or C₁-C₄ alkyl;
R⁸ is: hydrogen, halo, haloalkyl or haloalkyloxy, cyano, nitro, C₁-C₆alkyl, C₁-C₆ alkoxy or SR⁹; and
R⁹ is: hydrogen or C₁-C₄ alkyl or C₃-C₆ cycloalkyl.

9. A compound of Claim 1, having a structural formula VIII, 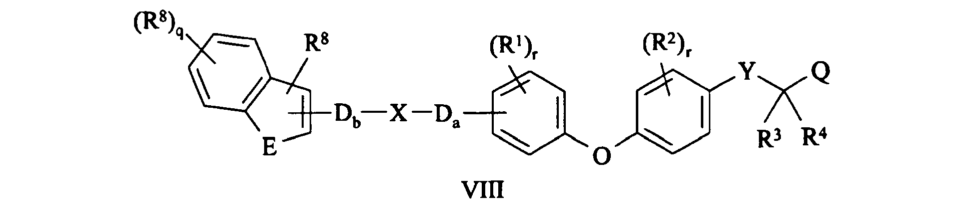 or a pharmaceutically acceptable salt or stereoisomer thereof, wherein:
q is 1, 2, 3 or 4; and
E is S, O or NR¹⁰ wherein R¹⁰ is hydrogen or C₁-C₄alkyl.

10. A compound of Claim 9, having a structural formula IX, 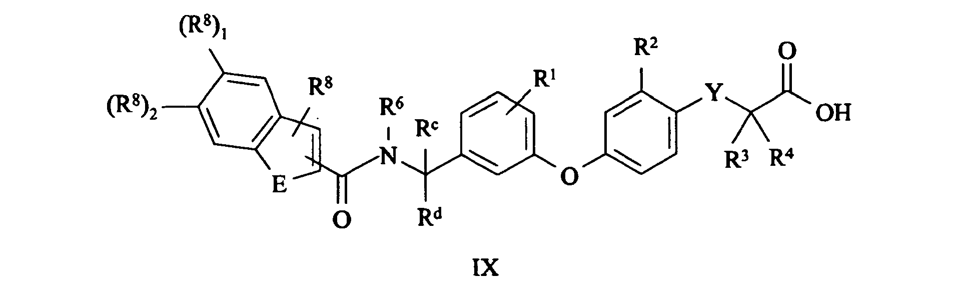 or a pharmaceutically acceptable salt or stereoisomer thereof, wherein:
Y is: O or CH₂;
E is: S, O, NH or NCH₃, NCH₂CH₃;
R¹ is: hydrogen, C₁-C₄ alkyl, halo or haloalkyl;
R², R³ and R⁴, R⁶, R^{c} and R^{d} are each independently: hydrogen or C₁-C₄ alkyl;
(R⁸)₁ and (R⁸)₂ are each independently: hydrogen, halo, haloalkyl, haloalkyloxy, cyano, nitro, C₁-C₆alkyl or C₁-C₆ alkoxy; and
R⁸ is: hydrogen or C₁-C₄ alkyl.

11. A compound of Claim 10, having a structural formula XIII, 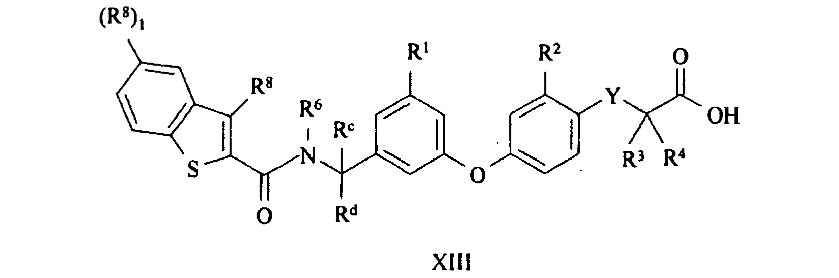 or a pharmaceutically acceptable salt or stereoisomer thereof, wherein:
Y is: O or CH₂;
R¹ is: hydrogen, C₁-C₄ alkyl, halo or haloalkyl;
R², R³, R⁴, R⁶, R^{c} and R^{d} are each independently: hydrogen or C₁-C₄ alkyl;
R⁸ are each independently: hydrogen or C₁-C₄ alkyl; and
(R⁸), is: hydrogen, halo, haloalkyl or haloalkyloxy, cyano, nitro, C₁-C₆alkyl or C₁-C₆ alkoxy.

12. A compound of Claim 1, having a structural formula XVI, 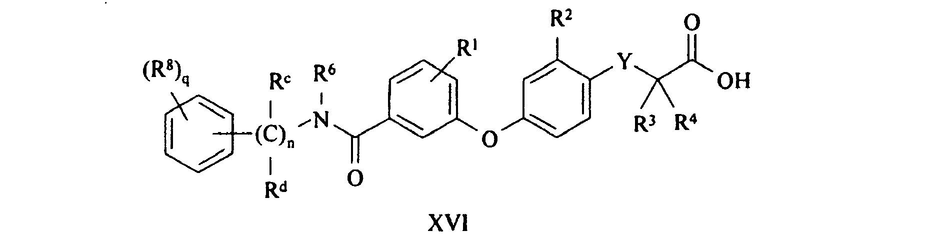 or a pharmaceutically acceptable salt or stereoisomer thereof, wherein:
n is 1, 2, 3, or 4.

13. A compound of Claim 12, wherein Y is O or CH₂; R¹, R², R³, R⁴ R^{c} and R^{d} are each independently hydrogen or C₁-C₄alkyl; n is I or 2; R⁶ is hydrogen, C₁-C₆ alkyl or arylalkyl; and R⁸ is hydrogen, C₁-C₆ alkoxy, halo or haloalkyl.

14. A compound of Claim 1, having a structural formula XVII, 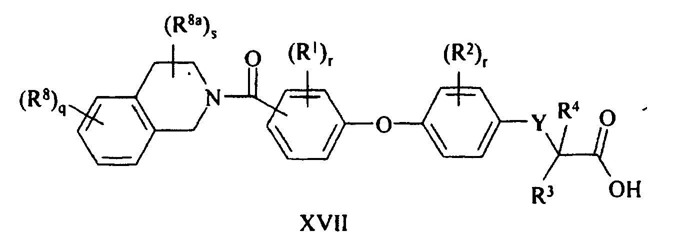 or a pharmaceutically acceptable salt or stereoisomer thereof, wherein:
R^{8a} is hydrogen, C₁-C₄ alkyl or aryl; and s is 1, 2, 3, 4, 5 or 6.

15. A compound of Claim 1, having a structural formula XIX, 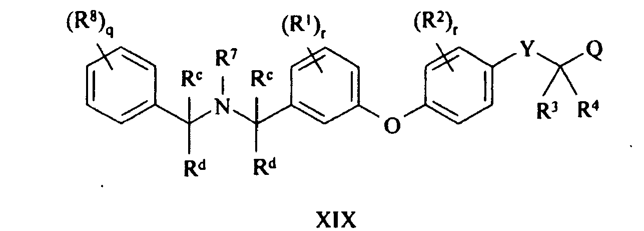 or a pharmaceutically acceptable salt or stereoisomer thereof.

16. A compound of Claim 15, wherein Q is COOH; R⁷ is hydrogen, methanesulfonyl or acetyl; and R^{c} and R^{d} are each hydrogen.

17. A compound of Claim 1 selected from:
2-(4-{3-[(2-Chloro-4-trifluoromethyl-benzoylamino)-methyl]-5-fluoro-phenoxy}-2-methyl-phenoxy)-2-methyl-propionic acid;
3-[4-(3-{[(5-Chloro-1H-indole-2-carbonyl)-amino]-methyl)-5-fluoro-phenoxy)-2-methylphenyl]-propionic acid;
2-(4-{3-Fluoro-5-[1-(2-methyl-4-trifluoromethyl-benzoylamino)-ethyl]-phenoxy}-2-methyl-phenoxy)-2-methyl-propionic acid (isomer 1);
2-[4-(3-{[(5-Chloro-3-methyl-benzo[b]thiophene-2-carbonyl)-amino]-methyl}-5-methyl-phenoxy)-2-methyl-phenoxy]-2-methyl-propionic acid;
(R)-3-[4-(3-{1-[(5-Chloro-1,3-dimethyl-1H-indole-2-carbonyl)-amino]-ethyl}-5-fluoro-phenoxy)-2-methyl-phenyl]-propionic acid;
3-(2-Ethyl-4-{3-fluoro-5-[(2-methyl-4-trifluoromethyl-benzoylamino)-methyl]-phenoxy}-phenyl)-propionic acid;
2-(4-{3-[(2-Fluoro-4-trifluoromethyl-benzoylamino)-methyl]-5-methyl-phenoxy}-2-methyl-phenoxy)-2-methyl-propionic acid;
(R)-2-[4-(3-{[(5-Chloro-1,3-dimethyl-1H-indole-2-carbonyl)-amino]-methyl}-5-methyl-phenoxy)-2-methyl-phenoxy]-2-methyl-propionic acid;
3-[4-(3-Fluoro-5-{[(5-fluoro-3-methyl-1H-indole-2-carbonyl)-amino]-methyl}-phenoxy)-2-methyl-phenyl]-propionic acid;
2-[4-(3-Fluoro-5-{[(5-fluoro-1,3-dimethyl- 1H-indole-2-carbonyl)-amino]-methyl}-phenoxy)-2-methyl-phenoxy]-2-methyl-propionic acid;
(R) -3-[4-(3-{1-[(5-Fluoro-1,3-dimethyl-1H-indole-2-carbonyl)-amino]-ethyl}-5-methyl-phenoxy)-2-methyl-phenyl]-propionic acid;
2-Methyl-2-(2-methyl-4-{3-[(2-methyl-4-trifluoromethyl-benzoylamino)-methyl]-phenoxy}-phenoxy)-propionic acid;
2-(4-{3-Fluoro-5-[(2-methyl-4-trifluoromethyl-benzoylamino)-methyl]-phenoxy}-2-methyl-phenoxy)-2-methyl-propionic acid;
(R) -3-[4-(3-Fluoro-5-{1-[(5-fluoro-1,3-dimethyl-1H-indole-2-carbonyl)-amino]-ethyl}-phenoxy)-2-methyl-phenyl]-propionic acid;
3-[4-(3-{[(5-Chloro-1,3-dimethyl-1H-indole-2-carbonyl)-amino]-methyl}-5-fluoro-phenoxy)-2-methyl-phenyl]-propionic acid;
3-[4-(3-{[(5-Chloro-1,3-dimethyl-1H-indole-2-carbonyl)-amino]-methyl}-phenoxy)-2-methyl-phenyl]-propionic acid;
3-[2-Ethyl-4-(3-fluoro-5-{[(5-fluoro-1,3-dimethyl-1H-indole-2-carbonyl)-amino]-methyl}-phenoxy)-phenyl]-propionic acid; and
3-(4-{3-[(2-Chloro-4-trifluoromethyl-benzoylamino)-methyl]-5-methyl-phenoxy}-2-ethyl-phenyl)-propionic acid.

18. A pharmaceutical composition comprising a pharmaceutically acceptable carrier and a compound of any one of claims 1-17 or a pharmaceutically acceptable salt.

19. Use of a compound of any one of Claims 1-17 or a pharmaceutically acceptable salt thereof for the manufacture of a medicament for treating hyperglycemia, dyslipidemia, Type II diabetes, Type I diabetes, hypertriglyceridemia, syndrome X, insulin resistance, heart failure, diabetic dyslipidemia, hyperlipidemia, hypercholesteremia, hypertension, obesity, anorexia bulimia, anorexia nervosa and cardiovascular disease.

20. Use of a compound of any one of Claims 1-17 or a pharmaceutically acceptable salt thereof for the manufacture of a medicament for treating diabetes mellitus in a mammal.

21. Use of a compound of any one of Claims 1-17 or a pharmaceutically acceptable salt thereof for the manufacture of a medicament for lowering blood glucose in a mammal.

22. Use of a compound of any one of Claims 1- 17 or a pharmaceutically acceptable salt, for the manufacture of a medicament for the treatment of a condition modulated by a PPAR.

## Patentansprüche

1. Verbindung der Formel I 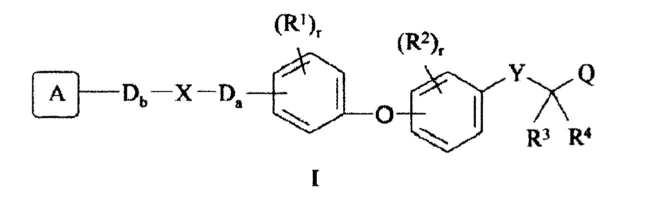 oder ein pharmazeutisch akzeptables Salz oder Stereoisomer hiervon, worin
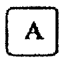 steht für a) Aryl,
b) ein 5- bis 10-gliedriges Heteroaryl, worin das Heteroaryl wenigstens ein Heteroatom enthält, das ausgewählt ist aus N, O oder S,
c) C₃-C₈-Cycloalkyl,
d) eine aliphatische Gruppe, oder
e) Heterocyclyl,
worin Aryl, Heteroaryl, Cycloalkyl, Heterocyclyl und eine aliphatische Gruppe optional substituiert sind mit ein oder mehr Gruppen, die unabhängig ausgewählt sind aus R⁸,
Dₐ und D_{b} jeweils unabhängig stehen für
eine Bindung oder -[C(R^{c})(R^{d})]ₙ, worin R^{c} und R^{d} jeweils unabhängig für Wasserstoff, C₁-C₆-Alkyl oder Aryl stehen,
Q für -C(O)OR⁵ oder R^{5A} steht,
X für NR⁶C[O]ₚ,
NR⁶S(O)₂,
C[O]ₚ, NR⁶,
S(O)₂NR⁶ oder
NR⁷ steht,
Y für eine Bindung, CH₂, S oder O steht, 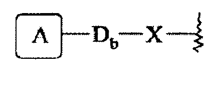
steht für 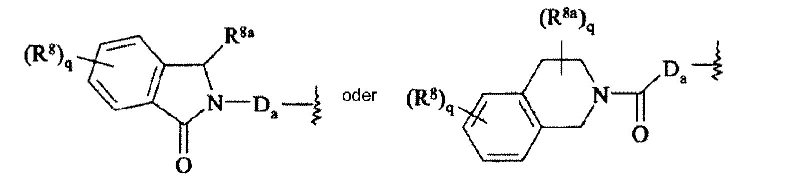
n und r jeweils unabhängig für 1, 2, 3 oder 4 stehen,
q für 1, 2, 3, 4 oder 5 steht,
p für 1 oder 2 steht,
R¹ und R² jeweils unabhängig für Wasserstoff, C₁-C₆-Alkyl, Halogen oder Halogenalkyl stehen,
R³ und R⁴ jeweils unabhängig für
Wasserstoff,
Halogen,
C₁-C₆-Alkyl,
C₁-C₆-Alkoxy oder
Aryloxy stehen,
R³ und R⁴ zusammen für ein 3- bis 6-gliedriges Carbocyclyl oder Heterocyclyl stehen,
R⁵ für Wasserstoff, C₁-C₆-Alkyl oder Aminoalkyl steht,
R^{5A} für Carboxamid, Sulfonamid, Acylsulfonamid, Tetrazol oder Reste der folgenden Formeln steht 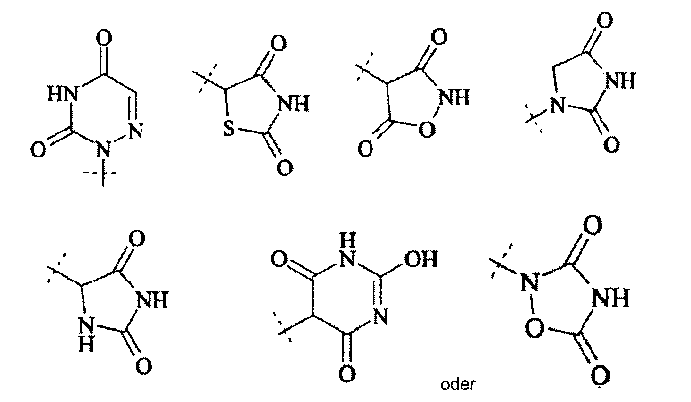
R⁶ jeweils unabhängig für
Wasserstoff,
C₁-C₁₂-Alkyl,
Arylalkyl,
C₃-C₆-Cycloalkyl oder
(CH₂)ₙC(O)-Aryl steht,
worin die Gruppen Alkyl, Arylalkyl und Cycloalkyl optional substituiert sind mit einer oder mehr Gruppen, die unabhängig ausgewählt sind aus R⁸,
R⁷ für Wasserstoff,
Acyl oder
Sulfonyl steht,
R⁸ und R^{8a} jeweils unabhängig stehen für
Wasserstoff,
C₁-C₆-Alkyl,
C₁-C₆-Alkoxy,
Nitro,
Cyano,
Halogen,
Halogenalkyl,
Halogenalkoxy,
Aryl,
Heteroaryl,
Benzyl,
Aryloxy,
SR⁹,
S[O]ₚR⁹ oder
C[O]ₚR⁹ und
R⁹ für Wasserstoff, C₁-C₆-Alkyl oder C₃-C₈-Cycloalkyl steht.

2. Verbindung nach Anspruch 1, worin Aryl oder Heteroaryl aus der Gruppe ausgewählt sind, die besteht aus Phenyl, Naphthyl, Indolyl, Isoindolyl, Benzimidazolyl, Chinolinyl, Isochinolinyl, Pyridyl, Benzothiophenyl und Benzofuranyl.

3. Verbindung nach Anspruch 2 der Strukturformel II 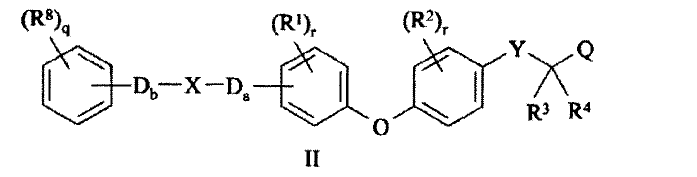 oder ein pharmazeutisch akzeptables Salz oder Stereoisomer hiervon, worin q für 1, 2, 3, 4 oder 5 steht.

4. Verbindung nach Anspruch 3, worin R⁸ in den Positionen 2 und 4 eines Phenylrings disubstituiert ist oder in den Positionen 2, 4 und 6 eines Phenylrings trisubstituiert ist im Verhältnis zur Brücke -D_{b}-.

5. Verbindung nach Anspruch 3 der Strukturformel III 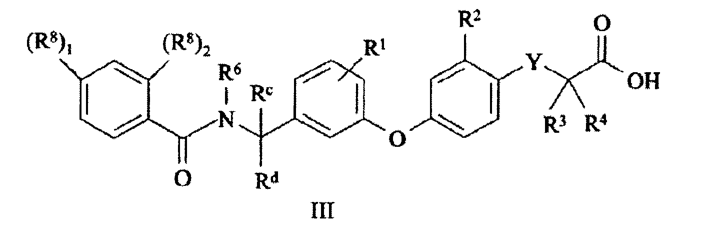 oder ein pharmazeutisch akzeptables Salz oder Stereoisomer hiervon, worin
Y für O oder CH₂ steht,
R¹ für Wasserstoff, Halogen oder C₁-C₄-Alkyl steht,
R², R³ und R⁴, R⁶, R^{c} und R^{d} jeweils unabhängig für Wasserstoff oder C₁-C₄-Alkyl stehen,
(R⁸), und (R⁸)₂ jeweils unabhängig für Wasserstoff, Halogen, Halogenalkyl oder Halogenalkyloxy, Cyano, Nitro, C₁-C₆-Alkyl, C₁-C₆-Alkoxy oder SR⁹ stehen,
R⁶ für Wasserstoff oder C₁-C₄-Alkyl steht und
R⁹ für Wasserstoff oder C₁-C₄-Alkyl oder C₃-C₆-Cycloalkyl steht.

6. Verbindung nach Anspruch 5 der Strukturformel IV 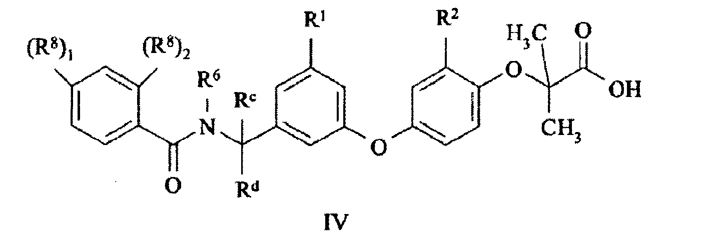 oder ein pharmazeutisch akzeptables Salz oder Stereoisomer hiervon, worin
R¹ und R² jeweils unabhängig für Wasserstoff, Halogen oder C₁-C₄-Alkyl stehen,
R^{c}, R^{d} und R⁶ jeweils unabhängig für Wasserstoff oder Methyl stehen, und
(R⁸)₁ und (R⁸)₂ jeweils unabhängig für Wasserstoff, F, Cl, Br, OMe, CF₃, OCF₃, SCH₃, NO₂, Cyano, Methyl, Ethyl, Isobutyl, Isopropyl oder tert-Butyl stehen.

7. Verbindung nach Anspruch 6 der Strukturformel V 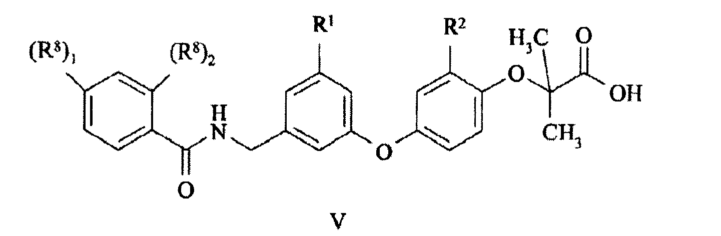 oder ein pharmazeutisch akzeptables Salz oder Stereoisomer hiervon, worin
R¹ und R² jeweils unabhängig für Wasserstoff, Methyl, Ethyl oder Fluor stehen, und
(R⁸), und (R⁸)₂ jeweils unabhängig für Wasserstoff, F, Cl, Br, OMe, CF₃, OCF₃, SCH₃, NO₂, Cyano, Methyl, Ethyl, Isobutyl, Isopropyl oder tert-Butyl stehen.

8. Verbindung nach Anspruch 3 der Strukturformel VII 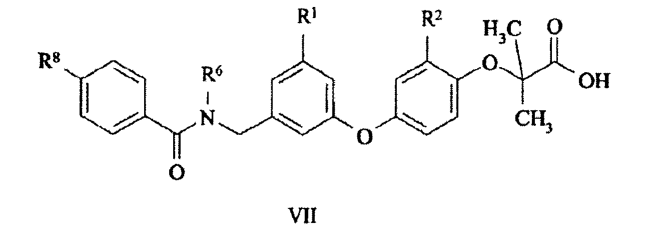 oder ein pharmazeutisch akzeptables Salz oder Stereoisomer hiervon, worin
R¹ und R² jeweils unabhängig für Wasserstoff, Halogen oder C₁-C₄-Alkyl stehen,
R⁶ für Wasserstoff oder C₁-C₄-Alkyl steht,
R⁸ für Wasserstoff, Halogen, Halogenalkyl oder Halogenalkoxy, Cyano, Nitro, C₁-C₆-Alkyl, C₁-C₆-Alkoxy oder SR⁹ steht, und
R⁹ für Wasserstoff oder C₁-C₄-Alkyl oder C₃-C₆-Cycloalkyl steht.

9. Verbindung nach Anspruch 1 der Strukturformel VIII 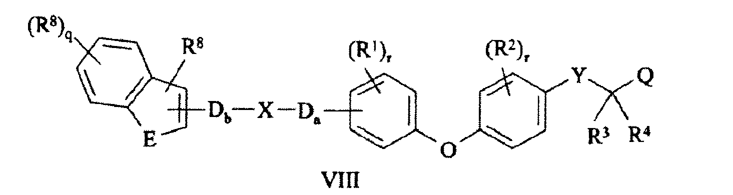 oder ein pharmazeutisch akzeptables Salz oder Stereoisomer hiervon, worin
q für 1, 2, 3 oder 4 steht, und
E für S, O oder NR¹⁰ steht, worin R¹⁰ für Wasserstoff oder C₁-C₄-Alkyl steht.

10. Verbindung nach Anspruch 9 der Strukturformel IX 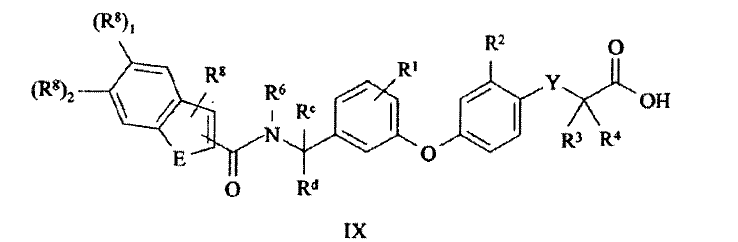 oder ein pharmazeutisch akzeptables Salz oder Stereoisomer hiervon, worin
Y für O oder CH₂ steht,
E für S, O, NH, NCH₃ oder NCH₂CH₃ steht,
R¹ für Wasserstoff, C₁-C₄-Alkyl, Halogen oder Halogenalkyl steht,
R², R³ und R⁴, R⁶, R^{c} und R^{d} jeweils unabhängig für Wasserstoff oder C₁-C₄-Alkyl stehen,
(R⁸), und (R⁸)₂ jeweils unabhängig für Wasserstoff, Halogen, Halogenalkyl, Halogenalkoxy, Cyano, Nitro, C₁-C₆-Alkyl oder C₁-C₆-Alkoxy stehen, und
R⁸ für Wasserstoff oder C₁-C₄-Alkyl steht.

11. Verbindung nach Anspruch 10 mit der Strukturformel XIII 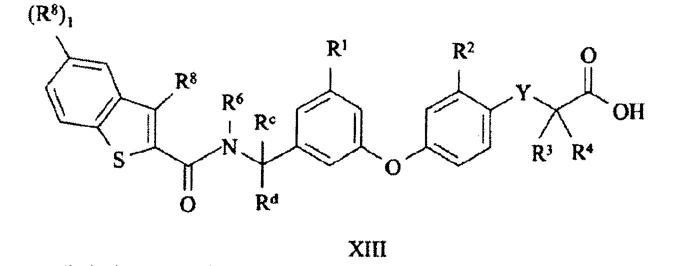 oder ein pharmazeutisch akzeptables Salz oder Stereoisomer hiervon, worin
Y für O oder CH₂ steht,
R¹ für Wasserstoff, C₁-C₄-Alkyl, Halogen oder Halogenalkyl steht,
R², R³, R⁴, R⁶, R^{c} und R^{d} jeweils unabhängig für Wasserstoff oder C₁-C₄-Alkyl stehen,
R⁸ jeweils unabhängig für Wasserstoff oder C₁-C₄-Alkyl steht, und
(R⁸)₁ für Wasserstoff, Halogen, Halogenalkyl oder Halogenalkoxy, Cyano, Nitro, C₁-C₆-Alkyl oder C₁-C₆-Alkoxy steht.

12. Verbindung nach Anspruch 1 der Strukturformel XVI 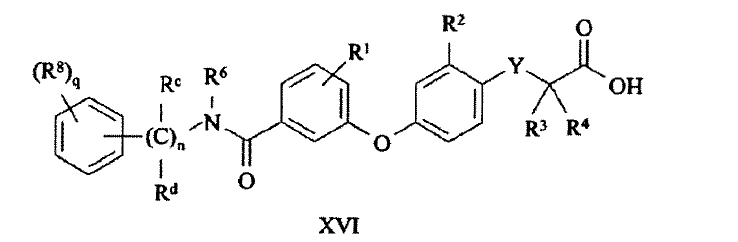 oder ein pharmazeutisch akzeptables Salz oder Stereoisomer hiervon, worin
n für 1, 2, 3 oder 4 steht.

13. Verbindung nach Anspruch 12, worin Y für O oder CH₂ steht, R¹, R², R³, R⁴, R^{c} und R^{d} jeweils unabhängig für Wasserstoff oder C₁-C₄-Alkyl stehen, n für 1 oder 2 steht, R⁶ für Wasserstoff, C₁-C₆-Alkyl oder Arylalkyl steht, und R⁸ für Wasserstoff, C₁-C₆-Alkoxy, Halogen oder Halogenalkyl steht.

14. Verbindung nach Anspruch 1 der Strukturformel XVII 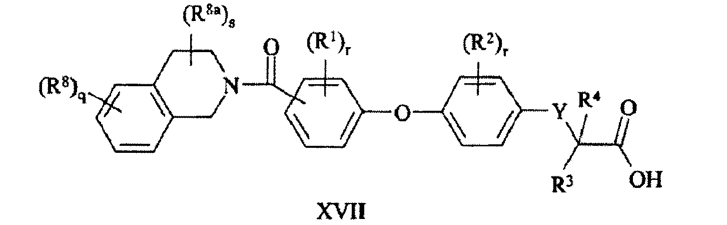 oder ein pharmazeutisch akzeptables Salz oder Stereoisomer hiervon, worin
R^{8a} für Wasserstoff, C₁-C₄-Alkyl oder Aryl steht und s für 1, 2, 3, 4, 5 oder 6 steht.

15. Verbindung nach Anspruch 1 der Strukturformel XIX 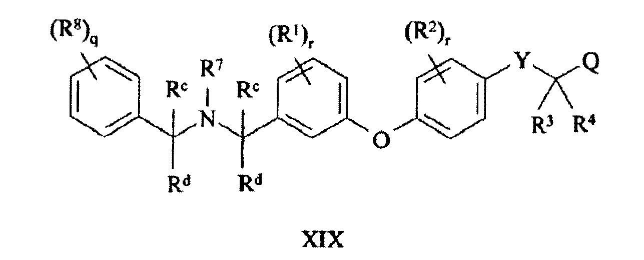 oder ein pharmazeutisch akzeptables Salz oder Stereoisomer hiervon.

16. Verbindung nach Anspruch 15, worin Q für COOH steht, R⁷ für Wasserstoff, Methansulfonyl oder Acetyl steht, und R^{c} und R^{d} jeweils für Wasserstoff stehen.

17. Verbindung nach Anspruch 1, die ausgewählt ist aus
2-(4-{3-[(2-Chlor-4-trifluormethylbenzoylamino)methyl]-5-fluorphenoxy}-2-methylphenoxy)-2-methylpropionsäure,
3-[4-(3-{[(5-Chlor-1H-indol-2-carbonyl)amino]methyl}-5-fluorphenoxy)-2-methylphenyl]propionsäure,
2-(4-{3-Fluor-5-[1-(2-methyl-4-trifluormethylbenzoylamino)ethyl]phenoxy}-2-methylphenoxy)-2-methylpropionsäure (Isomer 1),
2-[4-{3-[(5-Chlor-3-methylbenzo[b]thiophen-2-carbonyl)amino]methyl}-5-methylphenoxy)-2-methylphenoxy]-2-methylpropionsäure,
(R)-3-[4-(3-{1-[(5-Chlor-1,3-dimethyl-1H-indol-2-carbonyl)amino]ethyl}-5-fluorphenoxy)-2-methylphenyl]-propionsäure,
3-(2-Ethyl-4-{3-fluor-5-[(2-methyl-4-trifluormethylbenzoylamino)methyl]penoxy}phenyl)propionsäure,
2-(4-{3-[(2-Fluor-4-trifluormethylbenzoylamino)methyl]-5-methylphenoxy}-2-methylphenoxy)-2-methyl-propionsäure,
(R)-2-[4-(3-{[(5-Chlor-1,3-dimethyl-1H-indol-2-carbonyl)amino]methyl}-5-methylphenoxy)-2-methylphenoxy]-2-methylpropionsäure,
3-[4-(3-Fluor-5-{[(5-fluor-3-methyl-1H-indol-2-carbonyl)amino]methyl)phenoxy)-2-methylphenyl]propionsäure,
2-[4-(3-Fluor-5-{[(5-fluor-1,3-dimethyl-1H-indol-2-carbonyl)amino]methyl}phenoxy)-2-methylphenoxy]-2-methylpropionsäure,
(R)-3-[4-(3-{1-[(5-Fluor-1,3-dimethyl-1H-indol-2-carbonyl)amino]ethyl}-5-methylphenoxy)-2-methylphenyl]-propionsäure,
2-Methyl-2-(2-methyl-4-{3-[(2-methyl-4-trifluormethylbenzoylamino)methyl]phenoxy}phenoxy)propionsäure,
2-(4-{3-Fluor-5-[(2-methyl-4-trifluormethylbenzoylamino)methyl]phenoxy}-2-methylphenoxy)-2-methylpropionsäure,
(R)-3-[4-(3-Fluor-5-{1-[(5-fluor-1,3-dimethyl-1 H-indol-2-carbonyl)amino]ethyl}phenoxyr2-methylphenyl]-propionsäure,
3-[4-(3-{[(5-Chlor-1,3-dimethyl-1H-indol-2-carbonyl)amino]methyl}-5-fluorphenoxy}-2-methylphenyl]propionsäure,
3-[4-(3-{[(5-Chlor-1,3-dimethyl-1 H-indol-2-carbonyl)amino]methyl}phenoxy)-2-methylphenyl]propionsäure,
3-[2-Ethyl-4-(3-fluor-5-{[(5-fluor-1,3-dimethyl-1 H-indol-2-carbonyl)amino]methyl}phenoxy)phenyl]propionsäure und
3-(4-{3-[(2-Chlor-4-trifluormethylbenzoylamino)methyl]-5-methylphenoxy}-2-ethylphenyl)propionsäure.

18. Pharmazeutische Zusammensetzung umfassend einen pharmazeutisch akzeptablen Träger und eine Verbindung nach einem der Ansprüche 1 bis 17 oder ein pharmazeutisch akzeptables Salz hiervon.

19. Verwendung einer Verbindung nach einem der Ansprüche 1 bis 17 oder eines pharmazeutisch akzeptablen Salzes hiervon zur Herstellung eines Arzneimittels für die Behandlung von Hyperglykämie, Dyslipidämie, Diabetes Typ II, Diabetes Typ I, Hypertriglyceridämie, Syndrom X, Insulinresistenz, Herzversagen, diabetische Dyslipidämie, Hyperlipidämie, Hypercholesterämie, Hypertension, Obesität, Anorexia bulimia, Anorexia nervosa und Kardiovaskularkrankheit.

20. Verwendung einer Verbindung nach einem der Ansprüche 1 bis 17 oder eines pharmazeutisch akzeptablen Salzes hiervon zur Herstellung eines Arzneimittels für die Behandlung von Diabetes mellitus bei einem Säuger.

21. Verwendung einer Verbindung nach einem der Ansprüche 1 bis 17 oder eines pharmazeutisch akzeptablen Salzes hiervon zur Herstellung eines Arzneimittels für die Erniedrigung von Blutzucker in einem Säuger.

22. Verwendung einer Verbindung nach einem der Ansprüche 1 bis 17 oder eines pharmazeutisch akzeptablen Salzes hiervon zur Herstellung eines Arzneimittels für die Behandlung eines durch ein PPAR modulierten Zustands.

## Revendications

1. Composé de formule I, 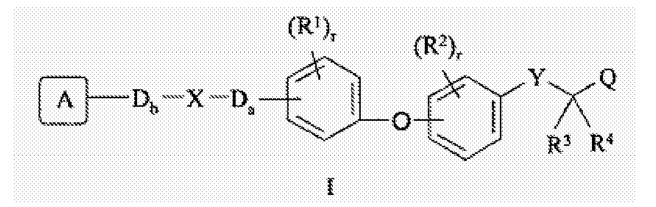 ou un sel ou un stéréo-isomère de celui-ci pharmaceutiquement acceptable, dans lequel :
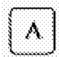 est : a) un groupe aryle,
b) un groupe hétéroaryle à 5 à 10 chaînons dans lequel le groupe hétéroaryle contenant au moins un hétéroatome choisi parmi N, O ou S,
c) un groupe cycloalkyle en C₃-C₈,
d) un groupe aliphatique, ou
e) un groupe hétérocyclyle,
dans lequel un groupe aryle, hétéroaryle, cycloalkyle, hétérocyclyle et aliphatique étant éventuellement substitué par un ou plusieurs groupe(s) indépendamment choisi(s) parmi R⁸;
Dₐ et D_{b} sont chacun indépendamment :
une liaison ou
-[C(R^{c})(R^{d})]ₙ, dans lequel R^{c} et R^{d} sont chacun indépendamment un atome d'hydrogène, un groupe alkyle en C₁-C₆ ou aryle ;
Q est : -C(O)OR⁵ ou R^{5A};
X est : NR⁶C[O]ₚ,
NR⁶S(O)₂,
C[O]ₚ, NR⁶,
S(O)₂NR⁶ ou
NR⁷;
Y est : une liaison, CH₂, S ou O ; 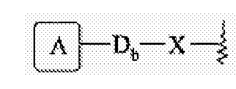
est: 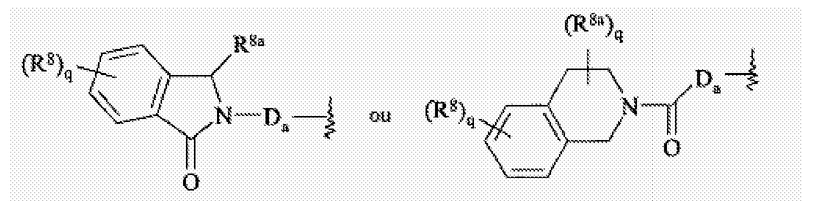
n et r sont chacun indépendamment : 1, 2, 3 ou 4 ;
q est : 1, 2, 3, 4 ou 5 ;
p est : 1 ou 2 ;
R¹ et R² sont chacun indépendamment : un atome d'hydrogène, un groupe alkyle en C₁-C₆, halogéno ou halogénoalkyle ;
R³ et R⁴ sont chacun indépendamment :
un atome d'hydrogène,
un groupe halogéno,
alkyle en C₁-C₆,
alcoxy en C₁-C₆ ou
aryloxy ;
R³ et R⁴ sont conjointement un groupe carbocyclyle ou hétérocyclyle à 3 à 6 chaînons ;
R⁵ est : un atome d'hydrogène, un groupe alkyle en C₁-C₆ ou aminoalkyle ;
R^{5A} est : un groupe carboxamide, sulfonamide, acylsulfonamide, tétrazole, 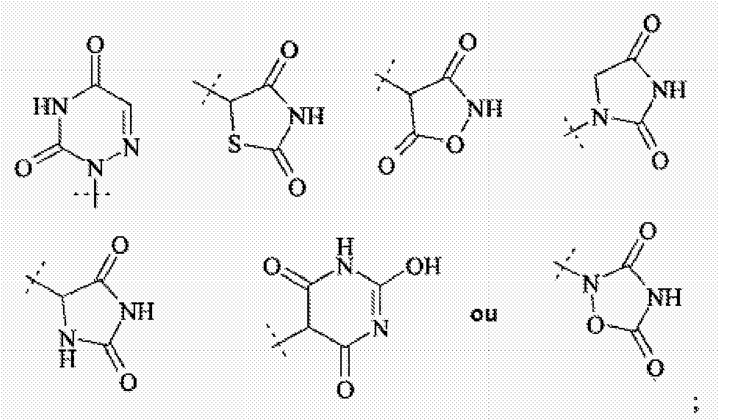
R⁶ est chacun indépendamment :
un atome d'hydrogène,
un groupe alkyle en C₁-C₁₂,
arylalkyle,
cycloalkyle en C₃-C₈, ou
(CH₂)ₙC(O)aryle,
dans lequel le groupe alkyle, arylalkyle et cycloalkyle étant éventuellement substitué par un ou plusieurs groupe(s) indépendamment choisi(s) parmi R⁸ ;
R⁷ est : un atome d'hydrogène,
un groupe acyle, ou
sulfonyle ;
R⁸ et R^{8a} sont chacun indépendamment :
un atome d'hydrogène,
un groupe alkyle en C₁-C₆,
alcoxy en C₁-C₆,
nitro,
cyano,
halogéno,
halogénoalkyle,
halogénoalkyloxy,
aryle,
hétéroaryle,
benzyle,
aryloxy,
SR⁹,
S[O]ₚR⁹ ou
C[O]ₚR⁹; et
R⁹ est : un atome d'hydrogène, un groupe alkyle en C₁-C₆, ou cycloalkyle en C₃-C₈.

2. Composé selon la revendication 1, dans lequel le groupe aryle ou hétéroaryle sont choisis parmi le groupe constitué du groupe phényle, naphtyle, indolyle, isoindolyle, benzoimidazolyle, quinoléinyle, isoquinoléinyle, pyridyle, benzothiophényle et benzofuranyle.

3. Composé selon la revendication 2, ayant une formule structurelle II, 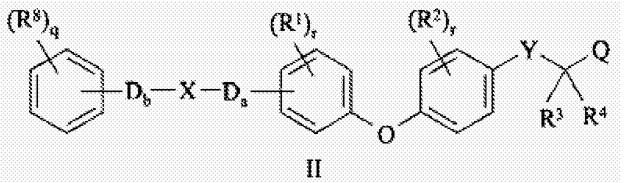 ou un sel ou un stéréo-isomère de celui-ci pharmaceutiquement acceptable, dans lequel :
q est 1, 2, 3, 4 ou 5.

4. Composé selon la revendication 3, dans lequel R⁸ est disubstitué en positions 2 et 4, ou trisubstitué en positions 2, 4 et 6 du cycle phényle par rapport à -D_{b}-.

5. Composé selon la revendication 3 ayant une formule structurelle III, 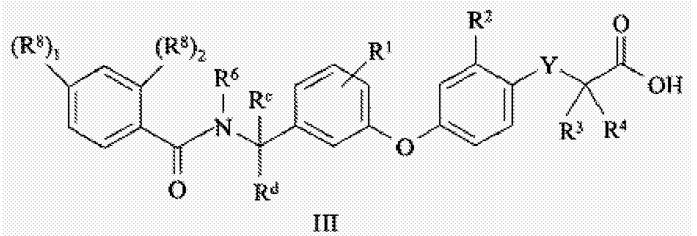 ou un sel ou un stéréo-isomère de celui-ci pharmaceutiquement acceptable, dans lequel :
Y est : - O ou CH₂ ;
R¹ est : un atome hydrogène, un groupe halogéno ou alkyle en C₁-C₄ ;
R², R³ et R⁴, R⁶, R^{c} et R^{d} sont chacun indépendamment : un atome d'hydrogène ou un groupe alkyle en C₁-C₄ ;
(R⁸)₁ et (R⁸)₂ sont chacun indépendamment : un atome d'hydrogène, un groupe halogéno, halogénoalkyle ou halogénoalkyloxy, cyano, nitro, alkyle en C₁-C₆, alcoxy en C₁-C₆ ou SR⁹ ;
R⁶ est : un atome d'hydrogène ou un groupe alkyle en C₁-C₄ ; et
R⁹ est : un atome d'hydrogène ou un groupe alkyle en C₁-C₄ ou cycloalkyle en C₃-C₆.

6. Composé selon la revendication 5, ayant une formule structurelle IV, 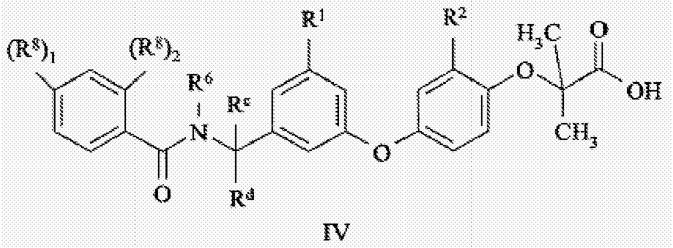 ou un sel ou un stéréo-isomère de celui-ci pharmaceutiquement acceptable, dans lequel :
R¹ et R² sont chacun indépendamment : un atome d'hydrogène, un groupe halogéno ou alkyle en C₁-C₄ ;
R^{c}, R^{d} et R⁶ sont chacun indépendamment : un atome d'hydrogène ou un groupe méthyle ; et
(R⁸)₁ et (R⁸)₂ sont chacun indépendamment :
un atome d'hydrogène, F, CI, Br, OMe, CF₃, OCF₃, SCH₃, NO₂, cyano, méthyle, éthyle, isobutyle, isopropyle, ou *tert*-butyle.

7. Composé selon la revendication 6, ayant une formule structurelle V, 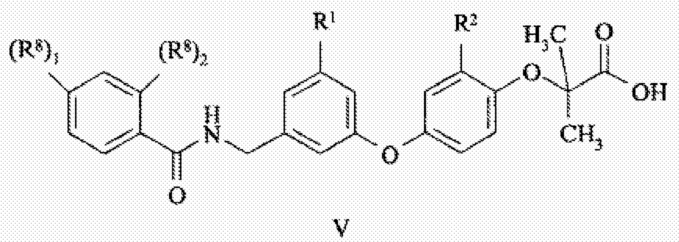 ou un sel ou un stéréo-isomère de celui-ci pharmaceutiquement acceptable, dans lequel :
R¹ et R² sont chacun indépendamment : un atome d'hydrogène, un groupe méthyle, éthyle ou fluoro ; et
(R⁸)₁ et (R⁸)₂ sont chacun indépendamment :
un atome d'hydrogène, F, CI, Br, OMe, CF₃, OCF₃, SCH₃, NO₂, un groupe cyano, méthyle, éthyle, isobutyle, isopropyle, ou *tert*-butyle.

8. Composé selon la revendication 3, ayant une formule structurelle VII, 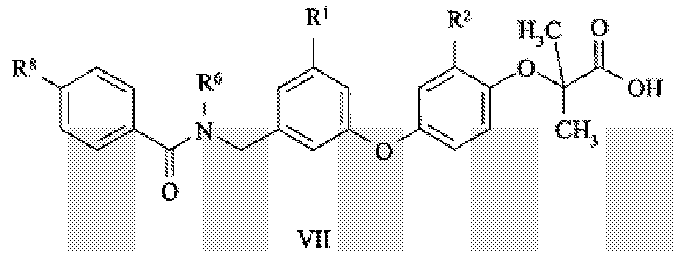 ou un sel ou un stéréo-isomère de celui-ci pharmaceutiquement acceptable, dans lequel :
R¹ et R² sont chacun indépendamment : un atome d'hydrogène, un groupe halogéno ou alkyle en C₁-C₄ ;
R⁶ est : un atome d'hydrogène ou un groupe alkyle en C₁-C₄;
R⁸ est : un atome d'hydrogène, un groupe halogéno, halogénoalkyle ou halogénoalkyloxy, cyano, nitro, alkyle en C₁-C₆, alcoxy en C₁-C₆ ou SR⁹ ; et
R⁹ est : un atome d'hydrogène ou un groupe alkyle en C₁-C₄ ou cycloalkyle en C₃-C₆.

9. Composé selon la revendication 1, ayant une formule structurelle VIII, 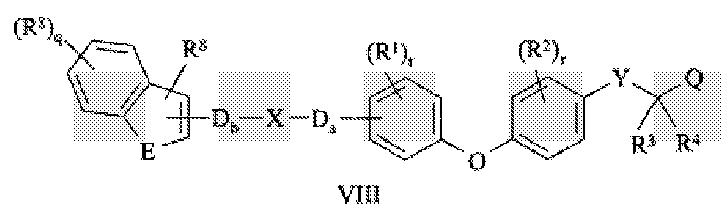 ou un sel ou un stéréo-isomère de celui-ci pharmaceutiquement acceptable, dans lequel :
q est 1, 2, 3 ou 4 ; et
E est S, O ou NR¹⁰ dans lequel R¹⁰ est un atome d'hydrogène ou un groupe alkyle en C₁-C₄.

10. Composé selon la revendication 9, ayant une formule structurelle IX, 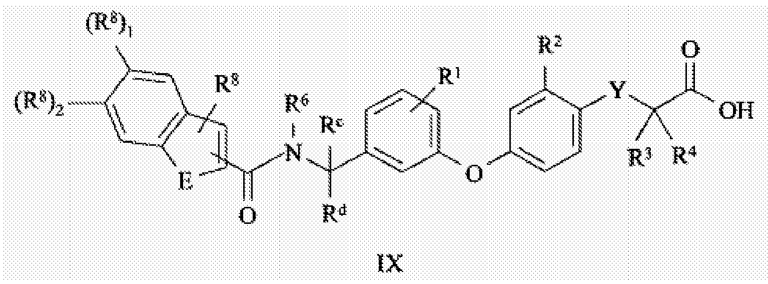 ou un sel ou un stéréo-isomère de celui-ci pharmaceutiquement acceptable, dans lequel :
Y est : O ou CH₂;
E est : S, O, NH ou NCH₃, NCH₂CH₃ ;
R¹ est : un atome d'hydrogène, un groupe alkyle en C₁-C₄, halogéno ou halogénoalkyle ;
R², R³ et R⁴, R⁶, R^{c} et R^{d} sont chacun indépendamment : un atome d'hydrogène ou un groupe alkyle en C₁-C₄ ;
(R⁸)₁ et (R⁸)₂ sont chacun indépendamment : un atome d'hydrogène, un groupe halogéno, halogénoalkyle, halogénoalkyloxy, cyano, nitro, alkyle en C₁-C₆ ou alcoxy en C₁-C₆ ; et
R⁸ est un atome d'hydrogène ou un groupe alkyle en C₁-C₄.

11. Composé selon la revendication 10, ayant une formule structurelle XIII, 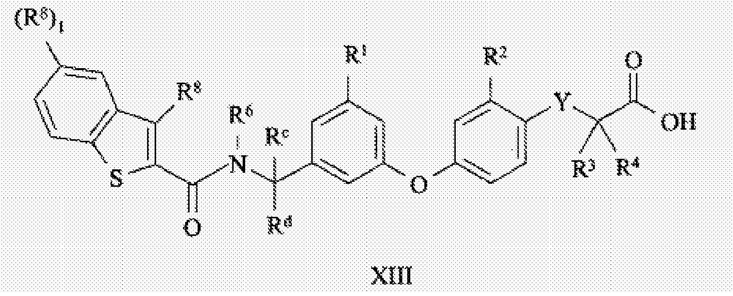 ou un sel ou un stéréo-isomère de celui-ci pharmaceutiquement acceptable, dans lequel :
Y est : O ou CH₂;
R¹ est : un atome d'hydrogène, un groupe alkyle en C₁-C₄, halogéno ou halogénoalkyle ;
R², R³, R⁴, R⁶, R^{c} et R^{d} sont chacun indépendamment : un atome d'hydrogène ou un groupe alkyle en C₁-C₄ ;
R⁸ sont chacun indépendamment : un atome d'hydrogène ou un groupe alkyle en C₁-C₄ ; et
(R⁸)₁ est : un atome d'hydrogène, un groupe halogéno, halogénoalkyle ou halogénoalkyloxy, cyano, nitro, alkyle en C₁-C₆ ou alcoxy en C₁-C₆).

12. Composé selon la revendication 1, ayant une formule structurelle XVI, 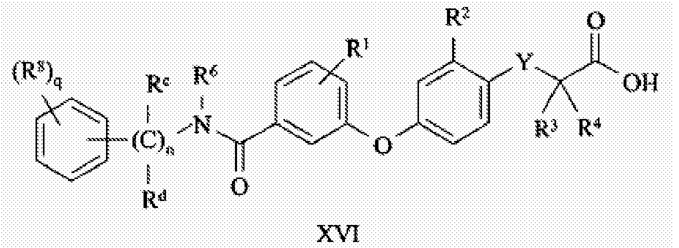 ou un sel ou un stéréo-isomère de celui-ci pharmaceutiquement acceptable, dans lequel :
n est 1, 2, 3 ou 4.

13. Composé selon la revendication 12, dans lequel Y est O ou CH₂ ; R¹, R², R³, R⁴, R^{c} et R^{d} sont chacun indépendamment un atome d'hydrogène ou un groupe alkyle en C₁-C₄ ; n est 1 ou 2 ; R⁶ est un atome d'hydrogène, un groupe alkyle en C₁-C₆ ou arylalkyle; et R⁸ est un atome d'hydrogène, un groupe alcoxy en C₁-C₆, halogéno ou halogénoalkyle.

14. Composé selon la revendication 1, ayant une formule structurelle XVII, 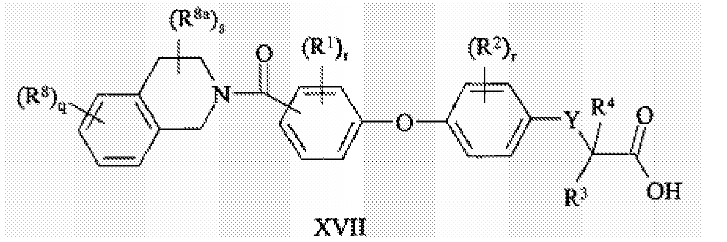 ou un sel ou un stéréo-isomère de celui-ci pharmaceutiquement acceptable, dans lequel :
R^{8a} est un atome d'hydrogène, un groupe alkyle en C₁-C₄ ou aryle ; et s est 1, 2, 3, 4, 5 ou 6.

15. Composé selon la revendication 1, ayant une formule structurelle XIX, 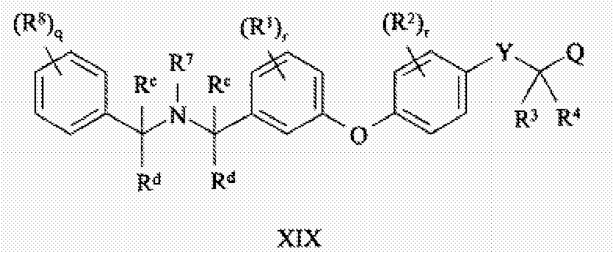 ou un sel ou un stéréo-isomère de celui-ci pharmaceutiquement acceptable.

16. Composé selon la revendication 15, dans lequel Q est COOH ; R⁷ est un atome d'hydrogène, un groupe méthane sulfonyle ou acétyle ; R^{c} et R^{d} représentent chacun un atome d'hydrogène.

17. Composé selon la revendication 1, choisi parmi : l'acide 2-(4-{3-[(2-chloro-4-trifluorométhylbenzoylamino)méthyl]-5-fluorophénoxy}-2-méthylphénoxy)-2-méthylpropionique ; l'acide 3-[4-(3-{[(5-chloro-1H-indole-2-carbonyl)amino]méthyl}-5-fluorophénoxy)-2-méthylphényl]propionique ; l'acide 2-(4-{3-fluoro-5-[1-(2-méthyl-4-trifluorométhylbenzoylamino)éthyl]phénoxy}-2-méthylphénoxy)-2-méthylpropionique (isomère 1) ; l'acide 2-[4-(3-{[(5-chloro-3-méthylbenzo[b]thiophène-2-carbonyl)amino]méthyl}-5-méthylphénoxy)-2-méthylphénoxy]-2-méthylpropionique ; l'acide (R)-3-[4-(3-{1-[(5-chloro-1,3-diméthyl-1 H-indole-2-carbonyl)amino]éthyl}-5-fluorophénoxy)-2-méthylphényl]propionique ; l'acide 3-(2-éthyl-4-{3-fluoro-5-[(2-méthyl-4-trifluorométhylbenzoylamino)méthyl]phénoxy}phényl)propionique; l'acide 2-(4-{3-[(2-fluoro-4-trifluorométhylbenzoylamino)méthyl]-5-méthylphénoxy}-2-méthylphénoxy)-2-méthylpropionique ; l'acide (R)-2-[4-(3-{[(5-chloro-1,3-diméthyl-1 H-indole-2-carbonyl)amino]méthyl}-5-méthylphénoxy)-2-méthylphénoxy]-2-méthylpropionique ; l'acide 3-(4-(3-fluoro-5-{[(5-flluoro-3-méthyl-1 H-indole-2-carbonyl)amino]méthyl}phénoxy)-2-méthylphényl]propionique ; l'acide 2-[4-(3-fluoro-5-{[(5-flluoro-1,3-diméthyl-1H-indole-2-carbonyl)amino]méthyl}phénoxy)-2-méthylphénoxy]-2-méthylpropionique ; l'acide (R)-3-[4-(3-{1-[(5-fluoro-1,3-diméthyl-1H-indole-2-carbonyl)amino]éthyl}-5-méthylphénoxy)-2-méthylphényl]propionique; l'acide 2-méthyl-2-(2-méthyl-4-{3-[(2-méthyl-4-trifluorométhylbenzoylamino)méthyl]phénoxylphénoxy)propionique ; l'acide 2-(4-{3-fluoro-5-[(2-méthyl-4-trifluorométhylbenzoylamino)méthyl]phénoxy}-2-méthylphénoxy)-2-méthylpropionique ; l'acide (R)-3-[4-(3-fluoro-5-{1-[(5-fluoro-1,3-diméthyl-1 H-indole-2-carbonyl)amino]éthyl}phénoxy)-2-méthylphényl]propionique ; l'acide 3-[4-(3-{[(5-chloro-1,3-diméthyl-1H-indole-2-carbonyl)amino]méthyl}-5-fluorophénoxy)-2-méthylphényl]propionique ; l'acide 3-[4-(3-{[(5-chloro-1,3-diméthyl-1H-indole-2-carbonyl)amino]méthyl}phénoxy)-2-méthylphényl]propionique ; l'acide 3-[2-éthyl-4-(3-fluoro-5-{[(5-fluoro-1,3-diméthyl-1 H-indole-2-carbonyl)amino]méthyl}phénoxy)phényl]propionique ; et l'acide 3-(4-{3-[(2-chloro-4-trifluorométhylbenzoylamino)méthyl]-5-méthylphénoxy}-2-éthylphényl)propionique.

18. Composition pharmaceutique comprenant un support pharmaceutiquement acceptable et un composé selon les revendications 1 à 17 ou un sel pharmaceutiquement acceptable.

19. Utilisation d'un composé selon l'une quelconque des revendications 1 à 17 ou d'un sel pharmaceutiquement acceptable de celui-ci pour la fabrication d'un médicament pour le traitement de l'hyperglycémie, de la dyslipidémie, du diabète de type II, du diabète de type I, de l'hypertriglycéridémie, du syndrome X, de la résistance à l'insuline, de l'insuffisance cardiaque, de la dyslipidémie diabétique, de l'hyperlipidémie, de l'hypercholestérolémie, de l'hypertension, de l'obésité, de l'anorexie-boulimie, de l'anorexie mentale et de la maladie cardio-vasculaire.

20. Utilisation d'un composé selon l'une quelconque des revendications 1 à 17 ou d'un sel pharmaceutiquement acceptable de celui-ci pour la fabrication d'un médicament pour le traitement du diabète sucré chez un mammifère.

21. Utilisation d'un composé selon l'une quelconque des revendications 1 à 17 ou d'un sel pharmaceutiquement acceptable de celui-ci pour la fabrication d'un médicament pour la réduction de la glycémie chez un mammifère.

22. Utilisation d'un composé selon l'une quelconque des revendications 1 à 17 ou d'un sel pharmaceutiquement acceptable de celui-ci pour la fabrication d'un médicament pour le traitement d'un état modulé par un PPAR.
